# EUROPEAN PATENT APPLICATION

(11) **EP 3 424 941 A1**
(43) Date of publication of application: **09.01.2019**
(21) Application number: 17760194.5
(22) Date of filing: 03.03.2017
(51) Int. Cl.: C07K 5/12, C07H 21/04, C12N 15/00, C12N 15/09, C07K 7/06, C07K 7/08

(54) **PRODUCTION METHOD FOR NONCYCLIC PEPTIDE-NUCLEIC ACID COMPLEX HAVING, AT N-TERMINAL, AMINO ACID WITH THIOL GROUP NEAR AMINO GROUP, LIBRARY THEREOF, AND CYCLIC PEPTIDE-NUCLEIC ACID COMPLEX LIBRARY DERIVED FROM SAME**

(30) Priority: 03.03.2016 JP 2016040876
(71) Applicant: Chugai Seiyaku Kabushiki Kaisha, Kita-ku Tokyo 115-8543 (JP)
(72) Inventor: NAKANO, Kazuhiko, Kamakura-shi Kanagawa 247-8530 (JP); OHTA, Atsushi, Kamakura-shi Kanagawa 247-8530 (JP); IIDA, Takeo, Kamakura-shi Kanagawa 247-8530 (JP); IIKURA, Hitoshi, Gotemba-shi Shizuoka 412-8513 (JP)
(74) Representative: Vossius & Partner Patentanwälte Rechtsanwälte mbB
(86) International application number: PCT/JP2017/008623
(87) International publication number: WO 2017/150732

(57) **Abstract**

When the initiation suppression method was used for translation of a peptide having at its N terminus an amino acid residue carrying a thiol group near its amino group with specific protecting groups being introduced to the thiol group and the amino group, it was found that not only the probability of initiation of amino acid translation reaction was improved, but also production of cleaved peptides was suppressed and translation efficiency and purity were improved. Furthermore, it was found that it is possible to efficiently promote the cyclization reaction of the peptide through amide bond formation. Based on these findings, the inventors discovered novel methods for preparing complexes between nucleic acids and peptides containing various unnatural amino acids and having an amide bond-mediated cyclized portion.

SEQUENCE LISTING

<110>  CHUGAI SEIYAKU KABUSHIKI KAISHA

<120>  Methods for producing a non-cyclic peptide-nucleic acid complex,
       a library of such complexes, and a library of cyclic
       peptide-nucleic acid complexes derived therefrom, wherein the
       non-cyclic peptide comprises at its N-terminus an amino acid
       having a thiol group near its amino group

<130>  C1-A1514P

<150>  JP 2016-040876
<151>  2016-03-03

<160>  43

<170>  PatentIn version 3.5

<210>  1
<211>  96
<212>  DNA
<213>  Artificial Sequence

<220>
<223>  DNA D-1: tRNAfMetCAT(-CA)

<400>  1
ggcgtaatac gactcactat aggcggggtg gagcagcctg gtagctcgtc gggctcataa      60

cccgaagatc gtcggttcaa atccggcccc cgcaac                                96


<210>  2
<211>  75
<212>  RNA
<213>  Artificial Sequence

<220>
<223>  RNA R-1: tRNAfMetCAU(-CA)

<400>  2
ggcggggugg agcagccugg uagcucgucg ggcucauaac ccgaagaucg ucgguucaaa      60

uccggccccc gcaac                                                       75


<210>  3
<211>  77
<212>  RNA
<213>  Artificial Sequence

<220>
<223>  tRNAfMetCAU

<400>  3
ggcggggugg agcagccugg uagcucgucg ggcucauaac ccgaagaucg ucgguucaaa      60

uccggccccc gcaacca                                                     77


<210>  4
<211>  73

<212>  RNA
<213>  Artificial Sequence

<220>
<223>  template mRNA R-4

<400>  4
ggguuaacuu uaagaaggag auauacauau gacucguacu aaggcuuacu ggagucuucc      60

ggguuaagcu ucg                                                          73


<210>  5
<211>  82
<212>  RNA
<213>  Artificial Sequence

<220>
<223>  template mRNA R-4D

<400>  5
ggguuaacuu uaagaaggag auauacauau gacucguacu aaggcuuacu ggagucuucc      60

gggugacgac gacuaagcuu cg                                                82


<210>  6
<211>  95
<212>  DNA
<213>  Artificial Sequence

<220>
<223>  DNA D-2: tRNAGluCTG(-CA)

<400>  6
ggcgtaatac gactcactat agtccccttc gtctagaggc ccaggacacc gccctctgac      60

ggcggtaaca ggggttcgaa tcccctaggg gacgc                                  95


<210>  7
<211>  95
<212>  DNA
<213>  Artificial Sequence

<220>
<223>  DNA D-3: tRNAGluAAG(-CA)

<400>  7
ggcgtaatac gactcactat agtccccttc gtctagaggc ccaggacacc gccctaagac      60

ggcggtaaca ggggttcgaa tcccctaggg gacgc                                  95


<210>  8
<211>  74
<212>  RNA
<213>  Artificial Sequence

<220>
<223>  RNA R-2: tRNAGluCUG(-CA)

<400>  8

guccccuucg ucuagaggcc caggacaccg cccucugacg gcgguaacag ggguucgaau 60

ccccuagggg acgc 74

<210> 9
<211> 74
<212> RNA
<213> Artificial Sequence

<220>
<223> RNA R-3: tRNAGluAAG(-CA)

<400> 9
guccccuucg ucuagaggcc caggacaccg cccuaagacg gcgguaacag ggguucgaau 60

ccccuagggg acgc 74

<210> 10
<211> 76
<212> RNA
<213> Artificial Sequence

<220>
<223> tRNAGluCUG

<400> 10
guccccuucg ucuagaggcc caggacaccg cccucugacg gcgguaacag ggguucgaau 60

ccccuagggg acgcca 76

<210> 11
<211> 76
<212> RNA
<213> Artificial Sequence

<220>
<223> tRNAGluAAG

<400> 11
guccccuucg ucuagaggcc caggacaccg cccuaagacg gcgguaacag ggguucgaau 60

ccccuagggg acgcca 76

<210> 12
<211> 76
<212> RNA
<213> Artificial Sequence

<220>
<223> template mRNA R-5-1

<400> 12
ggguuaacuu uaagaaggag auauacauau gaguuuuccg agucagauug uuuacacugg 60

ucguccguaa gcuucg 76

<210> 13
<211> 73

<212>  RNA
<213>  Artificial Sequence

<220>
<223>  template mRNA R-6-1

<400>  13
ggguuaacuu uaagaaggag auauacauau gaguuuuguu cagauuuuua guguuauucg      60

uccguaagcu ucg                                                        73


<210>  14
<211>  79
<212>  RNA
<213>  Artificial Sequence

<220>
<223>  template mRNA R-7-1

<400>  14
ggguuaacuu uaagaaggag auauacauau gacuacuccg uuuggucaga guuacauugu      60

uuaccguccg uaagcuucg                                                  79


<210>  15
<211>  76
<212>  RNA
<213>  Artificial Sequence

<220>
<223>  template mRNA R-8-1

<400>  15
ggguuaacuu uaagaaggag auauacauau guuuguuacu caggcuacuu acgguauuac      60

ucguccguaa gcuucg                                                     76


<210>  16
<211>  76
<212>  RNA
<213>  Artificial Sequence

<220>
<223>  template mRNA R-9-1

<400>  16
ggguuaacuu uaagaaggag auauacauau gaguuuuuuu guucagaguu acaguguuau      60

ucguccguaa gcuucg                                                     76


<210>  17
<211>  79
<212>  RNA
<213>  Artificial Sequence

<220>
<223>  template mRNA R-5-2

<400>  17

ggguuaacuu uaagaaggag auauacauau gugcaguuuu ccgagucaga uuguuuacac 60

uggucguccg uaagcuucg 79

<210> 18
<211> 76
<212> RNA
<213> Artificial Sequence

<220>
<223> template mRNA R-6-2

<400> 18
ggguuaacuu uaagaaggag auauacauau gugcaguuuu guucagauuu uuaguguuau 60

ucguccguaa gcuucg 76

<210> 19
<211> 82
<212> RNA
<213> Artificial Sequence

<220>
<223> template mRNA R-7-2

<400> 19
ggguuaacuu uaagaaggag auauacauau gugcacuacu ccguuugguc agaguuacau 60

uguuuaccgu ccguaagcuu cg 82

<210> 20
<211> 79
<212> RNA
<213> Artificial Sequence

<220>
<223> template mRNA R-8-2

<400> 20
ggguuaacuu uaagaaggag auauacauau gugcuuuguu acucaggcua cuuacgguau 60

uacucguccg uaagcuucg 79

<210> 21
<211> 79
<212> RNA
<213> Artificial Sequence

<220>
<223> template mRNA R-9-2

<400> 21
ggguuaacuu uaagaaggag auauacauau gugcaguuuu uuuguucaga guuacagugu 60

uauucguccg uaagcuucg 79

<210> 22
<211> 73

<212>  RNA
<213>  Artificial Sequence

<220>
<223>  template mRNA R-10

<400>  22
ggguuaacuu uaagaaggag auauacauau gaguuuuuuu guucagaguu acagucuucc      60

gcguuaagcu ucg                                                        73


<210>  23
<211>  73
<212>  RNA
<213>  Artificial Sequence

<220>
<223>  template mRNA R-11

<400>  23
ggguuaacuu uaagaaggag auauacauau gaguuuuguu cagauuuuua guguucuucc      60

gcguuaagcu ucg                                                        73


<210>  24
<211>  79
<212>  RNA
<213>  Artificial Sequence

<220>
<223>  template mRNA R-12

<400>  24
ggguuaacuu uaagaaggag auauacauau gacuacuccg uuuggucaga guuacauugu      60

ucuuccgcgu uaagcuucg                                                  79


<210>  25
<211>  76
<212>  RNA
<213>  Artificial Sequence

<220>
<223>  template mRNA R-13

<400>  25
ggguuaacuu uaagaaggag auauacauau guuuguuacu caggcuacuu acgguauucu      60

uccgcguuaa gcuucg                                                     76


<210>  26
<211>  76
<212>  RNA
<213>  Artificial Sequence

<220>
<223>  template mRNA R-14

<400>  26

ggguuaacuu uaagaaggag auauacauau gaguuuuccg agucagauug uuuacacucu      60

uccgcguuaa gcuucg                                                    76


<210>  27
<211>  79
<212>  RNA
<213>  Artificial Sequence

<220>
<223>  template mRNA R-15

<400>  27
ggguuaacuu uaagaaggag auauacauau guuuuauugg uuuccacagu ucaaguggcu      60

uccucguuau uaagcuucg                                                 79


<210>  28
<211>  123
<212>  DNA
<213>  Artificial Sequence

<220>
<223>  DNA D-16


<220>
<221>  misc_feature
<222>  (53)..(55)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (56)..(58)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (59)..(61)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (62)..(64)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (65)..(67)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (68)..(70)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (71)..(73)
<223>  nnn stands for ttt, ttg, cta, att, gtt, ccg, act, gct, tac, cat,
      cag, gaa, tgg, cgg, agt, agg or ggt

<220>
<221>  misc_feature
<222>  (74)..(76)
<223>  nnn stands for ttt, ttg, cta, att, gtt, ccg, act, gct, tac, cat,
      cag, gaa, tgg, cgg, agt, agg or ggt

<220>
<221>  misc_feature
<222>  (77)..(79)
<223>  nnn stands for ttt, ttg, cta, att, gtt, ccg, act, gct, tac, cat,
      cag, gaa, tgg, cgg, agt, agg or ggt

<400>  28
gtaatacgac tcactatagg gttaacttta agaaggagat atacatatgt gcnnnnnnnn      60

nnnnnnnnnn nnnnnnnnnt agccgaccgg caccggcacc ggcgataggg cggcggggac     120

aaa                                                                   123


<210>  29
<211>  105
<212>  RNA
<213>  Artificial Sequence

<220>
<223>  RNA R-16



<220>
<221>  misc_feature
<222>  (35)..(37)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (38)..(40)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (41)..(43)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (44)..(46)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (47)..(49)

<223>  nnn stands for uuu, uug, cua, auu, guu, ccg, acu, gcu, uac, cau,
      cag, gaa, ugg, cgg, agu, agg or ggu

<220>
<221>  misc_feature
<222>  (50)..(52)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (53)..(55)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (56)..(58)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (59)..(61)
<223>  nnn stands for uuu, uug, cua, auu, guu, ccg, acu, gcu, uac, cau,
      cag, gaa, ugg, cgg, agu, agg or ggu

<400>  29
ggguuaacuu uaagaaggag auauacauau gugcnnnnnn nnnnnnnnnn nnnnnnnnnn      60

nuagccgacc ggcaccggca ccggcgauag ggcggcgggg acaaa                     105


<210>  30
<211>  17
<212>  DNA
<213>  Artificial Sequence

<220>
<223>  linker sequence C-1


<220>
<221>  misc_feature
<223>  5' phosphorylated oligonucleotide

<400>  30
cccgtccccg ccgccct                                                     17


<210>  31
<211>  122
<212>  DNA
<213>  Artificial Sequence

<220>
<223>  mRNA - linker ligation product R-17


<220>
<221>  misc_feature
<223>  DNA/RNA

<220>
<221>  misc_feature
<222>  (35)..(37)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (38)..(40)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (41)..(43)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (44)..(46)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (47)..(49)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (50)..(52)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (53)..(55)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (56)..(58)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (59)..(61)
<223>  nnn stands for uuu, uug, cua, auu, guu, ccg, acu, gcu, uac, cau,
      cag, gaa, ugg, cgg, agu, agg or ggu

<400>  31
ggguuaacuu uaagaaggag auauacauau gugcnnnnnn nnnnnnnnnn nnnnnnnnnn      60

nuagccgacc ggcaccggca ccggcgauag ggcggcgggg acaaacccgt ccccgccgcc     120

ct                                                                    122


<210>  32

<211>  12
<212>  PRT
<213>  Artificial Sequence

<220>
<223>  peptide Pep-1


<220>
<221>  MOD_RES
<222>  (1)..(1)
<223>  FORMYLATION

<400>  32

Met Thr Arg Thr Lys Ala Tyr Trp Ser Leu Pro Gly
1               5                   10


<210>  33
<211>  11
<212>  PRT
<213>  Artificial Sequence

<220>
<223>  peptide Pep-2

<400>  33

Thr Arg Thr Lys Ala Tyr Trp Ser Leu Pro Gly
1               5                   10


<210>  34
<211>  12
<212>  PRT
<213>  Artificial Sequence

<220>
<223>  peptide Pep-3

<400>  34

Cys Thr Arg Thr Lys Ala Tyr Trp Ser Leu Pro Gly
1               5                   10


<210>  35
<211>  15
<212>  PRT
<213>  Artificial Sequence

<220>
<223>  peptide Pep-8

<400>  35

Met Thr Arg Thr Lys Ala Tyr Trp Ser Leu Pro Gly Asp Asp Asp
1               5                   10                  15


<210>  36
<211>  14

<212>  PRT
<213>  Artificial Sequence

<220>
<223>  peptide Pep-9

<400>  36

Thr Arg Thr Lys Ala Tyr Trp Ser Leu Pro Gly Asp Asp Asp
1               5                   10


<210>  37
<211>  79
<212>  RNA
<213>  Artificial Sequence

<220>
<223>  template mRNA R-18

<400>  37
ggguuaacuu uaagaaggag auauacauau gaguuuuugc ccgcagauua aauacguugg      60

ucuuccgcgu uaagcuucg                                                   79


<210>  38
<211>  74
<212>  RNA
<213>  Artificial Sequence

<220>
<223>  RNA T-1: tRNAGluCUA(-CA)

<400>  38
guccccuucg ucuagaggcc caggacaccg cccucuaacg gcgguaacag ggguucgaau      60

ccccuagggg acgc                                                        74


<210>  39
<211>  74
<212>  RNA
<213>  Artificial Sequence

<220>
<223>  RNA T-2: tRNAGluCAA(-CA)

<400>  39
guccccuucg ucuagaggcc caggacaccg cccucaaacg gcgguaacag ggguucgaau      60

ccccuagggg acgc                                                        74


<210>  40
<211>  74
<212>  RNA
<213>  Artificial Sequence

<220>
<223>  RNA T-3: tRNAGluUAG(-CA)

<400>  40

guccccuucg ucuagaggcc caggacaccg cccuuagacg gcgguaacag ggguucgaau 60

ccccuagggg acgc 74

<210> 41
<211> 74
<212> RNA
<213> Artificial Sequence

<220>
<223> RNA T-5: tRNAGluCCG(-CA)

<400> 41
guccccuucg ucuagaggcc caggacaccg cccuccgacg gcgguaacag ggguucgaau 60

ccccuagggg acgc 74

<210> 42
<211> 74
<212> RNA
<213> Artificial Sequence

<220>
<223> RNA T-6: tRNAGluCCU(-CA)

<400> 42
guccccuucg ucuagaggcc caggacaccg cccuccuacg gcgguaacag ggguucgaau 60

ccccuagggg acgc 74

<210> 43
<211> 75
<212> RNA
<213> Artificial Sequence

<220>
<223> RNA T-10: tRNAfMetCAU(-CA)

<400> 43
ggcggggugg agcagccugg uagcucgucg ggcucauaac ccgaagaucg ucgguucaaa 60

uccggccccc gcaac 75

SEQUENCE LISTING

<110>  CHUGAI SEIYAKU KABUSHIKI KAISHA

<120>  PRODUCTION METHOD FOR NONCYCLIC PEPTIDE-NUCLEIC ACID COMPLEX
       HAVING, AT N-TERMINAL, AMINO ACID WITH THIOL GROUP NEAR AMINO GROUP,
       LIBRARY THEREOF, AND CYCLIC PEPTIDE-NUCLEIC ACID COMPLEX LIBRARY
       DERIVED FROM SAME

<130>  AB2597 EP

<140>  EP 17 76 0194.5
<141>  2017-03-03

<150>  JP 2016-040876
<151>  2016-03-03

<160>  43

<170>  PatentIn version 3.5

<210>  1
<211>  96
<212>  DNA
<213>  Artificial Sequence

<220>
<223>  DNA D-1: tRNAfMetCAT(-CA)

<400>  1
ggcgtaatac gactcactat aggcggggtg gagcagcctg gtagctcgtc gggctcataa      60

cccgaagatc gtcggttcaa atccggcccc cgcaac                                96


<210>  2
<211>  75
<212>  RNA
<213>  Artificial Sequence

<220>
<223>  RNA R-1: tRNAfMetCAU(-CA)

<400>  2
ggcggggugg agcagccugg uagcucgucg ggcucauaac ccgaagaucg ucgguucaaa      60

uccggccccc gcaac                                                       75


<210>  3
<211>  77
<212>  RNA
<213>  Artificial Sequence

<220>
<223>  tRNAfMetCAU

<400>  3
ggcggggugg agcagccugg uagcucgucg ggcucauaac ccgaagaucg ucgguucaaa      60

uccggccccc gcaacca                                                     77

<210>  4
<211>  73
<212>  RNA
<213>  Artificial Sequence

<220>
<223>  template mRNA R-4

<400>  4
ggguuaacuu uaagaaggag auauacauau gacucguacu aaggcuuacu ggagucuucc      60

ggguuaagcu ucg                                                          73


<210>  5
<211>  82
<212>  RNA
<213>  Artificial Sequence

<220>
<223>  template mRNA R-4D

<400>  5
ggguuaacuu uaagaaggag auauacauau gacucguacu aaggcuuacu ggagucuucc      60

gggugacgac gacuaagcuu cg                                                82


<210>  6
<211>  95
<212>  DNA
<213>  Artificial Sequence

<220>
<223>  DNA D-2: tRNAGluCTG(-CA)

<400>  6
ggcgtaatac gactcactat agtccccttc gtctagaggc ccaggacacc gccctctgac      60

ggcggtaaca ggggttcgaa tcccctaggg gacgc                                  95


<210>  7
<211>  95
<212>  DNA
<213>  Artificial Sequence

<220>
<223>  DNA D-3: tRNAGluAAG(-CA)

<400>  7
ggcgtaatac gactcactat agtccccttc gtctagaggc ccaggacacc gccctaagac      60

ggcggtaaca ggggttcgaa tcccctaggg gacgc                                  95


<210>  8
<211>  74
<212>  RNA
<213>  Artificial Sequence

<220>
<223>  RNA R-2: tRNAGluCUG(-CA)

<400>  8
guccccuucg ucuagaggcc caggacaccg cccucugacg gcgguaacag ggguucgaau      60

ccccuagggg acgc                                                        74


<210>  9
<211>  74
<212>  RNA
<213>  Artificial Sequence

<220>
<223>  RNA R-3: tRNAGluAAG(-CA)

<400>  9
guccccuucg ucuagaggcc caggacaccg cccuaagacg gcgguaacag ggguucgaau      60

ccccuagggg acgc                                                        74


<210>  10
<211>  76
<212>  RNA
<213>  Artificial Sequence

<220>
<223>  tRNAGluCUG

<400>  10
guccccuucg ucuagaggcc caggacaccg cccucugacg gcgguaacag ggguucgaau      60

ccccuagggg acgcca                                                      76


<210>  11
<211>  76
<212>  RNA
<213>  Artificial Sequence

<220>
<223>  tRNAGluAAG

<400>  11
guccccuucg ucuagaggcc caggacaccg cccuaagacg gcgguaacag ggguucgaau      60

ccccuagggg acgcca                                                      76


<210>  12
<211>  76
<212>  RNA
<213>  Artificial Sequence

<220>
<223>  template mRNA R-5-1

<400>  12
ggguuaacuu uaagaaggag auauacauau gaguuuuccg agucagauug uuuacacugg      60

ucguccguaa gcuucg                                                      76

<210>  13
<211>  73
<212>  RNA
<213>  Artificial Sequence

<220>
<223>  template mRNA R-6-1

<400>  13
ggguuaacuu uaagaaggag auauacauau gaguuuuguu cagauuuuua guguuauucg      60

uccguaagcu ucg                                                        73


<210>  14
<211>  79
<212>  RNA
<213>  Artificial Sequence

<220>
<223>  template mRNA R-7-1

<400>  14
ggguuaacuu uaagaaggag auauacauau gacuacuccg uuuggucaga guuacauugu      60

uuaccguccg uaagcuucg                                                  79


<210>  15
<211>  76
<212>  RNA
<213>  Artificial Sequence

<220>
<223>  template mRNA R-8-1

<400>  15
ggguuaacuu uaagaaggag auauacauau guuuguuacu caggcuacuu acgguauuac      60

ucguccguaa gcuucg                                                     76


<210>  16
<211>  76
<212>  RNA
<213>  Artificial Sequence

<220>
<223>  template mRNA R-9-1

<400>  16
ggguuaacuu uaagaaggag auauacauau gaguuuuuuu guucagaguu acaguguuau      60

ucguccguaa gcuucg                                                     76


<210>  17
<211>  79
<212>  RNA
<213>  Artificial Sequence

<220>
<223>  template mRNA R-5-2

<400>  17
ggguuaacuu uaagaaggag auauacauau gugcaguuuu ccgagucaga uuguuuacac      60

uggucguccg uaagcuucg                                                   79


<210>  18
<211>  76
<212>  RNA
<213>  Artificial Sequence

<220>
<223>  template mRNA R-6-2

<400>  18
ggguuaacuu uaagaaggag auauacauau gugcaguuuu guucagauuu uuaguguuau      60

ucguccguaa gcuucg                                                      76


<210>  19
<211>  82
<212>  RNA
<213>  Artificial Sequence

<220>
<223>  template mRNA R-7-2

<400>  19
ggguuaacuu uaagaaggag auauacauau gugcacuacu ccguuugguc agaguuacau      60

uguuuaccgu ccguaagcuu cg                                               82


<210>  20
<211>  79
<212>  RNA
<213>  Artificial Sequence

<220>
<223>  template mRNA R-8-2

<400>  20
ggguuaacuu uaagaaggag auauacauau gugcuuuguu acucaggcua cuuacgguau      60

uacucguccg uaagcuucg                                                   79


<210>  21
<211>  79
<212>  RNA
<213>  Artificial Sequence

<220>
<223>  template mRNA R-9-2

<400>  21
ggguuaacuu uaagaaggag auauacauau gugcaguuuu uuuguucaga guuacagugu      60

uauucguccg uaagcuucg                                                   79

<210>  22
<211>  73
<212>  RNA
<213>  Artificial Sequence

<220>
<223>  template mRNA R-10

<400>  22
ggguuaacuu uaagaaggag auauacauau gaguuuuuuu guucagaguu acagucuucc      60

gcguuaagcu ucg                                                        73


<210>  23
<211>  73
<212>  RNA
<213>  Artificial Sequence

<220>
<223>  template mRNA R-11

<400>  23
ggguuaacuu uaagaaggag auauacauau gaguuuuguu cagauuuuua guguucuucc      60

gcguuaagcu ucg                                                        73


<210>  24
<211>  79
<212>  RNA
<213>  Artificial Sequence

<220>
<223>  template mRNA R-12

<400>  24
ggguuaacuu uaagaaggag auauacauau gacuacuccg uuuggucaga guuacauugu      60

ucuuccgcgu uaagcuucg                                                  79


<210>  25
<211>  76
<212>  RNA
<213>  Artificial Sequence

<220>
<223>  template mRNA R-13

<400>  25
ggguuaacuu uaagaaggag auauacauau guuuguuacu caggcuacuu acgguauucu      60

uccgcguuaa gcuucg                                                     76


<210>  26
<211>  76
<212>  RNA
<213>  Artificial Sequence

<220>
<223>  template mRNA R-14

<400>  26
ggguuaacuu uaagaaggag auauacauau gaguuuuccg agucagauug uuuacacucu      60

uccgcguuaa gcuucg                                                      76


<210>  27
<211>  79
<212>  RNA
<213>  Artificial Sequence

<220>
<223>  template mRNA R-15

<400>  27
ggguuaacuu uaagaaggag auauacauau guuuuauugg uuuccacagu ucaaguggcu      60

uccucguuau uaagcuucg                                                   79


<210>  28
<211>  123
<212>  DNA
<213>  Artificial Sequence

<220>
<223>  DNA D-16


<220>
<221>  misc_feature
<222>  (53)..(55)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (56)..(58)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (59)..(61)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (62)..(64)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (65)..(67)
<223>  nnn stands for ttt, ttg, cta, att, gtt, ccg, act, gct, tac, cat,
       cag, gaa, tgg, cgg, agt, agg or ggt

<220>
<221>  misc_feature
<222>  (68)..(70)

<223>  nnn stands for ttt, ttg, cta, att, gtt, ccg, act, gct, tac, cat,
      cag, gaa, tgg, cgg, agt, agg or ggt

<220>
<221>  misc_feature
<222>  (71)..(73)
<223>  nnn stands for ttt, ttg, cta, att, gtt, ccg, act, gct, tac, cat,
      cag, gaa, tgg, cgg, agt, agg or ggt

<220>
<221>  misc_feature
<222>  (74)..(76)
<223>  nnn stands for ttt, ttg, cta, att, gtt, ccg, act, gct, tac, cat,
      cag, gaa, tgg, cgg, agt, agg or ggt

<220>
<221>  misc_feature
<222>  (77)..(79)
<223>  nnn stands for ttt, ttg, cta, att, gtt, ccg, act, gct, tac, cat,
      cag, gaa, tgg, cgg, agt, agg or ggt

<400>  28
gtaatacgac tcactatagg gttaacttta agaaggagat atacatatgt gcnnnnnnnn      60

nnnnnnnnnn nnnnnnnnnt agccgaccgg caccggcacc ggcgataggg cggcggggac     120

aaa                                                                   123


<210>  29
<211>  105
<212>  RNA
<213>  Artificial Sequence

<220>
<223>  RNA R-16


<220>
<221>  misc_feature
<222>  (35)..(37)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (38)..(40)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (41)..(43)
<223>  nnn stands for uuu, uug, cua, auu, guu, ccg, acu, gcu, uac, cau,
      cag, gaa, ugg, cgg, agu, agg or ggu

<220>
<221>  misc_feature
<222>  (44)..(46)
<223>  nnn stands for uuu, uug, cua, auu, guu, ccg, acu, gcu, uac, cau,
      cag, gaa, ugg, cgg, agu, agg or ggu

<220>

<221>  misc_feature
<222>  (47)..(49)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (50)..(52)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (53)..(55)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (56)..(58)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (59)..(61)
<223>  nnn stands for uuu, uug, cua, auu, guu, ccg, acu, gcu, uac, cau,
       cag, gaa, ugg, cgg, agu, agg or ggu

<400>  29
ggguuaacuu uaagaaggag auauacauau gugcnnnnnn nnnnnnnnnn nnnnnnnnnn      60

nuagccgacc ggcaccggca ccggcgauag ggcggcgggg acaaa                     105


<210>  30
<211>  17
<212>  DNA
<213>  Artificial Sequence

<220>
<223>  linker sequence C-1


<220>
<221>  misc_feature
<223>  5' phosphorylated oligonucleotide

<400>  30
cccgtccccg ccgccct                                                     17


<210>  31
<211>  122
<212>  DNA
<213>  Artificial Sequence

<220>
<223>  mRNA - linker ligation product R-17


<220>
<221>  misc_feature

<223>  DNA/RNA

<220>
<221>  misc_feature
<222>  (35)..(37)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (38)..(40)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (41)..(43)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (44)..(46)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (47)..(49)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (50)..(52)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (53)..(55)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (56)..(58)
<223>  nnn stands for uuu, uug, cua, auu, guu, ccg, acu, gcu, uac, cau,
       cag, gaa, ugg, cgg, agu, agg or ggu

<220>
<221>  misc_feature
<222>  (59)..(61)
<223>  nnn stands for uuu, uug, cua, auu, guu, ccg, acu, gcu, uac, cau,
       cag, gaa, ugg, cgg, agu, agg or ggu

<400>  31
ggguuaacuu uaagaaggag auauacauau gugcnnnnnn nnnnnnnnnn nnnnnnnnnn      60

nuagccgacc ggcaccggca ccggcgauag ggcggcgggg acaaacccgt ccccgccgcc     120

ct                                                                    122

<210>  32
<211>  12
<212>  PRT
<213>  Artificial Sequence

<220>
<223>  peptide Pep-1


<220>
<221>  MOD_RES
<222>  (1)..(1)
<223>  FORMYLATION

<400>  32

Met Thr Arg Thr Lys Ala Tyr Trp Ser Leu Pro Gly
1               5                   10


<210>  33
<211>  11
<212>  PRT
<213>  Artificial Sequence

<220>
<223>  peptide Pep-2

<400>  33

Thr Arg Thr Lys Ala Tyr Trp Ser Leu Pro Gly
1               5                   10


<210>  34
<211>  12
<212>  PRT
<213>  Artificial Sequence

<220>
<223>  peptide Pep-3

<400>  34

Cys Thr Arg Thr Lys Ala Tyr Trp Ser Leu Pro Gly
1               5                   10


<210>  35
<211>  15
<212>  PRT
<213>  Artificial Sequence

<220>
<223>  peptide Pep-8

<400>  35

Met Thr Arg Thr Lys Ala Tyr Trp Ser Leu Pro Gly Asp Asp Asp
1               5                   10                  15

<210>  36
<211>  14
<212>  PRT
<213>  Artificial Sequence

<220>
<223>  peptide Pep-9

<400>  36

Thr Arg Thr Lys Ala Tyr Trp Ser Leu Pro Gly Asp Asp Asp
1               5                   10


<210>  37
<211>  79
<212>  RNA
<213>  Artificial Sequence

<220>
<223>  template mRNA R-18

<400>  37
ggguuaacuu uaagaaggag auauacauau gaguuuuugc ccgcagauua aauacguugg      60

ucuuccgcgu uaagcuucg                                                   79


<210>  38
<211>  74
<212>  RNA
<213>  Artificial Sequence

<220>
<223>  RNA T-1: tRNAGluCUA(-CA)

<400>  38
guccccuucg ucuagaggcc caggacaccg cccucuaacg gcgguaacag ggguucgaau      60

ccccuagggg acgc                                                        74


<210>  39
<211>  74
<212>  RNA
<213>  Artificial Sequence

<220>
<223>  RNA T-2: tRNAGluCAA(-CA)

<400>  39
guccccuucg ucuagaggcc caggacaccg cccucaaacg gcgguaacag ggguucgaau      60

ccccuagggg acgc                                                        74


<210>  40
<211>  74
<212>  RNA
<213>  Artificial Sequence

<220>
<223>  RNA T-3: tRNAGluUAG(-CA)

<400>  40
guccccuucg ucuagaggcc caggacaccg cccuuagacg gcgguaacag ggguucgaau      60

ccccuagggg acgc                                                        74


<210>  41
<211>  74
<212>  RNA
<213>  Artificial Sequence

<220>
<223>  RNA T-5: tRNAGluCCG(-CA)

<400>  41
guccccuucg ucuagaggcc caggacaccg cccuccgacg gcgguaacag ggguucgaau      60

ccccuagggg acgc                                                        74


<210>  42
<211>  74
<212>  RNA
<213>  Artificial Sequence

<220>
<223>  RNA T-6: tRNAGluCCU(-CA)

<400>  42
guccccuucg ucuagaggcc caggacaccg cccuccuacg gcgguaacag ggguucgaau      60

ccccuagggg acgc                                                        74


<210>  43
<211>  75
<212>  RNA
<213>  Artificial Sequence

<220>
<223>  RNA T-10: tRNAfMetCAU(-CA)

<400>  43
ggcggggugg agcagccugg uagcucgucg ggcucauaac ccgaagaucg ucgguucaaa      60

uccggccccc gcaac                                                       75

## Description

### Technical Field

The present invention relates to establishment of techniques for efficient translation of peptides and complexes of the peptides and nucleic acids, the peptides comprising a plurality of amino acid analogs, in which an amino acid carrying a thiol group near its amino group is positioned at the N-terminus. The present invention also relates to methods for producing peptides having a cyclic portion containing a plurality of amino acid analogs, complexes of the peptides and nucleic acids, and libraries containing the complexes.

### Background Art

In recent years, attention has been given to drug discovery using medium-sized molecules having a molecular weight of 500 to 2000 as techniques enabling drug discovery targeting molecules against which hit compounds are difficult to obtain from conventional small-molecule compounds. Peptides which are among the medium-sized molecules have been generally regarded as having low metabolic stability or low membrane permeability.

However, it has been becoming clear that metabolic stability and membrane permeability improve for cyclic peptides as represented by cyclosporine A and peptides containing amino acid analogs such as *N*-methyl amino acids and D-amino acids (Non-patent Documents 1 to 3). Furthermore, what is becoming clear is factors necessary for satisfying druglikeness (properties of having excellent metabolic stability and membrane permeability) of medium-sized peptides (for example, the range of total number of amino acids, number of N-substituted amino acids, and lipophilicity of the peptides) (Patent Document 1).

Accordingly, medium-sized molecule drug discovery may be made efficient by constructing a library containing medium-sized cyclic peptides satisfying required membrane permeability and metabolic stability, selecting peptides having drug efficacy (hit compounds) from the library through display library technologies, and structurally optimizing them.

In recent years, regarding libraries containing a great variety of peptides, research on libraries produced by the mRNA display method is advancing. This technology enables creation of a library containing 10¹² varied types of compounds within a short period of time. Furthermore recently, reports have been made on methods for preparing peptides containing amino acid analogs using a reconstituted cell-free translation system and also on examples of preparation of libraries for displaying cyclic peptides containing amino acid analogs (Patent Document 2, Non-patent Documents 4 and 5).

However, considering that peptides produced by conventional methods, which are cyclized through a thioether bond, are generally susceptible to oxidative metabolism (Non-patent Document 6), there is still room for improvement in terms of metabolic stability.

On the other hand, libraries of peptides cyclized through an amide bond as reported in Patent Document 1 have superior metabolic stability and membrane permeability compared to the aforementioned libraries of peptides having a thioether-cyclized moiety.

One of the methods for constructing a library of peptides having an amide-cyclized moiety is the method comprising translation of peptides carrying a cysteine or a cysteine analog at their N terminus and an active ester in the side chain of their C-terminal amino acid, followed by cyclization of the peptides by native chemical ligation (Patent Document 1).

The following three methods have been reported (Patent Document 1) for the translational synthesis (ribosomal synthesis) of peptides carrying a cysteine or a cysteine analog at their N terminus and serving as precursors of the cyclized peptides.

The first method is the one where a peptide having a cysteine at its N terminus is synthesized by enzymatically cleaving, immediately before a cysteine, the N-terminal amino acid or the N-terminal peptide of a peptide that begins with formylmethionine. As examples of the enzyme, peptide deformylase (PDF) and methionine amino peptidase (MAP) have been reportedly used in the method (Non-patent Document 7).

The second method is the one where a reconstituted cell-free translation system, from which methionine is removed in advance, is used and cysteine or a cysteine analog is positioned at the second position from the N terminus to perform ribosomal synthesis with skipping methionine which is the initiation codon (this is defined as the initiation read-through (iRT) method).

The third method performs ribosomal synthesis with adding, in advance as the initiation tRNA, aminoacyl-tRNA prepared by aminoacylation with cysteine or a cysteine analog having an optionally protected amino or thiol group, to a reconstituted cell-free translation system that does not contain methionine or translation initiation methionine tRNA (this is defined as the initiation suppression (iSP) method).

Patent Document 1 reports that when cysteine and cysteine analogs are unprotected, preparing their aminoacyl-tRNAs is difficult and it also reports the use of aminoacyl-tRNAs having a pentenoyl group or a 6-nitroveratryloxycarbanyl (NVOC) group as their amino protecting group.

On the other hand, as methods for ribosomally synthesizing peptides comprising a plurality of amino acid analogs, known methods include methods in which tRNAs are prepared in advance by artificial aminoacylation with amino acid analogs and then used for translation, and methods in which amino acid analogs are used to prepare aminoacyl-tRNAs with natural aminoacyl-tRNA synthetase (ARS) in the translation system and then translationally incorporated (Non-patent Documents 5, 8, and 9). These are methods that use aminoacyl-tRNAs carrying the amino acid analogs directly involved in the translational incorporation site.

However, even when such methods are used for ribosomal synthesis of peptides containing a plurality of amino acid analogs, there remain problems of inefficiency of translation or decrease in product purity. For example, the phenomena of production of peptides cleaved before or after the amino acid analog introduction site (these are defined as "cleaved peptides") have been known (Non-patent Document 10).

### Prior Art Documents

### [Patent Documents]

[Patent Document 1] WO 2013/100132
[Patent Document 2] WO 2008/117833

### [Non-patent Documents]

[Non-patent Document 1] J. Chatterjee, et al., Acc. Chem. Res. 2008, 41, 1331.
[Non-patent Document 2] J. E. Bock, et al., ACS Chem. Biol. 2013, 8, 488.
[Non-patent Document 3] K. Jpsephson, et al., Drug Discovery Today 2014, 19, 388-399.
[Non-patent Document 4] H. Suga et al., Chem. Bio. 2008, 15, 32.
[Non-patent Document 5] J. W. Szostak et al., J. Am. Chem. Soc. 2012, 134, 10469-10477
[Non-patent Document 6] R Kato, T Kamataki, T Yokoi, ed., Yakubutu-taishagaku (Drug Metabolomics) Iryoyakugaku/Iryohin Kaihatsu-no-kisotoshite (as Basis for Medical Pharmacy/Drug Development), 3rd edition
[Non-patent Document 7] Meinnel, T., et al., Biochimie (1993) 75, 1061-1075 [Non-patent Document 8] M. C. T. Hartman et al., PLoS one, 2007, 10, e972.
[Non-patent Document 9] T. Kawakami, ACS Chem Biol., 8, 1205-1214, 2013
[Non-patent Document 10] J. W. Szostak et al., J. Am. Chem. Soc. 2008, 130, 6131-6136

### Summary of the Invention

### [Problems to be Solved by the Invention]

The present invention was achieved in view of the above-described problems. An objective of the present invention is to provide efficient translation techniques for peptides comprising a plurality of amino acid analogs, in which an amino acid residue carrying a thiol group near its amino group is positioned at the N-terminus. Furthermore, an objective of the present invention is to provide methods for preparing peptides having a cyclic portion which contains a plurality of amino acid analogs and has an amide bond-cyclized moiety, complexes of the peptides and nucleic acids, and libraries containing the complexes.

### [Means for Solving the Problems]

The present inventors elucidated that the translation initiation method, which has been assumed to be not directly involved in translation elongation reaction, affects the translation efficiency of amino acid analogs in the downstream of translation, the purity of the products, and the production of cleaved peptides which are impurities. These facts could not be detected through conventional evaluation methods (*i.e.,* evaluation using peptides composed of naturally-occurring amino acids except for the N terminus), but was able to be detected for the first time through evaluation using peptides containing a plurality of amino acid analogs.

More specifically, under the coexistence of various functional groups in peptides or peptide-nucleic acid complexes, the present inventors carried out translation using the iSP method for a peptide having at the N terminus an amino acid residue carrying a thiol group near its amino group with both the thiol group and the amino group being protected by a specific protecting group that can be selectively and quantitatively deprotected, and elucidated that the probability of initiation of the translation reaction increases, production of cleaved peptides is suppressed, and translation efficiency and purity are improved. Furthermore, the present inventors discovered novel methods for producing peptides having cyclized moieties and complexes of the peptides and nucleic acids, the methods enabling efficient cyclization of peptides obtained by using the iSP method and peptide moieties of the complexes of the peptides and nucleic acids, and completed the present invention.

Specifically, the present invention includes the following:
[1] a method for preparing a peptide comprising two or more amino acid analog residues and having a cyclic portion, a complex of said peptide and a nucleic acid, or a library comprising said complex,
   wherein the method comprises the step of forming an amide bond by reacting a first reaction point with a second reaction point in a peptide, or in a complex of the peptide and a nucleic acid, wherein the peptide is synthesized by translating a nucleic acid encoding a noncyclic peptide which comprises at its N terminus an amino acid residue represented by general formula (I) or general formula (II) shown below comprising the first reaction point, and which comprises at a position at least four residues apart from the N terminus to the C-terminal side an amino acid residue comprising the second reaction point in one of its side chains: 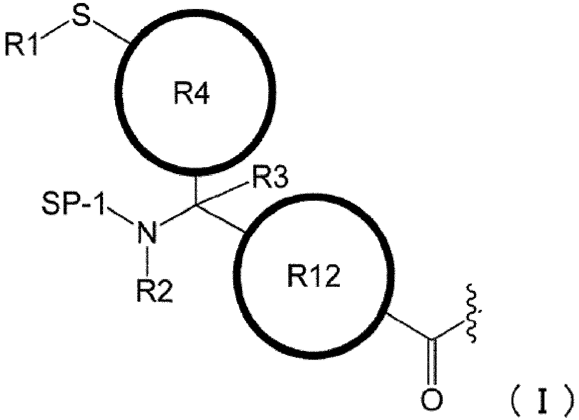 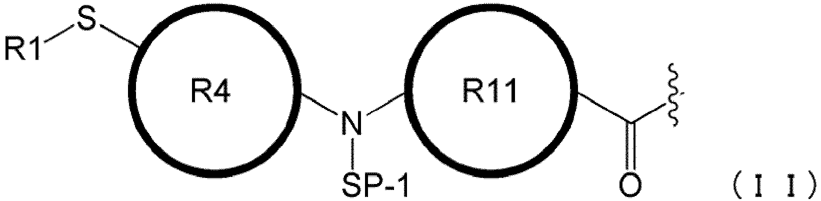 wherein,
   R1 is a thiol group-protecting group which can be ribosomally synthesized;
   R2 and R3 each independently are a hydrogen atom, an alkyl group, an alkenyl group, an alkynyl group, an aryl group, a heteroaryl group, an aralkyl group, or a cycloalkyl group, each of which is optionally substituted; or R2 and R3 form a ring together with atoms to which they are bonded; or R2 or R3 form a ring together with R4 and atoms to which they are bonded; with the proviso that when R2 forms an azido group (-N₃) together with the N atom to which it is bonded and SP-1, the above-mentioned definition does not apply to R2;
   R4 represents an alkylene, an arylene, a heteroarylene, an alkylenearylene, an alkyleneheteroarylene, an arylenealkylene, or a heteroarylenealkylene, each of which is optionally substituted;
   R11 and R12 each independently are a single bond, an alkylene, an arylene, a heteroarylene, an alkylene arylene, an alkyleneheteroarylene, an arylenealkylene, or a heteroarylenealkylene, each of which is optionally substituted;
   SP-1 forms an azido group together with the N atom to which it is bonded and R2, or is an amino group-protecting group represented by the following formula: 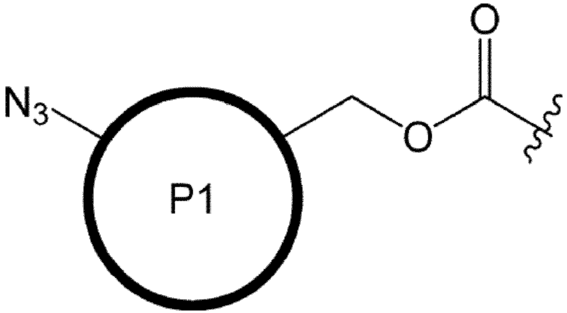
   wherein, P1 is a single bond, an arylene, or a heteroarylene,
   or represented by the following formula: 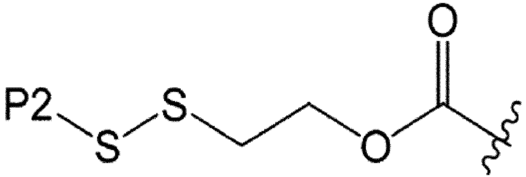
   wherein, P2 is an alkyl, an aryl, or a heteroaryl;
[2] the method of [1], wherein R1 is selected from the group consisting of S-R23, wherein R23 is an alkyl, an alkenyl, an alkynyl, an aryl, a heteroaryl or an aralkyl, each of which is optionally substituted; a sulfonate (-SO₃⁻); and a thiosulfonate (-S₂O₃⁻);
[3] the method of [2], wherein R23 is a methyl, an ethyl, an isopropyl, a tert-butyl, a phenyl, a *p*-trifluoromethylphenyl, a p-fluorophenyl, a benzyl, or a phenethyl, each of which is optionally substituted;
[4] the method of any one of [1] to [3], wherein R2 and R3 each independently are a hydrogen atom, a C1-C4 alkyl optionally substituted with a halogen or a C1-C4 alkoxy optionally substituted with a halogen;
[5] the method of any one of [1] to [4], wherein R4 is selected from the group consisting of the following: 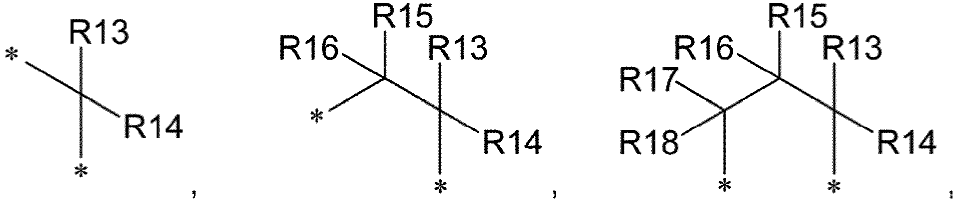 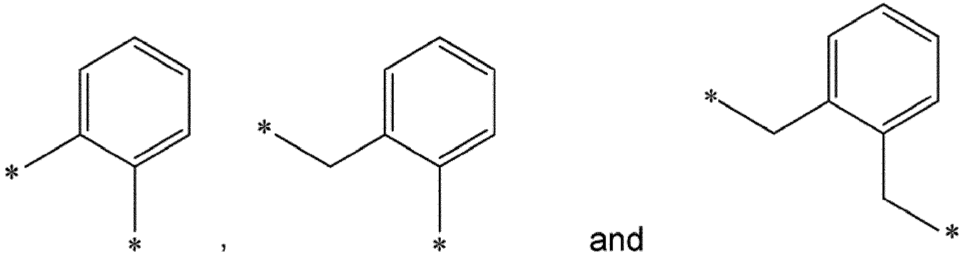 wherein R13 to R18 each independently are a hydrogen atom, an optionally substituted alkyl, or an optionally substituted alkoxy;
[6] the method of [5], wherein R13 to R18 each independently are a hydrogen atom or a methyl;
[7] the method of any one of [1] to [6], wherein R11 and R12 each independently are a single bond or are selected from the group consisting of the following: 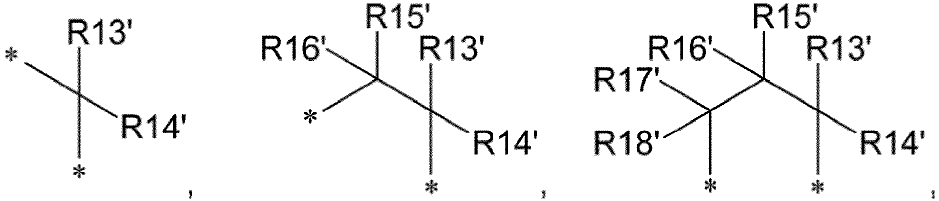 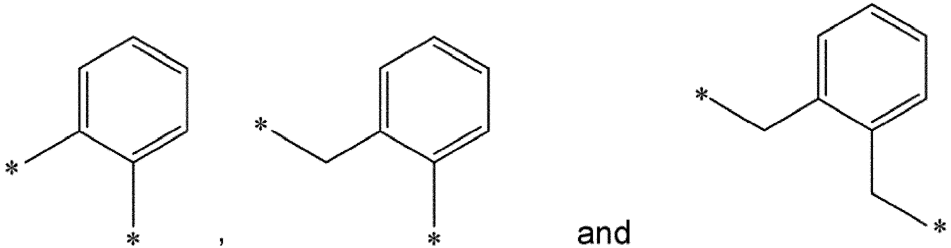 wherein R13' to R18' each individually are a hydrogen atom, an optionally substituted alkyl, or an optionally substituted alkoxy;
[8] the method of [7], wherein R13' to R18' each independently are a hydrogen atom or a methyl group;
[9] the method of any one of [1] to [8], wherein the amino acid residue represented by general formula (I) is an amino acid residue represented by any one of the following general formulas: 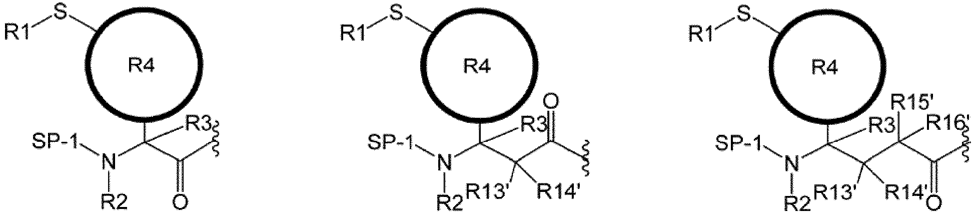 wherein
   R1 to R4 and SP-1 have the same meaning as R1 to R4 and SP-1 of [1], respectively, and
   R13' to R16' have the same meaning as R13' to R16' of [7], respectively; or
   the amino acid residue represented by general formula (II) is an amino acid residue represented by any one of the following general formulas: 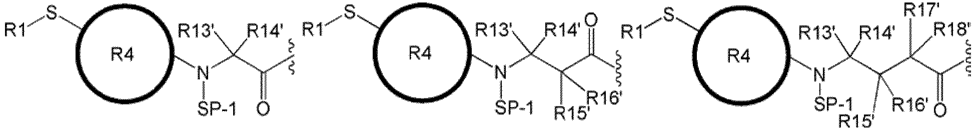 wherein
   R1, R4, and SP-1 have the same meaning as R1, R4, and SP-1 of [1], respectively, and
   R13' to R18' have the same meaning as R13' to R18' of [7], respectively;
[10] the method of any one of [1] to [9], wherein the amino acid residue represented by general formula (I) is an amino acid residue represented by any one of the following general formulas: 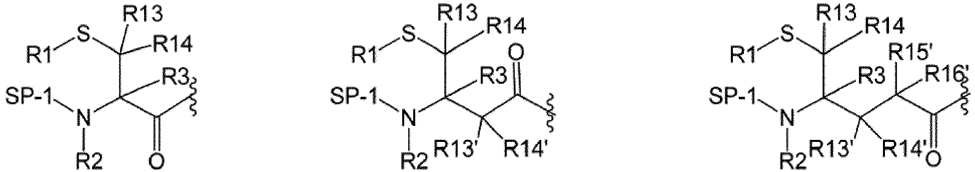 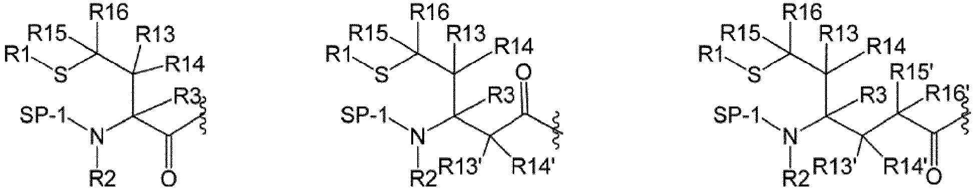 wherein
   R1 to R3 and SP-1 have the same meaning as R1 to R3 and SP-1 of [1], respectively;
   R13 to R16 have the same meaning as R13 to R16 of [5], respectively; and
   R13' to R16' have the same meaning as R13' to R16' of [7], respectively; or
   R2 forms a ring together with R13, R14, R15, or R16 and atoms to which they are bonded, with the proviso that when R2 forms an azido group (-N₃) together with the N atom to which it is bonded and SP-1, the above-mentioned definition does not apply to R2; or the amino acid residue represented by general formula (II) is an amino acid residue represented by any one of the following general formulas: 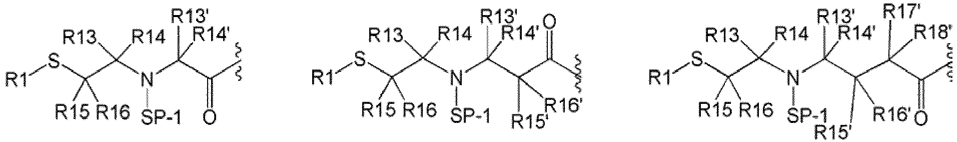 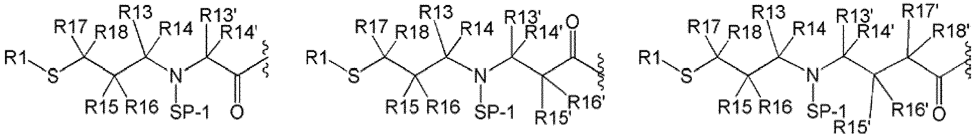
   wherein
   R1 and SP-1 have the same meaning as R1 and SP-1 of [1], respectively;
   R13 to R18 have the same meaning as R13 to R18 of [5], respectively; and
   R13' to R18' have the same meaning as R13' to R18' of [7], respectively;
[11] the method of any one of [1] to [10], wherein SP-1 is a 4-azidobenzyloxycarbonyl (*p*-Acbz), a 2-azidobenzyloxycarbonyl (*o*-Acbz), an azidomethoxycarbonyl (Azoc), a phenyldisulfanylethyloxycarbonyl (Phdec), a 2-pyridyldisulfanylethyloxycarbonyl (Pydec), or a 2-(*t*-butyldisulfanyl)ethyloxycarbonyl (Tbeoc); or SP-1 forms an azido group together with the N atom to which it is bonded and R2;
[12] the method of any one of [1] to [11], wherein the amino acid residue comprising the second reaction point in one of the side chains is represented by the following general formula: 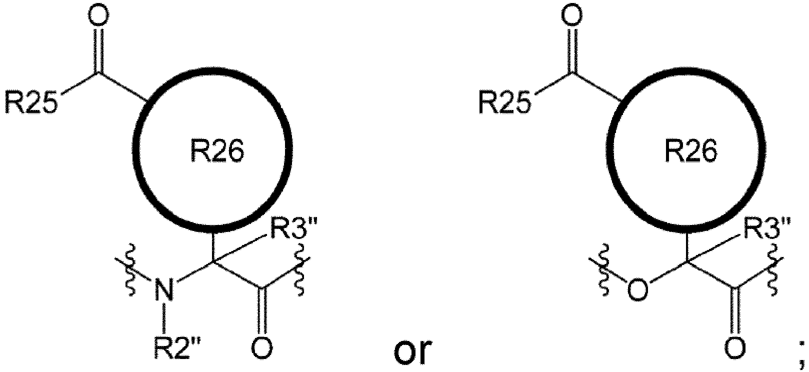 wherein
   R2" and R3" each independently are a hydrogen atom, an alkyl, an alkenyl, an alkynyl, an aryl, a heteroaryl, an aralkyl, or a cycloalkyl, each of which is optionally substituted; or R2" and R3" form a ring together with atoms to which they are bonded; or R2" or R3" form a ring together with R26 and atoms to which they are bonded;
   R25 is a hydroxyl group or forms an active ester with CO to which it is bonded; and
   R26 is an alkylene, an arylene, a heteroarylene, an arylenealkylene, or a heteroarylenealkylene, each of which is optionally substituted;
[13] the method of any one of [1] to [12], wherein R26 is selected from the group consisting of the following: 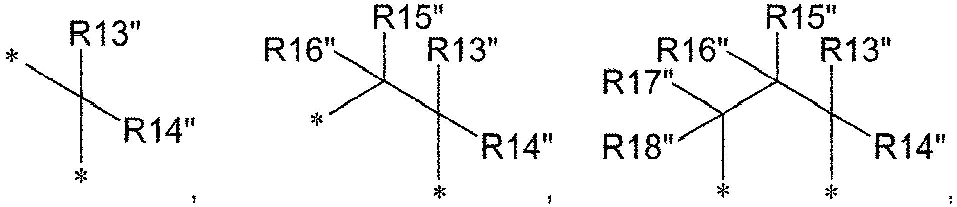 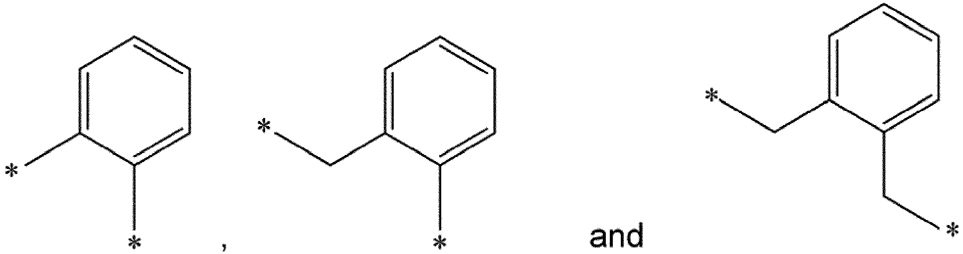 wherein, R13" to R18" each independently are a hydrogen atom, an optionally substituted alkyl, or an optionally substituted alkoxy;
[14] the method of any one of [1] to [13], wherein the amino acid residue comprising the second reaction point in one of the side chains is represented by the following general formula: 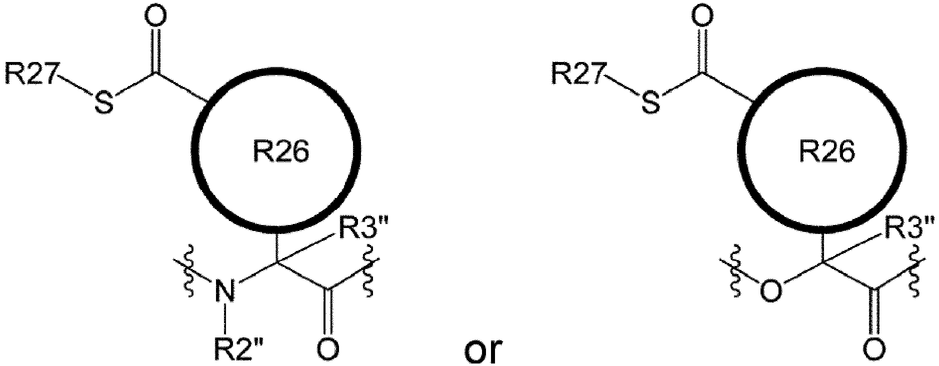 wherein
   R2" has the same meaning as R2" of [12];
   R3" is a hydrogen atom or an optionally substituted C1-C4 alkyl group; and
   R27 is selected from among a hydrogen atom, an optionally substituted alkyl group, an optionally substituted alkenyl group, an optionally substituted alkynyl group, an optionally substituted aryl group, an optionally substituted heteroaryl group, an optionally substituted cycloalkyl group, and an optionally substituted aralkyl group;
[15] the method of any one of [1] to [14], wherein the amino acid residue comprising the second reaction point in one of the side chains is represented by the following general formula: 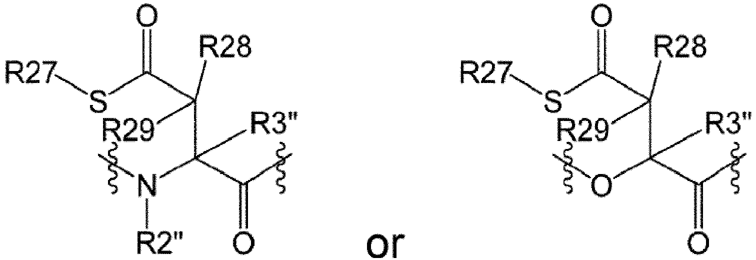 wherein
   R2" has the same meaning as R2" of [12];
   R3" is a hydrogen atom or an optionally substituted C1-C4 alkyl group;
   R28 and R29 each independently are a hydrogen atom, an optionally substituted C1-C6 alkyl group, an optionally substituted C2-C6 alkenyl group, an optionally substituted C2-C6 alkynyl group, an optionally substituted aryl group, an optionally substituted heteroaryl group, an optionally substituted aralkyl group, or an optionally substituted cycloalkyl group; and R27 is selected from among a hydrogen atom, an optionally substituted alkyl group, an optionally substituted alkenyl group, an optionally substituted alkynyl group, an optionally substituted aryl group, an optionally substituted heteroaryl group, an optionally substituted cycloalkyl group, and an optionally substituted aralkyl group;
[16] the method of any one of [1] to [15], which further comprises the step of desulfurizing an -SH group present in the cyclic portion;
[17] the method of any one of [1] to [16], wherein the complex of cyclic portion-comprising peptide and nucleic acid comprises a linker between the peptide and the nucleic acid;
[18] the method of any one of [1] to [17], wherein the peptide is synthesized by translation in a cell-free translation system not containing at least one of methionine, methionyl tRNA synthetase (MetRS), translation initiation tRNA for methionine, formyl donor, and methionyl tRNA transferase;
[19] a compound represented by the following general formula (IA) or general formula (IIA): 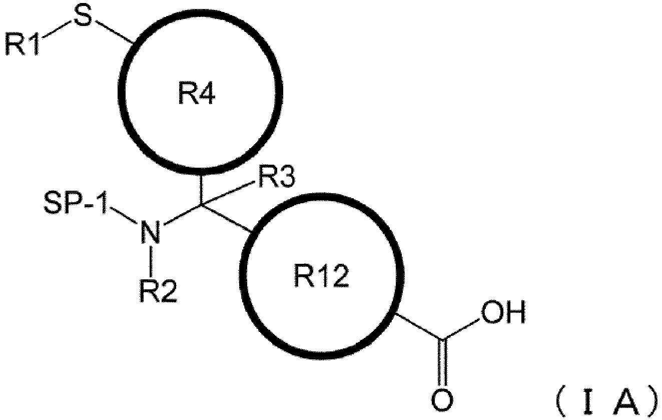 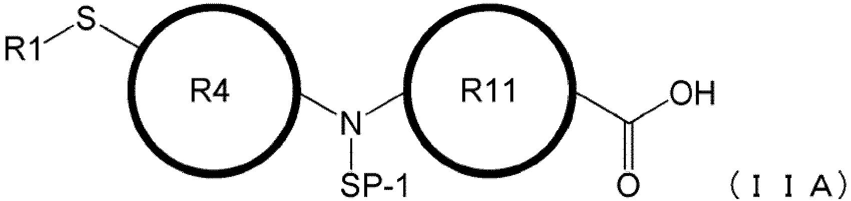 wherein R1 to R4, R11, R12, and SP-1 have the same meaning as R1 to R4, R11, R12, and SP-1 of [1], respectively;
[20] a compound represented by the following general formula (IB) or general formula (IIB): 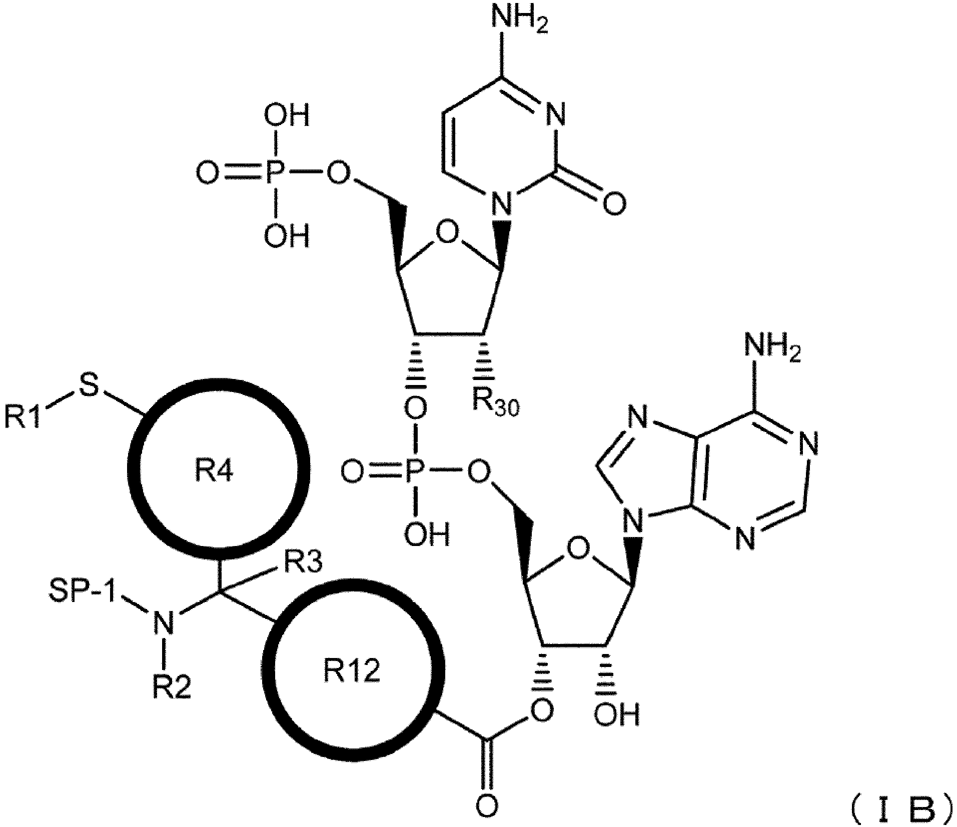 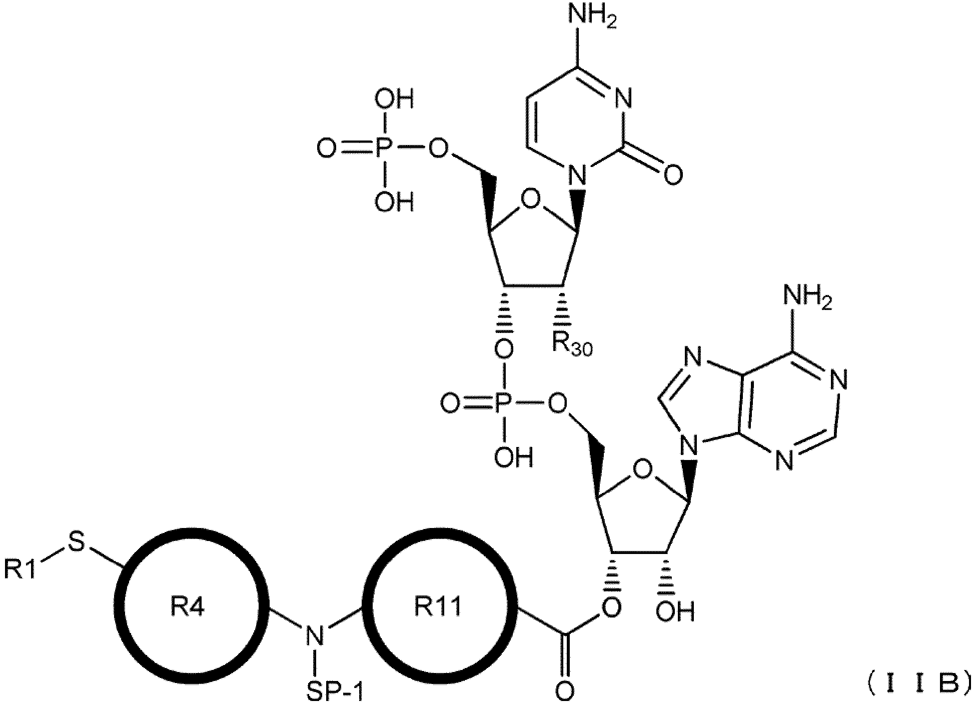 wherein, R1 to R4, R11, R12, and SP-1 have the same meaning as R1 to R4, R11, R12, and SP-1 of [1], respectively; and R30 represents H or a hydroxyl group;
[21] an aminoacyl-tRNA formed by bonding the compound of [19] and tRNA;
[22] a noncyclic peptide or a complex of the peptide and a nucleic acid, wherein the peptide comprises at its N terminus an amino acid residue represented by general formula (I) or general formula (II) shown below comprising a first reaction point, and which comprises at a position at least four residues apart from the N terminus to the C-terminal side an amino acid residue comprising a second reaction point in one of its side chains: 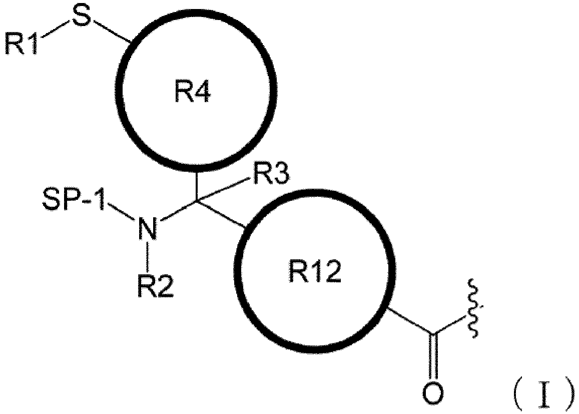 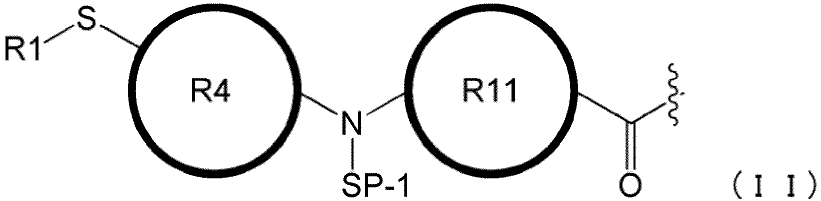 wherein R1 to R4, R11, R12, and SP-1 have the same meaning as R1 to R4, R11, R12, and SP-1 of [1], respectively;
[23] the peptide or the complex of [22], wherein the noncyclic peptide is obtained by a method comprising the step of synthesizing the noncyclic peptide by translating a nucleic acid encoding the noncyclic peptide which comprises at its N terminus an amino acid residue represented by general formula (I) or general formula (II) comprising a first reaction point, and which comprises at a position at least four residues apart from the N terminus to the C-terminal side an amino acid residue comprising a second reaction point in one of its side chains: 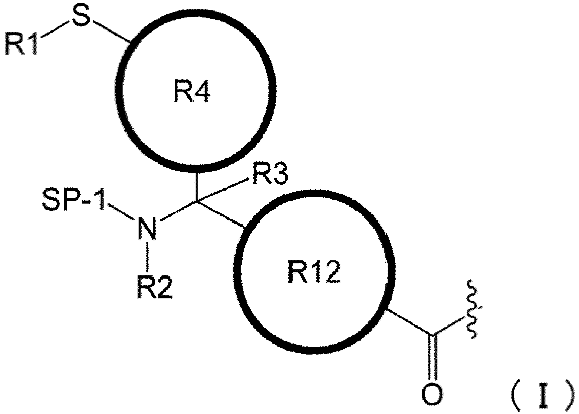 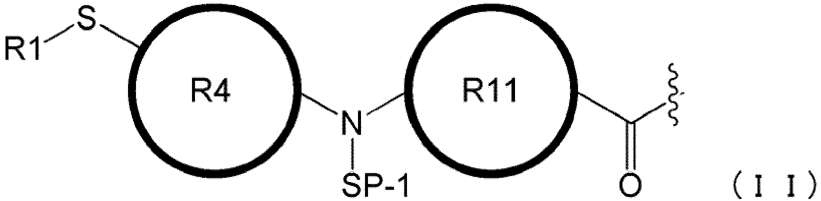 wherein, R1 to R4, R11, R12, and SP-1 have the same meaning as R1 to R4, R11, R12, and SP-1 of [1], respectively;
[24] a method of producing a peptide comprising two or more amino acid analog residues and having a cyclic portion, a complex of the peptide and a nucleic acid, or a library comprising the complex using the peptide or the complex of [22] or [23];
[25] the method of any one of [1] to [18], wherein the amino acid residue represented by general formula (I) is an amino acid residue represented by any one of the following formulas: 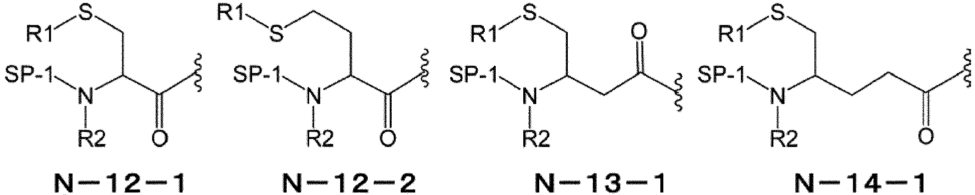 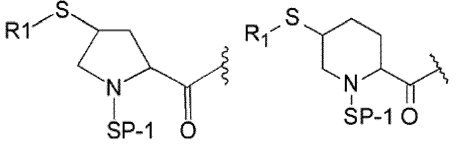 wherein R1, R2, and SP-1 have the same meaning as R1, R2, and SP-1 of [1], respectively; or the amino acid residue represented by general formula (II) is an amino acid residue represented by any one of the following formulas: 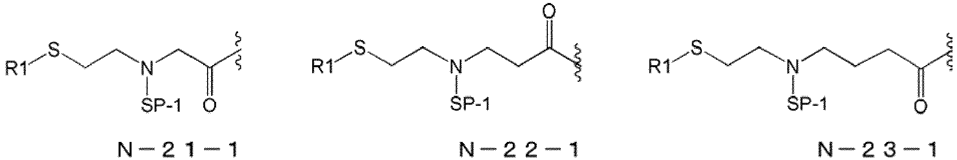 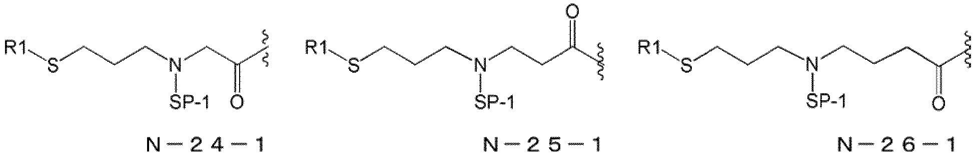 wherein, R1 is -S-R23;
   R23 is a C1-C20 alkyl group, an optionally substituted phenyl, or an optionally substituted benzyl; and
   SP-1 is p-Acbz, o-Acbz, or Azoc;
[26] the method of any one of [1] to [18] and [25], wherein the amino acid residue represented by general formula (I) is an amino acid residue represented by any one of the following formulas: 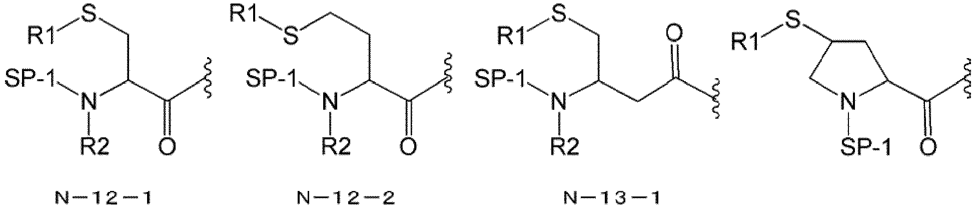 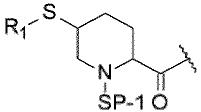
   wherein R1 and SP-1 have the same meaning as R1 and SP-1 of [1], respectively; and R2 is a hydrogen atom or an alkyl group which is optionally substituted; or
   the amino acid residue represented by general formula (II) is an amino acid residue represented by any one of the following formulas: 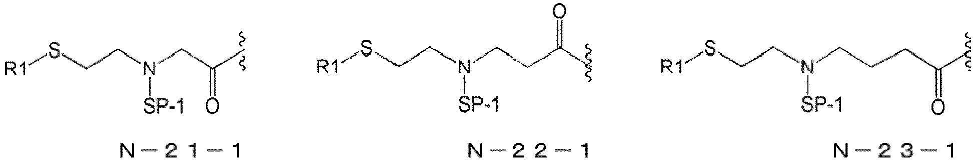
   wherein R1 is -S-R23;
      R23 is a methyl, an ethyl, an *n*-propyl, an *i*-propyl, an *n*-butyl, a *t*-butyl, or a *sec*-butyl; and
      SP-1 is *p*-Acbz, *o*-Acbz, or Azoc; and
[27] a method for preparing a peptide comprising two or more amino acid analog residues and having a cyclic portion, a complex of the peptide and a nucleic acid, or a library comprising the complex, wherein the method comprises the steps of:
   (a) synthesizing a noncyclic peptide, or a complex of the peptide and a nucleic acid, by translating a nucleic acid encoding the noncyclic peptide which comprises at its N terminus an amino acid residue represented by general formula (I) or general formula (II) shown below comprising a first reaction point, and which comprises at a position at least four residues apart from the N terminus to the C-terminal side an amino acid residue comprising a second reaction point in one of its side chains: 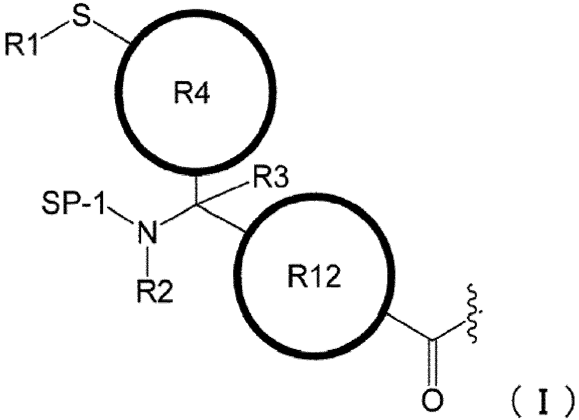 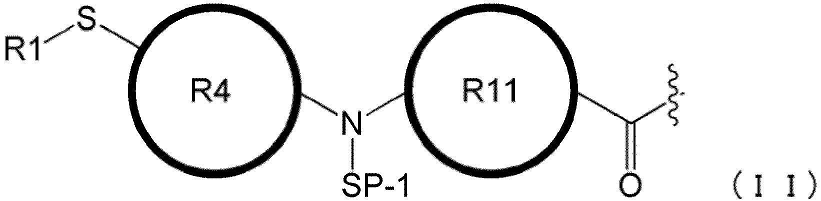 wherein,
      R1 is a thiol group-protecting group which can be ribosomally synthesized;
      R2 and R3 each independently are a hydrogen atom, an alkyl, an alkenyl, an alkynyl, an aryl, a heteroaryl, an aralkyl, or a cycloalkyl, each of which is optionally substituted; or R2 and R3 form a ring together with atoms to which they are bonded; or R2 or R3 form a ring together with R4 and atoms to which they are bonded, with the proviso that when R2 forms an azido group (-N₃) together with the N atom to which it is bonded and SP-1, the above-mentioned definition does not apply to R2;
      R4 is an alkylene, an arylene, a heteroarylene, an alkylenearylene, an alkyleneheteroarylene, an arylenealkylene, or a heteroarylenealkylene, each of which is optionally substituted;
      R11 and R12 each independently are a single bond, an alkylene, an arylene, a heteroarylene, an alkylenearylene, an alkyleneheteroarylene, an arylenealkylene, or a heteroarylenealkylene, each of which is optionally substituted;
      SP-1 forms an azido group together with the N atom to which it is bonded and R2, or is an amino group-protecting group represented by the following formula: 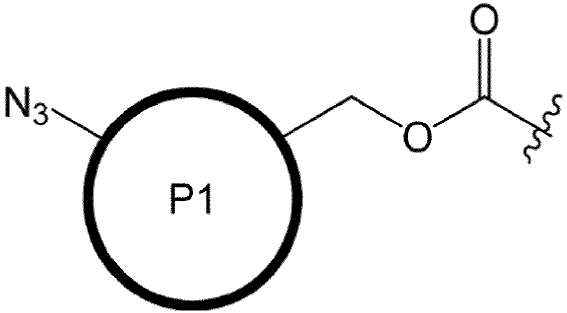
      wherein, P1 is a single bond, an arylene, or a heteroarylene,
      or the following formula: 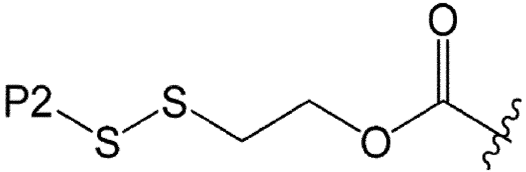 wherein P2 is an alkyl, an aryl, or a heteroaryl; and
   (b) removing R1 and SP-1 of general formula (I) from the noncyclic peptide or the peptide-nucleic acid complex obtained in step (a), and forming an amide bond by reacting the first reaction point with the second reaction point.

### [Effects of the Invention]

The present invention enables ribosomal synthesis of a peptide comprising a plurality of amino acid analogs and a complex of the peptide and a nucleic acid in high yield and purity with suppressing production of cleaved peptides, and also enables their efficient cyclization.

This results in the provision of peptides having a drug-like cyclic portion which comprises a plurality of amino acid analogs, complexes of the peptides and nucleic acids, and display libraries of the complexes. The display libraries constructed using the present invention enable ribosomal synthesis of peptides comprising a plurality of amino acid analogs with higher efficiency compared to conventional methods; therefore, the probability of obtaining a greater variety of drug-like hit compounds is increased further.

Furthermore, use of the present invention enables translationally introducing at the N terminus an amino acid residue carrying a thiol group near its amino group, including not only L cysteine but also D-cysteine and L or D-*N*-methylcysteine. Therefore, drug-like display libraries with higher diversity can be provided.

Furthermore, since hit compounds obtained from mRNA display libraries of the present invention already have excellent membrane permeability and metabolic stability, they can be efficiently optimized as pharmaceutical products.

### Brief Description of the Drawings

Fig. 1 shows electrophoretic confirmation of ribosomally synthesized peptides carrying a *p*-Acbz-protected cysteine analog at their N terminus.
Fig. 2-1 shows MALDI TOF MS confirmation of a ribosomally synthesized peptide carrying a *p*-Acbz-protected *N*-methylcysteine.
Fig. 2-2 shows MALDI TOF MS confirmation of a ribosomally synthesized peptide carrying a *p*-Acbz-protected *N*-methyl-D-cysteine.

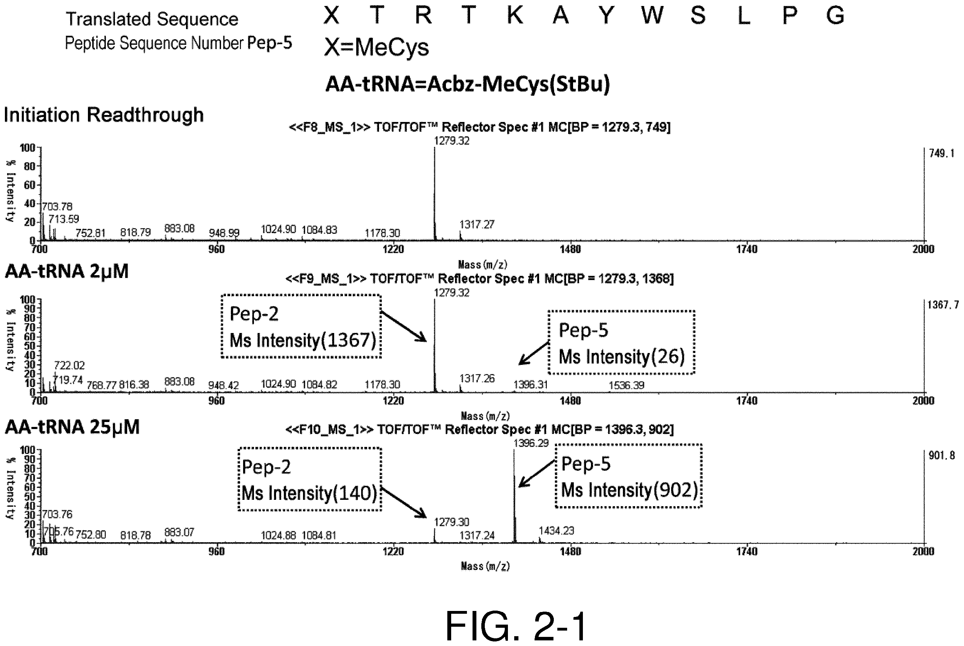

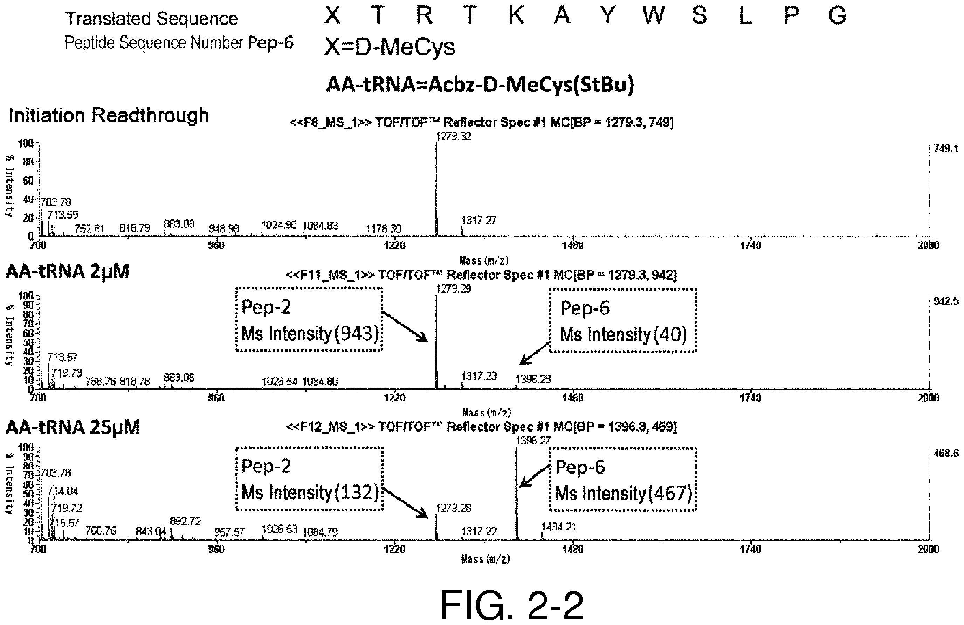

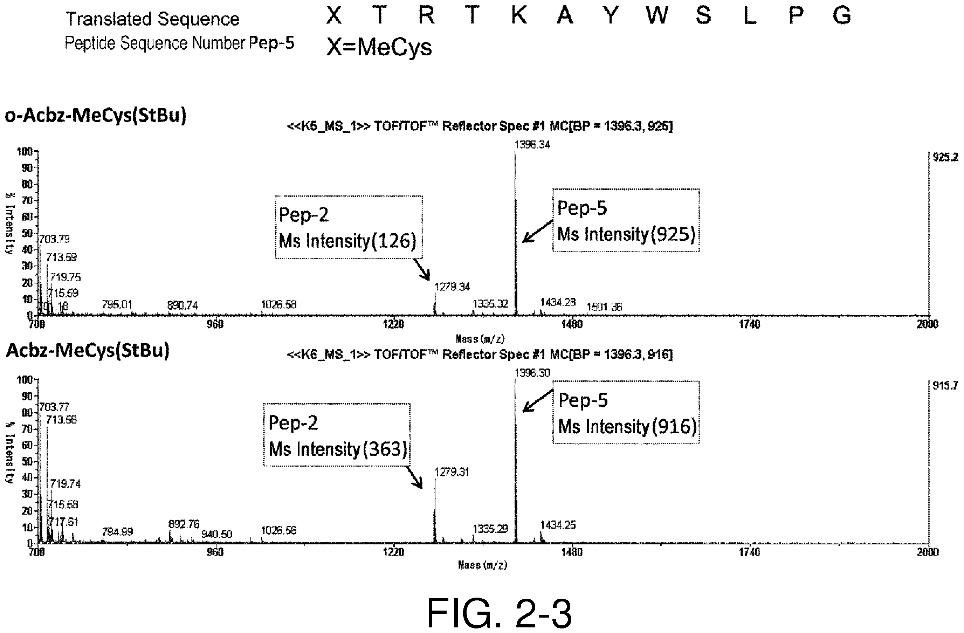

Fig. 2-3 shows MALDI TOF MS confirmation of ribosomally synthesized peptides carrying an o-Acbz or *p*-Acbz-protected *N*-methylcysteine.

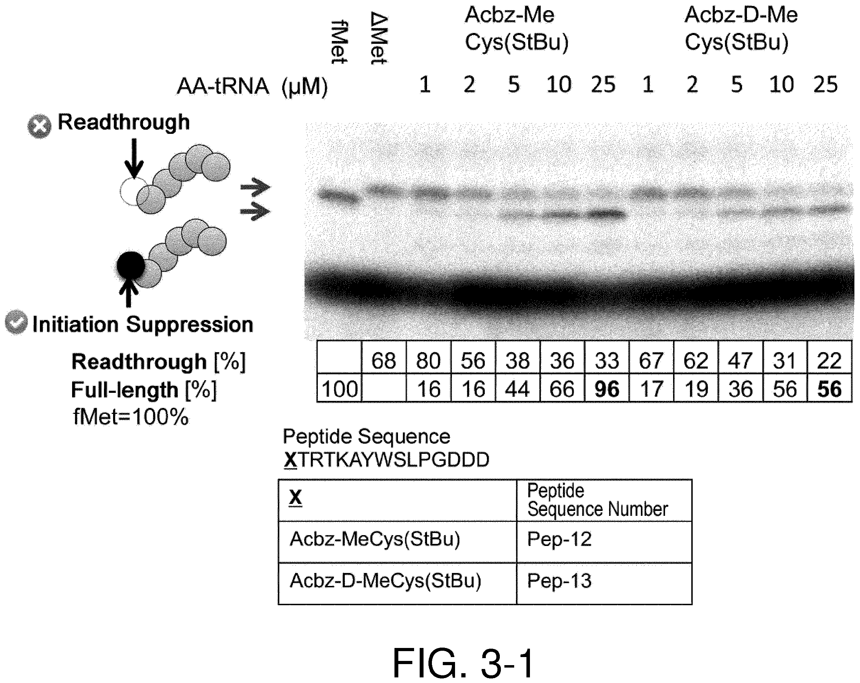

Fig. 3-1 shows electrophoretic confirmation of ribosomally synthesized peptides carrying an Acbz-protected *N*-methylcysteine.

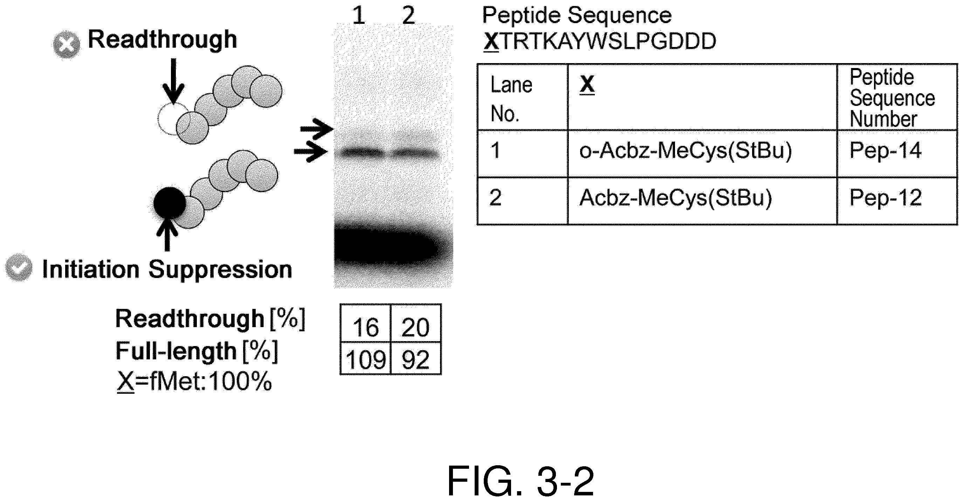

Fig. 3-2 shows electrophoretic confirmation of ribosomally synthesized peptides carrying an o-Acbz or *p*-Acbz-protected *N*-methylcysteine.

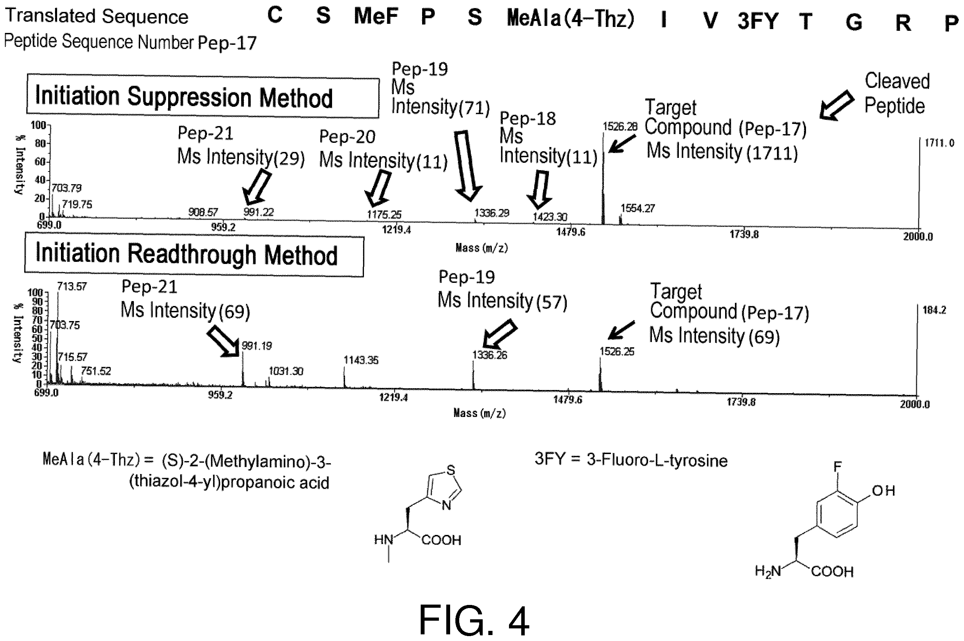

Fig. 4 shows the results of MALDI-TOF MS analyses performed on the same peptides ribosomally synthesized using the initiation suppression method or the initiation read-through method as the translation initiation methods. Comparative observation of the initiation read-through method and the initiation suppression method showed that the initiation suppression method produced the desired product exhibiting stronger MS intensity. MS intensities of the cleaved peptides were observed to be weaker, and the respective MS intensities were 1/20 or less than the intensity of the desired product.

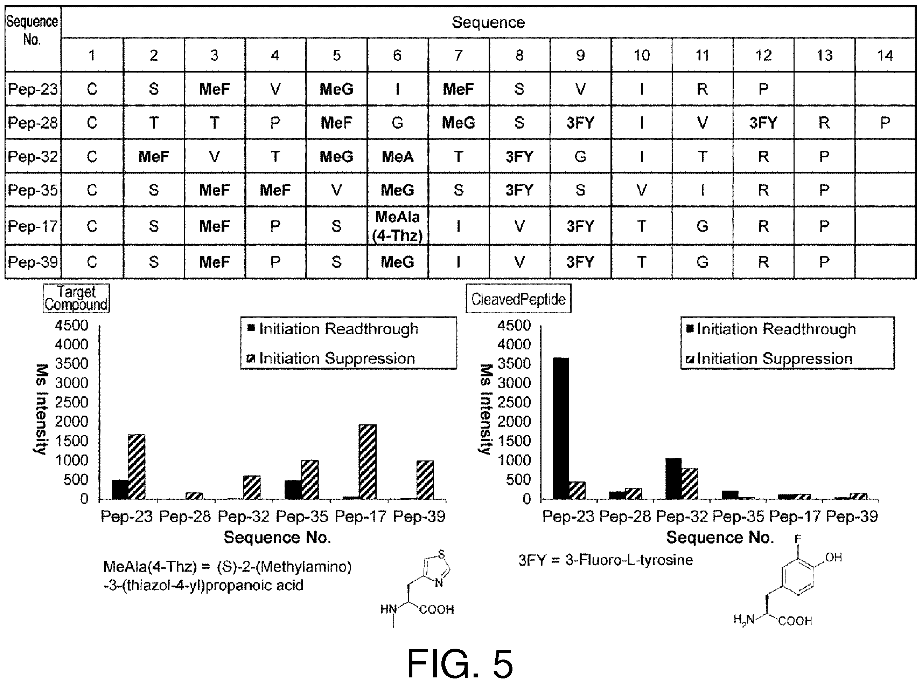

Fig. 5 shows the MS intensities of the desired product and of cleaved peptides which are side products obtained when peptides comprising a plurality of amino acid analogs were ribosomally synthesized by each of the translation initiation methods. In the graph showing the MS intensities of the cleaved peptides, when multiple cleaved peptides were observed, the total value of their MS intensities were presented. Comparative observation of the initiation read-through method and the initiation suppression method showed that the initiation suppression method produced the desired product exhibiting stronger MS intensity and the cleaved peptides exhibiting weaker MS intensities.

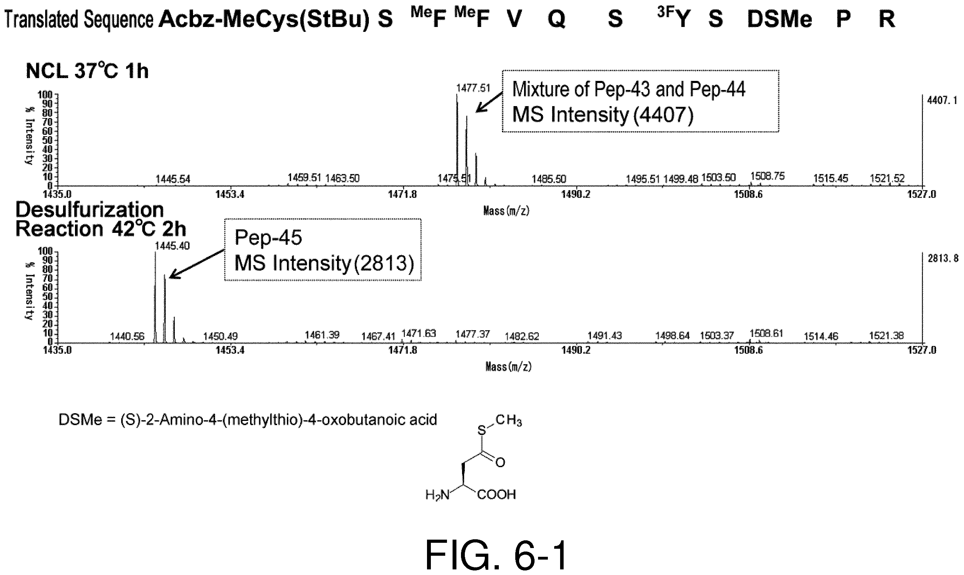

Fig. 6-1 shows the mass spectra of a compound obtained by ribosomally synthesizing a peptide carrying a protected *N*-methylcysteine at its N terminus and an active ester in the side chain of an amino acid at the C-terminal side, deprotecting the protected *N*-methylcysteine, and then performing a cyclization reaction by native chemical ligation; and of an amide-cyclized peptide obtained by a subsequent desulfurization reaction.

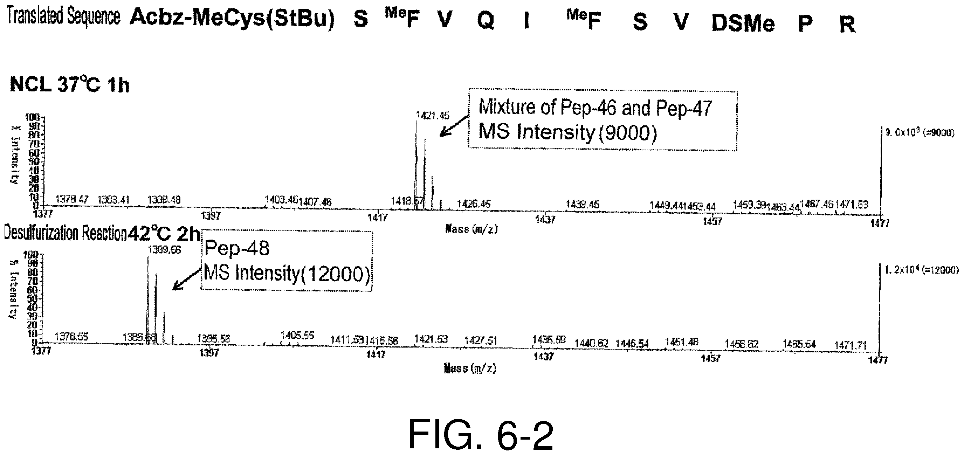

Fig. 6-2 shows a sequel to Fig. 6-1.

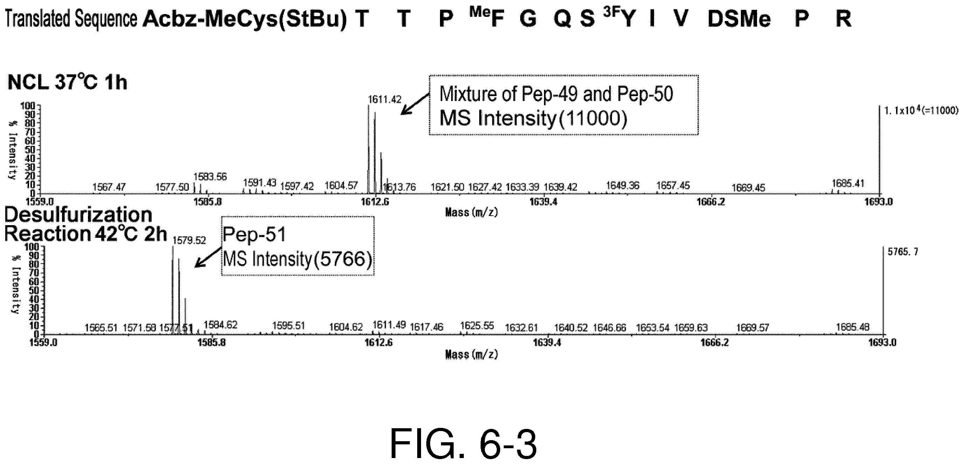

Fig. 6-3 shows a sequel to Fig. 6-2.

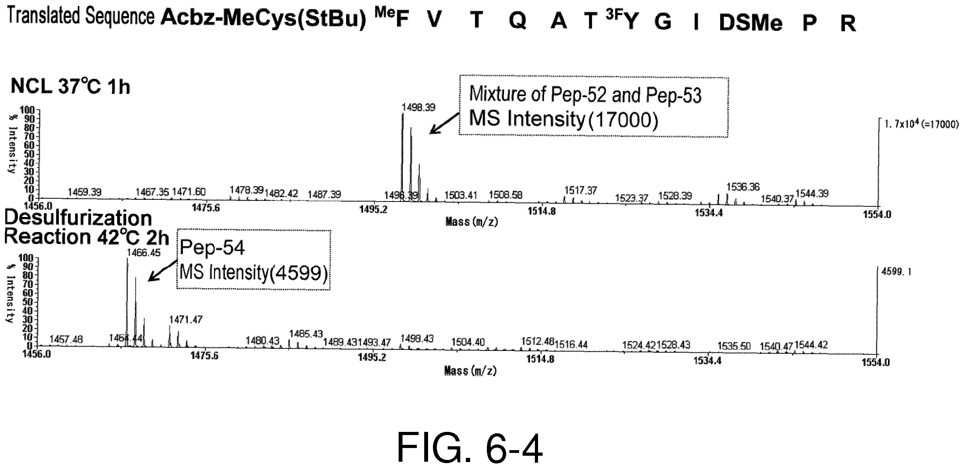

Fig. 6-4 shows a sequel to Fig. 6-3.

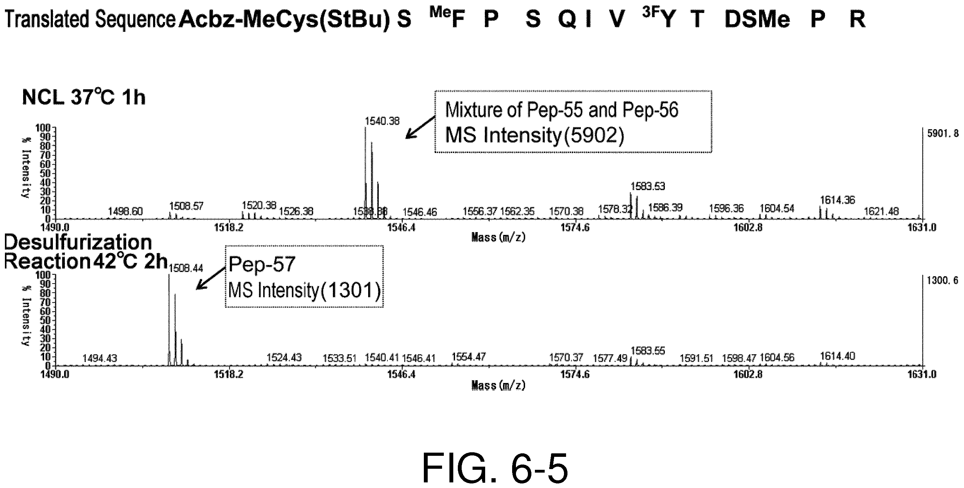

Fig. 6-5 shows a sequel to Fig. 6-4.

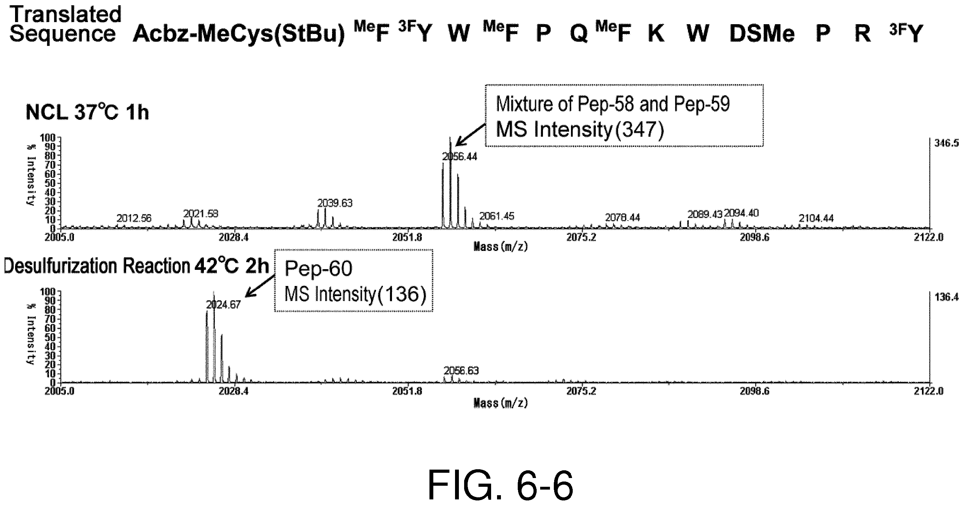

Fig. 6-6 shows a sequel to Fig. 6-5.

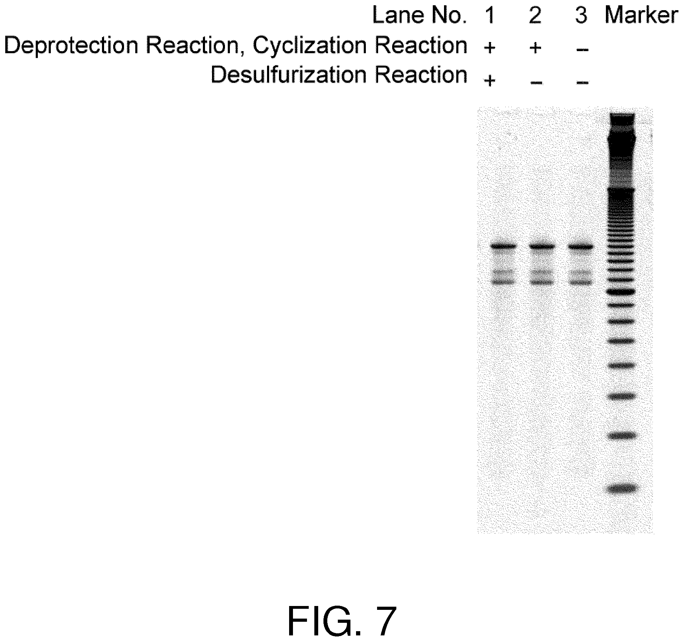

Fig. 7 shows the results of electrophoretic evaluation of the stability of RNA under conditions for Acbz deprotection reaction, cyclization reaction by native chemical ligation, and desulfurization reaction.

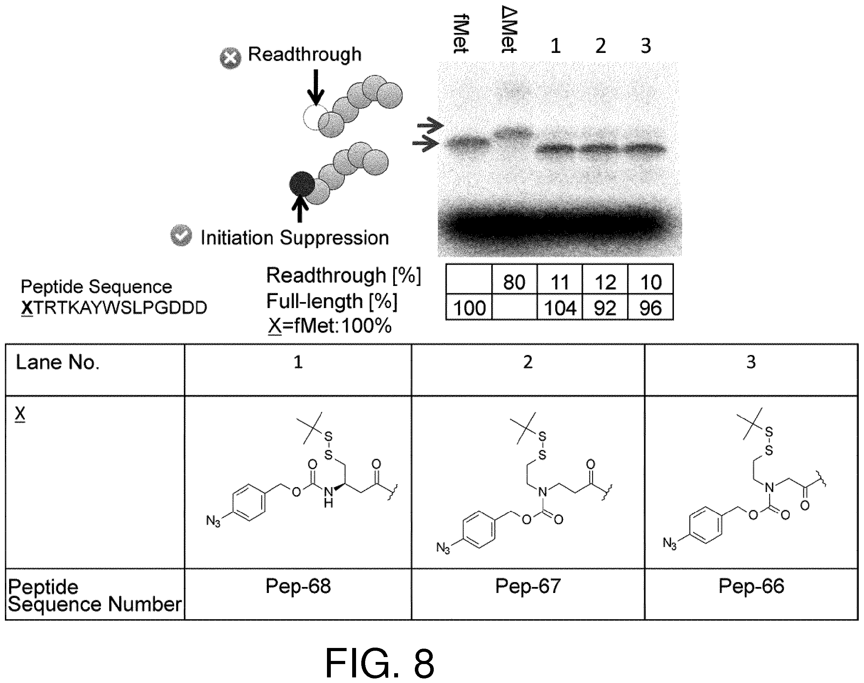

Fig. 8 shows an electrophoretic confirmation of peptides translationally incorporating at the N terminus an amino acid analog having a *p*-Acbz-protected amino group.

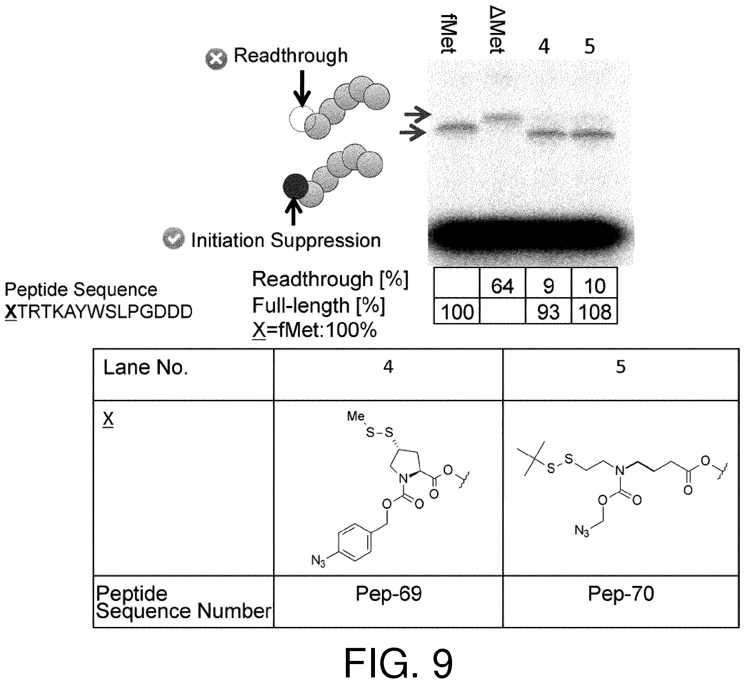

Fig. 9 shows an electrophoretic confirmation of peptides translationally incorporating at the N terminus an amino acid analog having a protecting group on each of the amino group and the thiol group.

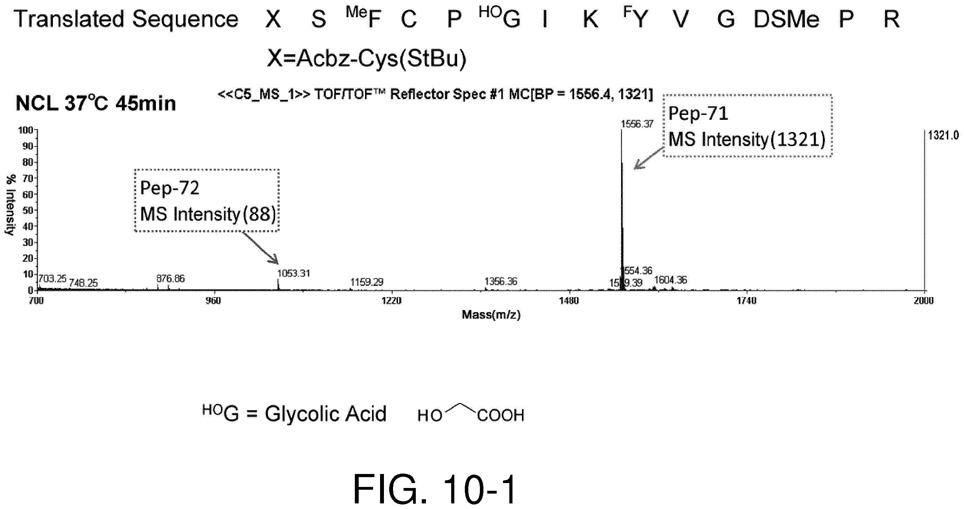

Fig. 10-1 shows the mass spectrum of cyclic peptide Pep-71 obtained by ribosomally synthesizing a peptide carrying Acbz-Cys(StBu) at its N terminus, Cys-Pro-^{HO}Gly, and active ester in the side chain of an amino acid at the C-terminal side, performing deprotection of the Acbz group and StBu, and then performing a cyclization reaction by native chemical ligation.

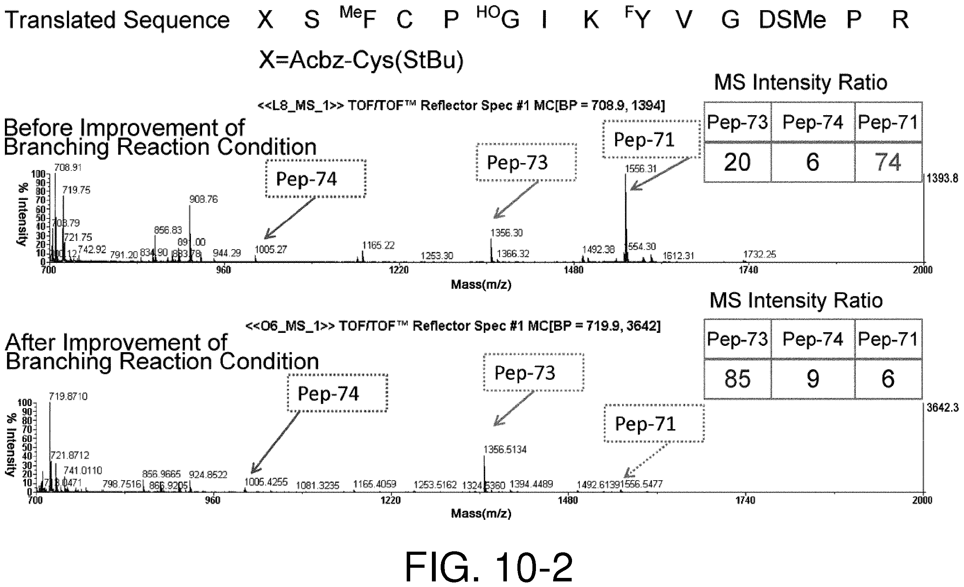

Fig. 10-2 shows the mass spectra of a branched peptide obtained by subjecting cyclic peptide Pep-71 synthesized from a translated peptide by native chemical ligation to branching reaction conditions. The upper part shows the mass spectrum taken before improving the branching reaction conditions and the lower part shows the mass spectrum taken after improving the branching reaction conditions.

### Mode for Carrying Out the Invention

### <Definition of substituent groups and such>

Herein, the term "alkyl" refers to a monovalent group derived from aliphatic hydrocarbon by removal of one arbitrary hydrogen atom and has a subset of a hydrocarbyl or hydrocarbon group structure containing neither heteroatoms nor unsaturated carbon-carbon bonds in the backbone and containing hydrogen and carbon atoms. Its carbon chain length n is in the range of one to 20. Examples of alkyl include "C1-C6 alkyl" and specifically include methyl, ethyl, propyl, butyl, pentyl, hexyl, isopropyl, *t*-butyl group, *sec*-butyl group, 1-methylpropyl group, 1,1-dimethylpropyl group, 2,2-dimethylpropyl, 1,2-dimethylpropyl, 1,1,2-trimethylpropyl, 1,2,2-trimethylpropyl, 1,1,2,2-tetramethylpropyl, 1-methylbutyl, 2-methylbutyl, 3-methylbutyl, 1,1-dimethylbutyl, 1,2-dimethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, isopentyl, and neopentyl.

Herein, the term "alkenyl" refers to a monovalent group having at least one double bond (two adjacent SP2 carbon atoms). The double bond can assume entgegen (*E*) or zusammen (*Z*) and *cis* or *trans* geometric forms depending on the arrangement of the double bond and substituents (if they exist). Examples of alkenyl include linear or branched chains, including straight chains containing internal olefins. Preferred examples thereof include C2-C10 alkenyl, and more preferably C2-C6 alkenyl.

Specific examples of alkenyl include vinyl, allyl, 1-propenyl, 2-propenyl, 1-butenyl, 2-butenyl (including *cis* and *trans* forms), 3-butenyl, pentenyl, and hexenyl.

Herein, the term "alkynyl" refers to a monovalent group having at least one triple bond (two adjacent SP carbon atoms). Examples thereof include linear or branched chain alkynyl including internal alkylene. Preferred examples thereof include C2-C10 alkynyl, and more preferably C2-C6 alkynyl.

Specific examples of alkynyl include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

Herein, the term "cycloalkyl" means a saturated or partially saturated cyclic monovalent aliphatic hydrocarbon group containing a single ring, bicyclo ring, or spiro ring. Preferred examples thereof include C3-C10 cycloalkyl. Specific examples of cycloalkyl include cyclopropyl, cyclobutyl, cyclopentyl, cyclohexyl, cycloheptyl, cyclooctyl, and bicyclo[2.2.1]heptyl.

Herein, the term "aryl" means a monovalent aromatic hydrocarbon ring. Preferred examples thereof include C6-C10 aryl. Specific examples of aryl include phenyl and naphthyl (*e.g*., 1-naphthyl and 2-naphthyl).

Herein, the term "heteroaryl" means an aromatic cyclic monovalent group containing preferably one to five heteroatoms among ring-constituting atoms and may be partially saturated. The ring may be a single ring or bicyclic condensed ring *(e.g.,* bicyclic heteroaryl formed by condensation with benzene or a monocyclic heteroaryl). The number of ring-constituting atoms is preferably five to ten (five- to ten-membered heteroaryl).

Specific examples of heteroaryl include furyl, thienyl, pyrrolyl, imidazolyl, pyrazolyl, thiazolyl, isothiazolyl, oxazolyl, isooxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

Herein, the term "arylalkyl (aralkyl)" means a group containing both an aryl and an alkyl, for example, a group derived from the above-mentioned alkyl by replacement of at least one hydrogen atom with an aryl. Preferred examples thereof include "C5-C10 aryl-C1-C6 alkyl", such as benzyl.

Herein, the term "alkylene" means a divalent group derived from the above-mentioned "alkyl" by removing another single arbitrary hydrogen atom. Preferred examples thereof include C1-C2 alkylene, C1-C3 alkylene, C1-C4 alkylene, C1-C5 alkylene, and C1-C6 alkylene. Specific examples of alkylene include methylene, 1,2-ethylene, 1,1-ethylene, 1,3-propylene, tetramethylene, pentamethylene, and hexamethylene.

Herein, the term "arylene" means a divalent group derived from the above-mentioned aryl by removing another single arbitrary hydrogen atom. An arylene may be a single ring or a condensed ring. The number of ring-constituting atoms is not particularly limited, but is preferably six to ten (C6-10 arylene). Specific examples of arylene include phenylene and naphthylene.

Herein, the term "heteroarylene" means a divalent group derived from the above-mentioned heteroaryl by removing another single arbitrary hydrogen atom. A heteroarylene may be a single ring or a condensed ring. The number of ring-constituting atoms is not particularly limited but is preferably five to ten (five- to ten-membered heteroarylene). Specific examples of heteroarylene include pyroldiyl, imidazoldiyl, pyrazoldiyl, pyridindiyl, pyridazindiyl, pyrimidindiyl, pyrazindiyl, triazoldiyl, triazindiyl, isooxazoldiyl, oxazoldiyl, oxadiazoldiyl, isothiazoldiyl, thiazoldiyl, thiadiazoldiyl, furandiyl, and thiophendiyl.

Herein, the term "alkylenearylene" means a divalent group formed by linking the above-mentioned alkylene and the above-mentioned arylene at an arbitrary position, in which the alkylene is linked to the basic skeletal structure. Specific examples of alkylenearylene include -C1-C6 alkylene-C6-C10 arylene.

Herein, the term "arylenealkylene" means a divalent group formed by linking the above-mentioned arylene and the above-mentioned alkylene at an arbitrary position, in which the arylene is linked to the basic skeletal structure. Specific examples of arylenealkylene include -C1-C6 arylene-C6-C10 alkylene.

Herein, the term "alkyleneheteroarylene" means a divalent group formed by linking the above-mentioned alkylene and the above-mentioned heteroarylene at an arbitrary position, in which the alkylene is linked to the basic skeletal structure. Specific examples of alkyleneheteroarylene include -C1-C6 alkylene-5-membered to 10-membered heteroarylene.

Herein, the term "heteroarylenealkylene" means a divalent group formed by linking the above-mentioned heteroarylene and the above-mentioned alkylene at an arbitrary position, in which the heteroarylene is linked to the basic skeletal structure. Specific examples of heteroarylenealkylene include -5-membered to 10-membered heteroarylene-C1-C6 alkylene.

Herein, the term "active ester (activated ester)" refers to a group containing a carbonyl that forms an amide bond by reaction with an amino group, in which the carbonyl group is bonded to, for example, OBt, OAt, OSu, OPfp, or SR1, and which is a group capable of promoting the reaction with an amine.

The term "reaction-promoting group" refers to a group that is introduced near a functional group to be bonded for the purpose of selectively causing reaction at a desired position and activates the functional group for binding reaction. For example, to allow carbonyl to react with amine, a reaction-promoting group can be introduced, for example, either on the carbonyl side or on the amine side, or both. These reaction-promoting groups may be eliminated concurrently with or after binding reaction.

### <Peptides and peptide-nucleic acid complexes>

### - Peptides having a cyclic portion and complexes between such peptides and nucleic acids

In the present invention, cyclic portions of cyclic portion-containing peptides and complexes of the peptides and nucleic acids are formed by subjecting ribosomally synthesized peptides or peptide-nucleic acid complexes to amidation reaction. In the present invention, the peptide portion of peptide-nucleic acid complexes may be described as the "peptide moiety". Furthermore, in the present invention, the term "nucleic acid" includes DNA and mRNA.

Peptides and peptide-nucleic acid complexes of the present invention also include peptides and peptide-nucleic acid complexes obtained by further chemical modification of the peptides and the peptide moieties of the complexes of the peptides and nucleic acids, in which the cyclic portions have been formed by amidation reaction following ribosomal synthesis.

Peptides and peptide-nucleic acid complexes of the present invention may have a linear portion as exemplified in Scheme A. The number of amide bonds (the number or length of amino acids) in the peptide and the peptide moiety is not particularly limited, but when a linear portion is present, the cyclic portion and the linear portion in combination have preferably 30 residues or less. The number of linear portions is not particularly limited, but one or two or more linear portions may be present. The total number of amino acids in the cyclic portion and the linear portion is preferably 13 residues or less for the ultimately obtained cyclic peptide to acquire high membrane permeability. The total number of amino acids is preferably nine or more for acquiring high metabolic stability. Considering the compatibility of membrane permeability and metabolic stability (druglikeness), the cyclic portion is preferably composed of five to twelve amino acids, more preferably five to eleven amino acids, even more preferably seven to eleven amino acids, and particularly preferably nine to eleven amino acids. The number of amino acids in the linear portion (number of units) is preferably 0 to eight, and more preferably 0 to three.

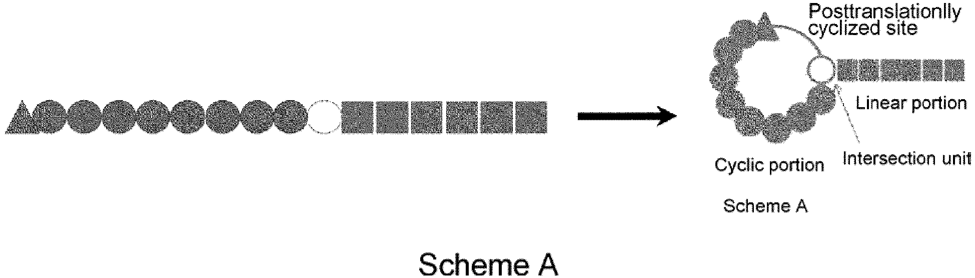

Scheme A describes the peptide moiety (before and after cyclization) in a peptide-nucleic acid complex of the present invention. The open circle (○) unit (intersection unit), filled circle (●) unit (cyclic portion main chain unit), square (■) unit (linear portion main chain unit), and the triangle (▲) unit (cyclized N-terminal unit) each denote an amino acid constituting the peptide moiety. The respective units may be the same or different amino acids.

In this context, a unit means an amino acid before cyclization following ribosomal synthesis, an amino acid after cyclization, or an amino acid at the completion of chemical modification after cyclization. The amino acid at the completion of chemical modification after cyclization includes a chemically modified or backbone-converted amino acid produced by posttranslational chemical modification of an amino acid translated by one tRNA.

In Scheme A, the cyclic portion is moiety composed of one triangle unit, eight filled circle units, and one open circle unit, and the linear portion is a moiety composed of six square units. The curved portion in Scheme A is a site cyclized after translation (posttranslationally cyclized site) and this portion is linked by an amide bond.

Peptides or peptide moieties of the present invention require a reactive functional group on the triangle unit or the intersection unit described in Scheme A; therefore, a drug-like amino acid is not necessarily selected for the triangle unit or the intersection unit. Furthermore, in the present invention, the cyclic portion preferably includes two or more amino acid analog residues.

Furthermore, a peptide or a peptide-nucleic acid complex of the present invention can be obtained as a cyclic peptide having two linear portions (linear portion 1 and linear portion 2) by carrying out a cyclization between the triangle unit and the intersection unit according to Scheme A, and then, for example, performing a further intramolecular cyclization reaction (see WO 2013/100132).

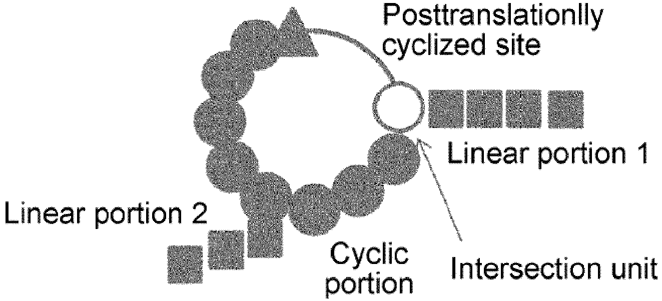

Herein, the term "translational synthesis (ribosomal synthesis)" means that a nucleic acid *(e.g.,* DNA or RNA) encoding a peptide or peptide moiety is translated to synthesize the peptide or peptide moiety. Translation is a process of obtaining a linear peptide through repetitive amide bond-forming reactions using mRNA as a template by the action of ribosomes.

Herein, the term "posttranslational modification" refers to a chemical reaction that is caused not by the action of ribosomes, but either automatically or by addition of reagents, after translation. Examples thereof can include cyclization reaction, desulfurization reaction, and deprotection reaction.

In the present invention, as the triangle unit in the peptide or peptide moiety before cyclization (pre-cyclized N-terminal unit), an amino acid residue having a thiol group near its amino group, with both the thiol and amino groups being protected by protecting groups, may be used. Specific examples of such amino acid residues include amino acid residues represented by general formula (I) 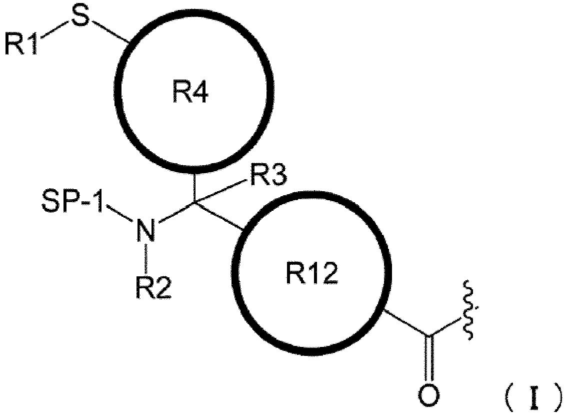 or general formula (II)

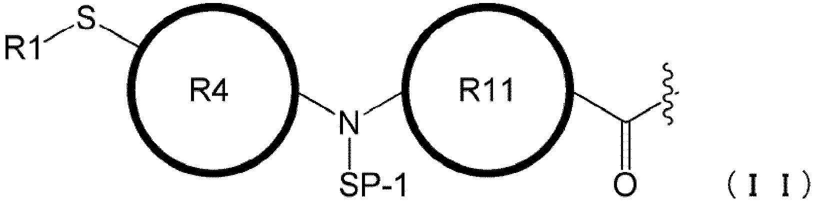

In the present invention, the intersection unit in the peptide or peptide moiety before cyclization may be an amino acid carrying in its side chain a functional group (second reaction point) that can form a ring with a functional group (first reaction point) on the amino acid of the triangle unit through amide bond formation. In the intersection unit, the amino group and carboxyl group in the main chain are used for covalent bond formation with other amino acid residues in ribosomal synthesis, and a third functional group is also necessary for posttranslational cyclization; therefore, the unit must have a total of three or more functional groups. Among these functional groups, a functional group in the side-chain moiety of the intersection unit is used for the posttranslational cyclization reaction.

The intersection unit may be incorporated into any position in the peptide moiety before cyclization as long as cyclization occurs at the position. The unit is preferably incorporated at a position that allows the cyclic portion to be composed of 5 to 12 amino acids, or more preferably composed of 5 to 11 amino acids, after cyclization or after posttranslational modification following cyclization. That is, in the present invention, the intersection unit is preferably incorporated at a position that is at least four amino acid residues (for example, four, five, six, seven, eight, nine, or ten amino acid residues) apart from the triangle unit to the C-terminal side.

Specific examples of such an intersection unit include amino acid residues represented by the following general formula (III) or (III-OH):

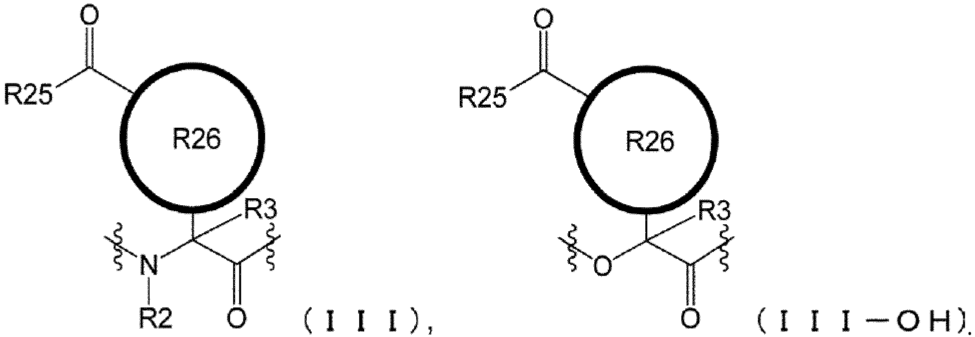

The filled circle unit and the square unit are selected from amino acids, and preferably selected from drug-like amino acids.

In the present invention, a "drug-like amino acid" refers to an amino acid that forms a drug-like peptide moiety and has a backbone identical to that of an "amino acid", *i.e.,* an amino acid having a backbone identical to that of an α-, β- or γ-amino acid. Specifically, the drug-like amino acid is selected from L-amino acids, D-amino acids, α,α-dialkylamino acids, and such. A preferable example is an amino acid in which one of the two hydrogen atoms in the main chain amino group (NH₂ group) is substituted with a methyl, and one or two of the hydrogen atoms of the methyl may be further replaced with an alkyl, a cycloalkyl, an alkenyl, an alkynyl, an aryl, a heteroaryl, an aralkyl, or such. Furthermore, examples include amino acids in which one or two of the hydrogen atoms in the main chain methylene (-CH₂-) are substituted with an alkyl, a cycloalkyl, an alkenyl, an alkynyl, an aryl, a heteroaryl, an aralkyl, or such, or are directly substituted with a "substituent contributing to druglikeness".

The above-mentioned substituents of the "drug-like amino acids" may be further substituted with a "substituent contributing to druglikeness". Here, examples of the "substituent contributing to druglikeness" include one or more substituents selected from among a hydroxyl (-OH), an oxy (-OR), an amide (-NR-CO-R' or -CO-NRR'), a sulfonyl (-SO₂-R), a sulfinyl (-SO-R), a halogen, an oxyamino (-NR-OR'), an aminooxy (-O-NRR'), an oxycarbonyl (-CO-OR), a thiocarbonyl (-CO-SR), a thiol (-SH), a thio (-SR), a primary amino (-NH₂), a secondary amino (-NHR), or a tertiary amino (-NRR'), a sulfonylamino (-NH-SO₂-R), a boryl (-BRR'), dioxyboryl (-B(OR)(OR')), an azide (-N₃), a carboxycarbonyl (-CO-CO₂H), a phosphorylcarbonyl (-CO-PO(R)(R')), a carbonyloxyamino (-NH-O-CO-R), and such.

The "drug-like amino acids" include all amino acids that can be chemically synthesized by the structural optimization of a side chain moiety in a peptide (*e.g*., when a hit compound is obtained using D-tyrosine, a D-amino acid chemically modified therefrom; or when a hit compound is obtained using β-alanine, a β-amino acid chemically modified therefrom) or a N-terminal substituted portion resulting from the chemical conversion of *N*-methyl amino acid.

In the present invention, the term "druglikeness" or "drug-like" means that the peptide moiety has membrane permeability and metabolic stability to the extent available as at least oral formulations or pharmaceuticals when targeting intracellular proteins, nucleic acids, intracellular regions of membrane proteins or transmembrane domains of membrane proteins.

Peptides or peptide moieties of the present invention may not have a linear portion but having a linear portion can strengthen the functions of the peptides or peptide moieties carrying a cyclic portion. For example, when the above-mentioned peptides or peptide moieties are used for inhibiting the binding between a certain receptor and its ligand, the peptides or peptide moieties having a linear portion can have higher binding activity towards the receptor or ligand than the peptides or peptide moieties not having a linear portion. Such potentiation of the binding activity can enhance the receptor-ligand binding inhibitory effect (see WO 2013/100132).

In the present invention, "amino acids" constituting the peptides or peptide moieties may be "natural amino acids" or "amino acid analogs". The "amino acids", "natural amino acids", and "amino acid analogs" are also referred to as "amino acid residues", "natural amino acid residues", and "amino acid analog residues", respectively.

"Natural amino acids" are α-aminocarboxylic acids (α-amino acids), and refer to the 20 types of amino acids contained in proteins. Specifically, they refer to Gly, Ala, Ser, Thr, Val, Leu, Ile, Phe, Tyr, Trp, His, Glu, Asp, Gln, Asn, Cys, Met, Lys, Arg and Pro.

"Amino acid analogs" include unnatural amino acids (for example, unnatural α-amino acids, β-amino acids, and γ-amino acids). An α-amino acid may be a D-amino acid, or an α,α-dialkylamino acid. In a similar manner to an α-amino acid, a β-amino acid and a γ-amino acid are also allowed to have any configuration. A side chain (with the main chain being methylene) of the amino acid analogs is not particularly limited, and may have, besides hydrogen atoms, for example, an alkyl, an alkenyl, an alkynyl, an aryl, a heteroaryl, an aralkyl, or a cycloalkyl. Each of these may have one or more substituents, and these substituents can be selected from any functional group containing, for example, a halogen atom, an N atom, an O atom, an S atom, a B atom, a Si atom, or a P atom. For example, herein, "C1-C6 alkyl optionally substituted with halogen" means "C1-C6 alkyl" substituted with one or more halogen atoms, and specific examples include trifluoromethyl, difluoromethyl, fluoromethyl, pentafluoroethyl, tetrafluoroethyl, trifluoroethyl, difluoroethyl, fluoroethyl, trichloromethyl, dichloromethyl, chloromethyl, pentachloroethyl, tetrachloroethyl, trichloroethyl, dichloroethyl, and chloroethyl. Furthermore, for example, "optionally substituted C5-C10 aryl C1-C6 alkyl" means that in which at least one hydrogen atom of the aryl and/or alkyl of "C5-C10 aryl C1-C6 alkyl" has been substituted with a substituent. Furthermore, "cases having two or more substituents" include cases having an S atom-containing functional group, which further has functional groups such as an amino or a halogen.

The main chain amino group of an amino acid analog may be unsubstituted (a NH₂ group), or it may be substituted (that is, an NHR group; in which R represents an alkyl, an alkenyl, an alkynyl, an aryl, a heteroaryl, an aralkyl, or a cycloalkyl, each of which optionally has a substituent; or a carbon chain bonded to the N atom and a carbon atom at the α position may form a ring as in proline. The substituent is similar to the substituent of the side chain, and examples include a halogen, an oxy, and a hydroxy.). Furthermore, for "an alkyl, an alkenyl, an alkynyl, an aryl, a heteroaryl, an aralkyl, or a cycloalkyl" in the definition of these substituents, the above-mentioned definitions for these functional groups are applied. For example, herein, "alkoxy" means a group in which a hydrogen atom in a hydroxy group is replaced with the above-mentioned alkyl group. Preferred examples thereof include "C1-C6 alkoxy".

Furthermore, "amino acid analogs" include hydroxycarboxylic acids in which an amino group of an "amino acid" has been replaced with a hydroxyl group. The hydroxycarboxylic acid may have various substituents, in a similar manner to other amino acid analogs. The hydroxycarboxylic acid may have a configuration corresponding to either an L- or D-form amino acid. While its side chain is not particularly limited, for example, it is selected from an alkyl, an alkenyl, an alkynyl, an aryl, a heteroaryl, an aralkyl, a cycloalkyl, and such, which are optionally substituted.

Examples of halogen-derived substituents include fluoro (-F), chloro (-Cl), bromo (-Br), and iodo (-I).

Examples of O atom-derived substituents include hydroxyl (-OH), oxy (-OR), carbonyl (-C=O-R), carboxyl (-CO₂H), oxycarbonyl (-C=O-OR), carbonyloxy (-O-C=O-R), thiocarbonyl (-C=O-SR), carbonylthio group (-S-C=O-R), aminocarbonyl (-C=O-NHR), carbonylamino (-NH-C=O-R), oxycarbonylamino (-NH-C=O-OR), sulfonylamino (-NH-SO₂-R), aminosulfonyl (-SO₂-NHR), sulfamoylamino (-NH-SO₂-NHR), thiocarboxyl (-C(=O)-SH), and carboxylcarbonyl (-C(=O)-CO₂H).

Examples of oxy (-OR) include alkoxy, cycloalkoxy, alkenyloxy, alkynyloxy, aryloxy, heteroaryloxy, and aralkyloxy.

Examples of carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.
(-C=O-OR)

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C=O-R) include, alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Additional examples include compounds produced by further substitution of an alkyl, a cycloalkyl, an alkenyl, an alkynyl, an aryl, a heteroaryl, or an aralkyl for the H atom bonded to the N atom in -C=O-NHR.

Examples of carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples include compounds produced by further substitution of an alkyl, a cycloalkyl, an alkenyl, an alkynyl, an aryl, a heteroaryl, or an aralkyl for the H atom bonded to the N atom in -NH-C=O-R.

Examples of oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples include compounds produced by further substitution of an alkyl, a cycloalkyl, an alkenyl, an alkynyl, an aryl, a heteroaryl, or an aralkyl for the H atom bonded to the N atom in -NH-C=O-OR.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples include compounds produced by further substitution of an alkyl, a cycloalkyl, an alkenyl, an alkynyl, an aryl, a heteroaryl, or an aralkyl for the H atom bonded to the N atom in -NH-SO₂-R.

Examples of aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples include compounds produced by further substitution of an alkyl, a cycloalkyl, an alkenyl, an alkynyl, an aryl, a heteroaryl, or an aralkyl for the H atom bonded to the N atom in -SO₂-NHR.

Examples of sulfamoylamino (-NH-SO₂-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Additionally, the two H atoms bonded to the N atoms in -NH-SO₂-NHR may be substituted with a substituent independently selected from the group consisting of an alkyl, a cycloalkyl, an alkenyl, an alkynyl, an aryl, a heteroaryl, and an aralkyl; or these two substituents may form a ring.

For S atom-derived substituents, examples include thiol (-SH), thio (-S-R), sulfinyl (-S=O-R), sulfonyl (-S(O)₂-R), and sulfo (-SO₃H).

Examples of thio (-S-R) are selected from among alkylthio, cycloalkylthio, alkenylthio, alkynylthio, arylthiol, heteroarylthio, aralkylthio, and such.

Examples of sulfinyl (-S=O-R) include alkylfulfinyl, cycloalkylsulfinyl, alkenylsulfinyl, alkynylsulfinyl, arylsulfinyl, heteroarylsulfinyl, and aralkylsulfinyl.

Examples of sulfonyl (-S(O)₂-R) include alkylsulfonyl, cycloalkylsulfonyl, alkenylsulfonyl, alkynylsulfonyl, arylsulfonyl, heteroarylsulfonyl, and aralkylsulfonyl.

For N atom-derived substituents, examples include azide (-N₃, also called "azido group"), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH-C(=NH)-NH₂), substituted guanidino (-NR-C(=NR"')-NR'R"), and aminocarbonylamino (-NR-CO-NR'R").

Examples of secondary amino (-NH-R) include alkylamino, cycloalkylamino, alkenylamino, alkynylamino, arylamino, heteroarylamino, and aralkylamino.

Examples of tertiary amino (-NR(R')) include amino groups, such as alkyl(aralkyl)amino, having any two substituents each independently selected from alkyl, cycloalkyl, alkenyl, alkynyl, aryl, heteroaryl, and aralkyl; and these two arbitrary substituents may form a ring.

Examples of substituted amidino (-C(=NR)-NR'R") include groups in which each of the three substituents R, R', and R" on the N atoms is independently selected from among alkyl, cycloalkyl, alkenyl, alkynyl, aryl, heteroaryl, and aralkyl; and such examples include alkyl(aralkyl)(aryl)amidino.

Examples of substituted guanidino (-NR-C(=NR"')-NR'R") include groups in which each of R, R', R", and R'" is independently selected from alkyl, cycloalkyl, alkenyl, alkynyl, aryl, heteroaryl, and aralkyl; or groups in which they form a ring.

Examples of aminocarbonylamino (-NR-CO-NR'R") include groups in which each of R, R', and R" is independently selected from a hydrogen atom, alkyl, cycloalkyl, alkenyl, alkynyl, aryl, heteroaryl, and aralkyl; or groups in which they form a ring.

Examples of B atom-derived substituents include boryl (-BR(R')) and dioxyboryl (-B(OR)(OR')). These two substituents, R and R', are each independently selected from alkyl, cycloalkyl, alkenyl, alkynyl, aryl, heteroaryl, and aralkyl; or they may form a ring.

At least one atom constituting an "amino acid" constituting the peptide or peptide moiety may be an atom (isotope) of the same atomic number (number of protons) and different mass number (total number of protons and neutrons). Examples of the isotope contained in the "amino acid" constituting the peptide moiety include a hydrogen atom, a carbon atom, a nitrogen atom, an oxygen atom, a phosphorus atom, a sulfur atom, a fluorine atom, and a chlorine atom, including ²H, ³H, ¹³C, ¹⁴C, ¹⁵N, ¹⁷O, ¹⁸O, ³¹P, ³²P, ³⁵S, ¹⁸F and ³⁶Cl.

Exemplary unnatural amino acids (amino acid analogs) that can be used other than at the N terminus in the present invention are shown below; however, the unnatural amino acids are not limited thereto. Many of these unnatural amino acids can be purchased with their side chains protected or unprotected and their amine moieties protected or unprotected. Those that cannot be purchased can be synthesized by known methods.

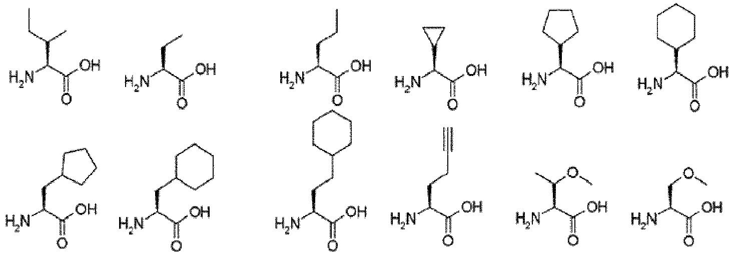

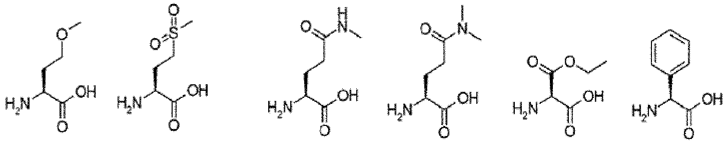

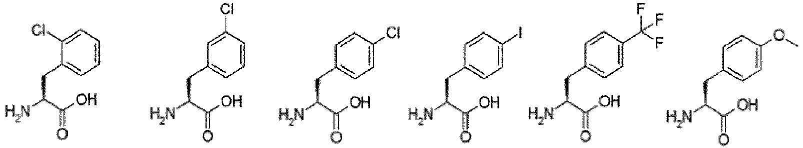

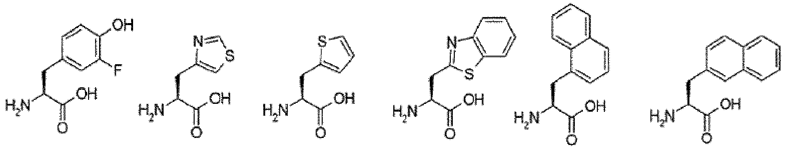

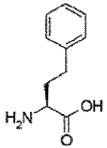

The following *N*-Me amino acids can be used as the unnatural amino acids: *N*-methylalanine; *N*-methylglycine; *N*-methylphenylalanine; *N*-methyltyrosine; N-methyl-3-chlorophenylalanine; *N*-methyl-4-chlorophenylalanine; N-methyl-4-methoxyphenylalanine; *N*-methyl-4-thiazolalanine; *N*-methylhistidine; N-methylserine; and N-methylaspartic acid.

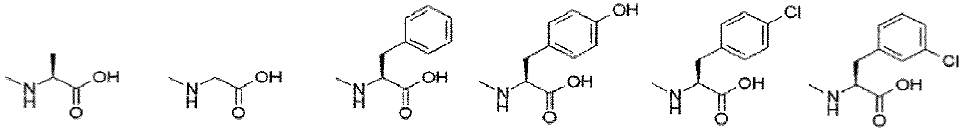

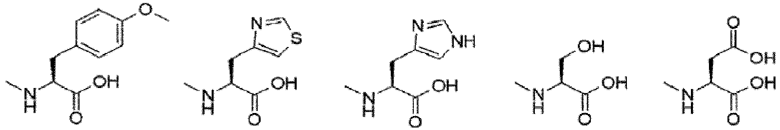

The following *N*-alkylamino acids may also be used as the unnatural amino acids.

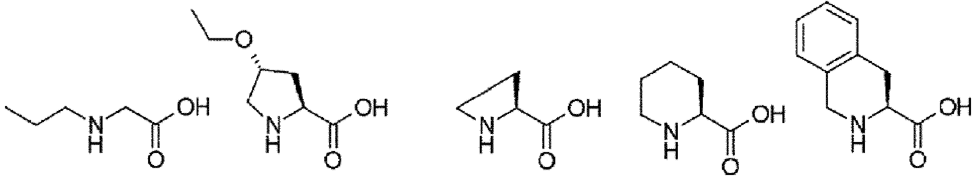

The following D-amino acid can also be used as the unnatural amino acid.

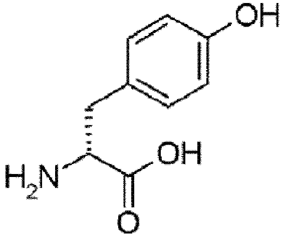

The following α,α-dialkylamino acid can also be used as the unnatural amino acid.

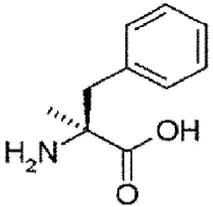

The following amino acid may also be used as the unnatural amino acid.

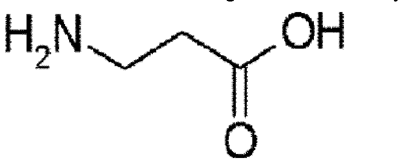

### <Method for preparing a peptide having a cyclic portion, and a complex between the peptide and a nucleic acid>

A peptide having a cyclic portion, a complex between the peptide and a nucleic acid (peptide-nucleic acid complex), or a library of peptide-nucleic acid complexes of the present invention can be prepared using, for example, a method comprising the steps described below.
1) Synthesizing a non-cyclic peptide or peptide moiety composed of amino acid residues by translating a nucleic acid encoding the peptide or peptide portion,
   wherein the non-cyclic peptide or peptide moiety comprises at its N terminus an amino acid residue represented by general formula (I) or general formula (II) shown below comprising a first reaction point 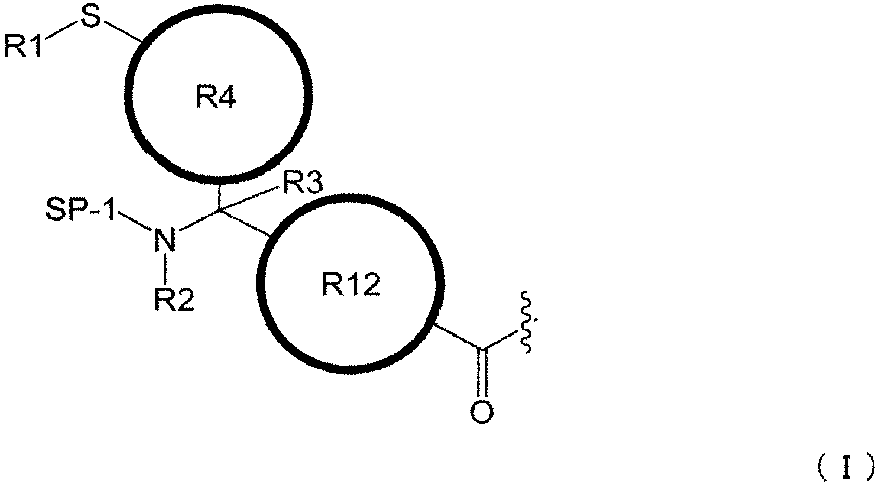 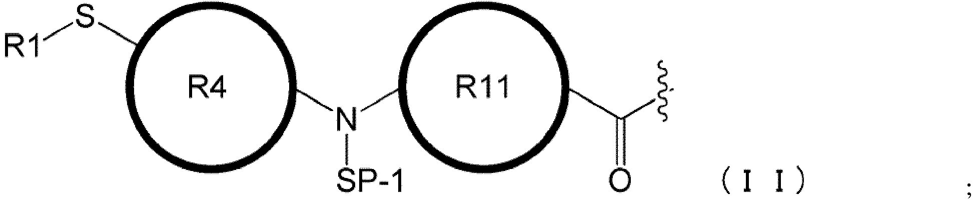 and also comprises at the C-terminal side an amino acid residue comprising a second reaction point in one of its side chains; and
2) forming an amide bond by reacting the first reaction point with the second reaction point.

In the present invention, the initiation suppression (iSP) method is used for the ribosomal synthesis of the noncyclic peptide or peptide moiety. In an ordinary translation, generally as the translation initiation amino acid, methionine is translated as the N-terminal amino acid. For initiation of translation, an exclusive "initiation tRNA" is present. The initiation tRNA binds with methionine (formylmethionine in prokaryotes) and is transported to the ribosome where translation is initiated, and the N-terminal amino acid becomes methionine (formylmethionine in prokaryotes). On the other hand, the method called the initiation suppression method ribosomally synthesizes a peptide carrying a desired amino acid at its N terminus by removing the initiation tRNA aminoacylated with methionine from the translation system (or preventing its synthesis in the translation system), and adding into the translation system an initiation tRNA aminoacylated with a desired amino acid prepared in advance. It has been known that during N-terminal introduction of an amino acid analog, amino acid tolerance is greater than during elongation, and an amino acid or amino acid analog having a structure largely different from natural amino acids have been used (Non-patent Document: J Am Chem Soc. 2009 Apr 15; 131(14): 5040-1. Translation initiation with initiator tRNA charged with exotic peptides. Goto Y, Suga H.).

A peptide which comprises at its N terminus an amino acid residue represented by general formula (I) mentioned above having a first reaction point and which comprises at its C-terminal side an amino acid residue having a second reaction point in one of its side chains can be obtained by the iSP method by translating a nucleic acid encoding this peptide.

Amino acids constituting the peptide or peptide moiety are preferably drug-like amino acids. To make the obtained peptide or peptide moiety drug-like, chemical modifications may be performed after ribosomal synthesis so that the amino acids become drug-like.

### <Introduction of a translatable amino acid to the N terminus>

In the present invention, translation can be performed so that the N-terminal amino acid becomes a desired amino acid using a translation initiation tRNA aminoacylated with an amino acid residue having a thiol group near its amino group with both the thiol and amino groups being protected (triangle unit; for example, a cysteine or cysteine analog protected by protecting groups). More specifically, the present invention provides methods for preparing a noncyclic peptide which comprises at its N terminus an amino acid residue represented by general formula (I) having a first reaction point and which comprises at the C-terminal side an amino acid residue having the second reaction point in one of its side chains.

The iSP method can be used in the present invention. More specifically, a pre-cyclized peptide moiety in which the N terminus is the triangle unit or a library of the peptide moiety can be constructed by adding an aminoacylated tRNA to a translation system not containing methionine, formyl donor, or methionyl transferase, making a translation initiation codon (*e.g.,* AUG) encode the triangle unit, and then translating it.

"Cysteine" in the present invention means L-cysteine having unprotected thiol and amino groups. "Cysteine analog" is an amino acid other than a natural amino acid and has a thiol in its side chain, where the thiol and amino groups are unprotected (it corresponds to an amino acid in which R1, R2, and SP-1 of formula (I) are hydrogen atoms). The conformation of the cysteine analog is arbitrary, and as long as it has a thiol group, the side chain structure is not particularly limited. Specific examples of such side chains include an alkyl, an alkenyl, an alkynyl, an aryl, a heteroaryl, an aralkyl, and a cycloalkyl, which are optionally substituted. The number of substituents is not limited to one and may be two or more.

Various combinations of a translation initiation tRNA having an anticodon other than CAU and a codon corresponding to the anticodon of the translation initiation tRNA can be used as the combination of the translation initiation tRNA and the initiation codon to diversify the N-terminus. That is, plural types of translation initiation tRNAs differing in anticodon are aminoacylated with the desired cysteine or cysteine analog to produce aminoacyl tRNAs. Using these aminoacyl tRNAs, mRNAs or mRNA libraries having, as initiation codons, codons corresponding to the aminoacyl tRNA anticodons can be translated to produce pre-cyclized peptide moieties having N-terminal residues that are not limited to a single type or libraries of the peptide moieties. Specifically, the pre-cyclized peptide moieties or libraries of peptide moieties can be prepared by a method described in, for example, Mayer C., et al., Anticodon sequence mutants of Escherichia coli initiator tRNA: effects of overproduction of aminoacyl-tRNA synthetases, methionyl-tRNA formyltransferase, and initiation factor 2 on activity in initiation. Biochemistry. 2003, 42, 4787-99 (translation initiation with an amino acid other than f-Met by variant *E. coli* initiation tRNA having anticodon other than CAU and expression of a protein involving the corresponding codon in the middle thereof).

The step of forming a cyclic portion in a peptide or peptide moiety of the present invention is described below. For obtaining reaction selectivity between a group in the triangle unit, which is to be reacted (a reaction point, *e.g.,* an amino group) and will form an amide bond with the reaction point in the intersection unit and basic functional groups in the amino acid residues constituting the peptide moiety, which are not to be reacted, a method of further activating the reaction point of the triangle unit is used. Specifically, in such a method, an amino acid having a reaction-promoting group (for example, a thiol group) near the reaction point is used at the N terminus. For example, since cysteine has a thiol at the β position of the amino group, it corresponds to such an amino acid. When using cysteine at the N terminus, as shown in Scheme C, the thiol and amino groups of cysteine are both protected in the translational incorporation process, but simultaneously to or before the cyclization reaction, free thiol and amino groups are generated by deprotection reaction. A peptide amide-cyclized at the desired position can be obtained by the reaction between the reaction point *(e.g.,* an amino group) in the triangle unit and the reaction point in the intersection unit *(e.g.,* an activated carboxyl group) mediated by the reaction-promoting group. The thiol group included in the triangle unit of the obtained cyclic peptide can be desulfurized by using suitable reagents such as TCEP (tris(2-carboxylethyl)phosphine) and VA-044 (2,2'-azobis-2-(2-imidazolin-2-yl)propane) dihydrochloride under mild reaction conditions which do not cause the reaction of RNA.

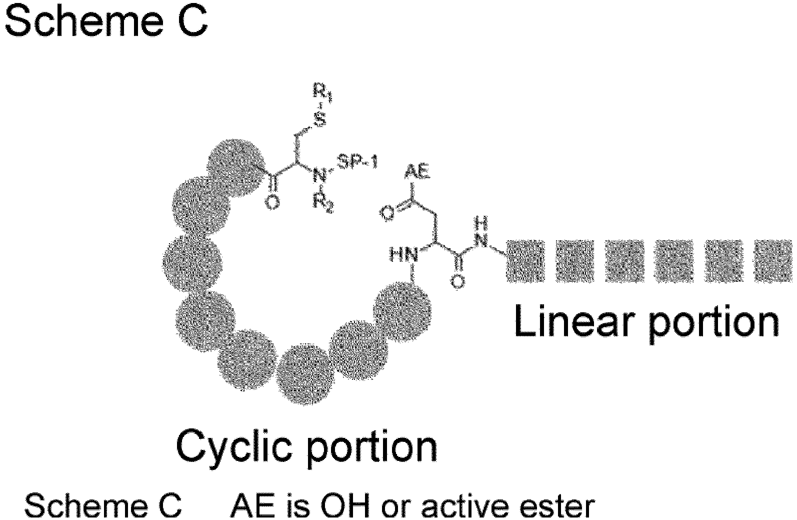

In preferred reaction conditions for the cyclization reaction, the pH is preferably 6.0 or higher to 9.2 or lower, and more preferably 7.0 or higher to 8.5 or lower. The reaction temperature is not particularly limited as long as usual chemical reactions can be carried out, and is preferably 4°C to 80°C, and more preferably 10°C to 50°C. For the purpose of suppressing air oxidation reaction (S-S forming reaction) which is a side reaction, a reducing agent such as tris(2-carboxylethyl)phosphine (=TCEP) may be added. When using TCEP, the amount used is not particularly limited, but 1 mM or more is preferred, and 10 mM or more to 100 mM or less is more preferred for enhancing its reactivity.

In preferred reaction conditions for the desulfurization reaction, the pH is preferably 3.0 or higher to 9.2 or lower, and more preferably 5.0 or higher to 8.5 or lower, considering making RNA exist with stability and suppressing hydrolysis. The reaction temperature is not particularly limited as long as usual chemical reactions can be carried out, and is preferably 4°C to 80°C, and more preferably 30°C to 60°C. The amount of TCEP used is not particularly limited, but 10 mM or more is preferred, 50 mM or more to 2 M or less is more preferred, and 100 mM or more to 500 mM or less is even more preferred for enhancing its reactivity. The amount of VA-044 used is not particularly limited, but 10 mM or more is preferred, 50 mM or more to 2 M or less is more preferred, and 100 mM or more to 500 mM or less is even more preferred for enhancing its reactivity. The reaction may be performed in a translation reaction solution alone such as the PURE system, or an organic solvent such as DMF or NMP may be added thereto. Alternatively, the reaction can be carried out after purifying the translation solution by column purification or the like and changing the solvent.

The N-terminal amino acid residue before cyclization, which can be used as the triangle unit, can be represented by the following general formula (I) or general formula (II).

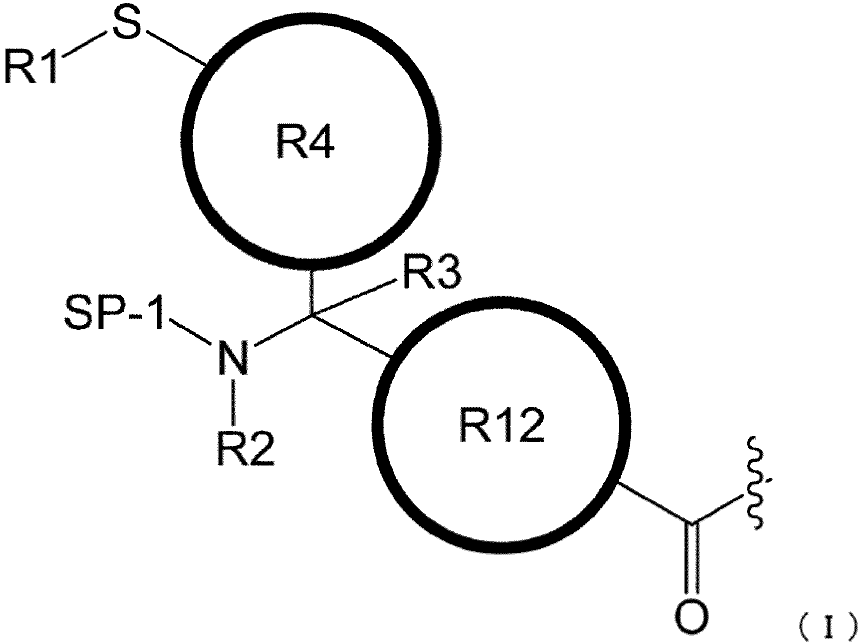

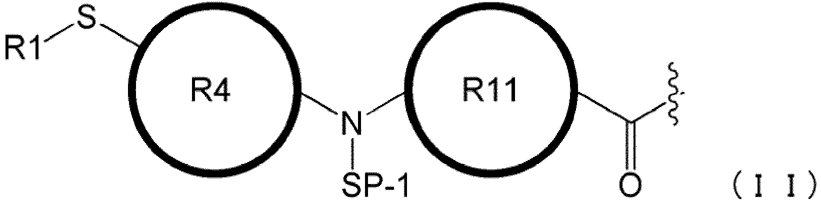

In the amino acid residues represented by general formula (I) or general formula (II) indicated above, R1 represents a thiol group-protecting group which can be ribosomally synthesized, and it is not particularly limited as long as it can be deprotected in a ribosomal synthesis solution to become a hydrogen atom before cyclization. Specific examples of such protecting groups include an S-R23 group, a sulfonate (-SO₃⁻), and a thiosulfonate (-S₂O₃⁻). When R1 is a sulfonate or a thiosulfonate, the counter cation is not particularly limited, and examples include Na⁺ and K⁺. R23 is an alkyl, an alkenyl, an alkynyl, an aryl, a heteroaryl, or an aralkyl, and these groups are optionally substituted. Specific examples of R23 include a methyl, an ethyl, an isopropyl, a *tert*-butyl, a phenyl, a *p*-trifluoromethylphenyl, a *p*-fluorophenyl, a benzyl, and a phenethyl. A protecting group such as an S-R23 group undergoes slow deprotection in a ribosomal synthesis solution; therefore, deprotection can be performed without the need for actively setting separate deprotection conditions. Employing such protecting groups enables performing deprotection and amide-cyclization in one step. For deprotection, a deprotecting agent can be added under the various reaction conditions described herein as necessary.

R2 and R3 are defined similarly to the side chain of the drug-like amino acid. Preferably, R2 and R3 each independently are, for example, a hydrogen atom, or an alkyl, an alkenyl, an alkyny, an aryl, a heteroaryl, an aralkyl, or a cycloalkyl, each of which optionally has a substituent; or R2 and R3 may form a ring together with the atoms to which they are bonded, or R2 or R3 may form a ring together with R4 and the atoms to which they are bonded. Any arrangement is acceptable for the configuration contained in those structures.

Examples of the case where "R2 and R3 form a ring together with the atoms to which they are bonded" include the following structure where a 4- to 7-membered ring is formed using R2, N bonded to R2, R3, and C bonded to R3 as part of the constituting atoms.

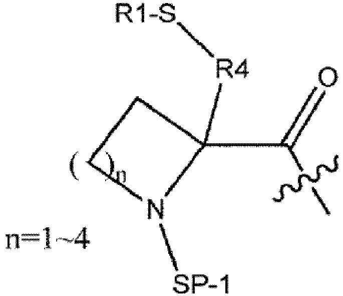

Examples of the case where "R2 forms a ring together with R4 and the atoms to which they are bonded" include the following structures where a 5- to 7-membered ring is formed using R2, N bonded to R2, R4, and C bonded to R4 as part of the constituting atoms.

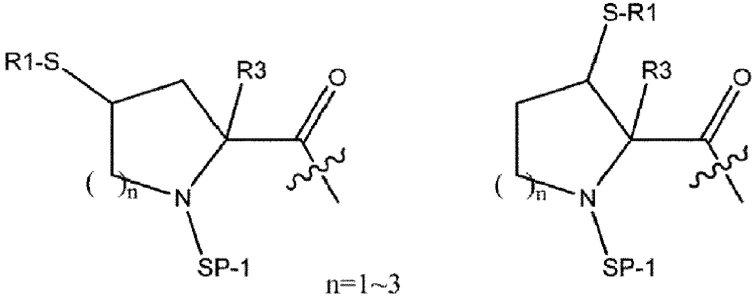

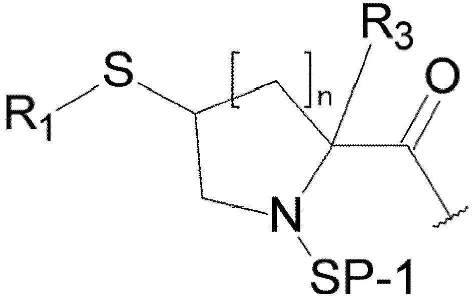

Examples of the case where "R3 forms a ring together with R4 and the atoms to which they are bonded" include the following structure where a 3- to 7-membered ring is formed using R3, R4, and C to which R3 and R4 are bonded as part of the constituting atoms.

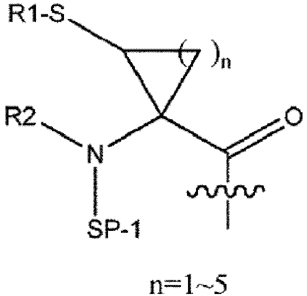

More preferably, R2 and R3 are a hydrogen atom, a C1-C4 alkyl, a C1-C4 alkyl optionally substituted with a C1-C4 alkoxy and/or a halogen, or a C5-C6-membered ring formed from two sites selected from R2, R3, and R4. Any arrangement is acceptable for the configuration contained in those structures.

R4 is a unit that links the S (sulfur) atom and the amino acid moiety, and for example, it is selected from the group consisting of an alkylene, an arylene, a heteroarylene, an alkylenearylene, an alkyleneheteroarylene, an arylenealkylene, and a heteroarylenealkylene, and these groups are optionally substituted.

N-3 to N-8 which are representative structures of R4 are shown below.

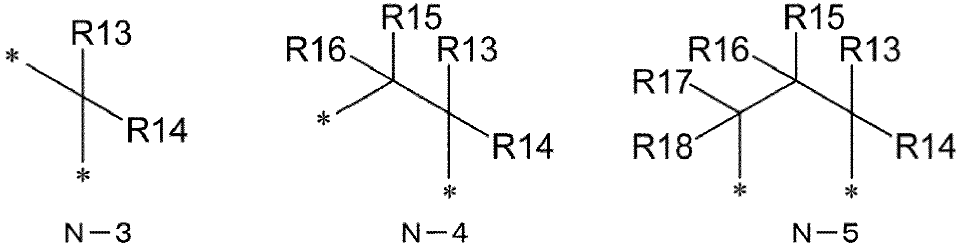

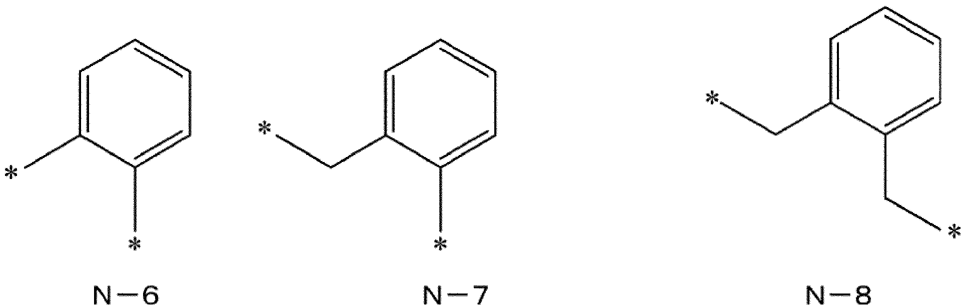

The S atom and the amino acid portion can be linked *via* one to five carbon atoms which are optionally substituted, such as optionally substituted methylene (N-3), optionally substituted ethylene (N-4), and optionally substituted propylene (N-5). Examples of methylene, ethylene, and propylene which are optionally substituted include groups in which R13 is methyl and R14 is a hydrogen atom, and dialkylated groups in which R13 and R14 are both methyl groups.

R13, R14, R15, R16, R17, and R18 are also defined similarly to R2 or R3, and for example, they are preferably selected from a hydrogen atom, an alkyl optionally substituted with a halogen atom or such, and an alkoxy optionally substituted with a halogen atom or such. A ring structure may be formed between these groups. More preferably, R13 to R18 are a hydrogen atom, a linear or branched C1-C4 alkyl, a C1-C5 alkoxy optionally substituted with one or more fluorine atoms, C1-C4 alkyl optionally substituted with one or more fluorine atoms or with C1-C5 alkoxy optionally substituted with one or more fluorine atoms, or form a C3-C7-membered ring composed of two sites selected from R13 to R18 and atoms bonded to them. Particularly preferably, R13 to R18 are a hydrogen atom or a methyl.

The S atom and the amino acid moiety can be linked directly *via* carbon atoms of an aromatic ring (N-6). The S atom and the amino acid moiety can also be linked *via* aralkyl structures (N-7 and N-8). Either one of the two bonding sites of N-7 may be on the amino acid moiety side or the S atom side.

In N-6 to N-8, benzene is used as the aromatic ring; however, an aromatic ring other than benzene (more specifically, various aromatic rings including heteroaromatic rings) may be used, and the aromatic ring may be substituted with substituents such as halogen, alkoxy, or trifluoromethyl.

R11 and R12 are also selected from partial structures similar to R4. For example, they can each be selected from among N-3, N-4, N-5, N-6, N-7 and N-8 type structures, and they have substituents R13' to R18', respectively. Furthermore, R11 and R12 may be single bonds.

Preferred structural formulas of formula (I) are indicated by N-9, N-10, and N-11. In N-9, R12 of formula (I) is a single bond. In N-10, the R12 portion of formula (I) is a single carbon atom (corresponding to the N-3 type). In N-11, R12 of formula (I) is two carbon atoms (corresponding to the N-4 type).

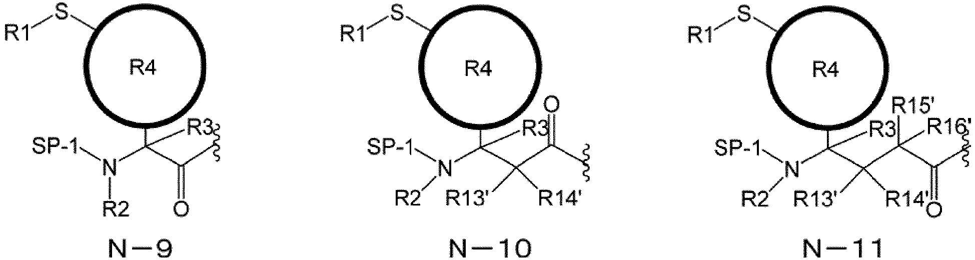

R1 to R4 in the formulas have the same meaning as the above-mentioned R1 to R4, respectively; R13' to R16' are defined similarly to the above-mentioned R13 to R16, respectively; and SP-1 in the formulas has the same meaning as SP-1 described later.

Preferred structural formulas of formula (II) are indicated by N-18, N-19, and N-20. In N-18, the R11 portion of formula (II) is a single carbon atom (corresponding to the N-3 type). In N-19, the R11 of formula (II) is two carbon atoms (corresponding to the N-4 type). In N-20, R11 of formula (II) is three carbon atoms (corresponding to the N-5 type).

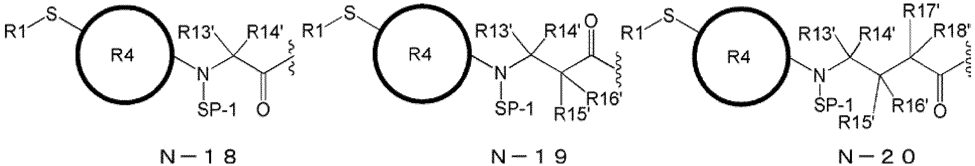

R1 and R4 in the formulas have the same meaning as the above-mentioned R1 and R4, respectively; R13' to R18' are defined similarly to the above-mentioned R13 to R18, respectively; and SP-1 in the formulas has the same meaning as SP-1 described later.

Furthermore, preferred structural formulas of formula (I) are indicated by N-12, N-13, N-14, N-15, N-16, and N-17. In N-12, R4 of N-9 is a single carbon atom (N-3). In N-13, R4 of N-10 is a single carbon atom (N-3). In N-14, R4 of N-11 is a single carbon atom (N-3). In N-15, R4 of N-9 is two carbon atoms (N-4). In N-16, R4 of N-10 is two carbon atoms (N-4). In N-17, R4 of N-11 is two carbon atoms (N-4).

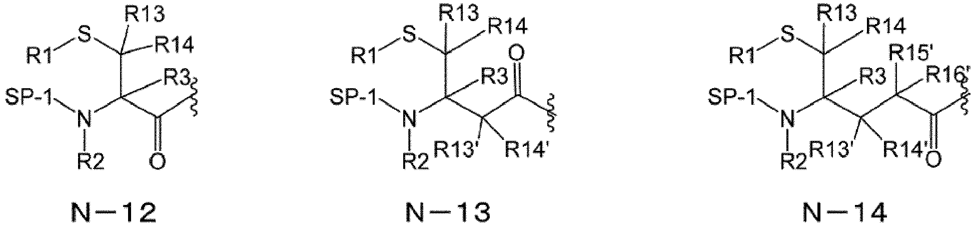

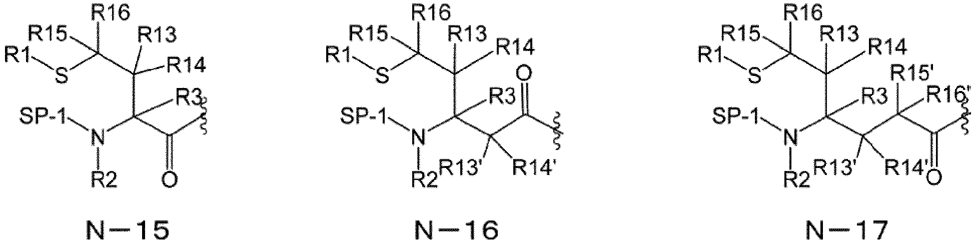

R1 to R3 and R13 to R16 in the formulas have the same meaning as the above-mentioned R1 to R3 and R13 to R16, respectively; R13' to R16' in the formulas are defined similarly to the above-mentioned R13 to R16, respectively; and SP-1 in the formulas has the same meaning as SP-1 described later. Alternatively, R2 forms a ring together with R13, R14, R15, or R16 and atoms to which they are bonded; however, the above-mentioned definition does not apply to R2 when R2 forms an azido group (-N₃) together with the N atom to which it is bonded and SP-1.

Furthermore, more preferred structural formulas of formula (II) are indicated by N-21 to N-26. In N-21, R4 of N-18 is two carbon atoms (N-4). In N-22, R4 of N-19 is two carbon atoms (N-4). In N-23, R4 of N-20 is two carbon atoms (N-4). In N-24, R4 of N-18 is three carbon atoms (N-5). In N-25, R4 of N-19 is three carbon atoms (N-5). In N-26, R4 of N-20 is three carbon atoms (N-5).

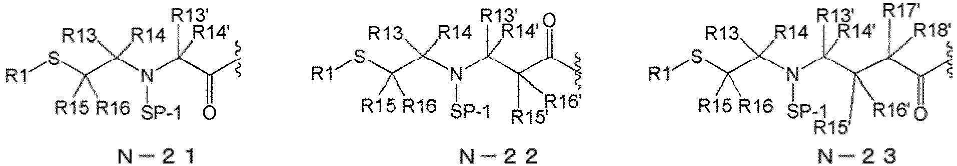

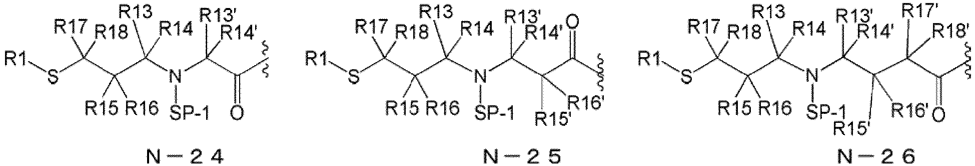

R1 and R13 to R18 in the formulas have the same meaning as the above-mentioned R1 and R13 to R18, respectively; R13' to R18' in the formulas are defined similarly to the above-mentioned R13 to R18, respectively; and SP-1 in the formulas has the same meaning as SP-1 described later.

In the present invention which uses the iSP method for ribosomal synthesis of the peptide moiety before cyclization, the amino protecting group "SP-1" in the triangle unit (for example, the amino acid residue represented by general formula (I)) before cyclization preferably satisfies all three conditions listed below:
(1) improves the stability of aminoacyl tRNA under translation reaction conditions;
(2) does not decrease the translation efficiency; and
(3) does not react with mRNA during the deprotection reaction and causes selective deprotection of only the desired functional group.

Examples of protecting groups satisfying the three conditions include protecting groups that can be deprotected under reductive conditions. Specific examples include protecting groups that can be deprotected in the presence of tris(2-carboxyethyl)phosphine (TCEP), dithiothreitol (DTT), and VA-044 (2,2'-azobis-2-(2-imidazolin-2-yl)propane dihydrochloride).

In a certain embodiment, SP-1 can be represented by the following formula.

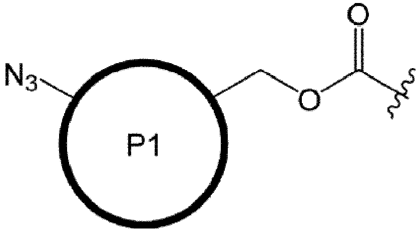

In this case, P1 is selected from a single bond, an arylene, a heteroarylene, and such, which may be further substituted with a halogen or an alkoxy. Furthermore, a methylene group positioned at the β position of the carbonyl group in the formula is also optionally substituted. Preferred examples of SP-1 in this embodiment include 4-azidobenzyloxycarbonyl (*p*-Acbz), 2-azidobenzyloxycarbonyl (*o*-Acbz), and azidomethoxycarbonyl (Azoc).

In another embodiment, SP-1 can be represented by the following formula.

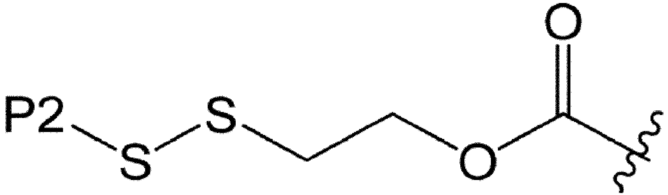

In this case, P2 is selected from an alkyl, an aryl, a heteroaryl, and such, which may be further substituted. Furthermore, the methylene group at the β position and/or γ position of the carbonyl group in the formula is also optionally substituted. Preferred examples of SP-1 in this embodiment include phenyldisulfanylethyloxycarbonyl (Phdec), 2-pyridyldisulfanylethyloxycarbonyl (Pydec), and 2-(*t*-butyldisulfanyl)ethyloxycarbonyl group (Tbeoc).

Furthermore, in another embodiment, when the N-terminal amino acid residue is represented by general formula (I), SP-1 together with the nitrogen atom to which it is bonded and R2 can form an azide (-N₃). The azide can be used as an amine equivalent.

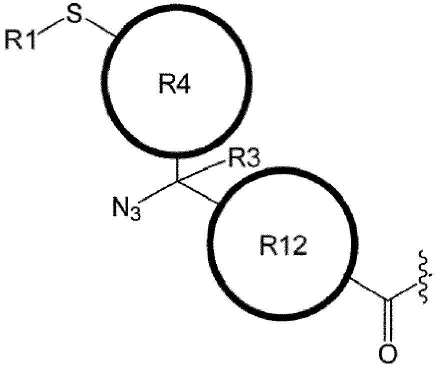

The detailed structures of *p*-Acbz, *o*-Acbz, Azoc, Phdec, Pydec, and Tbeoc are shown below. Among them, preferred are *p*-Acbz, *o*-Acbz, and Azoc.

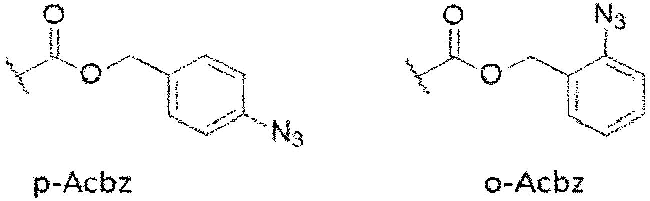

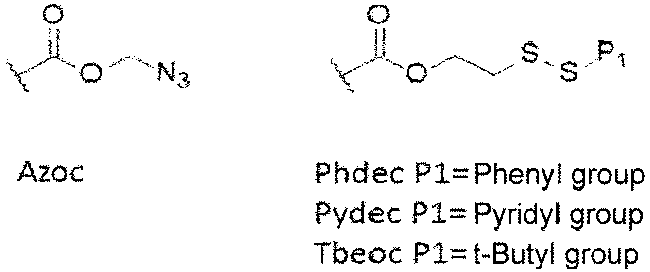

More favorable structural formulas and substituents of formula (I) are shown below: 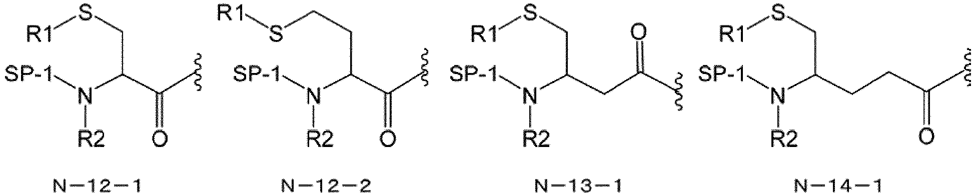 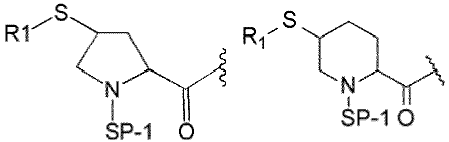 wherein,
R1 is -S-R23;
R23 is a C1-C20 alkyl, an optionally substituted phenyl, or an optionally substituted benzyl;
R2 is an C1-C6 alkyl optionally substituted with a substituent which contributes to druglikeness; and
SP-1 is *p*-Acbz, *o*-Acbz, or Azoc.

More favorable structural formulas and substituents of formula (II) are shown below: 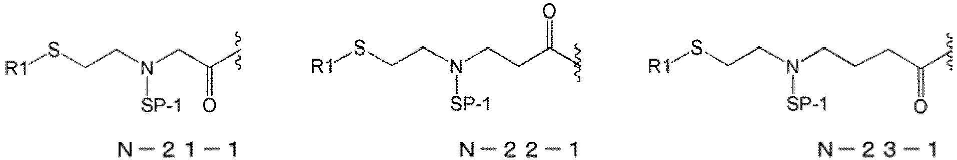 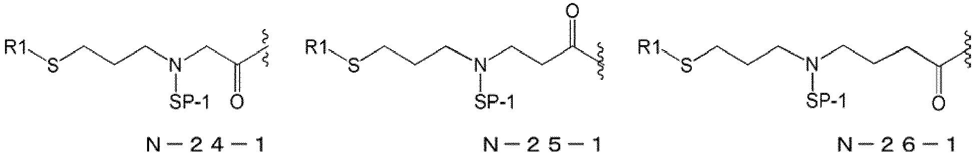 wherein,
R1 is -S-R23;
R23 is a C1-C20 alkyl, an optionally substituted phenyl, or an optionally substituted benzyl; and
SP-1 is *p*-Acbz, *o*-Acbz, or Azoc.

Even more favorable structural formulas and substituents of formula (I) are shown below: 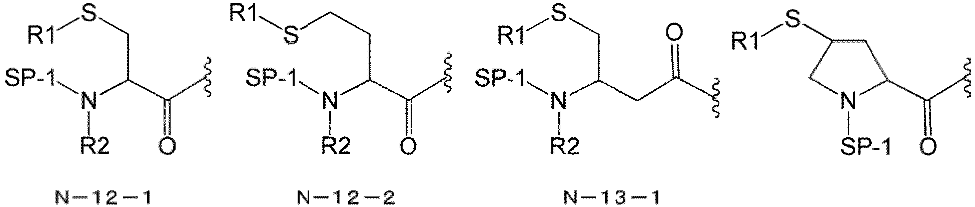 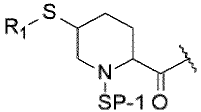 wherein,
R1 is -S-R23;
R23 is a methyl, an ethyl, an *n*-propyl, an *i*-propyl, an *n*-butyl, a *t*-butyl, or a *sec*-butyl;
R2 is a methyl, an ethyl, an *n*-propyl, or an *n*-butyl; and
SP-1 is *p*-Acbz or *o*-Acbz.

Even more favorable structural formulas and substituents of formula (II) are shown below: 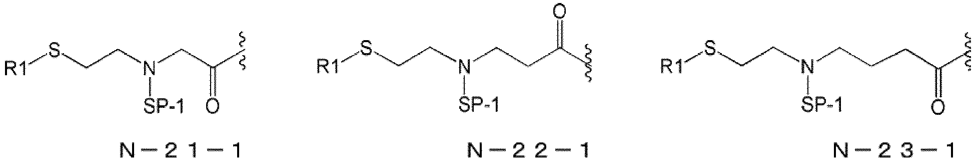 wherein,
R1 is -S-R23;
R23 is a methyl, an ethyl, an *n*-propyl, an *i*-propyl, an *n*-butyl, a *t*-butyl, or a *sec*-butyl; and
SP-1 is *p*-Acbz, *o*-Acbz, or Azoc.

Amino acid residues having a second reaction point in one of its side chains can be represented by general formula (III): 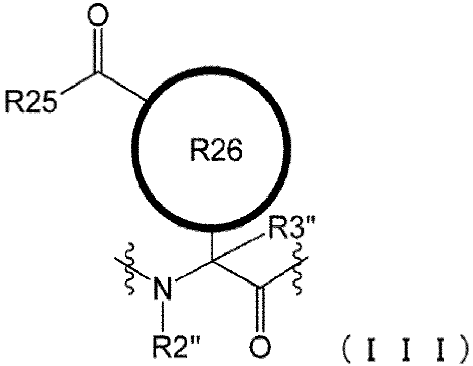 wherein, except for the active ester moiety (-COR25) which is the second reaction point, the substituents (*i.e.*, R2", R3", and R26) can be selected from groups that form drug-like amino acids. In the present invention, active ester means an ester or thioester that can be reacted with the amino group portion of the triangle unit *via* a thiol which is a reaction-promoting group, and it is not particularly limited as long as it has such properties.

Specifically, R25 is a hydroxyl group or forms an active ester together with the CO to which it is bonded. Substituents typically used in the art can be used as the R25, and specific examples include halogen, *N*-hydroxysuccinimide (-OSu), 1-hydroxy-7-azabenzotriazole (-OAt), 1-hydroxybenzotriazole (-OBt), and pentafluorophenol (-OPfp). Furthermore, when R25 is a hydroxyl group, amidation can be carried out using a condensing agent such as 4-(4,6-dimethoxy-1,3,5-triazin-2-yl)-4-methylmorpholinium chloride (DMT-MM) and 1-ethyl-3-(3-dimethylaminopropyl)carbodiimide (WSC). R25 together with the CO to which it is bonded may form a thioester (-C(=O)SR; favorable examples of R include a methyl, an ethyl, an isopropyl, a *t*-butyl, a benzyl, a CH₂-benzyl, and a phenyl), such as a methylthioester, an arylthioester, or an aralkylthioester. These active esters may have further substituents typically used in the art (for example, electron-withdrawing groups such as a halogen, nitro, trifluoromethyl, and nitrile, often used for the purpose of enhancing reactivity; electron-donating groups such as an alkoxy (*e.g.,* methoxy) and alkyl (*e.g.,* methyl) often used for the purpose of reducing reactivity and thereby enhancing reaction selectivity; bulky substituents typified by a *t*-butyl and isopropyl; and a sulfo group and a di-substituted amino group such as dimethylamino in consideration of hydrophilicity or highly lipid-soluble groups such as a long-chain alkyl in consideration of lipophilicity) as long as they show similar reactivities.

For introduction of an amino acid residue having such an active ester in one of its side chains to the peptide moiety, usable methods include, for example, a method in which, after translation of a carboxylic acid-containing amino acid residue in a PURE system or such, an active ester is generated by an activated ester-generating reaction in the reaction system, and a method of performing translation of an amino acid residue having an active ester in advance in a PURE system. Generally, an amino acid residue having among active esters, thioester or the like with a relatively low reactivity, is suitable for translation following preliminary isolation as active esters. On the other hand, an amino acid residue having a more highly reactive 1-hydroxy-7-azabenzotriazole (OAt) ester or the like is preferably generated posttranslationally as activated esters.

In the present invention, the S-R1 group carried by the amino acid residue of the triangle unit (for example, a residue represented by general formula (I)) is deprotected during the cyclization reaction and the SH group thus generated acts as a reaction promoting group so that the active ester of the intersection unit and the amino group of the triangle unit can be selectively and efficiently reacted to form an amide bond and accomplish the cyclization. That is, because of the high nucleophilicity of the SH group, first, the SH group of the triangle unit quickly reacts with the active ester of the intersection unit and is then converted to a thermodynamically more stable amide bond by an intramolecular transfer reaction. As a result, the triangle unit and the intersection unit can be amidated selectively (Chemical ligation method).

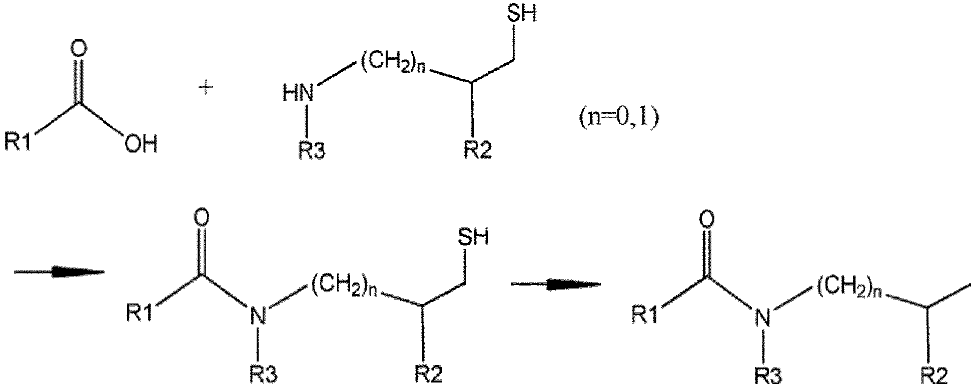

R2" and R3" of formula (III) are defined similarly to R2 and R3 defined for formula (I). Specifically, R2" and R3" each independently are a hydrogen atom, or an alkyl, an alkenyl, an alkynyl, an aryl, a heteroaryl, an aralkyl, or a cycloalkyl group, which are optionally substituted. Alternatively, R2" and R3" may form a ring together with the atoms to which they are bonded, or R2" or R3" may form a ring together with R26 and the atoms to which they are bonded. Any arrangement is acceptable for the configuration contained in those structures.

Examples of the case where "R2" and R3" form a ring together with the atoms to which they are bonded" include the following structure where a four- to seven-membered ring is formed using R2", N bonded to R2", R3", and C bonded to R3" as part of the constituting atoms.

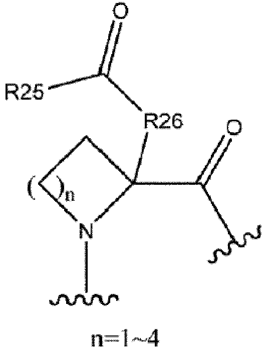

Examples of the case where "R2" forms a ring together with R26 and the atoms to which they are bonded" include the following structure where a four- to seven-membered ring is formed using R2", N bonded to R2", R26, and C bonded to R26 as part of the constituting atoms.

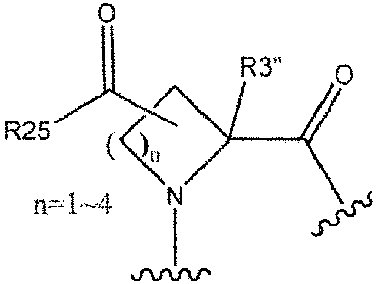

Examples of the case where "R3" forms a ring together with R26 and the atoms to which they are bonded" include the following structure where a three- to seven-membered ring is formed using R3", R26, and C to which R3" and R26 are bonded as part of the constituting atoms.

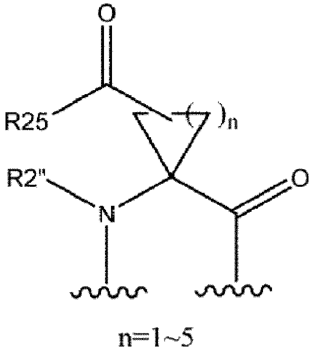

More preferably, R2" and R3" represent a hydrogen atom, a C1-C4 alkyl, a C1-C4 alkyl optionally substituted with a C1-C4 alkoxy and/or a halogen, or a C5-C6-membered ring formed from two sites selected from R2", R3", and R26. Any arrangement is acceptable for the configuration contained in those structures.

R26 is defined similarly to R4 defined for formula (I). Specifically, for example, R26 is selected from the group consisting of an alkylene, an arylene, a heteroarylene, an alkylenearylene, an alkyleneheteroarylene, an arylenealkylene, and a heteroarylenealkylene, and these groups are optionally substituted.

Representative structures of R26 are shown below.

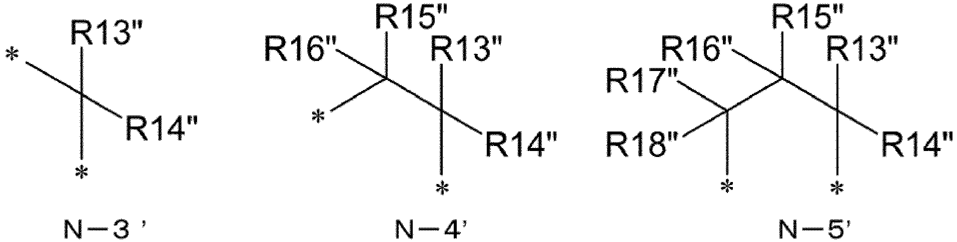

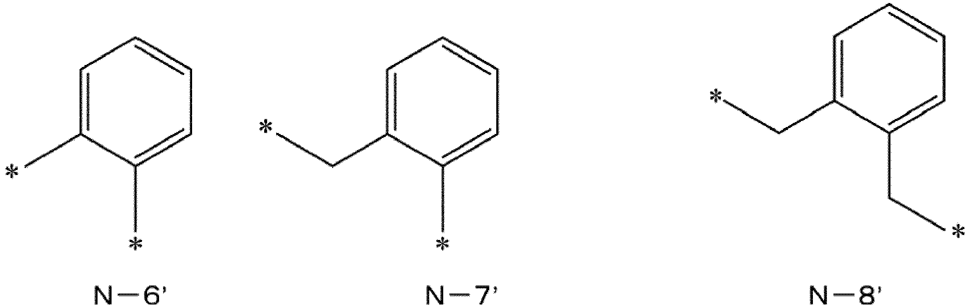

The second reaction point (for example, an active ester) and an amino acid portion can be linked through one to six optionally substituted carbon atoms, such as an optionally substituted methylene (N-3'), optionally substituted ethylene (N-4'), and optionally substituted propylene (N-5').

R13", R14", R15", R16", R17", and R18" are defined similarly to the side chains of drug-like amino acids defined above, and for example, they are selected from a hydrogen atom, an alkyl optionally substituted with a halogen atom or such, and an alkoxy optionally substituted with a halogen atom or such. A ring structure may be formed between these groups. More preferably, R13" to R18" each are a hydrogen atom, a linear or branched C1-C4 alkyl, a C1-C5 alkoxy optionally substituted with one or more fluorine atoms, C1-C4 alkyl optionally substituted with one or more fluorine atoms or with C1-C5 alkoxy optionally substituted with one or more fluorine atoms, or R13" to R18" form a C3-C7-membered ring composed of two sites selected from R13" to R18" and atoms bonded to them. Particularly preferably, R13" to R18" each are a hydrogen atom or a methyl.

R26 is more preferably a single-carbon-atom such as methylene (N-3'), four carbon atoms, five carbon atoms, or six carbon atoms. More preferably, R26 is a single carbon atom (N-3').

Furthermore, the second reaction point (for example, an active ester portion) and an amino acid portion can be linked directly through the carbon atoms of an aromatic ring (N-6'). Furthermore, the active ester portion and the amino acid portion can also be linked *via* an aralkyl structure (N-7' and N-8').

In N-6' to N-8', the linking position is limited to the *ortho*-position, but it may be, for example, the *meta-* or *para*-position without being limited to the *ortho*-position. Although benzene is used as the aromatic ring in N-6' to N-8', an aromatic ring other than benzene (i.e. various aromatic rings including heteroaromatic rings) may be used, and the aromatic ring is optionally substituted with a substituent such as a halogen or an alkoxy.

A favorable structure of formula (III) is indicated by formula (III-2).

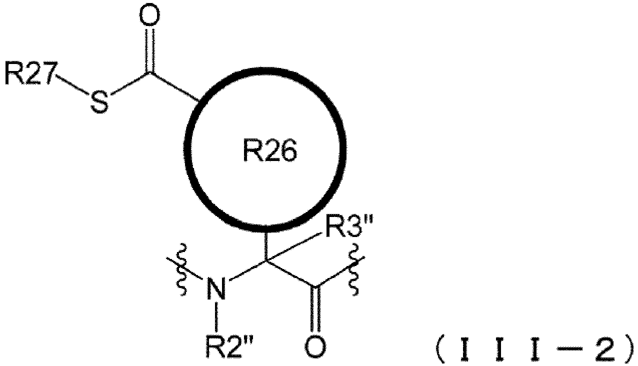

R27 is selected from among a hydrogen atom, an optionally substituted alkyl, an optionally substituted alkenyl, an optionally substituted alkynyl, an optionally substituted aryl, an optionally substituted heteroaryl, an optionally substituted cycloalkyl, and an aralkyl optionally substituted with an optionally substituted alkyl.

A substituent that may be carried by a group listed as examples of R27 is not particularly limited, as long as formula (III-2) carrying that substituent can be ribosomally synthesized. Examples of such substituents include electron-withdrawing groups such as a halogen, a nitro, a trifluoromethyl, and a nitrile often used for the purpose of enhancing reactivity; electron-donating groups such as an alkoxy (*e.g.,* methoxy) and alkyl (*e.g.,* methyl) often used for the purpose of reducing reactivity and thereby enhancing reaction selectivity; bulky substituents typified by a *t*-butyl and isopropyl; and a sulfo group and a di-substituted amino group such as dimethylamino in consideration of hydrophilicity or highly lipid-soluble groups such as a long-chain alkyl in consideration of lipophilicity. Examples of such substituents are preferably an optionally substituted alkyl, an optionally substituted cycloalkyl, and an optionally substituted aralkyl, and more preferably an aralkyl whose alkyl and/or aryl is/are optionally substituted.

R3" of formula (III-2) is defined similarly to the side chains of drug-like amino acids, and for example, it is preferably a hydrogen atom, a C1-C4 alkyl, or a C1-C4 alkyl optionally substituted with halogen or such, and particularly preferably a hydrogen atom. Both conformations corresponding to L- and D-amino acids are acceptable for the R3" provided that R3" is a hydrogen atom, but preferably, R3" has a conformation corresponding to an L-amino acid.

A more favorable structure among formula (III) is indicated by formula (III-3).

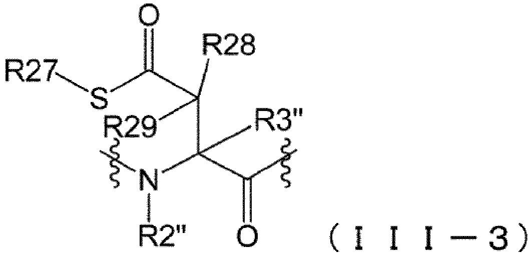

Each of R28 and R29 is defined similarly to the side chains of drug-like amino acids, and for example, it is selected from a hydrogen atom, an optionally substituted C1-C6 alkyl, an optionally substituted C2-C6 alkenyl, an optionally substituted C2-C6 alkynyl, an optionally substituted aryl, an optionally substituted heteroaryl, an aralkyl optionally substituted with an optionally substituted C1-C6 alkyl, or an optionally substituted cycloalkyl.

Preferred examples of R28 and R29 include monomethyl (R28 = Me, R29 = H), dimethyl (R28 = R29 = Me), and monotrifluoromethyl (R28 = CF₃, R29 = H).

R3" is selected from a hydrogen atom, a C1-C4 alkyl optionally substituted with halogen, or such, and is particularly preferably a hydrogen atom. Both conformations corresponding to L- and D-amino acids are acceptable for the R3" group provided that R3" is a hydrogen atom, but preferably, R3" has a conformation corresponding to an L-amino acid.

As amino acid residues having a second reaction point at one of its side chains, III-OH, III-2-OH, and III-3-OH may also be used in addition to III, III-2, and III-3: 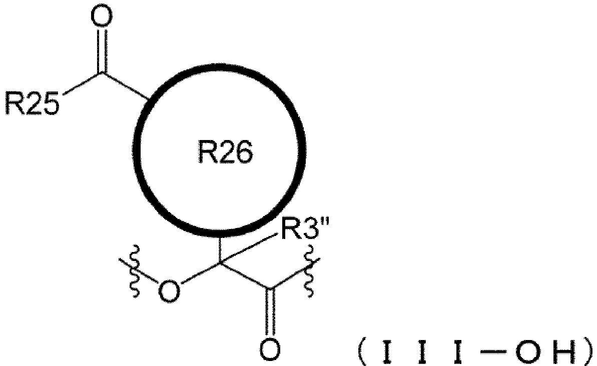 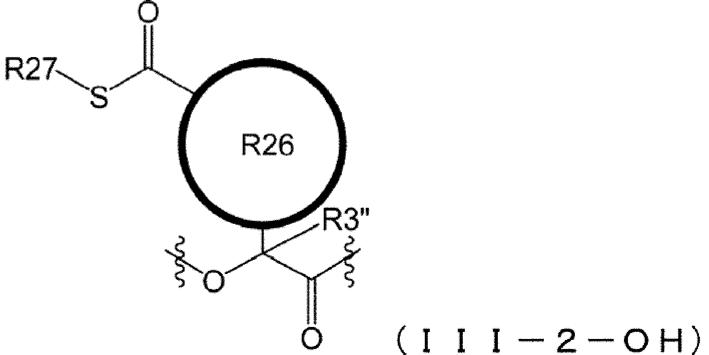 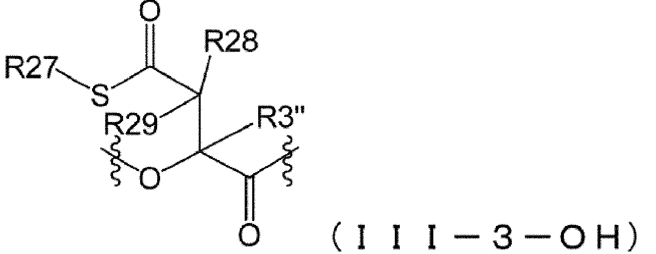 wherein, R3", R25, and R26 of formula III-OH, R3", R26, and R27 of formula III-2-OH, and R3", R27, R28, and R29 of formula III-3-OH have the same meaning as R3", R25, and R26 of formula III, R3", R26, and R27 of formula III-2, and R3", R27, R28, and R29 of formula III-3, respectively.

In the present invention, use of the amino acid residues of N-12 and N-15 as the triangle unit, and use of the amino acid residue of formula (III-3) as the intersection unit are favorable for methods for preparing peptides having a cyclic portion and complexes of the peptides and nucleic acids. Furthermore, to avoid side reactions, an *N*-alkylated amino acid (for example, proline and *N*-methylalanine) is preferably used for the amino acid residue adjacent to the intersection unit.

Examples of a method for preparing a complex between a nucleic acid and a drug-like peptide having the desired activity include a preparation method comprising the following steps (Patent Document 1):
(i) ribosomally synthesizing a noncyclic peptide moiety having nine to 13 amino acid residues in total to form a noncyclic peptide moiety-nucleic acid complex in which the noncyclic peptide moiety is linked to a nucleic acid sequence encoding thereof through a linker;
(ii) cyclizing through an amide bond the noncyclic peptide moiety of the complex ribosomally synthesized in step (i) to form a cyclic compound having a cyclic portion with five to twelve amino acid residues in total; and
(iii) bringing a library of the cyclic portion-containing peptide moiety-nucleic acid complexes obtained in step (ii) into contact with a biomolecule to select a complex having binding activity to the biomolecule.

Specifically, aminoacyl tRNAs used in the step of synthesizing peptides or peptide moiety-nucleic acid complexes can be prepared using the following methods.

A template DNA encoding a desired tRNA sequence and having a T7, T3 or SP6 promoter located upstream thereof is provided and transcribed to synthesize RNA using RNA polymerase compatible with the promoter, such as T7, T3 or SP6 RNA polymerase. tRNAs can also be extracted from cells and purified, and the generated tRNA of interest can be extracted by using a probe having a sequence complementary to the tRNA sequence. In this case, cells transformed with expression vectors for the tRNA of interest may be used as a source. The RNA sequence of interest may also be synthesized chemically. For example, a tRNA lacking CA at the 3'-terminal CCA sequence thus obtained can be ligated to separately prepared aminoacylated pdCpA or pCpA prepared separately with RNA ligase to obtain an aminoacyl-tRNA (pdCpA method, pCpA method). For example, tRNA for an amino acid represented by formula (I) can be prepared using pdCpA or pCpA linked to the amino acid represented by formula (I). The tRNA is useful in preparing a peptide, a peptide moiety-mRNA complex, and a library of peptide moiety-mRNA complexes of the present invention. Therefore, the present invention relates to pdCpA or pCpA linked to an amino acid represented by formula (I). Furthermore, the present invention relates to a tRNA aminoacylated with an amino acid represented by formula (I).

Alternatively, a full-length tRNA may be provided and aminoacylated by a flexizyme, which is a ribozyme capable of charging active esters of various amino acid analogs onto tRNAs. In addition, acylated tRNAs can be obtained by using the methods described later.

In the present invention, ribosomal synthesis can be carried out, for example, by adding mRNA to a known cell-free translation system such as the PURE system mixed with protein factors necessary for translation in *E. coli* (methionyl tRNA transformylase, EF-G, RF1, RF2, RF3, RRF, IF1, IF2, IF3, EF-Tu, EF-Ts and ARS (necessary ones are selected from AlaRS, ArgRS, AsnRS, AspRS, CysRS, GlnRS, GluRS, GlyRS, HisRS, IleRS, LeuRS, LysRS, MetRS, PheRS, ProRS, SerRS, ThrRS, TrpRS, TyrRS and ValRS)), ribosome, amino acids, creatine kinase, myokinase, inorganic pyrophosphatase, nucleoside diphosphate kinase, *E. coli-*derived tRNA, creatine phosphate, potassium glutamate, HEPES-KOH (pH 7.6), magnesium acetate, spermidine, dithiothreitol, GTP, ATP, CTP, UTP and the like. Moreover, when adding T7 RNA polymerase in advance, coupled transcription and translation may be performed from a template DNA containing a T7 promoter. In this case, desired acylated tRNA population and amino acid analog population (*e.g.,* F-Tyr) acceptable by ARS can be added to the system to ribosomally synthesize peptides containing the amino acid analog population (Kawakami T, et al. Ribosomal synthesis of polypeptoids and peptoid-peptide hybrids. J Am Chem Soc. 2008, 130, 16861-3; Kawakami T, et al. Diverse backbone-cyclized peptides via codon reprogramming. Nat Chem Biol. 2009, 5, 888-90). Alternatively, the translational incorporation efficiency of amino acid analogs may be enhanced by using variants of ribosome, EF-Tu, and the like (Dedkova LM, et al. Construction of modified ribosomes for incorporation of D-amino acids into proteins. Biochemistry. 2006, 45, 15541-51; Doi Y, et al. Elongation factor Tu mutants expand amino acid tolerance of protein biosynthesis system. J Am Chem Soc. 2007, 129, 14458-62; Park HS, et al. Expanding the genetic code of Escherichia coli with phosphoserine. Science. 2011, 333, 1151-4).

The cell-free translation system refers to a combination of ribosomes extracted from cells as well as protein factors involved in translation, tRNAs, amino acids, energy sources such as ATPs and a regenerating system thereof, and is not limited as long as it can translate mRNAs into proteins. The cell-free translation system of the present invention can additionally contain an initiation factor, an elongation factor, a dissociation factor, aminoacyl-tRNA synthetase, and such. These factors can be obtained by purification from various cell extracts. Examples of cells for use in the purification of the factors can include prokaryotic cells and eukaryotic cells. Examples of the prokaryotic cells can include *E. coli* cells, extreme thermophile cells, and *Bacillus subtilis* cells. Those obtained using eukaryotic cells, such as yeast cells, wheat germs, rabbit reticulocytes, plant cells, insect cells, or animal cells, as a source are known.

Meanwhile, PURE system is a reconstituted cell-free translation system prepared by individually extracting and purifying protein factors necessary for translation in *E. coli,* energy-regenerating enzymes and ribosomes and mixing them with tRNAs, amino acids, ATPs, GTPs, and such. Since this system has a low content of impurities and is a reconstituted system, a system free from protein factors and amino acids to be excluded can be created easily ((i) Nat Biotechnol. 2001; 19: 751-5. Cell-free translation reconstituted with purified components. Shimizu Y, Inoue A, Tomari Y, Suzuki T, Yokogawa T, Nishikawa K, Ueda T; (ii) Methods Mol Biol. 2010; 607: 11-21. PURE technology. Shimizu Y, Ueda T.).

An mRNA display library can be prepared, for example, as follows. First, a library of DNAs in which a desired sequence is positioned downstream of a promoter such as T7 promoter is chemically synthesized, and this library is used as templates to prepare double-stranded DNAs by primer extension reaction. The double-stranded DNAs are used as templates and transcribed into mRNAs by using RNA polymerase such as T7 RNA polymerase. To the 3-ends of the resulting RNAs, a linker (spacer) joined to an aminoacyl-tRNA analog such as antibiotic puromycin is conjugated. The resulting conjugates are added to a known cell-free translation system, such as the above-mentioned PURE system, and incubated so that the mRNAs are translated to link each mRNA to the peptide encoded thereby through a linker containing puromycin or such. In this way, a display library composed of complexes formed between mRNAs and their corresponding products can be constructed. The linkers can additionally include spacers well-known to those skilled in the art.

In addition, the library is brought into contact with desired immobilized targets and molecules that did not bind to the targets are washed off to thereby enable concentrating the target-binding molecules (panning). cDNAs are synthesized from the mRNAs serving as a tag containing genetic information attached to the molecule thus selected, amplified by PCR, and subjected to sequencing to thereby enable determining sequences of the bound peptides.

In the present invention, for example, methods shown in the following embodiments can be performed specifically for construction of a display library of the complexes formed between the cyclized peptide moieties and nucleic acids and acquisition of cyclized peptide moieties or cyclized branched peptides, which bind to targets, from the constructed display library.

More specifically, a method for preparing a library of complexes between a peptide having a cyclic portion and a nucleic acid and an amide-cyclized peptide library thereof according to the present invention can comprise one or a plurality of the following steps:
A) providing pdCpA or pCpA aminoacylated with formula (I);
B) providing an initiation tRNA lacking 3'-terminal CA;
C) linking the pdCpA or pCpA of step A) to the initiation tRNA of step B) to provide an initiation tRNA aminoacylated with formula (I);
D) providing pdCpA or pCpA aminoacylated with formula (III);
E) providing a tRNA lacking 3'-terminal CA;
F) linking the pdCpA or pCpA of step D) to the tRNA of step E) to provide a tRNA aminoacylated with formula (III);
G) providing a cell-free translation system containing the initiation aminoacyl-tRNA of step C) and the aminoacyl-tRNA of step F) and not containing either one of methionine, methionyl tRNA synthetase (MetRS), translation initiation tRNA for methionine, formyl donor, and methionyl tRNA transferase;
H) providing a peptide sequence-encoding template DNA library comprising, downstream of a promoter, a codon corresponding to the anticodon of the initiation aminoacyl-tRNA of step C) as the first codon, and further downstream thereof, a codon corresponding to the anticodon of the aminoacyl-tRNA of step F);
I) providing an mRNA library from the template DNA library of step H);
J) conjugating linkers to the 3'-ends of the mRNA library of step I);
K) adding the linker-conjugated mRNA library of step J) to the cell-free translation system of step G) to perform translation, thereby providing an pre-cyclized (noncyclic) peptide moiety-mRNA complex display library; and
L) forming cyclic portions and then performing desulfurization.

The method of the present invention can also comprise, following step J), a step of synthesizing cDNAs using primers annealing to the 3'-regions of the mRNA library. Furthermore, the method of the present invention can include the following steps:
M) concentrating the mRNA library that is bound to a target substance by panning;
N) synthesizing cDNA with reverse transcriptase; and
O) analyzing the nucleotide sequence.

Such a method for preparing a library of a cyclic portion-comprising peptide-mRNA complexes and an amide-cyclized peptide library thereof according to the present invention is preferably a method comprising one or a plurality of the following steps:
A) providing pdCpA or pCpA aminoacylated with N-9;
B) providing an initiation tRNA lacking 3'-terminal CA;
C) linking the pdCpA or pCpA of step A) to the initiation tRNA of step B) to provide an initiation tRNA aminoacylated with N-9;
D) providing pdCpA or pCpA aminoacylated with formula (III)-2 or formula (III)-2-OH;
E) providing a tRNA lacking 3'-terminal CA;
F) linking the pdCpA or pCpA of step D) to the tRNA of step E) to provide a tRNA aminoacylated with formula (III)-2 or formula (III)-2-OH;
G) providing a cell-free translation system containing the initiation aminoacyl-tRNA of step C) and the aminoacyl-tRNA of step F) and not containing either one of methionine, methionyl tRNA synthetase (MetRS), translation initiation tRNA for methionine, formyl donor, and methionyl tRNA transferase;
H) providing a peptide sequence-encoding template DNA library comprising, downstream of a promoter, ATG which is a codon corresponding to the anticodon of the initiation aminoacyl-tRNA of step C) as the first codon, and further downstream thereof, a codon corresponding to the anticodon of the aminoacyl-tRNA of step F), and on the 3' side thereof, a codon for proline or *N*-methylamino acid serving as another ARS substrate;
I) providing an mRNA library from the template DNA library of step H);
J) conjugating linkers to the 3'-ends of the mRNA library of step I);
K) adding the linker-conjugated mRNA library of step J) to the cell-free translation system of step G) to perform translation, thereby providing an pre-cyclized (noncyclic) peptide moiety-mRNA complex display library; and
L) forming cyclic portions and then performing desulfurization.

More preferably, such a method for preparing a library of cyclic portion-comprising peptide-mRNA complexes and an amide-cyclized peptide library thereof according to the present invention is a method comprising one or a plurality of the following steps:
A) providing pdCpA or pCpA aminoacylated with N-12 or N-15;
B) providing an initiation tRNA lacking 3'-terminal CA;
C) linking the pdCpA or pCpA of step A) to the initiation tRNA of step B) to provide an initiation tRNA aminoacylated with N-12 or N-15;
D) providing pdCpA or pCpA aminoacylated with formula (III)-3 or formula (III)-3-OH;
E) providing a tRNA lacking 3'-terminal CA;
F) linking the pdCpA or pCpA of step D) to the tRNA of step E) to provide a tRNA aminoacylated with formula (III)-3 or formula (III)-3-OH;
G) providing a cell-free translation system containing the initiation aminoacyl-tRNA of step C) and the aminoacyl-tRNA of step F) and not containing either one of methionine, methionyl tRNA synthetase (MetRS), translation initiation tRNA for methionine, formyl donor, and methionyl tRNA transferase;
H) providing a peptide sequence-encoding template DNA library comprising, downstream of a promoter, ATG which is a codon corresponding to the anticodon of the initiation aminoacyl-tRNA of step C) as the first codon, and further downstream thereof, a codon corresponding to the anticodon of the aminoacyl-tRNA of step F), and on the 3' side thereof, a codon for proline or *N*-methylamino acid serving as another ARS substrate;
I) providing an mRNA library from the template DNA library of step H);
J) conjugating linkers to the 3'-ends of the mRNA library of step I);
K) adding the linker-conjugated mRNA library of step J) to the cell-free translation system of step G) to perform translation, thereby providing an pre-cyclized (noncyclic) peptide moiety-mRNA complex display library; and
L) forming cyclic portions and then performing desulfurization.

Even more preferably, such a method for preparing a library of cyclic portion-comprising peptide-mRNA complexes and an amide-cyclized peptide library thereof according to the present invention is a method comprising one or a plurality of the following steps:
A) providing pdCpA or pCpA aminoacylated with N-12-1, N-12-2, N-13-1, or N-14-1;
B) providing an initiation tRNA lacking 3'-terminal CA;
C) linking the pdCpA or pCpA of step A) to the initiation tRNA of step B) to provide an initiation tRNA aminoacylated with N-12-1, N-12-2, N-13-1, or N-14-1;
D) providing pdCpA or pCpA aminoacylated with formula (III)-3;
E) providing a tRNA lacking 3'-terminal CA;
F) linking the pdCpA or pCpA of step D) to the tRNA of step E) to provide a tRNA aminoacylated with formula (III)-3;
G) providing a cell-free translation system containing the initiation aminoacyl-tRNA of step C) and the aminoacyl-tRNA of step F) and not containing either one of methionine, methionyl tRNA synthetase (MetRS), translation initiation tRNA for methionine, formyl donor, and methionyl tRNA transferase;
H) providing a peptide sequence-encoding template DNA library comprising, downstream of a promoter, ATG which is a codon corresponding to the anticodon of the initiation aminoacyl-tRNA of step C) as the first codon, and further downstream thereof, a codon corresponding to the anticodon of the aminoacyl-tRNA of step F), and on the 3' side thereof, a codon for proline or *N*-methylamino acid serving as another ARS substrate;
I) providing an mRNA library from the template DNA library of step H);
J) conjugating linkers to the 3'-ends of the mRNA library of step I);
K) adding the linker-conjugated mRNA library of step J) to the cell-free translation system of step G) to perform translation, thereby providing an pre-cyclized (noncyclic) peptide moiety-mRNA complex display library; and
L) forming cyclic portions and then performing desulfurization.

### Method for suppressing aspartimide formation

In the translational incorporation of aspartic acid-type thioester, the thioester reacts with an amide bond immediately following the C-terminal amino acid residue to form aspartimide. Accordingly, a desired thioester-containing full-length peptide can be ribosomally synthesized by using an amino acid having an *N*-alkyl group (*e.g.,* proline) as an amino acid residue immediately following the C-terminal amino acid residue when translationally incorporating the aspartic acid-type thioester.

Furthermore, the present invention relates to compounds represented by general formula (IA) and general formula (IIA) shown below.

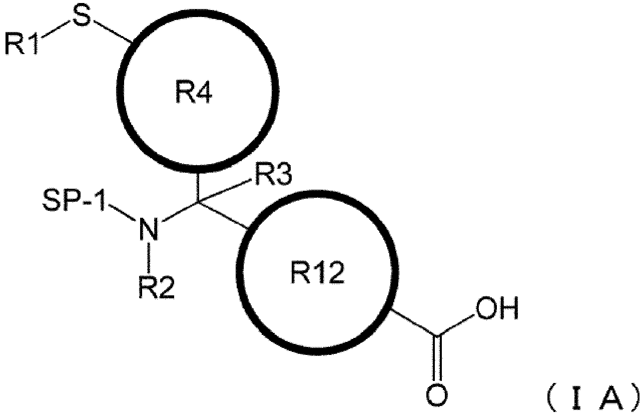

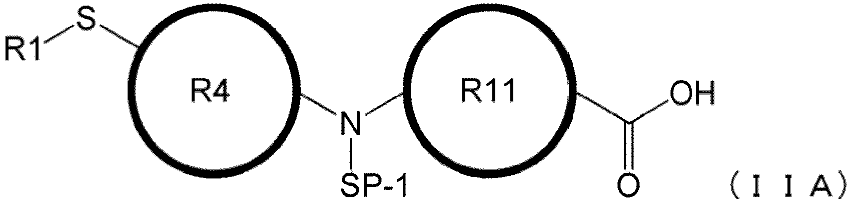

Furthermore, the present invention relates to compounds represented by general formula (IB) and general formula (IIB) shown below.

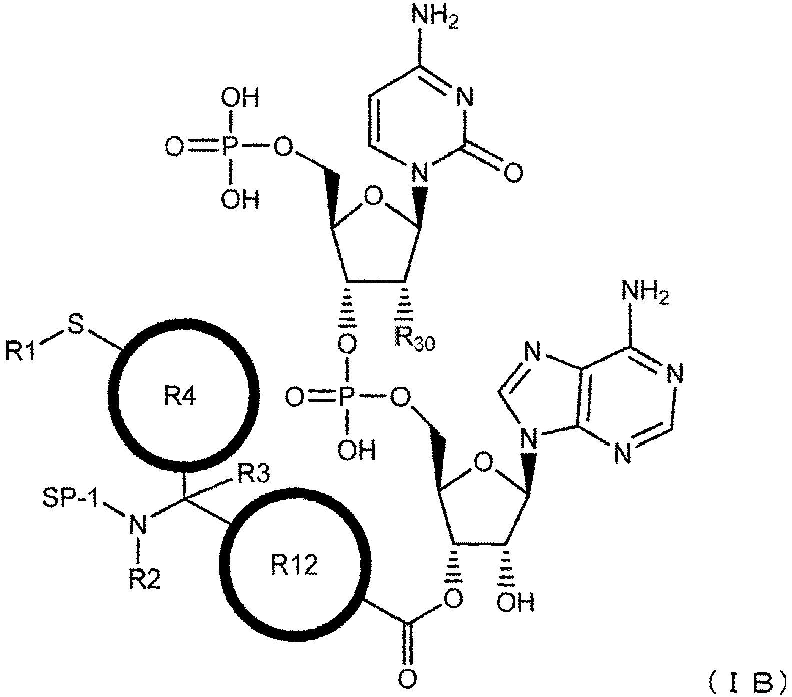

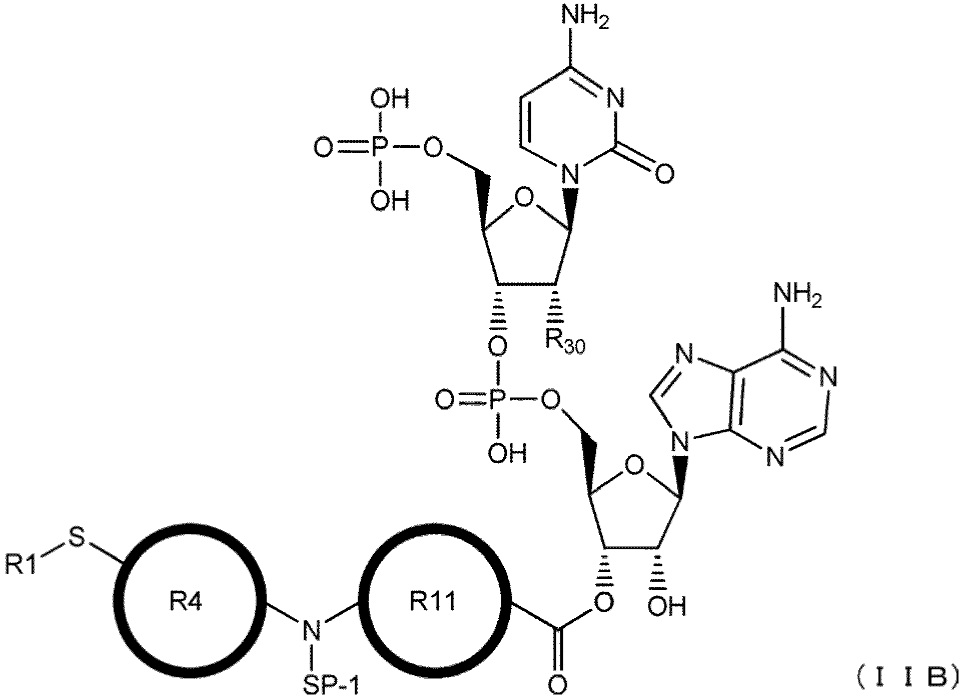

In general formulas (IA) and (IB) shown above, R1 to R4, R12, and SP-1 have the same meaning as R1 to R4, R12, and SP-1 of general formula (I), and R30 represents H or a hydroxyl group. R30 is particularly preferably OH.

Furthermore, in general formulas (IIA) and (IIB) shown above, R1, R4, R11, and SP-1 have the same meaning as R1, R4, R11, and SP-1 of general formula (II), and R30 represents H or a hydroxyl group. R30 is particularly preferably OH.

Furthermore, the present invention relates to conjugates between aminoacyl-tRNA and general formula (IA) or general formula (IIA) described above.

Compounds represented by the above-mentioned general formulas (IA), (IB), (IIA), and (IIB) and conjugates formed between aminoacyl-tRNA and general formula (IA) or general formula (IIA) are useful in efficient translation of peptides comprising a plurality of amino acid analogs, in which cysteine or a cysteine analog is positioned at the N terminus. Furthermore, they are useful in methods for preparing a complex formed between a peptide having a cyclic portion containing a plurality of amino acid analogs and a nucleic acid, and a library containing such complexes.

Compounds represented by the above-mentioned general formulas (IA), (IB), (IIA), and (IIB) and conjugates formed between aminoacyl-tRNA and general formula (IA) or general formula (IIA) can be obtained by methods well known to those skilled in the art.

Furthermore, the present invention relates to a complex formed between a nucleic acid and a noncyclic peptide which comprises at its N terminus an amino acid residue represented by general formula (I) or general formula (II) shown below and comprising a first reaction point, and which comprises at a position at least four residues apart from the N terminus to the C-terminal side an amino acid residue comprising a second reaction point in one of its side chains.

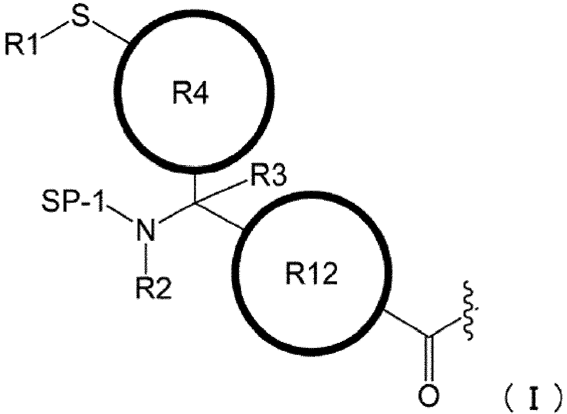

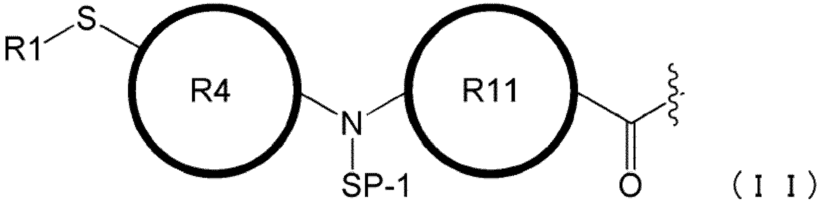

The complex can be obtained by a method comprising a step of translating a nucleic acid encoding a noncyclic peptide which comprises at its N terminus an amino acid residue represented by general formula (I) or general formula (II) shown above and comprising a first reaction point, and which comprises at a position at least four residues apart from the N terminus to the C-terminal side an amino acid residue comprising a second reaction point in one of its side chains to synthesize the complex. In formula (I) shown above, R1 to R4, R11, R12, and SP-1 are defined according to the definitions described herein.

By using nucleic acids encoding noncyclic peptides constituting the complexes, complexes between a nucleic acid and a peptide comprising two or more amino acid analogs and having a cyclic portion, or a library of those complexes, can be prepared efficiently.

All prior art references cited herein are incorporated by reference into this description.

### Examples

The present invention will be further illustrated with reference to the following Examples but is not limited thereto.

The following abbreviations are used in the Examples.
- DCM: Dichloromethane
- DIPEA: *N,N*-diisopropylethylamine
- DMF: Dimethylformamide
- DMSO: Dimethylsulfoxide
- DTT: Dithiothreitol
- FA: Formic acid
- TFA: Trifluoroacetic acid
- THF: Tetrahydrofuran
- TCEP: Tris(2-carboxyethyl)phosphine
- NMP: *N*-Methyl-2-pyrrolidone
- DBU: 1,8-Diazabicyclo[5.4.0]-7-undecene
- Acbz: 4-Azidobenyloxycarbonyl group 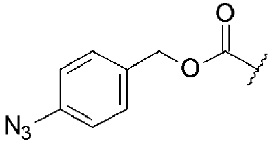
*o*-Acbz 2-Azidobenzyloxycarbonyl group 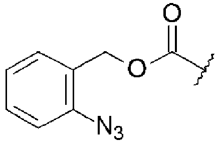
- Pen: 4-Pentenoyl group
- CH₂CN: Cyanomethyl group
LCMS analysis conditions are as follows.

**[Table 1]**

| Fractionation condition | Device | Column (I. D. x Length (mm)) | Mobile phase | Gradient (A/B) | Flow rate (mL/min) | Column temperature (°C) | Wave length |
|---|---|---|---|---|---|---|---|
| SQD AA05 | Acquity UPLC/SQD | Aldrich Ascent is Express C18 (2.1 x 50) | A) 10mM AcONH4, H20 | 95/5 => 0/100 (1.0 minute) => 0/100 ( 0.4 minute) | 1.0 | 35 | 210-400nm PDA total |
| | | | B) MeOH | | | | |
| SQD AA50 | Acquity UPLC/SQD | Aldrich Ascent is Express C18 (2.1 x 50) | A) 10mM AcONH4, H2O | 50/50 => 0/100 (0.7 minutes) => 0/100 (0.7 minutes) | 1.0 | 35 | 210-400nm PDA total |
| | | | B) MeOH | | | | |
| SQD FAO5 | Acquity UPLC/SQD | Aldrich Ascent is Express C18 (2.1 x 50) | A) 0.1% FA, H2O | 95/5 => 0/100 (1.0 minute) => 0/100 ( 0.4 minute) | 1.0 | 35 | 210-400nm PDA total |
| | | | B) 0. 1% FA CH3CN | | | | |
| SQD FA50 | Acquity UPLC/SQD | Aldrich Ascent is Express C18 (2.1 x 50) | A) 0.1% FA, H2O | 50/50 => 0/100 (0.7 minutes) => 0/100 (0.7 minutes) | 1.0 | 35 | 210-400nm PDA total |
| | | | B) 0.1% FA CH3CN | | | | |
| SMD method 1 | Shimadzu LCMS-202 0 LC-20AD | Shim-Pack XR-ODS (3.0 x 50) | A) 0.05% TFA, H2O | 95/5 => 0/100 (2.2 minute) => 0/100 ( 1.0 minute) | 1.0 | 40 | 210-800nm PDA total |
| | | | B) 0.05% TFA CH3CN | | | | |
| SMD method 2 | Shimadzu LCMS-202 0 LC-20AD | Shim-Pack XR-ODS (3.0 x 50) | A) 0.05% TFA, H2O | 95/5 => 0/80 (2.2 minute) => 0/80 ( 1.0 minute) | 1.0 | 40 | 210-800nm PDA total |
| | | | B) 0.05% TFA CH3CN | | | | |
| SMD method 3 | Nexera/2 020 | Aldrich Ascent is Express C18 (2.1 x 50) | A) 0.1% FA. H2O | 95/5 => 0/100 (1.5 minute) => 0/100 ( 0.7 minute) | 1.0 | 35 | 210-400nm PDA total |
| | | | B) 0.1% FA CH3CN | | | | |
| SMD method 4 | Shimadzu LCMS-202 0 LC-30AD | ACQUITY UPLC BEH C18 (1.7x50) | A)0.1% FA, H2O | 95/5=> 20/80(4.0 minute) => 20/80 ( 1.2 minute) | 0.7 | 45 | 190-800nm PDA total |
| | | | B)0.05% FA. MeCN | | | | |
| SMD method 5 | Shimadzu LCMS-202 0 LC-20AD | Shim-Pack XR-ODS (2. 2x50) | A)0.05% TFA, H2O | 95/5=> 0/100(2.2 minute) => 0/110 ( 1.0 minute) | 1.0 | 40 | 190-800nm PDA total |
| | | | B) 0.05% TFA, MeCN | | | | |

### [Example 1] Synthesis of pCpA-amino acids for use in a cell-free translation system

### 1-1. Synthesis of pCpA-amino acids for translational incorporation of cysteine or a cysteine analog to the N terminus by the initiation suppression method

To ribosomally synthesize peptides having cysteine or a cysteine analog at the N terminus by the initiation suppression method, pCpAs aminoacylated with cysteine or a cysteine analog were synthesized. That is, aminoacylated pCpAs (nk-05, 09, 14, 19, and 22) were synthesized according to the following scheme.

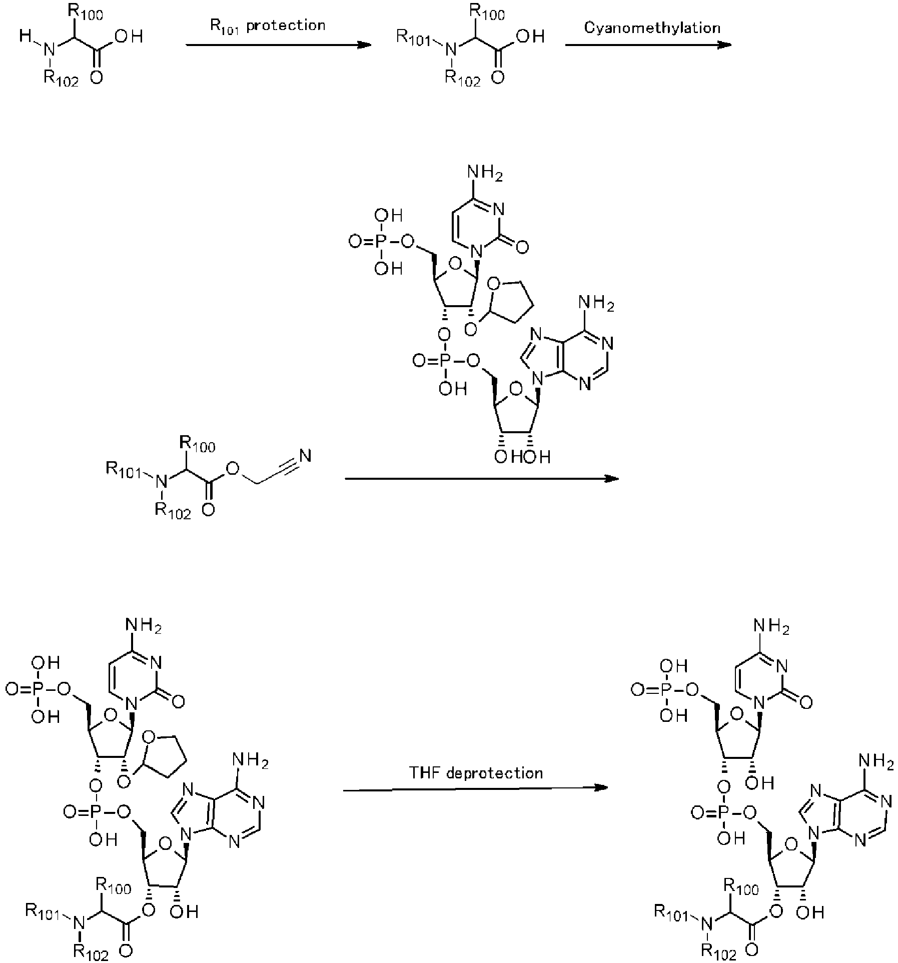

### Synthesis of (R)-2-((((4-azidobenzyl)oxy)carbonyl)amino)-3-(tert-butyldisulfanyl)propanoic acid (Compound nk02, Acbz-Cvs(StBu)-OH)

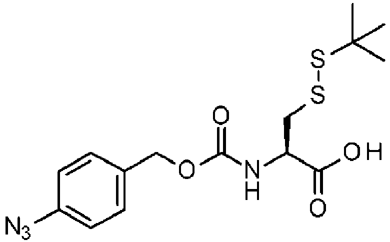

Under nitrogen atmosphere, DMF (0.6 mL) was added at room temperature to a mixture of *S*-*tert*-butylmercapto-L-cysteine (Compound nk01, H-Cys(StBu)-OH)) (126 mg, 0.60 mmol) and 4-azidobenzyl (4-nitrophenyl)carbonate (207 mg, 0.66 mmol) synthesized by the method described in the literature (Bioconjugate Chem., 2008, 19, 714). The mixture was cooled in an ice bath, and then triethylamine (251 µL, 1.80 mmol) was added to it. The reaction mixture was stirred at 25°C for 12 hours and then purified by reverse-phase silica gel column chromatography (0.1% aqueous formic acid solution/0.1% formic acid-acetonitrile solution) to give (R)-2-((((4-azidobenzyl)oxy)carbonyl)amino)-3-(*tert*-butyldisulfanyl)propanoic acid (Compound nk02, Acbz-Cys(StBu)-OH) (220.1 mg, 95%).
LCMS (ESI) m/z = 383 (M-H)-
Retention time: 0.84 minutes (analysis condition SQDFA05)

### Synthesis of (R)-cyanomethyl 2-((((4-azidobenzyl)oxy)carbonyl)amino)-3-(tert-butyldisulfanyl)propanoate (Compound nk03, Acbz-Cys(StBu)-OCH₂CN)

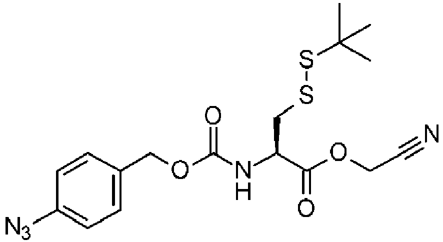

Under nitrogen atmosphere, (R)-2-((((4-azidobenzyl)oxy)carbonyl)amino)-3-(*tert*-butyldisulfanyl)propanoic acid (Compound nk02, Acbz-Cys(StBu)-OH) (1.15 g, 3.00 mmol) and *N*-ethyl-isopropylpropan-2-amine (DIPEA) (0.576 mL, 3.30 mmol) were dissolved in acetonitrile (6.0 mL), 2-bromoacetonitrile (0.627 mL, 9.00 mmol) was added, and the mixture was stirred at room temperature for five hours. The reaction solution was concentrated, and the resulting residue was purified by column chromatography (ethyl acetate:hexane = 1:9 -> 1:1) to give (R)-cyanomethyl 2-((((4-azidobenzyl)oxy)carbonyl)amino)-3-(*tert*-butyldisulfanyl)propanoate (Compound nk03, Acbz-Cys(StBu)-OCH₂CN) (1.21 g, 95%).
LCMS (ESI) m/z = 422 (M-H)-
Retention time: 0.90 minutes (analysis condition SQDFA05)

### Synthesis of (2R)-(2R,3S,4R,5R)-2-((((((2R,3R,4R,5R)-5-(4-amino-2-oxopyrimidin-1(2H)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (Compound nk04)

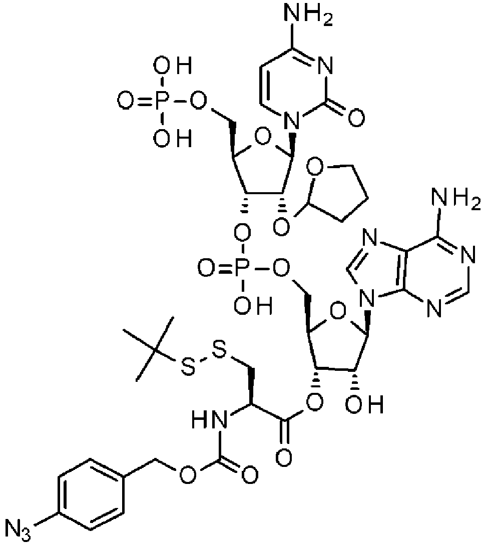

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (120.4 mg, 0.167 mmol) synthesized by the method described in the literature (Helv. Chim. Acta, 90, 297-310) was dissolved in Buffer A (60 mL), a solution of (R)-cyanomethyl 2-((((4-azidobenzyl)oxy)carbonyl)amino)-3-(*tert*-butyldisulfanyl)propanoate (Compound nk03, Acbz-Cys(StBu)-OCH₂CN) (212 mg, 0.500 mmol) in acetonitrile (2.5 mL) was added to it in three portions (0.83-mL each was added at the start of the reaction, and five minutes and 30 minutes after starting the reaction, three times additions in total), and the mixture was stirred at room temperature for 70 minutes. The reaction solution was lyophilized, and the resulting residue was purified by reverse-phase silica gel column chromatography (0.1% aqueous formic acid solution/0.1% formic acid-acetonitrile) to give a mixture (566 mg) of the titled compound (Compound nk04) and *N,N,N*-trimethylhexadecan-1-aminium chloride.
LCMS (ESI) m/z = 1087 (M-H)-
Retention time: 0.62 minutes (analysis condition SQDFA05)

Buffer A was prepared as follows.

Acetic acid was added to an aqueous solution of *N,N,N*-trimethylhexadecan-1-aminium chloride (6.40 g, 20 mmol) and imidazole (6.81 g, 100 mmol) to give Buffer A of 20 mM *N,N,N*-trimethylhexadecan-1-aminium and 100 mM imidazole at pH 8 (1 L).

### Synthesis of (2R)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (nk05, Acbz-Cys(StBu)-pCpA)

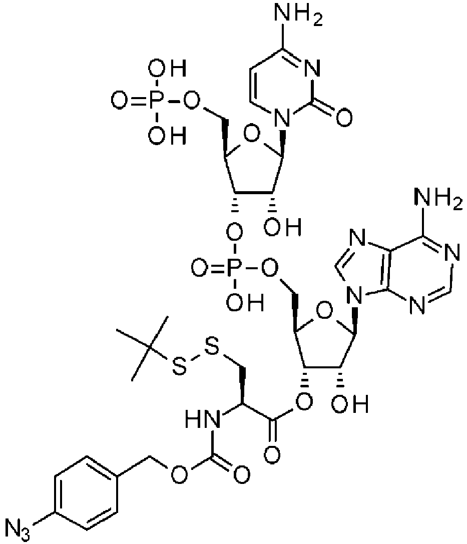

80% Aqueous acetic acid solution (5 mL) was added to the mixture (270 mg) of (2R)-(2R,3S,4R,5R)-2-((((((2R,3R,4R,5R)-5-(4-amino-2-oxopyrimidin-1(2*H*)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9*H*-purin-9-yl)-4-hydroxytetrahydrofuran-3-yl 2-((((4-azidobenzyl)oxy)carbonyl)amino)-3-(*tert*-butyldisulfanyl)propanoate (Compound nk04) and *N,N,N*-trimethylhexadecane-1-aminium chloride obtained in the previous step and stirred at room temperature for two hours. The reaction solution was lyophilized, and the resulting residue was purified by reverse-phase silica gel column chromatography (0.05% aqueous trifluoroacetic acid solution/0.05% trifluoroacetic acid-acetonitrile) to give the titled compound (Compound nk05, Acbz-Cys(StBu)-pCpA) (17.8 mg, 21%, two steps).
LCMS (ESI) m/z = 1019 (M+H)+
Retention time: 0.54 minutes (analysis condition SQDFA05)

### Synthesis of (S)-2-((((4-azidobenzyl)oxy)carbonyl)amino)-3-(tert-butyldisulfanyl)propanoic acid (Compound nk06, Acbz-D-Cys(StBu)-OH)

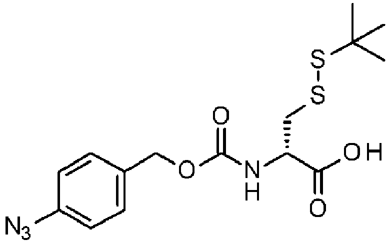

Under nitrogen atmosphere, DMF (1.91 mL) was added at room temperature to a mixture of S-*tert*-butylmercapto-D-cysteine (H-D-Cys(StBu)-OH) (400 mg, 1.91 mmol) and 4-azidobenzyl (4-nitrophenyl)carbonate (661 mg, 2.10 mmol). The mixture was cooled in an ice bath, and then triethylamine (799 µL, 5.73 mmol) was added to it. The reaction mixture was stirred at 25°C for two hours and then purified by reverse-phase silica gel column chromatography (0.1% aqueous formic acid solution/0.1% formic acid-acetonitrile solution) to give (S)-2-((((4-azidobenzyl)oxy)carbonyl)amino)-3-(*tert*-butyldisulfanyl)propanoic acid (Compound nk06, Acbz-D-Cys(StBu)-OH) (658.2 mg, 90%).
LCMS (ESI) m/z = 383 (M-H)-
Retention time: 0.81 minutes (analysis condition SQDFA05)

### Synthesis of (S)-cyanomethyl 2-((((4-azidobenzyl)oxy)carbonyl)amino)-3-(tert-butyldisulfanyl)propanoate (Compound nk07, Acbz-D-Cys(StBu)-OCH₂CN)

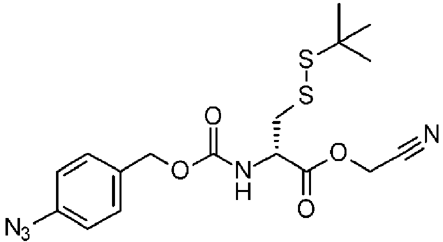

Under nitrogen atmosphere, (S)-2-((((4-azidobenzyl)oxy)carbonyl)amino)-3-(tert-butyldisulfanyl)propanoic acid (Compound nk06, Acbz-D-Cys(StBu)-OH) (0.658 g, 1.71 mmol) and *N*-ethyl-isopropylpropan-2-amine (DIPEA) (0.329 mL, 1.88 mmol) were dissolved in acetonitrile (3.42 mL), 2-bromoacetonitrile (0.358 mL, 5.13 mmol) was added, and the mixture was stirred at room temperature for five hours. The reaction solution was concentrated, and the resulting residue was purified by column chromatography (ethyl acetate:hexane = 1:9 -> 1:1) to give (S)-cyanomethyl 2-((((4-azidobenzyl)oxy)carbonyl)amino)-3-(*tert*-butyldisulfanyl)propanoate (Compound nk07, Acbz-D-Cys(StBu)-OCH₂CN) (0.715 g, 99%).
LCMS (ESI) m/z = 422 (M-H)-
Retention time: 0.90 minutes (analysis condition SQDFA05)

### Synthesis of (2S)-(2R,3S,4R,5R)-2-((((((2R,3R,4R,5R)-5-(4-amino-2-oxopyrimidin-1(2H)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (Compound nk08)

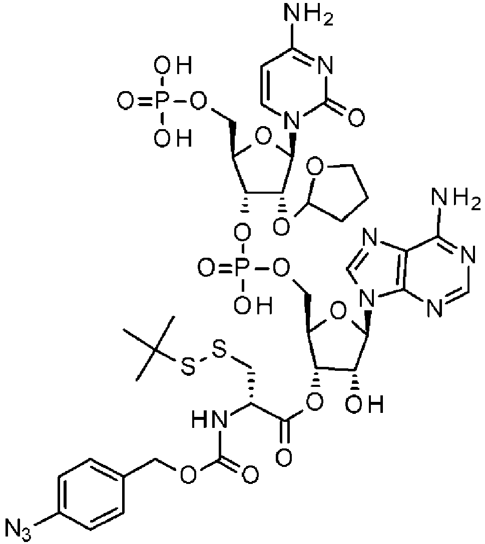

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (60.2 mg, 0.083 mmol) was dissolved in Buffer A (40 mL), a solution of (S)-cyanomethyl 2-((((4-azidobenzyl)oxy)carbonyl)amino)-3-(*tert*-butyldisulfanyl)propanoate (Compound nk07, Acbz-D-Cys(StBu)-OCH₂CN) (106 mg, 0.250 mmol) in acetonitrile (1.26 mL) was added to it in three portions (0.42-mL each was added at the start of the reaction, and five minutes and 30 minutes after starting the reaction, three times additions in total), and the mixture was stirred at room temperature for 70 minutes. The reaction solution was lyophilized, and the obtained residue was purified by reverse-phase silica gel column chromatography (0.1% aqueous formic acid solution/0.1% formic acid-acetonitrile) to give a mixture (242.5 mg) of the titled compound (Compound nk08) and *N,N,N*-trimethylhexadecan-1-aminium chloride.
LCMS (ESI) m/z = 1087 (M-H)-
Retention time: 0.59 minutes (analysis condition SQDFA05)

### Synthesis of (2S)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)amino)-3-(tert-butyldisulfanyl)propanoate (nk09, Acbz-D-Cys(StBu)-pCpA)

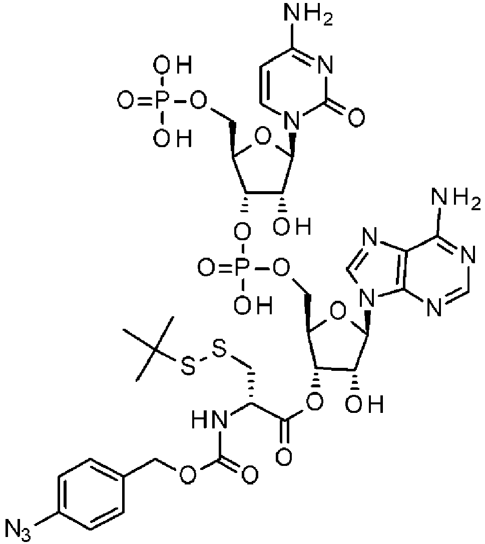

80% Aqueous acetic acid solution (5 mL) was added to the mixture (242.5 mg) of (2S)-(2R,3S,4R,5R)-2-((((((2R,3R,4R,5R)-5-(4-amino-2-oxopyrimidin-1(2*H*)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9*H*-purin-9-yl)-4-hydroxytetrahydrofuran-3-yl 2-((((4-azidobenzyl)oxy)carbonyl)amino)-3-(*tert*-butyldisulfanyl)propanoate (Compound nk08) and *N,N,N*-trimethylhexadecane-1-aminium chloride obtained in the previous step and stirred at room temperature for two hours. The reaction solution was lyophilized, and the resulting residue was purified by reverse-phase silica gel column chromatography (0.05% aqueous trifluoroacetic acid solution/0.05% trifluoroacetic acid-acetonitrile) to give the titled compound (Compound nk09, Acbz-D-Cys(StBu)-pCpA) (24.7 mg, 29%, two steps).
LCMS (ESI) m/z = 1019 (M+H)+
Retention time: 0.55 minutes (analysis condition SQDFA05)

### Synthesis of (R)-3-(tert-butyldisulfanyl)-2-(methylamino)propanoic acid (Compound nk10, H-MeCys(StBu)-OH)

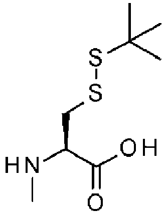

1,8-Diazabicyclo[5.4.0]-7-undecene (DBU) (1.12 mL, 7.41 mmol) was added to a solution of (R)-2-((((9*H*-fluoren-9-yl)methoxy)carbonyl)(methyl)amino)-3-(*tert*-butyldisulfanyl)propanoic acid (Fmoc-MeCys(StBu)-OH) (3.00 g, 6.73 mmol) in *N,N*-dimethylformamide (13.4 mL), and the mixture was stirred at room temperature for 30 minutes. The reaction solution was purified by reverse-phase silica gel column chromatography (0.1% aqueous formic acid solution/0.1% formic acid-acetonitrile) to give (R)-3-(*tert*-butyldisulfanyl)-2-(methylamino)propanoic acid (Compound nk10, H-MeCys(StBu)-OH) (1.46 g, 97%).
LCMS (ESI) m/z = 224 (M+H)+
Retention time: 0.34 minutes (analysis condition SQDFA05)

### Synthesis of (R)-2-((((4-azidobenzyl)oxy)carbonyl)(methyl)amino)-3-(tert-butyldisulfanyl)propanoic acid (Compound nk11, Acbz-MeCvs(StBu)-OH)

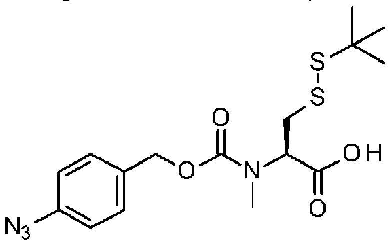

Under nitrogen atmosphere, DMF (3.13 mL) was added at room temperature to a mixture of (R)-3-(*tert*-butyldisulfanyl)-2-(methylamino)propanoic acid (Compound nk10, H-MeCys(StBu)-OH)) (700 mg, 3.13 mmol) and 4-azidobenzyl (4-nitrophenyl)carbonate (1.034 g, 3.29 mmol). The mixture was cooled in an ice bath, and then triethylamine (1.31 mL, 9.40 mmol) was added to it. The reaction mixture was stirred at 25°C for four days and then purified by reverse-phase silica gel column chromatography (0.1% aqueous formic acid solution/0.1% formic acid-acetonitrile solution) to give (R)-2-((((4-azidobenzyl)oxy)carbonyl)(methyl)amino)-3-(*tert*-butyldisulfanyl)propanoic acid (Compound nk11, Acbz-MeCys(StBu)-OH) (1.15 g, 92%).
LCMS (ESI) m/z = 397 (M-H)-
Retention time: 0.87 minutes (analysis condition SQDFA05)

### Synthesis of (R)-cyanomethyl 2-((((4-azidobenzyl)oxy)carbonyl)(methyl)amino)-3-(tert-butyldisulfanyl)propanoate (Compound nk12, Acbz-MeCys(StBu)-OCH₂CN)

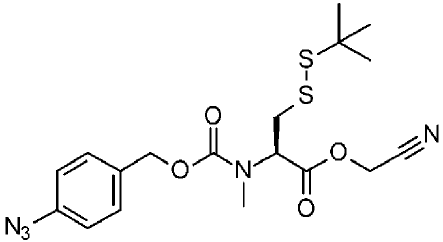

Under nitrogen atmosphere, (R)-2-((((4-azidobenzyl)oxy)carbonyl)(methyl)amino)-3-(*tert*-butyldisulfanyl)propanoic acid (Compound nk11, Acbz-MeCys(StBu)-OH) (1.14 g, 2.86 mmol) and *N*-ethyl-isopropylpropan-2-amine (DIPEA) (0.49 mL, 2.80 mmol) were dissolved in acetonitrile (5.72 mL), 2-bromoacetonitrile (0.598 mL, 8.58 mmol) was added, and the mixture was stirred at room temperature for seven hours. The reaction solution was concentrated, and the resulting residue was purified by column chromatography (ethyl acetate:hexane = 1:9 -> 1:1) to give (R)-cyanomethyl 2-((((4-azidobenzyl)oxy)carbonyl)(methyl)amino)-3-(*tert*-butyldisulfanyl)propanoate (Compound nk12, Acbz-MeCys(StBu)-OCH₂CN) (1.17 g, 93%).
LCMS (ESI) m/z = 436 (M-H)-
Retention time: 0.96 minutes (analysis condition SQDFA05)

### Synthesis of (2R)-(2R,3S,4R,5R)-2-((((((2R,3R,4R,5R)-5-(4-amino-2-oxopyrimidin-1(2H)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (Compound nk13)

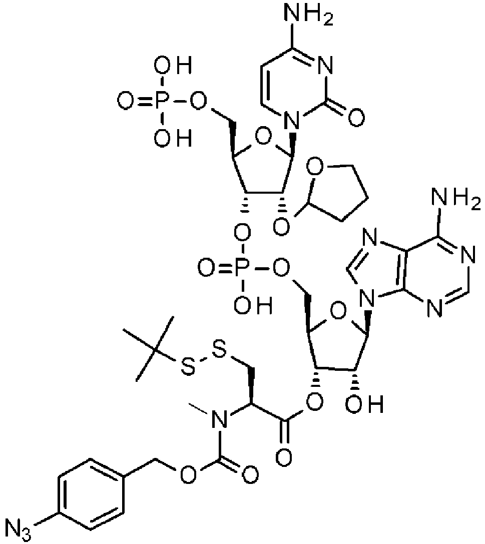

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (60.2 mg, 0.083 mmol) was dissolved in Buffer A (40 mL), a solution of (R)-cyanomethyl 2-((((4-azidobenzyl)oxy)carbonyl)(methyl)amino)-3-(*tert*-butyldisulfanyl)propanoate (Compound nk12, Acbz-MeCys(StBu)-OCH₂CN) (109 mg, 0.250 mmol) in acetonitrile (1.26 mL) was added to it in three portions (0.42-mL each was added at the start of the reaction, and five minutes and 30 minutes after starting the reaction, three times additions in total), and the mixture was stirred at room temperature for 120 minutes. The reaction solution was lyophilized, and the resulting residue was purified by reverse-phase silica gel column chromatography (0.1% aqueous formic acid solution/0.1% formic acid-acetonitrile) to give a mixture (90 mg) of the titled compound (Compound nk13) and *N,N,N*-trimethylhexadecan-1-aminium chloride.
LCMS (ESI) m/z = 1101 (M-H)-
Retention time: 0.69 minutes (analysis condition SQDFA05)

### Synthesis of (2R)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (Compound nk14, Acbz-MeCys(StBu)-pCpA)

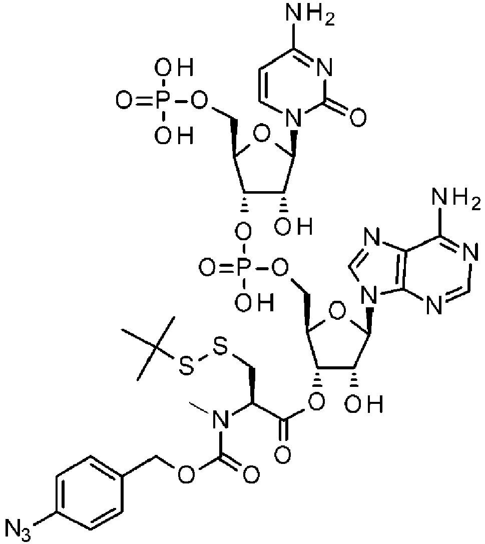

80% Aqueous acetic acid solution (5 mL) was added to the mixture (90 mg) of (2R)-(2R,3S,4R,5R)-2-((((((2R,3R,4R,5R)-5-(4-amino-2-oxopyrimidin-1(2*H*)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9*H*-purin-9-yl)-4-hydroxytetrahydrofuran-3-yl 2-((((4-azidobenzyl)oxy)carbonyl)(methyl)amino)-3-(*tert*-butyldisulfanyl)propanoate (Compound nk13) and *N,N,N*-trimethylhexadecane-1-aminium chloride obtained in the previous step and stirred at room temperature for 150 minutes. The reaction solution was lyophilized, and the resulting residue was purified by reverse-phase silica gel column chromatography (0.05% aqueous trifluoroacetic acid solution/0.05% trifluoroacetic acid-acetonitrile) to give the titled compound (Compound nk14, Acbz-MeCys(StBu)-pCpA) (26.7 mg, 31%, two steps).
LCMS (ESI) m/z = 1033 (M+H)+
Retention time: 0.65 minutes (analysis condition SQDFA05)

### Synthesis of (S)-3-(tert-butyldisulfanyl)-2-(methylamino)propanoic acid (Compound nk15, H-D-MeCys(StBu)-OH)

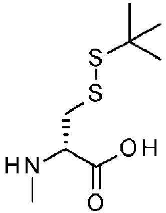

1,8-Diazabicyclo[5.4.0]-7-undecene (DBU) (0.186 mL, 1.234 mmol) was added to a solution of (S)-2-((((9*H*-fluoren-9-yl)methoxy)carbonyl)(methyl)amino)-3-(*tert*-butyldisulfanyl)propanoic acid (Fmoc-D-MeCys(StBu)-OH) (0.5 g, 1.12 mmol) in *N,N*-dimethylformamide (2.24 mL), and the mixture was stirred at room temperature for 90 minutes. The reaction solution was purified by reverse-phase silica gel column chromatography (0.1% aqueous formic acid solution/0.1% formic acid-acetonitrile) to give (S)-3-(*tert*-butyldisulfanyl)-2-(methylamino)propanoic acid (Compound nk15, H-D-MeCys(StBu)-OH) (0.22 g, 88%).
LCMS (ESI) m/z = 224 (M+H)+
Retention time: 0.38 minutes (analysis condition SQDFA05)

### Synthesis of (S)-2-((((4-azidobenzyl)oxy)carbonyl)(methyl)amino)-3-(tert-butyldisulfanyl)propanoic acid (Compound nk16, Acbz-D-MeCvs(StBu)-OH)

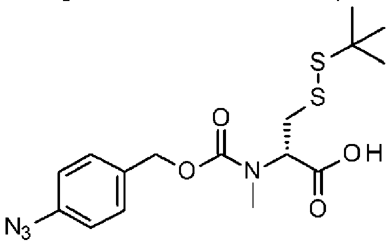

Under nitrogen atmosphere, DMF (0.90 mL) was added at room temperature to a mixture of (S)-3-(*tert*-butyldisulfanyl)-2-(methylamino)propanoic acid (Compound nk15, H-D-MeCys(StBu)-OH)) (200 mg, 0.90 mmol) and 4-azidobenzyl (4-nitrophenyl)carbonate (295 mg, 0.94 mmol). The mixture was cooled in an ice bath, and then triethylamine (374 µL, 2.69 mmol) was added to it. The reaction mixture was stirred at 40°C for two hours and then purified by reverse-phase silica gel column chromatography (0.1% aqueous formic acid solution/0.1% formic acid-acetonitrile solution) to give (S)-2-((((4-azidobenzyl)oxy)carbonyl)(methyl)amino)-3-(*tert*-butyldisulfanyl)propanoic acid (Compound nk16, Acbz-D-MeCys(StBu)-OH) (350 mg, 98%).
LCMS (ESI) m/z = 397 (M-H)-
Retention time: 0.90 minutes (analysis condition SQDFA05)

### Synthesis of (S)-cyanomethyl 2-((((4-azidobenzyl)oxy)carbonyl)(methyl)amino)-3-(tert-butyldisulfanyl)propanoate (Compound nk17, Acbz-D-MeCys(StBu)-OCH₂CN)

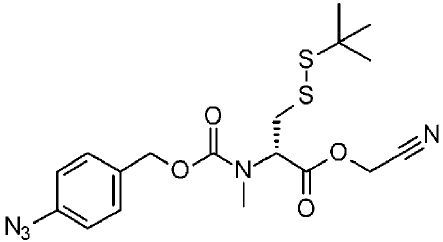

Under nitrogen atmosphere, (S)-2-((((4-azidobenzyl)oxy)carbonyl)(methyl)amino)-3-(*tert*-butyldisulfanyl)propanoic acid (Compound nk16, Acbz-D-MeCys(StBu)-OH) (350 mg, 0.88 mmol) and *N*-ethyl-isopropylpropan-2-amine (DIPEA) (0.15 mL, 0.86 mmol) were dissolved in acetonitrile (1.75 mL), 2-bromoacetonitrile (0.18 mL, 2.63 mmol) was added, and the mixture was stirred at room temperature for two hours. The reaction solution was concentrated to give (R)-cyanomethyl 2-((((4-azidobenzyl)oxy)carbonyl)(methyl)amino)-3-(*tert*-butyldisulfanyl)propanoate (Compound nk17, Acbz-D-MeCys(StBu)-OCH₂CN) (1.17 g) as a crude product. (R)-cyanomethyl 2-((((4-azidobenzyl)oxy)carbonyl)(methyl)amino)-3-(*tert*-butyldisulfanyl)propanoate (Compound nk17, Acbz-D-MeCys(StBu)-OCH₂CN) obtained as a crude product was dissolved in acetonitrile (4.4 mL) and was directly used in the next step.
LCMS (ESI) m/z = 436 (M-H)-
Retention time: 0.96 minutes (analysis condition SQDFA05)

### Synthesis of (2S)-(2R,3S,4R,5R)-2-((((((2R,3R,4R,5R)-5-(4-amino-2-oxopyrimidin-1(2H)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (Compound nk18)

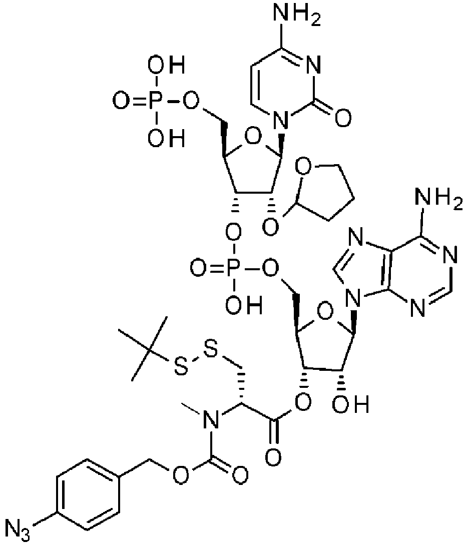

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (85.0 mg, 0.118 mmol) was dissolved in Buffer A (56.4 mL), a solution of (R)-cyanomethyl 2-((((4-azidobenzyl)oxy)carbonyl)(methyl)amino)-3-(*tert*-butyldisulfanyl)propanoate (Compound nk17, Acbz-D-MeCys(StBu)-OCH₂CN) in 0.2 M acetonitrile (1.77 mL, 0.353 mmol) was added to it in three portions (0.59-mL each was added at the start of the reaction, and ten minutes and 30 minutes after starting the reaction, three times additions in total), and the mixture was stirred at room temperature for 120 minutes. The reaction solution was lyophilized, and the resulting residue was purified by reverse-phase silica gel column chromatography (0.1% aqueous formic acid solution/0.1% formic acid-acetonitrile) to give a mixture (105.2 mg) of the titled compound (Compound nk18) and *N,N,N*-trimethylhexadecan-1-aminium chloride.
LCMS (ESI) m/z = 1101 (M-H)-
Retention time: 0.69 minutes (analysis condition SQDFA05)

### Synthesis of (2S)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((4-azidobenzyl)oxy)carbonyl)(methyl)amino)-3-(tert-butyldisulfanyl)propanoate (nk19, Acbz-D-MeCys(StBu)-pCpA)

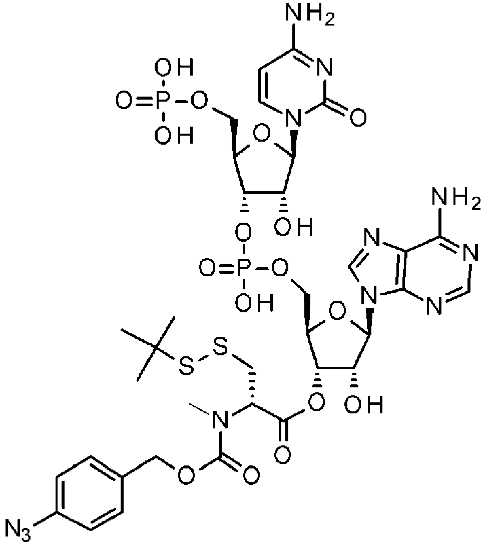

80% aqueous acetic acid solution (5 mL) was added to a mixture (100 mg) of (2S)-(2R,3S,4R,5R)-2-((((((2R,3R,4R,5R)-5-(4-amino-2-oxopyrimidin-1(2*H*)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9*H*-purin-9-yl)-4-hydroxytetrahydrofuran-3-yl 2-((((4-azidobenzyl)oxy)carbonyl)(methyl)amino)-3-(*tert*-butyldisulfanyl)propanoate (Compound nk18) and *N,N,N*-trimethylhexadecane-1-aminium chloride obtained in the previous step, and this was stirred for 80 minutes at room temperature. The reaction solution was lyophilized and the resulting residue was purified by reverse-phase silica gel column chromatography (0.05% aqueous trifluoroacetic acid solution/0.05% trifluoroacetic acid-acetonitrile) to give the titled compound (Compound nk19, Acbz-D-MeCys(StBu)-pCpA) (39.8 mg, 43%, two steps).
LCMS (ESI) m/z = 1033 (M+H)+
Retention time: 0.62 minutes (analysis condition SQDFA05)

### Synthesis of (R)-2-((((2-azidobenzyl)oxy)carbonyl)(methyl)amino)-3-(tert-butyldisulfanyl)propanoic acid (Compound nk20, o-Acbz-MeCys(StBu)-OH)

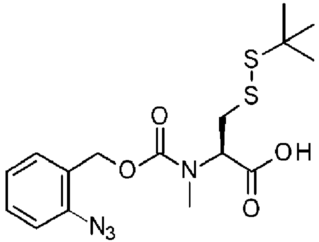

Under nitrogen atmosphere, DMF (0.5 mL) was added at room temperature to a mixture of (R)-3-*tert*-butyldisulfanyl)-2-(methylamino)propanoic acid (Compound nk10, H-MeCys(StBu)-OH) (112 mg, 0.50 mmol) and 2-azidobenzyl (4-nitrophenyl)carbonate (165 mg, 0.53 mmol). The mixture was cooled in an ice bath, and then triethylamine (0.21 mL, 1.50 mmol) was added to it. The reaction mixture was stirred at 30°C for four hours and then purified by reverse-phase silica gel column chromatography (0.1% aqueous formic acid solution/0.1% formic acid-acetonitrile solution) to give (R)-2-((((2-azidobenzyl)oxy)carbonyl)(methyl)amino)-3-(*tert*-butyldisulfanyl)propanoic acid (Compound nk20, *o*-Acbz-MeCys(StBu)-OH) (197 mg, 99%).
LCMS (ESI) m/z = 397 (M-H)-
Retention time: 0.91 minutes (analysis condition SQDFA05)

### Synthesis of (R)-cyanomethyl 2-((((2-azidobenzyl)oxy)carbonyl)(methyl)amino)-3-(tert-butyldisulfanyl)propanoate (Compound nk21, o-Acbz-MeCys(StBu)-OCH₂CN)

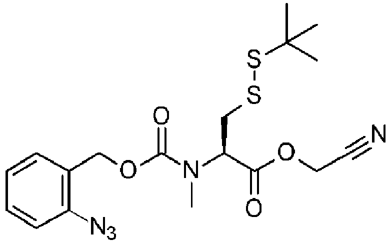

Under nitrogen atmosphere, (R)-2-((((2-azidobenzyl)oxy)carbonyl)(methyl)amino)-3-(*tert*-butyldisulfanyl)propanoic acid (Compound nk20, *o*-Acbz-MeCys(StBu)-OH) (196 mg, 0.49 mmol) and *N*-ethyl-isopropylpropan-2-amine (DIPEA) (0.084 mL, 0.48 mmol) were dissolved in acetonitrile (984 µL), 2-bromoacetonitrile (0.103 mL, 1.48 mmol) was added, and the mixture was stirred at room temperature for three hours. The reaction solution was concentrated to give (R)-cyanomethyl 2-((((2-azidobenzyl)oxy)carbonyl)(methyl)amino)-3-(*tert*-butyldisulfanyl)propanoate (Compound nk21, *o*-Acbz-MeCys(StBu)-OCH₂CN) as a crude product. (R)-cyanomethyl 2-((((2-azidobenzyl)oxy)carbonyl)(methyl)amino)-3-(*tert*-butyldisulfanyl)propanoate (Compound nk21, *o*-Acbz-MeCys(StBu)-OCH₂CN) obtained as a crude product was dissolved in acetonitrile (2.46 mL) and was directly used in the next step.
LCMS (ESI) m/z = 438 (M+H)+
Retention time: 1.00 minute (analysis condition SQDFA05)

### Synthesis of (2R)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-((((2-azidobenzyl)oxy)carbonyl)(methyl)amino)-3-(tert-butyldisulfanyl)propanoate (Compound nk22, o-Acbz-MeCys(StBu)-pCpA)

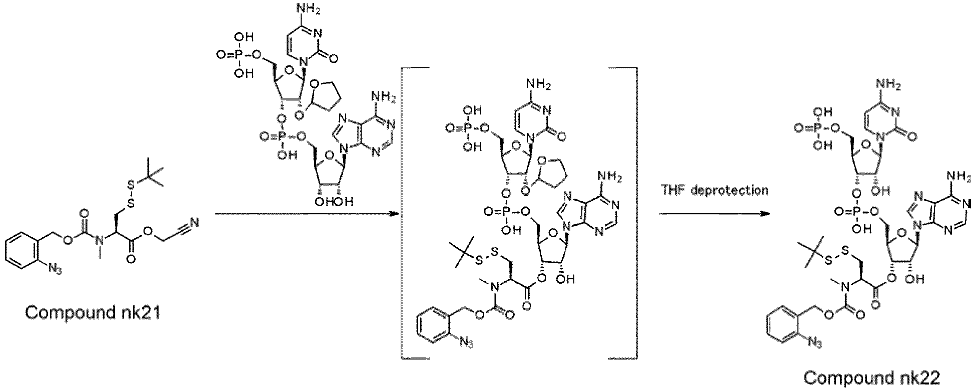

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (60.2 mg, 0.083 mmol) was dissolved in Buffer A (40 mL), a solution of (R)-cyanomethyl 2-((((2-azidobenzyl)oxy)carbonyl)(methyl)amino)-3-(*tert*-butyldisulfanyl)propanoate (Compound nk21, *o*-Acbz-MeCys(StBu)-OCH₂CN) in 0.2 M acetonitrile (1.25 mL, 0.25 mmol) was added to it in three portions (0.42-mL each was added at the start of the reaction, and five minutes and 30 minutes after starting the reaction, three times additions in total), and the mixture was stirred at room temperature for 90 minutes. The reaction solution was cooled to 0°C, and then trifluoroacetic acid (2 mL) was added to it. After stirring the reaction solution at 0°C for ten minutes, this was purified by reverse-phase silica gel column chromatography (0.05% aqueous trifluoroacetic acid solution/0.05% trifluoroacetic acid-acetonitrile) to give the titled compound (Compound nk22, o-Acbz-MeCys(StBu)-pCpA) (26.3 mg, 31%).
LCMS (ESI) m/z = 1033 (M+H)+
Retention time: 0.60 minutes (analysis condition SQDFA05)

### 1-2. Synthesis of pCpA-amino acids for translation elongation Synthesis of (2S)-2-(N-methylpent-4-enamido)-3-(1,3-thiazol-4-yl)propanoic acid (Compound nk23, Pen-MeAla(4-Thz)-OH)

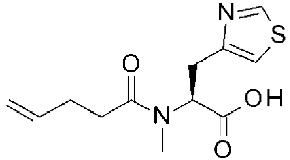

A 20% solution of piperidine in DMF (100 mL) was added to (S)-2-(((9*H*-fluoren-9-yl)methoxy)carbonyl)(methyl)amino)-3-(thiazol-4-yl)propanoic acid (Fmoc-MeAla(4-Thz)-OH) (4.20 g, 10.28 mmol), and this was stirred at room temperature for two hours. The reaction solution was concentrated under reduced pressure, diethyl ether was added to the concentrated residue, this mixture was filtered and washed with diethyl ether to obtain (2S)-2-(methylamino)-3-(1,3-thiazol-4-yl)propanoic acid as a crude product.

After dissolving the obtained (2S)-2-(methylamino)-3-(1,3-thiazol-4-yl)propanoic acid in 1,4-dioxane (40 mL)/water (40 mL), 2,5-dioxopyrrolidin-1-yl pent-4-enoate (3.200 g, 16.23 mmol) and sodium hydrogen carbonate (1.80 g, 21.43 mmol) were added. Then, the reaction solution was stirred at 30°C for twelve hours. After completion of the reaction, water was added to the reaction solution, this was washed with ethyl acetate, and a 1 M aqueous sodium hydrogen sulfate solution was added until the pH of the aqueous layer reached 2. The resulting mixture was extracted with ethyl acetate, and the organic layer was washed with saturated saline solution. The organic layer was dried over anhydrous sodium sulfate, filtered, and then concentrated under reduced pressure. The resulting residue was purified by normal-phase silica gel column chromatography (dichloromethane/methanol) to give (2S)-2-(*N*-methylpent-4-enamido)-3-(1,3-thiazol-4-yl)propanoic acid (Compound nk23, Pen-MeAla(4-Thz)-OH) (0.60 g, 22%).
LCMS (ESI) m/z = 269 (M+H)+
Retention time: 1.32 minutes (analysis condition SMD method 1)

### Synthesis of (S)-cyanomethyl 2-(N-methylpent-4-enamido)-3-(thiazol-4-yl)propanoate (Compound nk24, Pen-MeAla (4-Thz)-OCH₂CN)

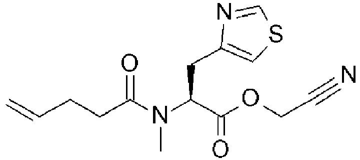

Under nitrogen atmosphere, (2S)-2-(*N*-methylpent-4-enamido)-3-(1,3-thiazol-4-yl)propanoic acid (Compound nk23, Pen-MeAla(4-Thz)-OH) (1.50 g, 2.80 mmol) and *N*-ethyl-isopropylpropan-2-amine (DIPEA) (680 mg, 5.26 mmol) were dissolved in dichloromethane (80 mL), 2-bromoacetonitrile (1.25 g, 10.42 mmol) was added to it, and the mixture was stirred overnight at room temperature. The reaction solution was concentrated, and the resulting residue was purified by normal-phase silica gel column chromatography (petroleum ether/ethyl acetate) to give (S)-cyanomethyl 2-(*N*-methylpent-4-enamido)-3-(thiazol-4-yl)propanoate (Compound nk24, Pen-MeAla(4-Thz)-OCH₂CN) (0.23 g, 27%).
LCMS (ESI) m/z = 308 (M+H)+
Retention time: 1.61 minutes (analysis condition SMD method 1)

### Synthesis of (2S)-(2R,3S,4R,5R)-2-((((((2R,3R,4R,5R)-5-(4-amino-2-oxopyrimidin-1(2H)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9H-purin-9-yl)-4-hydroxytetrahydrofuran-3-yl 2-(N-methylpent-4-enamido)-3-(thiazol-4-yl)propanoate (Compound nk25)

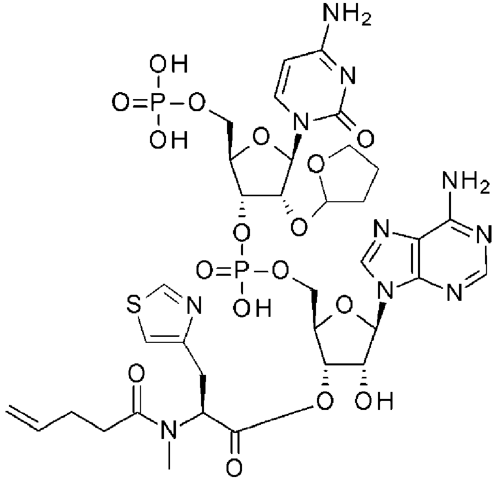

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (300 mg, 0.415 mmol) was dissolved in Buffer A (120 mL), a solution of (S)-cyanomethyl 2-(*N*-methylpent-4-enamido)-3-(thiazol-4-yl)propanoate (Compound nk24, Pen-MeAla(4-Thz)-OCH₂CN) (311 mg, 1.013 mmol) in acetonitrile (3.6 mL) was added to it, and the mixture was stirred at room temperature for four hours. The reaction solution was lyophilized and the obtained residue was purified by reverse-phase silica gel column chromatography (10 mM aqueous ammonium acetate solution/methanol) to give a mixture (300 mg) of the titled compound (Compound nk25) and *N,N,N*-trimethylhexadecan-1-aminium chloride.
LCMS (ESI) m/z = 971 (M-H)-
Retention time: 0.59 minutes (analysis condition SQDAA05)

### Synthesis of (2S)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 2-(N-methylpent-4-enamido)-3-(thiazol-4-yl)propanoate (Compound nk26, Pen-MeAla(4-Thz)-pCpA)

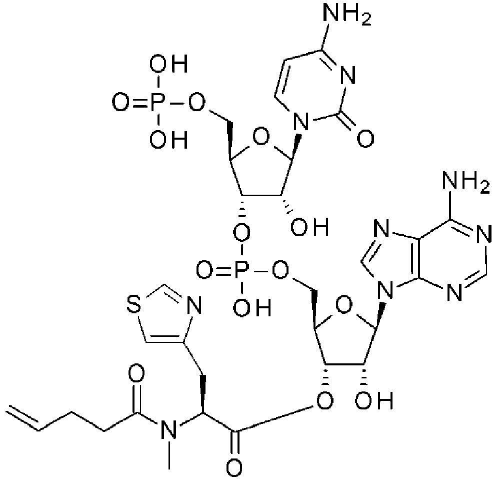

80% Aqueous acetic acid solution (6 mL) was added to the mixture (300 mg) of (2S)-(2R,3S,4R,5R)-2-((((((2R,3R,4R,5R)-5-(4-amino-2-oxopyrimidin-1(2*H*)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9*H*-purin-9-yl)-4-hydroxytetrahydrofuran-3-yl 2-(*N*-methylpent-4-enamido)-3-(thiazol-4-yl)propanoate (Compound nk25) and *N,N,N*-trimethylhexadecane-1-aminium chloride obtained in the previous step and this was stirred at room temperature for seven hours. The reaction solution was lyophilized and the resulting residue was purified by reverse-phase silica gel column chromatography (0.05% aqueous trifluoroacetic acid solution/0.05% trifluoroacetic acid-acetonitrile) to give the titled compound (Compound nk26, Pen-MeAla(4-Thz)-pCpA) (46 mg, 17%, two steps).
LCMS (ESI) m/z = 903 (M+H)+
Retention time: 0.37 minutes (analysis condition SQDFA05)

### Synthesis of cyanomethyl 2-(N-methylpent-4-enamido)acetate (Compound nk27, Pen-MeGly-OCH₂CN)

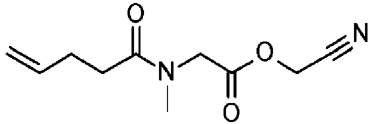

*N*-methyl glycine (1.5 g, 16.8 mmol) and *N*-ethyl-isopropylpropan-2-amine (DIPEA) (7.35 mL, 42.1 mmol) were added to dichloromethane (33.7 mL). Subsequently, the mixture was cooled to 0°C, then 2,5-dioxopyrrolidin-1-yl pent-4-enoate (1.95 mL, 17.7 mmol) was added, and the reaction solution was stirred at 25°C for three days. After completion of the reaction, *N*-ethyl-isopropylpropan-2-amine (DIPEA) (2.94 mL, 16.8 mmol) and 2-bromoacetonitrile (2.35 mL, 33.7 mmol) were added to the reaction mixture, and this was stirred at 25°C for four hours. The reaction solution was diluted with dichloromethane, then a saturated aqueous ammonium chloride solution was added, and the organic layer was washed with saturated saline solution. Subsequently, the organic layer was dried over anhydrous sodium sulfate, filtered, and then concentrated under reduced pressure, and the resulting residue was purified by normal-phase silica gel column chromatography (hexane/ethyl acetate) to give cyanomethyl 2-(*N*-methylpent-4-enamido)acetate (Compound nk27, Pen-MeGly-OCH₂CN) (1.1 g, 31%).
LCMS (ESI) m/z = 211 (M+H)+
Retention time: 0.72 minutes (analysis condition SMD method 3)

### Synthesis of (2R,3S,4R,5R)-2-((((((2R,3S,4R,5R)-5-(4-amino-2-oxopyrimidin-1(2H)-yl)-4-hydroxy-2-((phos phonooxy)methyl)tetrahydrofuran-3-yl)oxy)(hydroxy)phosphoryl)oxy)methyl)-5-(6-amino-9H-p urin-9-yl)-4-hydroxytetrahydrofuran-3-yl N-methyl-N-(pent-4-enoyl)glycinate (Compound nk28, Pen-MeGly-pCpA)

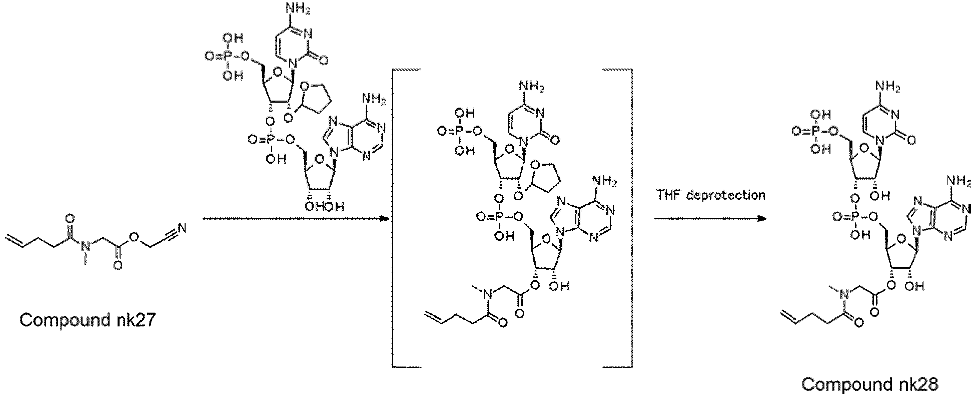

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (250 mg, 0.35 mmol) was dissolved in Buffer A (100 mL), a solution of cyanomethyl 2-(*N*-methylpent-4-enamido)acetate (Compound nk27, Pen-MeGly-OCH₂CN) (290 mg, 1.38 mmol) in acetonitrile (1.5 mL) was added to it, and the mixture was stirred at room temperature for four hours. Furthermore, an 80% aqueous acetic acid solution (15 mL) was added to the reaction solution and this was stirred at room temperature for four hours.

The reaction solution was purified by reverse-phase silica gel column chromatography (0.05% aqueous trifluoroacetic acid solution/0.05% trifluoroacetic acid-acetonitrile) to give the titled compound (Compound nk28, Pen-MeGly-pCpA) (37.6 mg, 13%).
LCMS (ESI) m/z = 806 (M+H)+
Retention time: 0.60 minutes (analysis condition SMD method 2)

### 1-3. Synthesis of pCpA-amino acids for the intersection unit

### Synthesis of (2S)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 4-(methylthio)-4-oxo-2-(pent-4-enamido)butanoate (Compound nk30, Pen-AspSMe-pCpA)

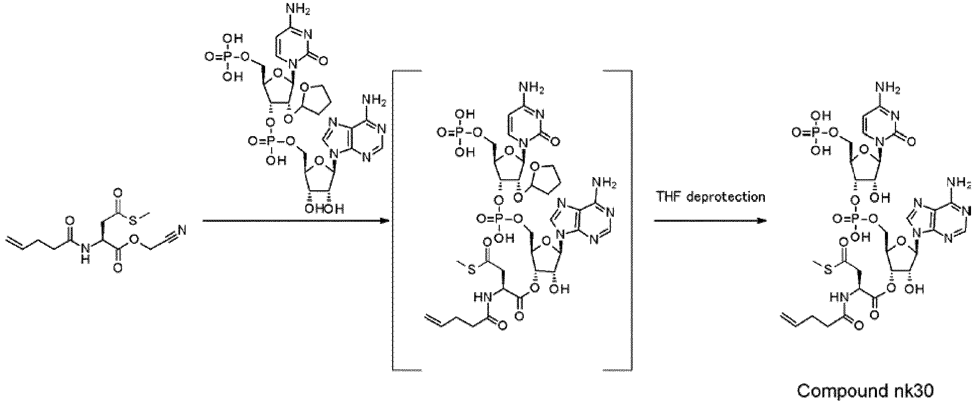

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (400 mg, 0.55 mmol) was dissolved in Buffer A (200 mL), a solution of (S)-cyanomethyl 4-(methylthio)-4-oxo-2-(pent-4-enamido)butanoate (630 mg, 2.22 mmol) synthesized by the method described in the literature (WO 2013/100132 A) in THF (4.0 mL) was added to it, and the mixture was stirred at room temperature for one hour. Furthermore, trifluoroacetic acid (4.6 mL, 60 mmol) was added to the reaction solution, the reaction solution was lyophilized, and the resulting residue was purified by reverse-phase silica gel column chromatography (0.05% aqueous trifluoroacetic acid solution/0.05% trifluoroacetic acid-acetonitrile) to give the titled compound (Compound nk30, Pen-AspSMe-pCpA) (20 mg, 4.2%).
LCMS (ESI) m/z = 880.4 (M+H)+
Retention time: 0.38 minutes (analysis condition SQDFA05)

### [Example 2] Synthesis of aminoacyl-tRNAs for translation initiation, which carry cysteine or a cysteine analog

### 2-1. Synthesis of tRNA (lacking CA) by transcription

tRNAfMetCAU(-CA) (sequence number: R-1) lacking 3'-end CA was synthesized from template DNA (sequence number: D-1) by *in vitro* transcription using RiboMAX Large Scale RNA production System T7 (Promega, P1300) and purified with RNeasy Mini kit (Qiagen).
Sequence number: D-1 (SEQ ID NO: 1):
tRNAfMetCAT(-CA) DNA sequence: 
GGCGTAATACGACTCACTATAGGCGGGGTGGAGCAGCCTGGTAGCTCGTCGGGCTCAT
AACCCGAAGATCGTCGGTTCAAATCCGGCCCCCGCAAC
 Sequence number: R-1 (SEQ ID NO: 2):
tRNAfMetCAU(-CA) RNA sequence:

GGCGGGGUGGAGCAGCCUGGUAGCUCGUCGGGCUCAUAACCCGAAGAUCGUCGGU
UCAAAUCCGGCCCCCGCAAC

### 2-2. Synthesis of aminoacyl-tRNAs using pCpA aminoacylated with cysteine or a cysteine analog

10x ligation buffer (500 mM HEPES-KOH (pH 7.5), 200 mM MgCl₂) (4 µL), 10 mM ATP (4 µL), and nuclease free water (5.6 µL) were added to 50 µM transcribed tRNAfMetCAU(-CA) (sequence number: R-1) (20 µL). The mixture was heated at 95°C for two minutes and then incubated at room temperature for five minutes, and the tRNA was refolded. 10 units/µL T4 RNA ligase (New England Biolabs) (2.4 µL) and a 5 mM solution of aminoacylated pCpA (nk-05, 09, 14, 19, or 22) in DMSO (4 µL) were added, and ligation reaction was carried out at 16°C for 45 minutes. Aminoacylated tRNA (Compound AAtR-1, 2, 3, 4, or 5) was collected by phenol-chloroform extraction and ethanol precipitation. Aminoacylated tRNA (Compound AAtR-1, 2, 3, 4, or 5) was dissolved in 1 mM sodium acetate immediately before the addition to the translation mixture.

### Compound AAtR-1

Acbz-Cys(StBu)-tRNAfMetCAU (SEQ ID NO: 3)

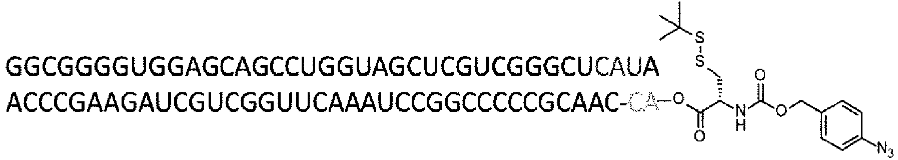
GGCGGGGUGGAGCAGCCUGGUAGCUCGUCGGGCUCAUA
ACCCGAAGAUCGUCGGUUCAAAUCCGGCCCCCGCAAC-CA-

### Compound AAtR-2

Acbz-D-Cys(StBu)-tRNAfMetCAU (SEQ ID NO: 3)

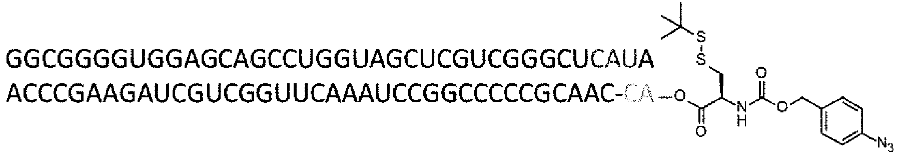
GGCGGGGUGGAGCAGCCUGGUAGCUCGUCGGGCUCAUA
ACCCGAAGAUCGUCGGUUCAAAUCCGGCCCCCGCAAC-CA

### Compound AAtR-3

Acbz-MeCys(StBu)-tRNAfMetCAU (SEQ ID NO: 3)

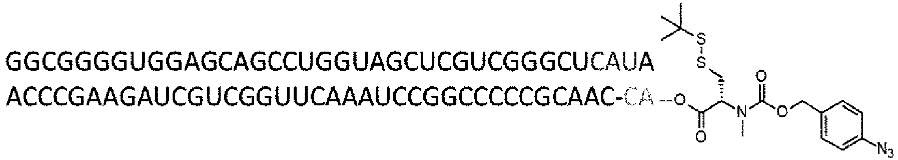
GGCGGGGUGGAGCAGCCUGGUAGCUCGUCGGGCUCAUA
ACCCGAAGAUCGUCGGUUCAAAUCCGGCCCCCGCAAC-CA
S
S
O
O
N
O
O
N3

### Compound AAtR-4

Acbz-D-MeCys(StBu)-tRNAfMetCAU (SEQ ID NO: 3)

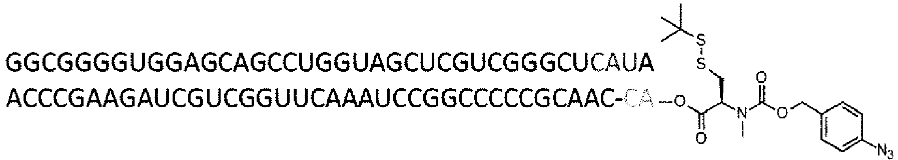
GGCGGGGUGGAGCAGCCUGGUAGCUCGUCGGGCUCAUA
ACCCGAAGAUCGUCGGUUCAAAUCCGGCCCCCGCAAC-CA

### Compound AAtR-5

o-Acbz-MeCys(StBu)-tRNAfMetCAU (SEQ ID NO: 3)

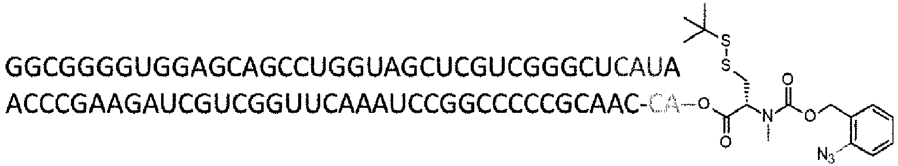
GGCGGGGUGGAGCAGCCUGGUAGCUCGUCGGGCUCAUA
ACCCGAAGAUCGUCGGUUCAAAUCCGGCCCCCGCAAC-CA-
S
S
O
O
N
O
O
O
N3

### [Example 3] Ribosomal synthesis of a peptide to which cysteine or a cysteine analog with a protecting group was attached to each of its amino group and thiol group, using the initiation suppression method

As shown in the following experiments, it was demonstrated that cysteine or a cysteine analog having an amino group protected by an Acbz group and a thiol group protected by StBu can be translationally incorporated at the N terminus. More specifically, it was shown that not only L-cysteine, but also a cysteine analog including D-cysteine, or L- or D-*N*-methyl cysteine can be translationally incorporated at the N terminus. WO 2013/100132 A1 has shown that the Acbz group which is an amino protecting group can be selectively deprotected under reaction conditions where RNAs can stably exist, and conditions necessary for efficiently synthesizing a library of peptides with amide cyclization are satisfied.

### 3-1. Ribosomal synthesis of peptides having cysteine or a cysteine analog at their N terminus, using the initiation suppression method

To confirm that cysteine or a cysteine analog is translationally incorporated at the N terminus using the initiation suppression method, tRNAfMet aminoacylated with cysteine or a cysteine analog was added to a cell-free translation system to carry out ribosomal synthesis. The translation system used was PURE system, an E. coli-derived reconstituted cell-free protein synthesis system. Specifically, the synthesis was carried out by adding 1 µM template mRNA and 250 µM each of amino acids, Thr, Arg, Lys, Ala, Tyr, Trp, Ser, Leu, Pro, and Gly, to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 6 mM magnesium acetate, 2 mM spermidine, 1 mM dithiothreitol, 1.5 mg/mL *E. coli* MRE600 (RNase-negative)-derived tRNA (Roche), 0.1 mM 10-HCO-H4 folate, 4 µg/ml creatine kinase, 3 µg/ml myokinase, 2 units/ml inorganic pyrophosphatase, 1.1 µg/ml nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), and 20 µM aminoacyl-tRNAfMet (Acbz-Cys(StBu)-tRNAfMetCAU (Compound AAtR-1), Acbz-D-Cys(StBu)-tRNAfMetCAU (Compound AAtR-2), or Acbz-MeCys(StBu)-tRNAfMetCAU (Compound AAtR-3)) for translation initiation was further added to the translation solution mixture, this was incubated at 37°C for one hour.

### <Detection by mass spectrometry>

MALDI-TOF MS was used for mass spectrometric analyses of the peptides ribosomally synthesized in the cell-free translation system. Specifically, the analyses were carried out through preparation of a solution by adding 1 µM template mRNA (R-4) and Thr, Arg, Lys, Ala, Tyr, Trp, Ser, Leu, Pro, and Gly as each amino acid (each at a final concentration of 250 µM) to the above-described cell-free translation system, and also 20 µM aminoacyl-tRNAfMet (Acbz-Cys(StBu)-tRNAfMetCAU (Compound AAtR-1), Acbz-D-Cys(StBu)-tRNAfMetCAU (Compound AAtR-2), or Acbz-MeCys(StBu)-tRNAfMetCAU (Compound AAtR-3)) for translation initiation.

It was reported in WO 2013/100132A1 that removal of the Acbz protecting group takes place during MALDI measurement or during treatment operations prior to the measurement. Therefore, in translation experiments using Acbz-Cys(StBu)-tRNAfMetCAU (Compound AAtR-1), Acbz-D-Cys(StBu)-tRNAfMetCAU (Compound AAtR-2), and Acbz-MeCys(StBu)-tRNAfMetCAU (Compound AAtR-3), respectively, mass spectrometric analyses by MALDI-TOF MS were performed after deprotection under reductive conditions using Tris(2-carboxyethyl)phosphine (TCEP). Specifically, a Tris(2-carboxyethyl)phosphine (TCEP) solution (pH 7.0) (final concentration of 20 mM) was added to the obtained translation reaction product, and then this was incubated at 37°C for one hour. Translation products were analyzed by MALDI-TOF MS using α-cyano-4-hydroxycinnamic acid (CHCA) as the matrix.

As a result, MS peaks indicating the peptides introduced with cysteine and a cysteine analog at the N terminus (Table 2, Peptide sequence numbers: Pep-3, Pep-4, and Pep-5), respectively, were observed. Furthermore, an MS peak indicating a peptide resulting from skipping the first letter (Table 2, Peptide sequence number: Pep-2) was observed as a side product.

Furthermore, as control experiments, a translation solution to which 250 µM Met was added instead of aminoacyl-tRNA for translation initiation, and a translation solution to which aminoacyl-tRNA for translation initiation was not added were prepared, and they were incubated at 37°C for one hour. Translation reaction products were analyzed by MALDI-TOF MS. The matrix used in the MALDI-TOF MS was α-cyano-4-hydroxycinnamic acid (CHCA). When 250 µM Met was used instead of aminoacyl-tRNA for translation initiation, an MS peak indicating the peptide starting from an N-terminal formylmethionine (hereinafter, formylmethionine is abbreviated as fMet) (Table 2, Peptide sequence number: Pep-1) was observed. Furthermore, when aminoacyl-tRNA for translation initiation was not added, an MS peak indicating the peptide in which the first letter was skipped (Table 2, Peptide sequence number: Pep-2) due to initiation read-through (Patent Document (WO 2013/100132A1)) was observed.
Template mRNA sequence number: R-4 (SEQ ID NO: 4)
RNA sequence: 
GGGUUAACUUUAAGAAGGAGAUAUACAUAUGACUCGUACUAAGGCUUACUGGAG
UCUUCCGGGUUAAGCUUCG
 Peptide sequence
[Xaa]ThrArgThrLysAlaTyrTrpSerLeuProGly

**[Table 2]**

| Aminoacyl -tRNAfMetCAU (Compound No.) | Peptide sequence number | Peptide sequence | MS theoretical value | MS measured value[M+H]+ |
|---|---|---|---|---|
| | | [Xaa]ThrArgThrLysAlaTyrTrpSerLeuProGly | | |
| | Pep-1 | fMetThrArgThrLysAlaTyrTrpSerLeuProGly (SEQ ID NO:3 2) | 1437.7 | 1438.6 |
| | Pep-2 | ThrArgThrLysAlaTyrTrpSerLeuProGly (SEQ ID NO:3 3) | 1278.7 | 1279.3 |
| Acbz-Cys(StBu) (AAtR-1) | Pep-3 | [Cys]ThrArgThrLysAlaTyrTrpSerLeuProGly (SEQ ID NO:3 4) | 1381.7 | 1382.5 |
| Acbz-D-Cys(StBu) (AAtR-2) | Pep-4 | [D-Cys]ThrArgThrLysAlaTyrTrpSerLeuProGly | 1381.7 | 1382.5 |
| Acbz-MeCys(StBu) (AAtR-3) | Pep-5 | [MeCys]ThrArgThrLysAlaTyrTrpSerLeuProGly | 1395.7 | 1396.3 |

### <Detection by electrophoresis>

For detection of peptides into which cysteine or a cysteine analog is translationally incorporated at the N terminus, radioisotope-labeled aspartic acid was used to perform peptide translation experiments. Specifically, the experiments were carried out as follows: a solution in which 1 µM template mRNA (R-4D), Thr, Arg, Lys, Ala, Tyr, Trp, Ser, Leu, Pro, and Gly as each amino acid (each at a final concentration of 250 µM), and ¹⁴C-aspartic acid (final concentration of 37 µM, Moravek Biochemicals, MC139) was added to the above-described cell-free translation system was prepared; 20 µM tRNAfMet aminoacylated with cysteine or a cysteine analog (Acbz-Cys(StBu)-tRNAfMetCAU (Compound AAtR-1),
Acbz-D-Cys(StBu)-tRNAfMetCAU (Compound AAtR-2), or
Acbz-MeCys(StBu)-tRNAfMetCAU (Compound AAtR-3)) was added to the translation solution mixture, and this was incubated at 37°C for one hour.

Furthermore, as control experiments, a translation solution to which 250 µM Met was added instead of aminoacyl-tRNA for translation initiation and a translation solution to which aminoacyl-tRNA for translation initiation was not added were prepared, and they were incubated at 37°C for one hour.

An equivalent amount of 2x sample buffer (TEFCO, cat No. 06-323) was added to the obtained translation reaction solution, this was heated at 95°C for three minutes, and then subjected to electrophoresis (16% Peptide-PAGE mini, TEFCO, TB-162). The electrophoresed gel was dried using a Clear Dry Quick Dry Starter KIT (TEFCO, 03-278), exposed for 24 hours to an Imaging Plate (GE Healthcare, 28-9564-75), subjected to detection using a bioanalyzer system (Typhoon FLA 7000, GE Healthcare), and analyzed using ImageQuantTL (GE Healthcare).

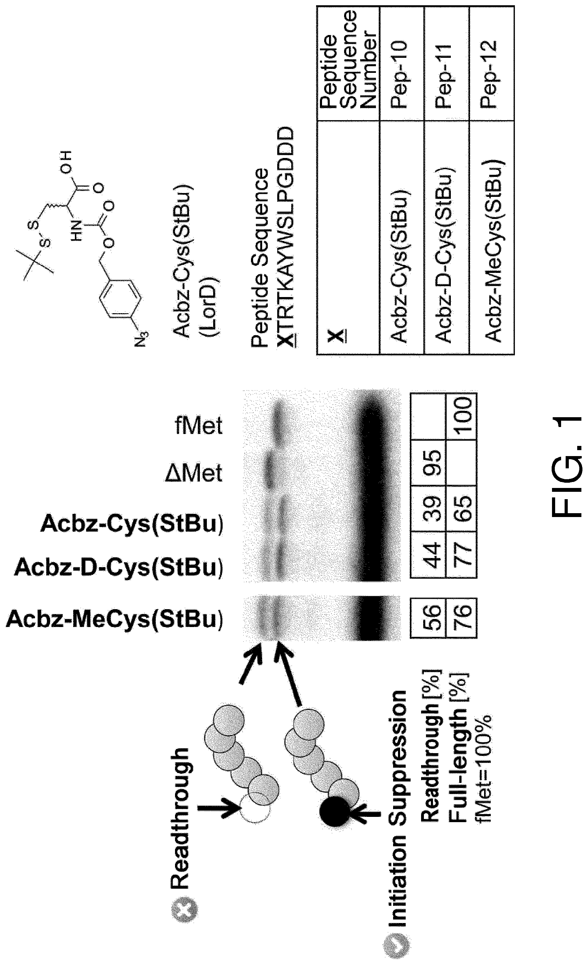

When Acbz-Cys(StBu), Acbz-D-Cys(StBu), and Acbz-MeCys(StBu) were respectively translationally incorporated at the N terminus, bands for the desired peptides (Peptide sequence numbers: Pep-10, 11, 12, and 15) were detected respectively as the major products (in Fig. 1, the relative translation efficiencies when the translation efficiency of the peptide started by formylmethionine (Peptide sequence number: Pep-8), which was used as a control experiment, is set as 100). Furthermore, a band indicating the peptide in which the first letter was skipped (Peptide sequence number: Pep-9), produced as a side product, was observed.
Template mRNA sequence number: R-4D (SEQ ID NO: 5)
RNA sequence: 
GGGUUAACUUUAAGAAGGAGAUAUACAUAUGACUCGUACUAAGGCUUACUGGAG
UCUUCCGGGUGACGACGACUAAGCUUCG
 Peptide sequence
[Xaa]ThrArgThrLysAlaTyrTrpSerLeuProGlyAspAspAsp

**[Table 3]**

| Aminoacyl -tRNAfMetCAU (Compound No.) | Peptide sequence number | Peptide sequence |
|---|---|---|
| | Pep-8 | MetThrArgThrLysAlaTyrTrpSerLeuProGlyAspAspAsp (SEQ ID NO:3 5) |
| | Pep-9 | ThrArgThrLysAlaTyrTrpSerLeuProGlyAspAspAsp (SEQ ID NO:3 6) |
| Acbz-Cys(StBu) (AAtR-1) | Pep-10 | [Acbz-Cys(StBu)]ThrArgThrLysAlaTyrTrpSerLeuProGlyAspAspAsp |
| Acbz-D-Cys(StBu) (AAtR-2) | Pep-11 | [Acbz-D-Cys(StBu)]ThrArgThrLysAlaTyrTrpSerLeuProGlyAspAspA sp |
| Acbz-MeCys(StBu) (AAtR-3) | Pep-12 | [Acbz-MeCys(StBu)]ThrArgThrLysAlaTyrTrpSerLeuProGlyAspAspA sp |

### 3-2. Ribosomal synthesis of peptides having an N-methyl cysteine at their N terminus using the initiation suppression method under ribosomal synthesis conditions with reduced production of side-products

As described above, when translationally synthesizing a peptide containing cysteine or a cysteine analog at its N terminus by the initiation suppression method, a peptide in which translation was initiated from an amino acid encoded by the second codon and then translated over the full length was observed as a side product. Next, the translation conditions were optimized to improve the translation efficiency of the peptide of interest by reducing the production of the side product. Specifically, the concentration conditions for the aminoacyl-tRNA for translation initiation (1, 2, 5, 10, and 25 µM) were examined, and translation conditions yielding the best production ratio between the peptide of interest and the side product were set.

To confirm whether cysteine or a cysteine analog is translationally incorporated at the N terminus using the initiation suppression method, tRNAfMet aminoacylated with *N*-methyl cysteine having a protecting group was added to a cell-free translation system and ribosomal synthesis was carried out. The translation system used was PURE system, an *E. coli*-derived reconstituted cell-free protein synthesis system. Specifically, the synthesis was carried out by adding 1 µM template mRNA and 250 µM each of Thr, Arg, Lys, Ala, Tyr, Trp, Ser, Leu, Pro, and Gly to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 10 mM magnesium acetate, 2 mM spermidine, 1 mM dithiothreitol, 0.5 mg/mL *E*. *coli* MRE600 (RNase-negative)-derived tRNA (Roche), 0.1 mM 10-HCO-H4 folate, 4 µg/ml creatine kinase, 3 µg/ml myokinase, 2 units/mL inorganic pyrophosphatase, 1.1 µg/mL nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), and upon further adding tRNAfMet aminoacylated with *N*-methyl cysteine to the translation solution mixture, this was incubated at 37°C for one hour.

### <Detection by mass spectrometry>

MALDI-TOF MS was used for mass spectrometric analyses of the peptides ribosomally synthesized in the cell-free translation system. Specifically, the analyses were carried out through preparation of a solution by adding 1 µM template mRNA (R-4) and Thr, Arg, Lys, Ala, Tyr, Trp, Ser, Leu, Pro, and Gly as each amino acid (each at a final concentration of 250 µM) to the above-described cell-free translation system, and 2 or 25 µM tRNAfMet aminoacylated with an *N*-methyl cysteine analog (Acbz-MeCys(StBu)-tRNAfMetCAU (Compound AAtR-3), Acbz-D-MeCys(StBu)-tRNAfMetCAU (Compound AAtR-4), or o-Acbz-MeCys(StBu)-tRNAfMetCAU (Compound AAtR-5)) was added to the translation solution mixture, and this was incubated at 37°C for one hour.

Tris(2-carboxyethyl)phosphine (TCEP) (final concentration of 20 mM) was added to the obtained translation reaction product, and then this was incubated at 37°C for one hour. Translation products were analyzed by MALDI-TOF MS using α-cyano-4-hydroxycinnamic acid (CHCA) as the matrix.

As a result, when 25 µM each of Acbz-MeCys(StBu)-tRNAfMetCAU (Compound AAtR-3) and Acbz-D-MeCys(StBu)-tRNAfMetCAU (Compound AAtR-4) were used, MS peaks indicating the peptides introduced with NMe cysteine or NMe-D-cysteine at the N terminus (Table 4, Peptide sequence numbers: Pep-5 and Pep-6), respectively, were observed as the major products (Figs. 2-1 and 2-2). Furthermore, when 2 µM each of
Acbz-MeCys(StBu)-tRNAfMetCAU (Compound AAtR-3) and
Acbz-D-MeCys(StBu)-tRNAfMetCAU (Compound AAtR-4) were used, an MS peak indicating a peptide in which the first letter was skipped (Table 2, Peptide sequence number: Pep-2) was observed as the main product (Figs. 2-1 and 2-2). MS peaks indicating the peptides into which NMe-cysteine or NMe-D-cysteine was introduced at the N terminus (Table 4, Peptide sequence numbers: Pep-5 and 6) were observed; however, their MS intensity ratios were 1/10 or less than that of the peptide in which the first letter was skipped (Table 1, Peptide sequence number: Pep-2).

Furthermore, when 25 µM *o*-Acbz-MeCys(StBu)-tRNAfMetCAU (Compound AAtR-5) was used, an MS peak indicating a peptide into which NMe-cysteine was introduced at the N-terminus (Table 4, Peptide sequence number: Pep-5) was observed as the main product (Fig. 2-3).

**[Table 4]**

| Aminoacyl -tRNAfMetCAU (Compound No.) | Peptide sequence number | Peptide sequence | MS theoretical value | MS measured value[M+H]+ |
|---|---|---|---|---|
| | | [Xaa]ThrArgThrLysAlaTyrTrpSerLeuProGly | | |
| Acbz-MeCys(StBu) (AAtR-3) | Pep-5 | [MeCys]ThrArgThrLysAlaTyrTrpSerLeuProGly | 1395.7 | 1396.3 |
| Acbz-D-MeCys(StBu) (AAtR-4) | Pep-6 | [D-MeCys]ThrArgThrLysAlaTyrTrpSerLeuProGl y | 1395.7 | 1396.3 |
| o-Acbz-MeCys(StBu) (AAtR-5) | Pep-5 | [MeCys]ThrArgThrLysAlaTyrTrpSerLeuProGly | 1395.7 | 1396.3 |
| | Pep-2 | ThrArgThrLysAlaTyrTrpSerLeuProGly | 1278.7 | 1279.3 |

### <Detection by electrophoresis>

For detection of peptides into which cysteine or a cysteine analog is translationally incorporated at the N terminus, radioisotope-labeled aspartic acid was used to perform peptide translation experiments. Specifically, the experiments were carried out as follows: a solution to which 1 µM template mRNA (R-4D), Thr, Arg, Lys, Ala, Tyr, Trp, Ser, Leu, Pro, and Gly as each amino acid (each at a final concentration of 250 µM), and ¹⁴C-aspartic acid (final concentration of 37 µM, Moravek Biochemicals, MC139) were added to the above-described cell-free translation system was prepared, and tRNAfMet aminoacylated with cysteine or a cysteine analog (performed under five conditions, 1, 2, 5, 10, and 25 µM) were added to the translation solution mixture, this was incubated at 37°C for one hour. Furthermore, as control experiments, a translation solution to which 250 µM Met was added instead of aminoacyl-tRNA for translation initiation and a translation solution to which aminoacyl-tRNA for translation initiation was not added were prepared, and they were incubated at 37°C for one hour.

An equivalent amount of 2x sample buffer (TEFCO, cat No. 06-323) was added to the obtained translation reaction solution, this was heated at 95°C for three minutes, and then subjected to electrophoresis (16% Peptide-PAGE mini, TEFCO, TB-162). The electrophoresed gel was dried using a Clear Dry Quick Dry Starter KIT (TEFCO, 03-278), exposed for 24 hours to an Imaging Plate (GE Healthcare, 28-9564-75), subjected to detection using a bioanalyzer system (Typhoon FLA 7000, GE Healthcare), and analyzed using ImageQuantTL (GE Healthcare).

In the ribosomal syntheses of Acbz-MeCys(StBu) and Acbz-D-MeCys(StBu), both cases showed the highest translation efficiency for the peptide of interest and lowest translation efficiency for the side product when the aminoacyl-tRNA concentration was 25 µM (Fig. 3-1). Specifically, for Acbz-MeCys(StBu), the production ratio of the peptide of interest (Peptide sequence number: Pep-12) and the side product (Peptide sequence number: Pep-9) was 96:33 (this shows the relative translation efficiency when the translation efficiency of the peptide started with formylmethionine (Peptide sequence number: Pep-8), a control experiment, is set as 100). Furthermore, for Acbz-D-MeCys(StBu), the production ratio of the peptide of interest (Peptide sequence number: Pep-13) and the side product (Peptide sequence number: Pep-9) was 56:22. In addition, for o-Acbz-MeCys(StBu), the production ratio of the peptide of interest (Peptide sequence number: Pep-14) and the side product (Peptide sequence number: Pep-9) was 109:16 (Fig. 3-2).

**[Table 5]**

| Aminoacyl -tRNAfMetCAU (Compound No.) | Peptide sequence number | Peptide sequence |
|---|---|---|
| | | [Xaa]ThrArgThrLysAlaTyrTrpSerLeuProGlyAspAspAsp |
| | Pep-8 | MetThrArgThrLysAlaTyrTrpSerLeuProGlyAspAspAsp |
| | Pep-9 | ThrArgThrLysAlaTyrTrpSerLeuProGlyAspAspAsp |
| Acbz-MeCys(StBu) (AAtR-3) | Pep-12 | [Acbz-MeCys(StBu)]ThrArgThrLysAlaTyrTrpSerLeuProGlyAspA spAsp |
| Acbz-D-MeCys(StBu) (AAtR-4) | Pep-13 | [Acbz-D-MeCys(StBu)]ThrArgThrLysAlaTyrTrpSerLeuProGlyAs pAspAsp |
| o-Acbz-MeCys(StBu) (AAtR-5) | Pep-14 | [o-Acbz-MeCys(StBu)]ThrArgThrLysAlaTyrTrpSerLeuProGlyAs pAspAsp |

### [Example 4] Ribosomal synthesis for evaluating the product purity and translation efficiency of an amino acid analog in translation elongation reaction depending on the method of initiating translation

Synthesis of tRNAs aminoacylated with amino acid analogs were performed according to the methods described below.

### 4-1-1. Synthesis of tRNA (lacking CA) by transcription

tRNAGluCUG(-CA) (sequence number: R-2) lacking 3'-end CA was synthesized from template DNA (sequence number: D-2) by *in vitro* transcription using RiboMAX Large Scale RNA production System T7 (Promega, P1300) and purified with RNeasy Mini kit (Qiagen).

### Sequence number: D-2 (SEQ ID NO: 6)

tRNAGluCTG(-CA) DNA sequence:

GGCGTAATACGACTCACTATAGTCCCCTTCGTCTAGAGGCCCAGGACACCGCCCTCTG
ACGGCGGTAACAGGGGTTCGAATCCCCTAGGGGACGC

### Sequence number: D-3 (SEQ ID NO: 7)

tRNAGluAAG(-CA) DNA sequence:

GGCGTAATACGACTCACTATAGTCCCCTTCGTCTAGAGGCCCAGGACACCGCCCTAAG
ACGGCGGTAACAGGGGTTCGAATCCCCTAGGGGACGC

### Sequence number: R-2 (SEQ ID NO: 8)

tRNAGluCUG(-CA) RNA sequence:

GUCCCCUUCGUCUAGAGGCCCAGGACACCGCCCUCUGACGGCGGUAACAGGGGUU
CGAAUCCCCUAGGGGACGC

### Sequence number: R-3 (SEQ ID NO: 9)

tRNAGluAAG(-CA) RNA sequence:

GUCCCCUUCGUCUAGAGGCCCAGGACACCGCCCUAAGACGGCGGUAACAGGGGUU
CGAAUCCCCUAGGGGACGC

### 4-1-2. Synthesis of aminoacyl-tRNA by reaction between a tRNA (lacking CA) and a pCpA-amino acid

10x ligation buffer (500 mM HEPES-KOH (pH 7.5), 200 mM MgCl₂) (4 µL), 10 mM ATP (4 µL), and nuclease free water (5.6 µL) were added to 50 µM transcribed tRNAGluCUG(-CA) (sequence number: R-2) or tRNAGluAAG(-CA) (sequence number: R-3) (20 µL). The mixture was heated at 95°C for two minutes and then incubated at room temperature for five minutes to perform the refolding of tRNA. 10 units/µL T4 RNA ligase (New England Biolabs) (2.4 µL) and a 5 mM solution of aminoacylated pCpA (nk-026, 28, or 30) in DMSO (4 µL) were added, and ligation reaction was carried out at 16°C for 45 minutes. A 3 M sodium acetate solution (4 µL) and 44 µL of 62.5 mM iodine (solution in water:THF = 1:1) were added to 40 µL of the ligation reaction solution, and deprotection was carried out at room temperature for one hour. Aminoacylated tRNA (Compound AAtR-7, 8, or 9) was collected by phenol-chloroform extraction followed by ethanol precipitation. Aminoacylated tRNA (Compound AAtR-7, 8, or 9) was dissolved in 1 mM sodium acetate immediately before adding to the translation mixture.

### Compound AAtR-7 (SEQ ID NO: 10)

MeAla(4-Thz)-tRNAGluCUG

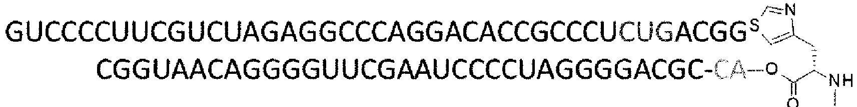
GUCCCCUUCGUCUAGAGGCCCAGGACACCGCCCUCUGACGG
CGGUAACAGGGGUUCGAAUCCCCUAGGGGACGC-CA-O

### Compound AAtR-8

MeGly-tRNAGluCUG (SEQ ID NO: 10)

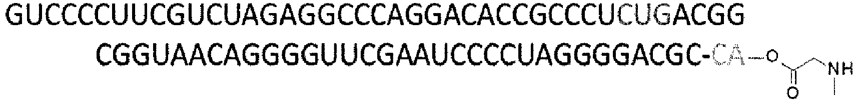
GUCCCCUUCGUCUAGAGGCCCAGGACACCGCCCUCUGACGG
CGGUAACAGGGGUUCGAAUCCCCUAGGGGACGC-CA-O
NH

### Compound AAtR-9 (SEQ ID NO: 11)

AspSMe-tRNAGluAAG

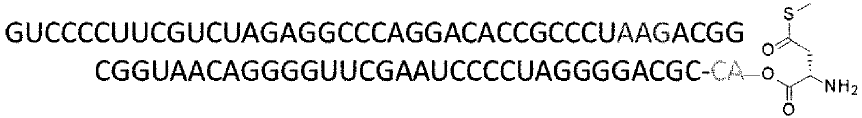
GUCCCCUUCGUCUAGAGGCCCAGGACACCGCCCUAAGACGG
CGGUAACAGGGGUUCGAAUCCCCUAGGGGACGC-CA-O
S
O
NH2
O

Next, experiments were performed to confirm how product purity and translation efficiency of an amino acid analog are affected in translation elongation reaction depending on the method of initiating translation. Specifically, the same peptide compound (peptide sequence number: Pep-17) containing multiple amino acid analogs was ribosomally synthesized using the initiation suppression method or the initiation read-through method. MS analyses were performed on the translation solutions containing the product, and translation efficiencies were compared by the MS intensities for the product of interest, which was the same peptide compound. Furthermore, to confirm the product purity, MS intensities for the cleaved peptides included in the products were compared.

### 4-2-1. Ribosomal synthesis of peptides having cysteine at the N terminus using the initiation read-through method

After ribosomal synthesis of a peptide into which Cys was translationally incorporated at the N terminus using the initiation read-through method, purity of the peptide obtained from the ribosomal synthesis was confirmed using MALDI-TOF MS.

The translation system used was PURE system, an *E*. *coli*-derived reconstituted cell-free protein synthesis system. Specifically, the synthesis was carried out as follows: 1 µM template mRNA (R-5-2 (SEQ ID NO: 17)), 250 µM each of amino acids, Ala, Arg, Cys, Gly, His, Ile, Lys, Pro, Ser, Thr, Trp, Val, and 3-fluoro-L-tyrosine (Tyr(3-F)), were added to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 6 mM magnesium acetate, 2 mM spermidine, 1 mM dithiothreitol, 1.5 mg/mL *E. coli* MRE600 (RNase-negative)-derived tRNA (Roche), 0.1 mM 10-HCO-H4 folate, 4 µg/ml creatine kinase, 3 µg/ml myokinase, 2 units/mL inorganic pyrophosphatase, 1.1 µg/mL nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), 5mM *N*-methyl phenylalanine (MePhe) as well as 20 µM MeAla(4-Thz)-tRNAGluCUG (Compound AAtR-7) were added to the translation solution mixture, and this was incubated at 37°C for one hour.

The obtained translation reaction products were analyzed by MALDI-TOF MS using α -cyano-4-hydroxycinnamic acid (CHCA) as the matrix. As a result, while an MS peak for the substance of interest into which Cys was introduced at the N terminus (Peptide sequence number: Pep-17) was observed, MS peaks for two types of cleaved peptides (Peptide sequence numbers: Pep-19 and Pep-21) were also observed (Fig. 4). The MS intensity ratio for the respective peptides was the following: substance of interest (Peptide sequence number: Pep-17):cleaved peptide (Peptide sequence number: Pep-19):cleaved peptide (Peptide sequence number: Pep-21) = 1:1:1
Template mRNA sequence number: R-5-2 (SEQ ID NO: 17)
RNA sequence:

GGGUUAACUUUAAGAAGGAGAUAUACAUAUGUGCAGUUUCCGAGUCAGAUUGU
UUACACUGGUCGUCCGUAAGCUUCG

**[Table 6]**

| | Peptide sequence number | Peptide sequence | MS theoretical value | MS measured value [M+H]+ |
|---|---|---|---|---|
| Target compound | Pep-17 | CysSer[MePhe]ProSer[MeA4Tz]IleVal[Tyr(3-F)]ThrGly ArgPro | 1525. 7 | 1526. 3 |
| Cleaved peptide | Pep-19 | [MePhe]ProSer[MeA4Tz]IleVal[Tyr(3-F)]ThrGlyArgPro | 1335. 6 | 1336.3 |
| Cleaved peptide | Pep-21 | [MeA4Tz]IleVal[Tyr(3-F)]ThrGlyArgPro | 990.5 | 991.2 |

### 4-2-2. Ribosomal synthesis of a peptide having cysteine at the N terminus using the initiation suppression method

After ribosomally synthesizing a peptide into which Acbz-Cys(StBu) was translationally incorporated at the N terminus using the initiation suppression method, Acbz was deprotected using Tris(2-carboxyethyl)phosphine (TCEP). Purity of the peptide obtained from the ribosomal synthesis was confirmed using MALDI-TOF MS.

The translation system used was PURE system, an *E. coli*-derived reconstituted cell-free protein synthesis system. Specifically, the synthesis was carried out as follows: 1 µM template mRNA (R-5-1 (SEQ ID NO: 12)) and 250 µM each of each amino acids, Ala, Arg, Cys, Gly, His, Ile, Lys, Pro, Ser, Thr, Trp, Val, and 3-fluoro-L-tyrosine (Tyr(3-F)), were added to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 6 mM magnesium acetate, 2 mM spermidine, 1 mM dithiothreitol, 1.5 mg/mL *E. coli* MRE600 (RNase-negative)-derived tRNA (Roche), 0.1 mM 10-HCO-H4 folate, 4 µg/ml creatine kinase, 3 µg/ml myokinase, 2 units/mL inorganic pyrophosphatase, 1.1 µg/mL nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), 5mM *N*-methyl phenylalanine (MePhe) as well as 20 µM Acbz-Cys(StBu)-tRNAfMetCAU (Compound AAtR-1) and 20 µM MeAla(4-Thz)-tRNAGluCUG (Compound AAtR-7) were added to the translation solution mixture, and this was incubated at 37°C for one hour.

Tris(2-carboxyethyl)phosphine (TCEP) (final concentration of 20 mM) was added to the obtained translation reaction product, and then this was incubated at 37°C for one hour. Translation products were analyzed by MALDI-TOF MS using α-cyano-4-hydroxycinnamic acid (CHCA) as the matrix.

As a result, an MS peak for the substance of interest into which Cys was introduced at the N terminus (Peptide sequence number: Pep-17) was observed as the main peak, and the MS intensity was 28 times that observed in the initiation read-through method (Fig. 4).

Furthermore, while MS peaks for the four types of cleaved peptides (Peptide sequence numbers: Pep-18, Pep-19, Pep-20, and Pep-21) were observed, each of their MS intensities were 1/20 or less than that of the substance of interest (Peptide sequence number: Pep-17).
Template mRNA sequence number: R-5-1 (SEQ ID NO: 12)
RNA sequence:

GGGUUAACUUUAAGAAGGAGAUAUACAUAUGAGUUUCCGAGUCAGAUUGUUUA
CACUGGUCGUCCGUAAGCUUCG

**[Table 7]**

| | Peptide sequence number | Peptide sequence | MS theoretical value | MS measured value[M+H]+ |
|---|---|---|---|---|
| Target compound | Pep-17 | CysSer[MePhe]ProSer[MeA4Tz]IleVal[Tyr(3-F)]ThrGlyArgPro | 1525.7 | 1526.3 |
| Cleaved peptide | Pep-18 | Ser[MePhe]ProSer[MeA4Tz]IleVal[Tyr(3-F)]ThrGlyArgPro | 1422.7 | 1423.3 |
| Cleaved peptide | Pep-19 | [MePhe]ProSer[MeA4Tz]IleVal[Tyr(3-F)]ThrGlyArgPro | 1335.6 | 1336.3 |
| Cleaved peptide | Pep-20 | ProSer[MeA4Tz]IleVal[Tyr(3-F)]ThrGlyArgPro | 1174.6 | 1175.3 |
| Cleaved peptide | Pep-21 | [MeA4Tz]IleVal[Tyr(3-F)]ThrGlyArgPro | 990.5 | 991.2 |

The above-mentioned results show that use of the initiation suppression method can enable more efficient and high-purity ribosomal synthesis of peptides having cysteine at the N terminus and containing multiple amino acid analogs.

### 4-3-1. Ribosomal synthesis of peptides having a cysteine or a cysteine analog at the N terminus using the initiation suppression method or the initiation read-through method

Experiments performed to confirm whether the above-mentioned observed phenomena can be observed when ribosomally synthesizing other peptides are shown below. As a control experiment, 250 µM Met was added instead of Acbz-Cys(StBu)-tRNAfMetCAU (Compound AAtR-1), and peptides in which the N terminus is initiated from fMet were ribosomally synthesized. The template mRNA sequences used in the ribosomal syntheses are indicated in Table 8 shown below.

**[Table 8]**

| Template mRNA sequence number | Sequence |
|---|---|
| R-5-1 (SEQ ID NO:12) | GGGUUAACUUUAAGAAGGAGAUAUACAUAUGAGUUUUCCGAGUCAGAUUGUUUACACUGGUCGUCCG UAAGCUUCG |
| R-6-1 (SEQ ID NO:13) | GGGUUAACUUUAAGAAGGAGAUAUACAUAUGAGUUUUGUUCAGAUUUUUAGUGUUAUUCGUCCGUAA GCUUCG |
| R-7-1 (SEQ ID NO:14) | GGGUUAACUUUAAGAAGGAGAUAUACAUAUGACUACUCCGUUUGGUCAGAGUUACAUUGUUUACCGU CCGUAAGCUUCG |
| R-8-1 (SEQ ID NO:15) | GGGUUAACUUUAAGAAGGAGAUAUACAUAUGUUUGUUACUCAGGCUACUUACGGUAUUACUCGUCCG UAAGCUUCG |
| R-9-1 (SEQ ID NO:16) | GGGUUAACUUUAAGAAGGAGAUAUACAUAUGAGUUUUUUGUUCAGAGUUACAGUGUUAUUCGUCCG UAAGCUUCG |
| R-5-2 (SEQ ID NO:17) | GGGUUAACUUUAAGAAGGAGAUAUACAUAUGUGCAGUUUCCGAGUCAGAUUGUUUACACUGGUCGU CCGUAAGCUUCG |
| R-6-2 (SEQ ID NO:18) | GGGUUAACUUUAAGAAGGAGAUAUACAUAUGUGCAGUUUGUUCAGAUUUUAGUGUUAUUCGUCCG UAAGCUUCG |
| R-7-2 (SEQ ID NO:19) | GGGUUAACUUUAAGAAGGAGAUAUACAUAUGUGCACUACUCCGUUUGGUCAGAGUUACAUUGUUUAC CGUCCGUAAGCUUCG |
| R-8-2 (SEQ ID NO:20) | GGGUUAACUUUAAGAAGGAGAUAUACAUAUGUGCUUUGUUACUCAGGCUACUUACGGUAUUACUCGU CCGUAAGCUUCG |
| R-9-2 (SEQ ID NO:21) | GGGUUAACUUUAAGAAGGAGAUAUACAUAUGUGCAGUUUUUUGUUCAGAGUUACAGUGUUAUUCGU CCGUAAGCUUCG |

### 4-3-1-1. Ribosomal synthesis of peptides having a cysteine at the N terminus by using the initiation read-through method

The translation system used was PURE system, an *E. coli-*derived reconstituted cell-free protein synthesis system. Specifically, the synthesis was carried out as follows: 1 µM template mRNA (R-6-2 (SEQ ID NO: 18), R-7-2 (SEQ ID NO: 19), R-8-2 (SEQ ID NO: 20), R-9-2 (SEQ ID NO: 21), or R-5-2 (SEQ ID NO: 17)) and 250 µM each of amino acids, Arg, Cys, Gly, His, Ile, Lys, Pro, Ser, Thr, Trp, Val, and 3-fluoro-L-tyrosine (Tyr(3-F)), were added to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 6 mM magnesium acetate, 2 mM spermidine, 1 mM dithiothreitol, 1.5 mg/mL *E. coli* MRE600 (RNase-negative)-derived tRNA (Roche), 0.1 mM 10-HCO-H4 folate, 4 µg/ml creatine kinase, 3 µg/ml myokinase, 2 units/mL inorganic pyrophosphatase, 1.1 µg/mL nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), 5mM each of *N*-methyl phenylalanine (MePhe) and *N*-methyl alanine (MeAla), as well as 20 µM MeGly-tRNAGluCUG (Compound AAtR-8) (SEQ ID NO: 10) were added to the translation solution mixture, and this was incubated at 37°C for one hour.

The obtained translation reaction products were analyzed by MALDI-TOF MS using α -cyano-4-hydroxycinnamic acid (CHCA) as the matrix. The sequence number of the template mRNA used in the ribosomal synthesis, the theoretical MS values for the ribosomally synthesized substances of interest and cleaved peptides, and the measured MALDI-TOF MS values are shown in Tables 9 to 13. Furthermore, the MS intensities for the ribosomally synthesized substance of interest and cleaved peptides are shown in Fig. 5. In the graph showing the MS intensities of the cleaved peptides in Fig. 5, when multiple cleaved peptides were observed, the total values of MS intensities of each of cleaved peptides were presented.

**[Table 9]**

| Template mRNA sequence number R-6-2 | | | | |
|---|---|---|---|---|
| | Peptide sequence number | Sequence | MS theoretical value | MS measured value [M+H]+ |
| Target compound | Pep-23 | CysSer[MePhe]Val[MeGly]Ile[MePhe]SerValIleArgPro | 1365.7 | 1366.5 |
| Cleaved peptide | Pep-24 | Ser[MePhe]Val[MeGly]Ile[MePhe]SerValIleArgPro | 1262.7 | 1263.5 |
| Cleaved peptide | Pep-25 | [MePhe]Val[MeGly]Ile[MePhe]SerValIleArgPro | 1175.7 | 1176.5 |
| Cleaved peptide | Pep-26 | [MeGly]Ile[MePhe]SerValIleArgPro | 915.6 | 916.4 |

**[Table 10]**

| Template mRNA sequence number R-7-2 | | | | |
|---|---|---|---|---|
| | Peptide sequence number | Sequence | MS theoretical value | MS measured value[M+H]+ |
| Target compound | Pep-28 | CysThrThrPro[MePhe]Gly[MeGly]Ser[Tyr(3-F)]Ile Val[Tyr(3-F)]ArgPro | 1623.8 | 1624.4 |
| Cleaved peptide | Pep-29 | [MePhe]Gly[MeGly]Ser[Tyr(3-F)]IleVal[Tyr(3-F) ]ArgPro | 1221.6 | 1222.3 |
| Cleaved peptide | Pep-30 | [MeGly]Ser[Tyr(3-F)]IleVal[Tyr(3-F)]ArgPro | 1003.5 | 1004.3 |

**[Table 11]**

| Template mRNA sequence number R-8-2 | | | | |
|---|---|---|---|---|
| | Peptide sequence number | Sequence | MS theoretical value | MS measured value[M+H] + |
| Target compound | Pep-32 | Cys[MePhe]ValThr[MeGly][MeAla]Thr[Tyr(3-F)]Gl yIleThrArgPro | 1444.7 | 1445.3 |
| Cleaved peptide | Pep-33 | [MePhe]ValThr[MeGly][MeAla]Thr[Tyr(3-F)]GlyIl eThrArgPro | 1341.7 | 1342.4 |

**[Table 12]**

| Template mRNA sequence number R-9-2 | | | | |
|---|---|---|---|---|
| | Peptide sequence number | Sequence | MS theoretical value | MS measured value[M+H]+ |
| Target compound | Pep-35 | CysSer[MePhe][MePhe]Val[MeGly]Ser[Tyr(3-F)]Se rValIleArgPro | 1520.7 | 1521.3 |
| Cleaved peptide | Pep-36 | Ser[MePhe][MePhe]Val[MeGly]Ser[Tyr(3-F)]SerVa lIleArgPro | 1417.7 | 1418.4 |
| Cleaved peptide | Pep-37 | [MePhe]Val[MeGly]Ser[Tyr(3-F)]SerValIleArgPro | 1169.6 | 1170.3 |

**[Table 13]**

| Template mRNA sequence number R-5-2 | | | | |
|---|---|---|---|---|
| | Peptide sequence number | Sequence | theoretical value | MS measured value[M+H]+ |
| Target compound | Pep-39 | CysSer[MePhe]ProSer[MeGly]IleVal[Tyr(3-F)]Thr GlyArgPro | 1428.7 | 1429.3 |
| Cleaved peptide | Pep-40 | Ser[MePhe]ProSer[MeGly]IleVal[Tyr(3-F)]ThrGly ArgPro | 1325.7 | 1326.4 |
| Cleaved peptide | Pep-41 | [MePhe]ProSer[MeGly]IleVal[Tyr(3-F)]ThrGlyArg Pro | 1238.6 | 1239.4 |
| Cleaved peptide | Pep-42 | [MeGly]IleVal[Tyr(3-F)]ThrGlyArgPro | 893.5 | 894.3 |

### 4-3-1-2. Ribosomal synthesis of a peptide having cysteine at the N terminus using the initiation suppression method

After ribosomally synthesizing a peptide into which Acbz-Cys(StBu) was translationally incorporated at the N terminus using the initiation suppression method, Acbz was deprotected using Tris(2-carboxyethyl)phosphine (TCEP). Purity of the peptide obtained from the ribosomal synthesis was confirmed by subjecting the obtained reaction mixture to mass spectrometry using MALDI.

The translation system used was PURE system, an *E. coli*-derived reconstituted cell-free protein synthesis system. Specifically, the synthesis was carried out as follows: 1 µM template mRNA (R-6-1 (SEQ ID NO: 18), R-7-1 (SEQ ID NO: 19), R-8-1 (SEQ ID NO: 20), R-9-1 (SEQ ID NO: 21), or R-5-1 (SEQ ID NO: 17)) and 250 µM each of amino acids, Arg, Cys, Gly, His, Ile, Lys, Pro, Ser, Thr, Trp, Val, and 3-fluoro-L-tyrosine (Tyr(3-F)), were added to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 6 mM magnesium acetate, 2 mM spermidine, 1 mM dithiothreitol, 1.5 mg/mL *E. coli* MRE600 (RNase-negative)-derived tRNA (Roche), 0.1 mM 10-HCO-H4 folate, 4 µg/ml creatine kinase, 3 µg/ml myokinase, 2 units/mL inorganic pyrophosphatase, 1.1 µg/mL nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), 5mM each of *N*-methyl phenylalanine (MePhe) and *N*-methyl alanine (MeAla), as well as 20 µM MeGly-tRNAGluCUG (Compound AAtR-8) (SEQ ID NO: 10) and 20 µM Acbz-Cys(StBu)-tRNAfMetCAU (Compound AAtR-1) were added to the translation solution mixture, and this was incubated at 37°C for one hour.

The obtained translation reaction products were analyzed by MALDI-TOF MS using α -cyano-4-hydroxycinnamic acid (CHCA) as the matrix. The sequence numbers of the template mRNAs used in the ribosomal synthesis, the theoretical MS values for the ribosomally synthesized substance of interest and cleaved peptides, and the measured MALDI-TOF MS values are shown in Tables 14 to 18. Furthermore, the MS intensities for the ribosomally synthesized substance of interest and cleaved peptides are shown in Fig. 5. In the graph showing the MS intensities of the cleaved peptides in Fig. 5, when multiple cleaved peptides were observed, the total values of the MS intensities of each of cleaved peptides were presented.

**[Table 14]**

| Template mRNA sequence number R-6-1 | | | | |
|---|---|---|---|---|
| | Peptide sequence number | Sequence | MS theoretical value | MS measured value[M+H]+ |
| Target compound | Pep-23 | CysSer[MePhe]Val[MeGly]Ile[MePhe]SerValIleArgPro | 1365.7 | 1366.5 |
| Cleaved peptide | Pep-24 | Ser[MePhe]Val[MeGly]Ile[MePhe]SerValIleArgPro | 1262.7 | 1263.5 |
| Cleaved peptide | Pep-25 | [MePhe]Val[MeGly]Ile[MePhe]SerValIleArgPro | 1175.7 | 1176.5 |
| Cleaved peptide | Pep-26 | [MeGly]Ile[MePhe]SerValIleArgPro | 915.6 | 916.4 |

**[Table 15]**

| Template mRNA sequence number R-7-1 | | | | |
|---|---|---|---|---|
| | Peptide sequence number | Sequence | MS theoretical value | MS measured value[M+H]+ |
| Target compound | Pep-28 | CysThrThrPro[MePhe]Gly[MeGly]Ser[Tyr(3-F)]IleVa l[Tyr(3-F)]ArgPro | 1623.8 | 1624.4 |
| Cleaved peptide | Pep-29 | [MePhe]Gly[MeGly]Ser[Tyr(3-F)]IleVal[Tyr(3-F)]A rgPro | 1221.6 | 1222.3 |
| Cleaved peptide | Pep-30 | [MeGly]Ser[Tyr(3-F)]IleVal[Tyr(3-F)]ArgPro | 1003.5 | 1004.3 |

**[Table 16]**

| Template mRNA sequence number R-8-1 | | | | |
|---|---|---|---|---|
| | Peptide sequence number | Sequence | MS theoretical value | MS measured value[M+H]+ |
| Target compound | Pep-32 | Cys[MePhe]ValThr[MeGly][MeAla]Thr[Tyr(3-F)]GlyI leThrArgPro | 144.7 | 1445.3 |
| Cleaved peptide | Pep-33 | [MePhe]ValThr[MeGly][MeAla]Thr[Tyr(3-F)]GlyIleT hrArgPro | 1341.7 | 1342.4 |

**[Table 17]**

| Template mRNA sequence number R-9-1 | | | | |
|---|---|---|---|---|
| | Peptide sequence number | Sequence | MS theoretical value | MS measured value[M+H]+ |
| Target compound | Pep-35 | CysSer[MePhe][MePhe]Val[MeGly]Ser[Tyr(3-F)]SerV alIleArgPro | 1520.7 | 1521.3 |
| Cleaved peptide | Pep-36 | Ser[MePhe][MePhe]Val[MeGly]Ser[Tyr(3-F)]SerVal I leArgPro | 1417.7 | 1418.4 |
| Cleaved peptide | Pep-37 | [MePhe]Val[MeGly]Ser[Tyr(3-F)]SerValIleArgPro | 1169.6 | 1170.3 |

**[Table 18]**

| Template mRNA sequence number R-5-1 | | | | |
|---|---|---|---|---|
| | Peptide sequence number | Sequence | MS theoretical value | MS measured value [M+H] + |
| Target compound | Pep-39 | CysSer[MePhe]ProSer[MeGly]IleVal[Tyr(3-F)]Thr GlyArgPro | 1428.7 | 1429.3 |
| Cleaved peptide | Pep-40 | Ser[MePhe]ProSer[MeGly]IleVal[Tyr(3-F)]ThrGly ArgPro | 1325.7 | 1326.4 |
| Cleaved peptide | Pep-41 | [MePhe]ProSer[MeGly]IleVal[Tyr(3-F)]ThrGlyArg Pro | 1238.6 | 1239.4 |
| Cleaved peptide | Pep-42 | [MeGly]IleVal[Tyr(3-F)]ThrGlyArgPro | 893.5 | 894.3 |

As shown in Fig. 5, when comparing the initiation read-through method and the initiation suppression method for all of the peptides containing multiple amino acid analogs, of which translation were evaluated this time, MS intensities of the substances of interest were observed to be stronger and the MS intensities of the cleaved peptides were observed to be weaker in the initiation suppression method. That is, the initiation suppression method is demonstrated to be a technique for efficient ribosomal synthesis of peptides containing multiple amino acid analogs and having cysteine at their N terminus. Furthermore, the observed cleaved peptides were those cleaved before or after translational incorporation of an amino acid analog, and thus the initiation suppression method was shown to be able to ribosomally synthesize peptides containing multiple amino acid analogs with high purity.

### [Example 5] Synthesis of amide cyclic peptides by ribosomally synthesizing peptides having NMe cysteine with a protecting group at the N terminus and performing native chemical ligation after deprotection of the protecting group

### 5-1. Synthesis of amide cyclic peptides by ribosomally synthesizing peptides having NMe cysteine with a protecting group at the N terminus and an active ester in the side chain of the amino acid at the C-terminal side, and performing native chemical ligation and desulfurization reaction after deprotection of the protecting group from the ribosomally synthesized peptide

As shown in the following reaction formula, after ribosomally synthesizing a peptide having Acbz-MeCys(StBu) at the N terminus using the initiation suppression method, and an amide cyclic peptide was synthesized by deprotection of Acbz using Tris(2-carboxyethyl)phosphine (TCEP), native chemical ligation, and desulfurization. Specifically, in native chemical ligation which uses MeCys as the substrate, there is equilibrium between the thiolactone compound produced from thioester exchange and the NCL amide compound produced from a subsequent S-N shift, and a mixture of two compounds is obtained. By subjecting the mixture to desulfurization reaction of the NCL amide compound, the equilibrium was shifted to make the reaction proceed; thereby the cyclic peptide of interest was obtained. Thus, reaction conditions that enable synthesis of amide cyclic peptides of interest through successive conversion from peptides obtained by ribosomal synthesis were set.

The obtained reaction mixture was analyzed by MALDI, and this confirmed that the respective reactions proceeded and the amide cyclic peptides of interest could be obtained. The template mRNA sequences used for the ribosomal synthesis are shown in Table 19 below.

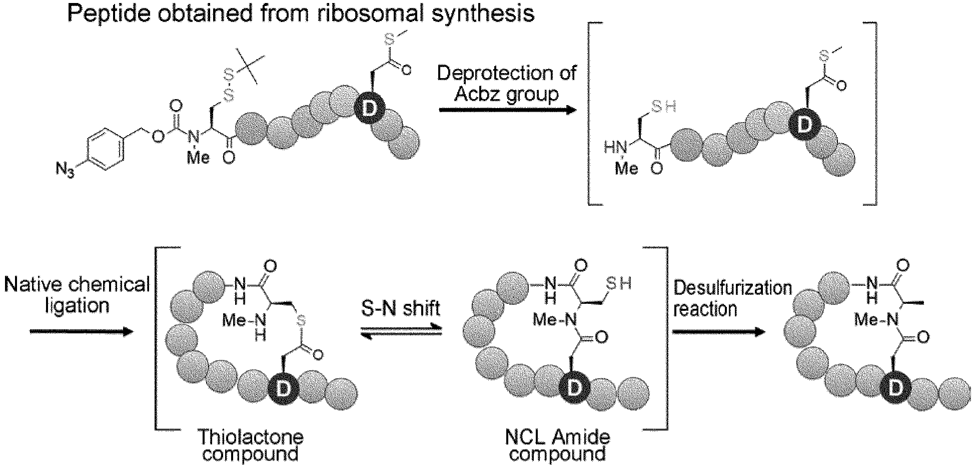

**[Table 19]**

| Template mRNA Sequence number | Sequence |
|---|---|
| R-10 (SEQ ID NO: 22) | GGGUUAACUUUAAGAAGGAGAUAUACAUAUGAGUUUUUUGUUCAGAGUUACAGUCUUCCGCGUUA AGCUUCG |
| R-11 (SEQ ID NO: 23) | GGGUUAACUUUAAGAAGGAGAUAUACAUAUGAGUUUUGUUCAGAUUUUUAGUGUUCUUCCGCGUUA AGCUUCG |
| R-12 (SEQ ID NO: 24) | GGGUUAACUUUAAGAAGGAGAUAUACAUAUGACUACUCCGUUUGGUCAGAGUUACAUUGUUCUUCC GCGUUAAGCUUCG |
| R-13 (SEQ ID NO: 25) | GGGUUAACUUUAAGAAGGAGAUAUACAUAUGUUUGUUACUCAGGCUACUUACGGUAUUCUUCCGCG UUAAGCUUCG |
| R-14 (SEQ ID NO: 26) | GGGUUAACUUUAAGAAGGAGAUAUACAUAUGAGUUUCCGAGUCAGAUUGUUUACACUCUUCCGCG UUAAGCUUCG |
| R-15 (SEQ ID NO: 27) | GGGUUAACUUUAAGAAGGAGAUAUACAUAUGUUUAUUGGUUUCCACAGUUCAAGUGGCUUCCUCG UUAUUAAGCUUCG |

The translation system used was PURE system, an *E. coli*-derived reconstituted cell-free protein synthesis system. Specifically, the synthesis was carried out as follows: 1 µM template mRNA (R-10, R-11, R-12, R-13, R-14, or R-15) and 250 µM each of amino acids, Ala, Arg, Cys, Gln, Gly, His, Ile, Lys, Pro, Ser, Thr, Trp, Val, and 3-fluoro-L-tyrosine (Tyr(3-F)), were added to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 6 mM magnesium acetate, 2 mM spermidine, 1 mM dithiothreitol, 1.5 mg/mL *E. coli* MRE600 (RNase-negative)-derived tRNA (Roche), 0.1 mM 10-HCO-H4 folate, 4 µg/ml creatine kinase, 3 µg/ml myokinase, 2 units/mL inorganic pyrophosphatase, 1.1 µg/mL nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), 5mM of *N*-methyl phenylalanine (MePhe), as well as 20 µM AspSMe-tRNAGluAAG (Compound AAtR-9) and 20 µM Acbz-MeCys(StBu)-tRNAfMetCAU (Compound AAtR-3) were added to the translation solution mixture, and this was incubated at 37°C for one hour.

Next, 150 mM TCEP cyclization reaction reagent solution (2 µL) was mixed into the translation reaction solution (8 µL), and this was incubated at 37°C for one hour. Progress of the Acbz deprotection reaction and cyclization reaction by native chemical ligation was confirmed by MALDI-TOF MS (Fig. 6). For analyses by MALDI-TOF MS, 1 µL reaction solution was sampled and purified using SPE C-TIP (Nikkyo Technos) using α -cyano-4-hydroxycinnamic acid (CHCA) as the matrix. Furthermore, the 150 mM TCEP cyclization reaction reagent solution was prepared by mixing a TCEP solution (pH 7.0) (500 mM, 12 µL), a Tris solution (pH 8.3) (2 M, 3 µL), an EDTA solution (pH 8.0) (500 mM, Nacalai Tesque, product number 14362-24, 5 µL), and an aqueous potassium hydroxide solution (200 mM, 20 µL).

Furthermore, a TCEP solution (pH 7.0) (500 mM, 7 µL) and a glutathione (GSH) solution (250 mM, 1 µL) were added to the cyclization reaction solution (7 µL), this was incubated at 42°C for one minute, and then a 2,2'-azobis[2-(2-imidazolin-2-yl)propane] dihydrochloride salt (VA-044) solution (1 M, 4 µL) and an aqueous potassium hydroxide solution (1 M, 1 µL) were added to the reaction mixture, and this was incubated at 42°C for two hours.

MALDI-TOF MS was used to confirm that the desulfurization reaction was progressing (Fig. 6). For the analyses by MALDI-TOF MS, 1 µL of reaction solution was sampled and purified using SPE C-TIP (Nikkyo Technos) using α-cyano-4-hydroxycinnamic acid (CHCA) as the matrix.

Mixture of compounds obtained by ribosomal synthesis from template mRNA sequence number: R-10 followed by Acbz-group deprotection reaction and cyclization reaction by native chemical ligation:

### [MeCys*]Ser[MePhe][MePhe]ValGlnSer[Tyr(3-F)]SerAsp*ProArg (cyclized at the two * sites) (mixture of Pep-43 and Pep-44)

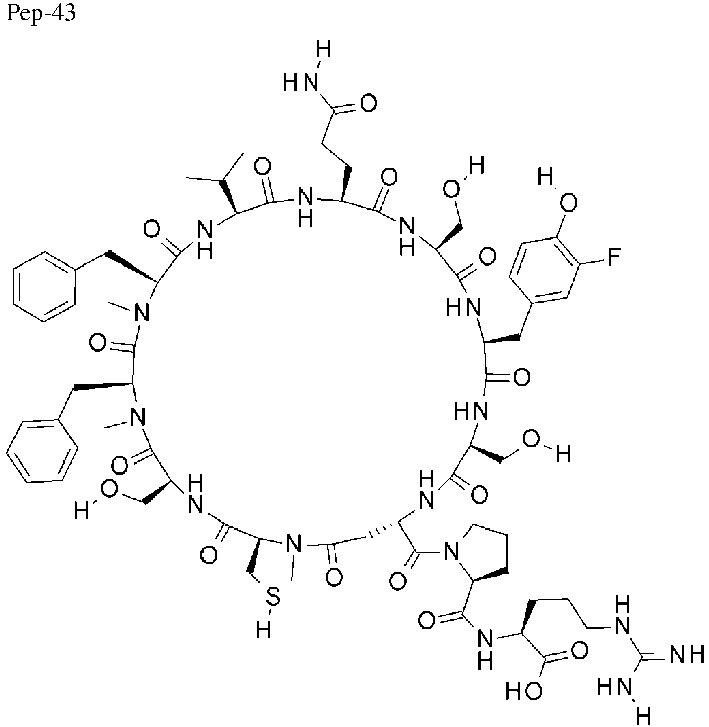

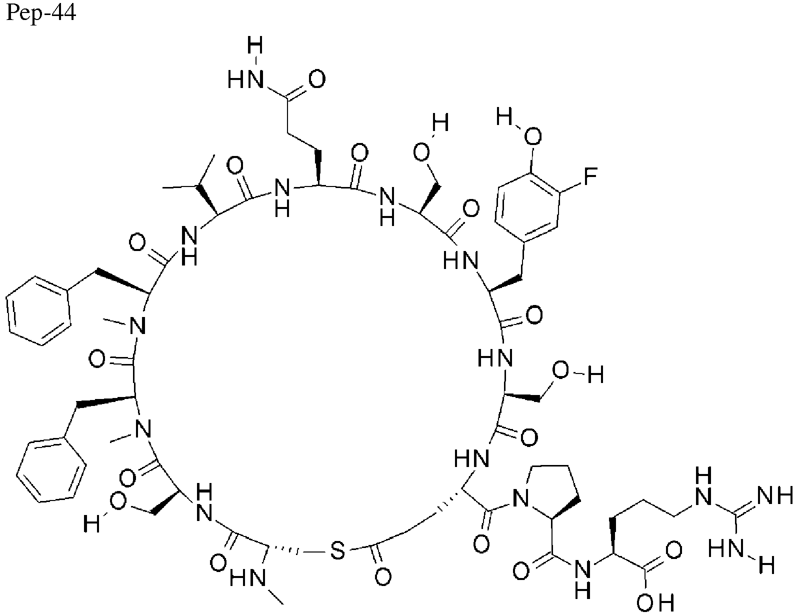

MALDI-TOF MS
m/z = 1477.5 (M+H)+ Calc. 1476.7

Compound obtained by subjecting a mixture of compounds (Pep-43 and Pep-44) derived from template mRNA sequence number: R-10 to a desulfurization reaction:

### [MeAla*]Ser[MePhe][MePhe]ValGlnSer[Tyr(3-F)]SerAsp*ProArg (cyclized at the two * sites) (Pep-45)

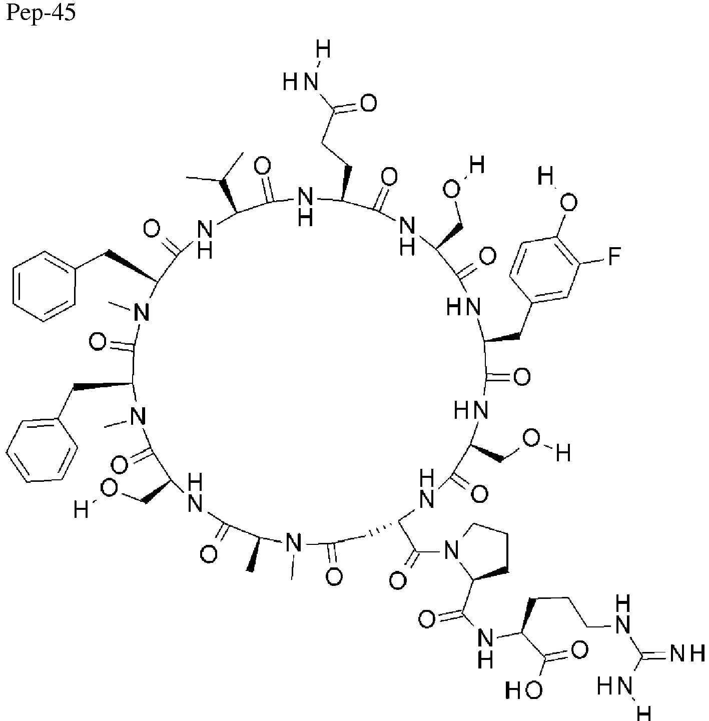

MALDI-TOF MS
m/z = 1445.5 (M+H)+ Calc. 1444.7

Mixture of compounds obtained by ribosomal synthesis from template mRNA sequence number: R-11 followed by Acbz-group deprotection reaction and cyclization reaction by native chemical ligation:

### [MeCys*]Ser[MePhe]ValGlnIle[MePhe]SerValAsp*ProArg (cyclized at the two * sites) (Pep-46 and Pep-47)

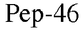

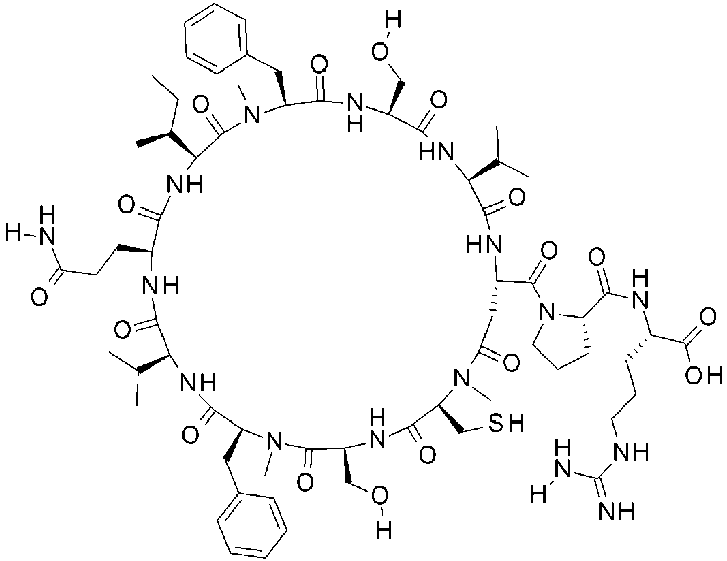

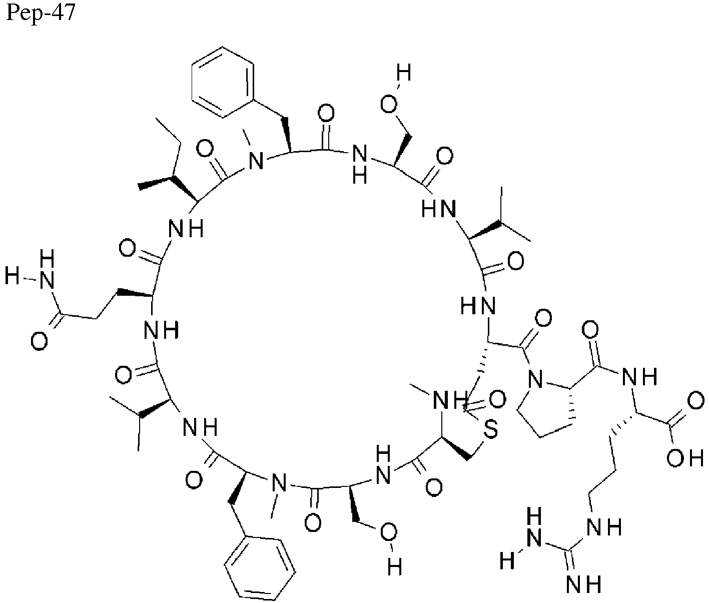

MALDI-TOF MS
m/z = 1421.5 (M+H)+ Calc. 1420.7

Compound obtained by subjecting a mixture of compounds (Pep-46 and Pep-47) derived from template mRNA sequence number: R-11 to a desulfurization reaction:

### [MeAla*]Ser[MePhe]ValGlnIle[MePhe]SerValAsp*ProArg (cyclized at the two * sites) (Pep-48)

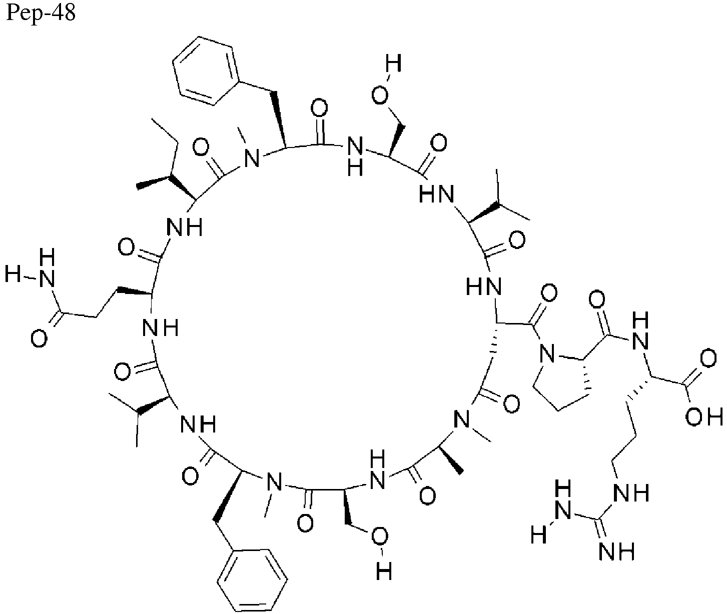

MALDI-TOF MS
m/z = 1389.6 (M+H)+ Calc. 1388.7

Mixture of compounds obtained by ribosomal synthesis from template mRNA sequence number: R-12 followed by Acbz-group deprotection reaction and cyclization reaction by native chemical ligation:

### [MeCys*]ThrThrPro[MePhe]GlyGlnSer[Tyr(3-F)]IleValAsp*ProArg (cyclized at the two * sites) (Pep-49 and Pep-50)

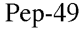

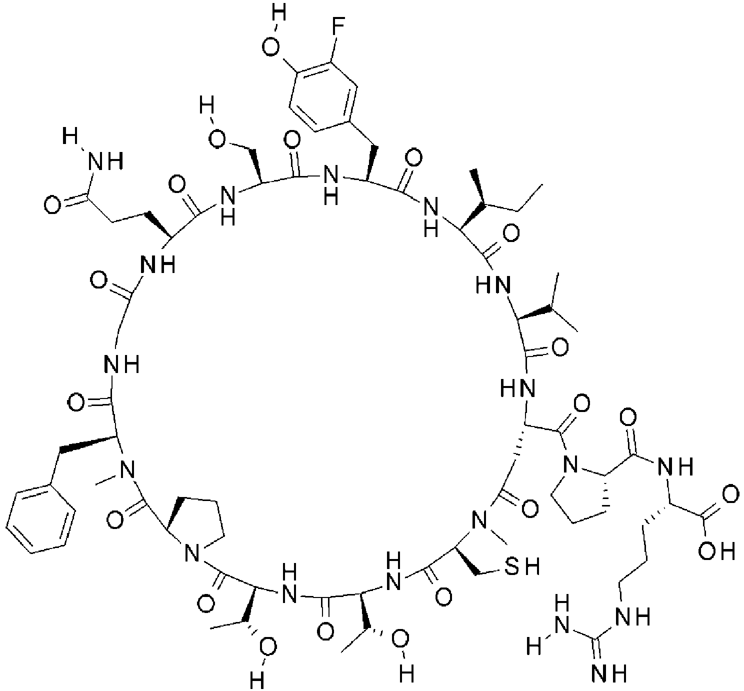

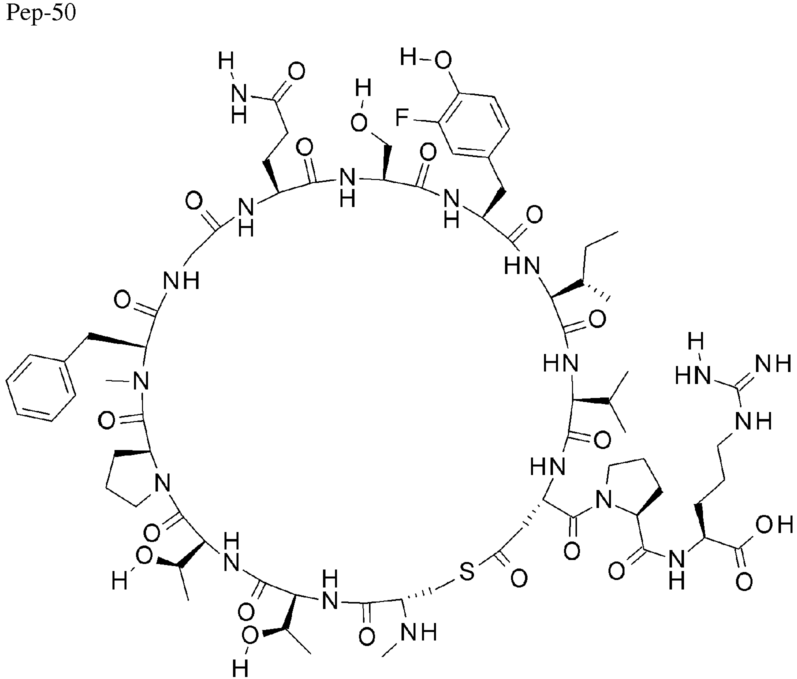

MALDI-TOF MS
m/z = 1611.4 (M+H)+ Calc. 1610.8

Compound obtained by subjecting a mixture of compounds (Pep-49 and Pep-50) derived from template mRNA sequence number: R-12 to a desulfurization reaction:

### [MeAla*]ThrThrPro[MePhe]GlyGlnSer[Tyr(3-F)]IleValAsp*ProArg (cyclized at the two * sites) (Pep-51)

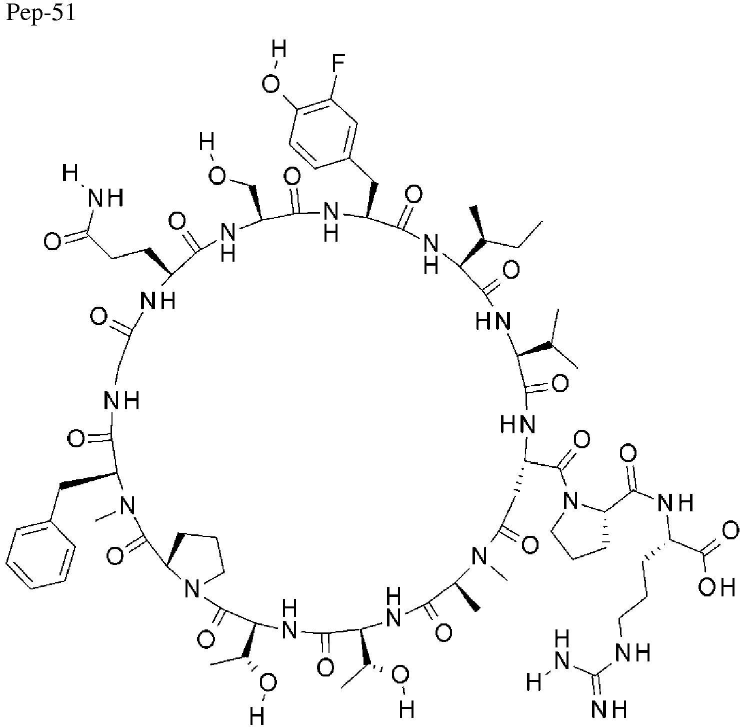

MALDI-TOF MS
m/z = 1579.5 (M+H)+ Calc. 1578.8

Mixture of compounds obtained by ribosomal synthesis from template mRNA sequence number: R-13 followed by Acbz-group deprotection reaction and cyclization reaction by native chemical ligation:

### [MeCys*][MePhe]ValThrGlnAlaThr[Tyr(3-F)]GlyIleAsp*ProArg (cyclized at the two * sites) (Pep-52 and Pep-53)

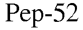

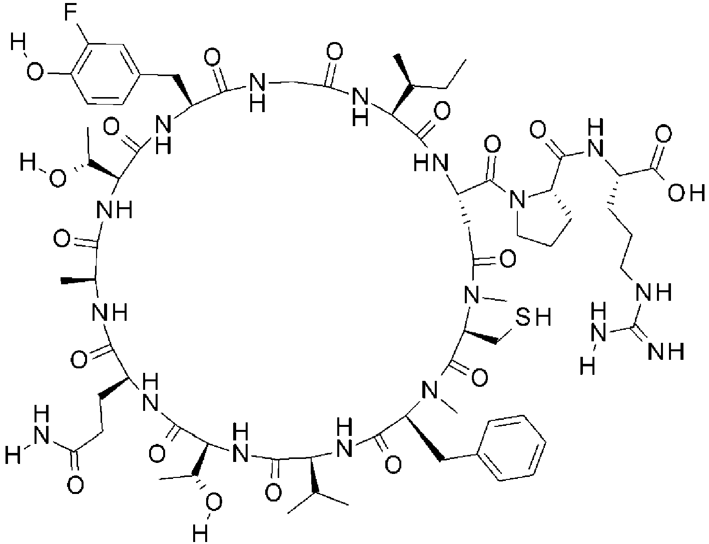

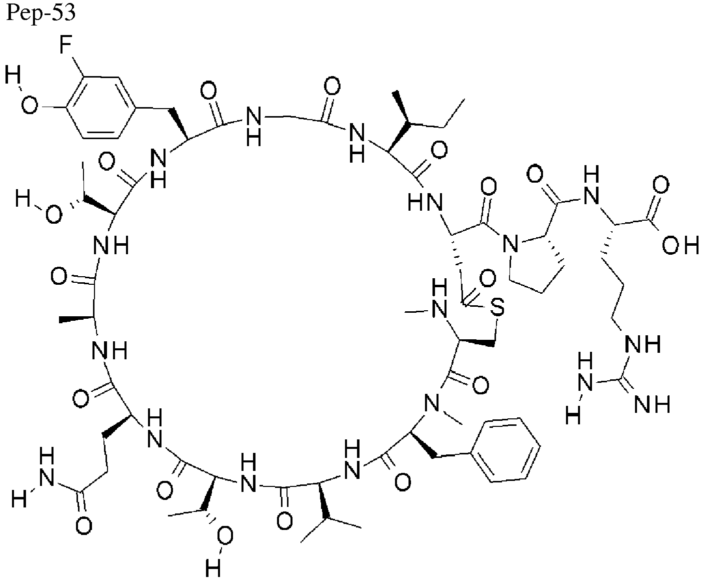

MALDI-TOF MS
m/z = 1498.4 (M+H)+ Calc. 1497.7

Compound obtained by subjecting a mixture of compounds (Pep-52 and Pep-53) derived from template mRNA sequence number: R-13 to a desulfurization reaction:

### [MeAla*][MePhe]ValThrGlnAlaThr[Tyr(3-F)]GlyIleAsp*ProArg (cyclized at the two * sites) (Pep-54)

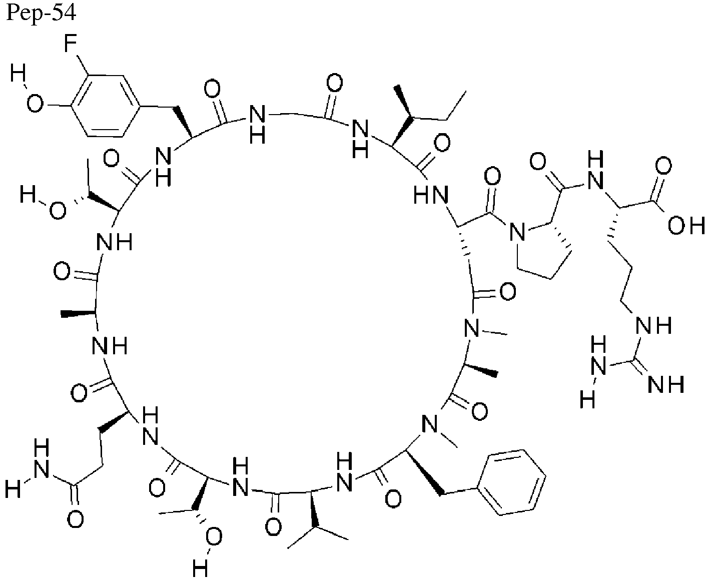

MALDI-TOF MS
m/z = 1466.5 (M+H)+ Calc. 1465.7

Mixture of compounds obtained by ribosomal synthesis from template mRNA sequence number: R-14 followed by Acbz-group deprotection reaction and cyclization reaction by native chemical ligation:

### [MeCys*]Ser[MePhe]ProSerGlnIleVal[Tyr(3-F)]ThrAsp*ProArg (cyclized at the two * sites) (Pep-55 and Pep-56)

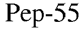

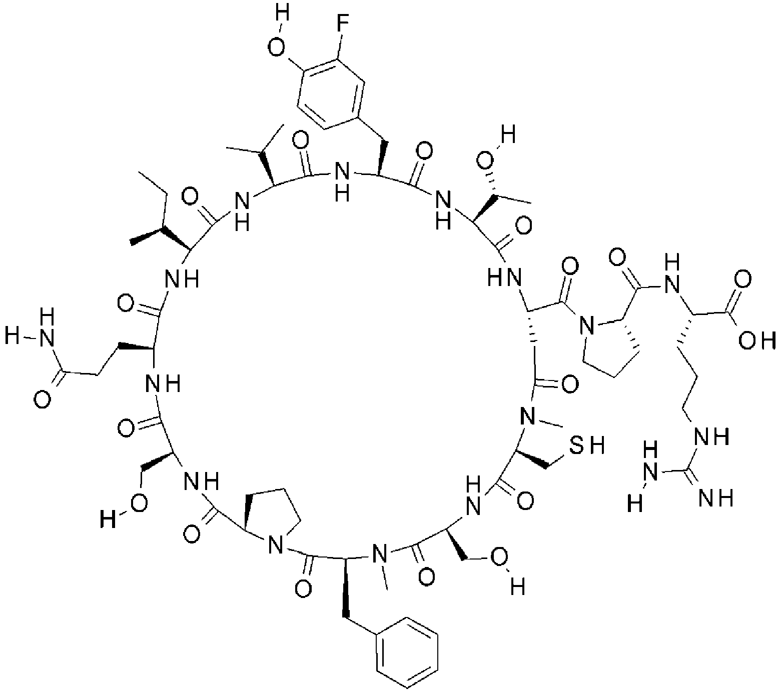

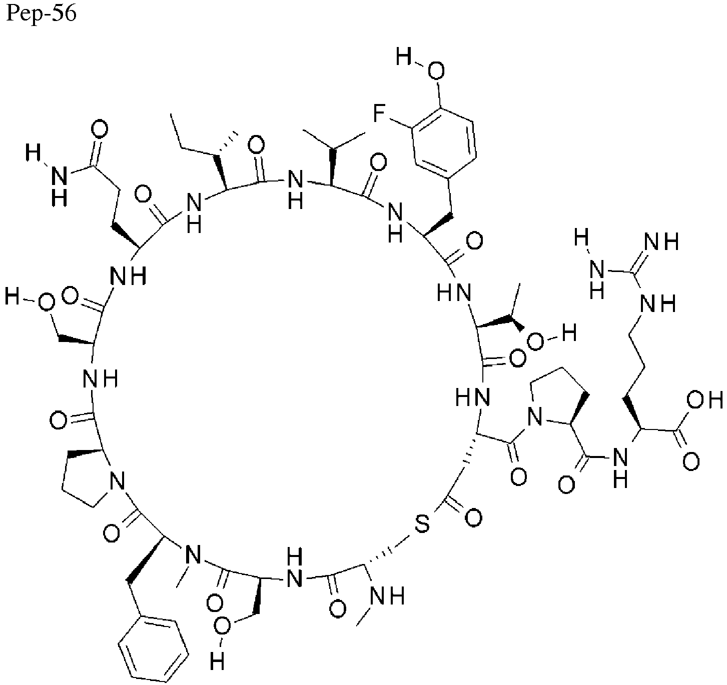

MALDI-TOF MS
m/z = 1540.4 (M+H)+ Calc. 1539.7

Compound obtained by subjecting compounds (Pep-55 and Pep-56) derived from template mRNA sequence number: R-14 to a desulfurization reaction:

### [MeAla*]Ser[MePhe]ProSerGlnIleVal[Tyr(3-F)]ThrAsp*ProArg (cyclized at the two * sites)

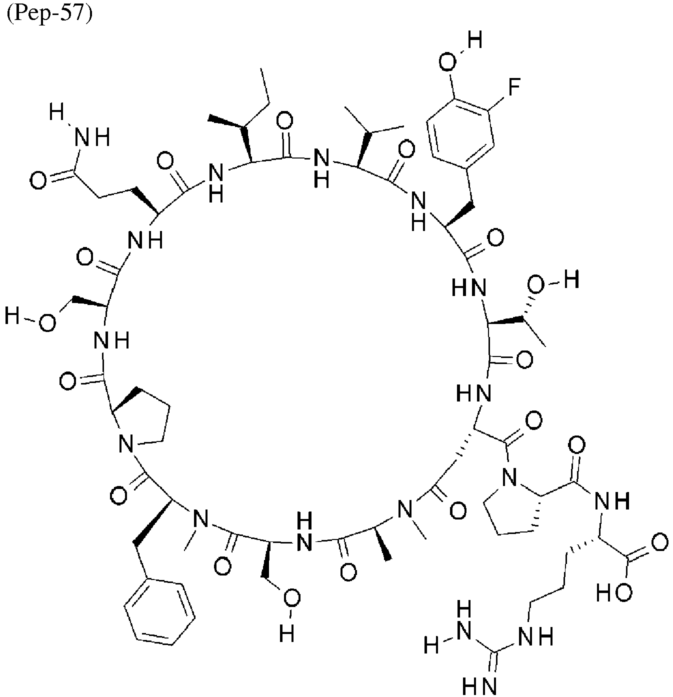

MALDI-TOF MS
m/z = 1508.4 (M+H)+ Calc. 1507.7

Mixture of compounds obtained by ribosomal synthesis from template mRNA sequence number: R-15 followed by Acbz-group deprotection reaction and cyclization reaction by native chemical ligation:

### [MeCys*][MePhe][Tyr(3-F)]Trp[MePhe]ProGln[MePhe]LysTrpAsp*ProArg[Tyr(3-F)] (cyclized at the two * sites) (Pep-58 and Pep-59)

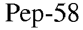

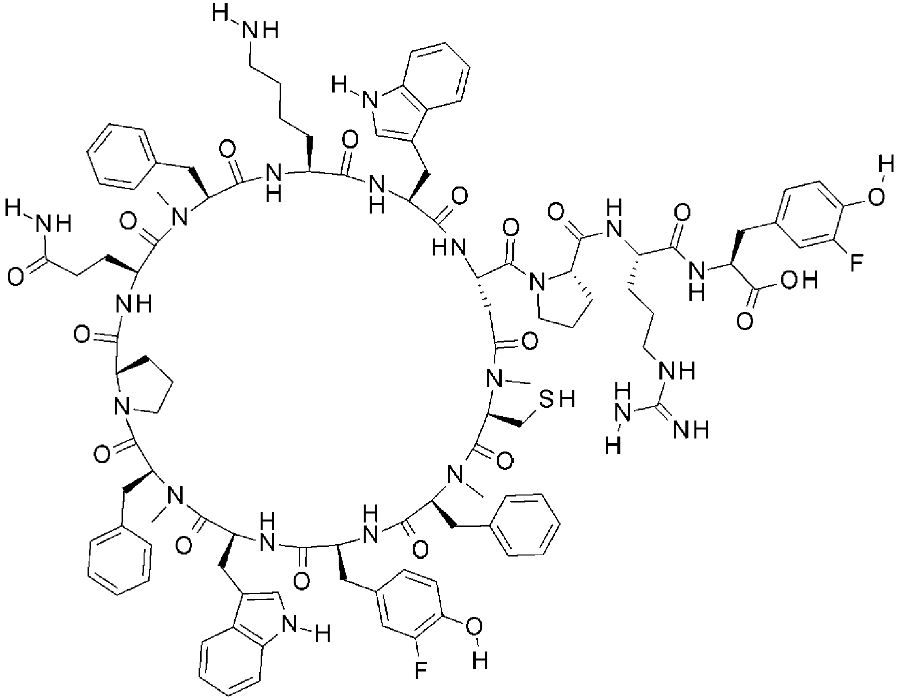

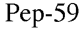

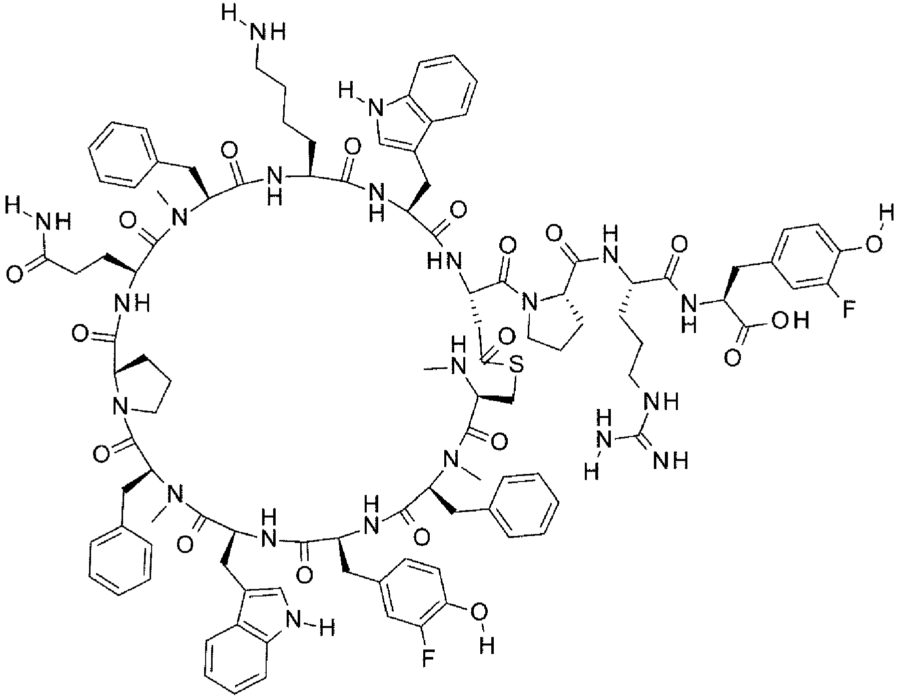

MALDI-TOF MS
m/z = 2056.4 (M+H)+ Calc. 2055.9

Compound obtained by subjecting a mixture of compounds (Pep-58 and Pep-59) derived from template mRNA sequence number: R-15 to a desulfurization reaction:

### [MeAla*][MePhe][Tyr(3-F)]Trp[MePhe]ProGln[MePhe]LysTrpAsp*ProArg[Tyr(3-F)] (cyclized at the two * sites) (Pep-60)

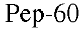

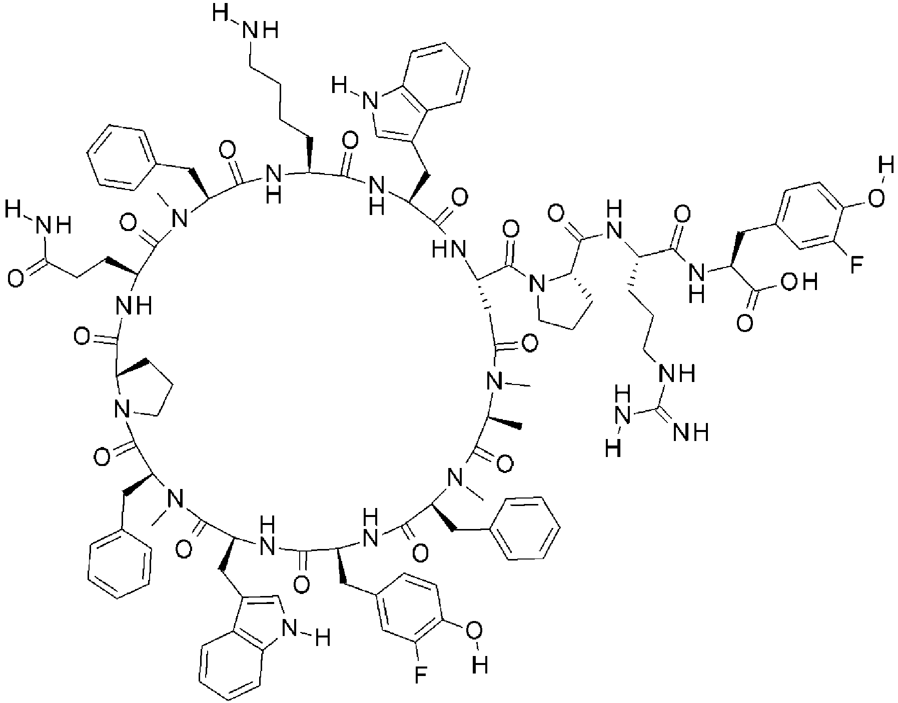

MALDI-TOF MS
m/z = 2024.7 (M+H)+ Calc. 2024.0

### [Example 6] Evaluation of RNA stability under posttranslational modification conditions

Experiments were performed to confirm that RNA was stable under each of the reaction conditions that allow the synthesis of amide cyclic peptide of interest by successive conversion from peptides obtained by ribosomal synthesis.

### 6-1. Preparation of mRNA-puromycin liker ligation products for evaluation of stability under posttranslational modification conditions

mRNA (sequence number: R-16) was prepared by *in vitro* transcription using T7 RiboMAX™ Express Large Scale RNA Production System (Promega, P1320) and by using a DNA library (sequence number: D-16) prepared by the method described in the literature (Patent Document WO 2013/100132) as a template, and was purified using RNeasy MinElute kit (Qiagen).

Ligation reaction was carried out on 6 µM mRNA (sequence number: R-16) under conditions of 9 µM puromycin linker (Sigma) (sequence number: C-1), 1x T4 RNA ligase reaction buffer (New England Biolabs), 1 mM ATP, 10% DMSO, 0.625 units/µL T4 RNA ligase (New England Biolabs) at 37°C for 30 minutes, and then the reaction mixture was purified by RNeasy MinElute kit (Qiagen) to obtain a mRNA-puromycin linker ligation product (sequence number: R-17).

### DNA sequence number: D-16 (SEQ ID NO: 28)

GTAATACGACTCACTATAGGGTTAACTTTAAGAAGGAGATATACATATGTGC(NNN)9TA
GCCGACCGGCACCGGCACCGGCGATAGGGCGGCGGGACAAA

The site indicated as (NNN) in the sequence means that TTT, TTG, CTA, ATT, GTT, CCG, ACT, GCT, TAC, CAT, CAG, GAA, TGG, CGG, AGT, AGG, and GGT randomly appear. Furthermore, (NNN)₉ means that any NNNs are bound nine times, and does not only refer to (ATT)₉ but schematically illustrates that, for example, diversity of 17⁹ is possible when each NNN is selected from 17 types.

### RNA sequence number: R-16 (SEQ ID NO: 29)

GGGUUAACUUUAAGAAGGAGAUAUACAUAUGUGC(PPP)₉UAGCCGACCGGCACCGG
CACCGGCGAUAGGGCGGCGGGACAAA

The site indicated as (PPP) in the sequence means that UUU, UUG, CUA, AUU, GUU, CCG, ACU, GCU, UAC, CAU, CAG, GAA, UGG, CGG, AGU, AGG, and GGU randomly appear. Furthermore, (PPP)₉ means that any PPPs are bound nine times, and does not only refer to (AUU)₉ but schematically illustrates that, for example, diversity of 17⁹ is possible when each PPP is selected from 17 types.

### Puromycin linker sequence number: C-1 (SEQ ID NO: 30)

[P]CCCGTCCCCGCCGCCCT[Spacer18][Spacer18][Spacer18][Spacer18][Spacer18]CC[Purom ycin] ([P]:5'-phosphorylated)

The detailed partial structure is as shown below.

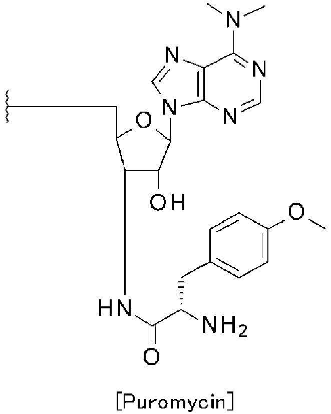

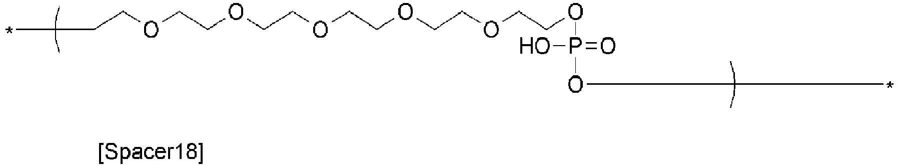

### mRNA-puromycin linker ligation product (sequence number: R-17) (SEQ ID NO: 31)

GGGUUAACUUUAAGAAGGAGAUAUACAUAUGUGC(PPP)₉UAGCCGACCGGCACCGG CACCGGCGAUAGGGCGGCGGGGACAAACCCGTCCCCGCCGCCCT[Spacer18][Spacer18 ][Spacer18][Spacer18][Spacer18]CC[Puromycin]

### 6-2. Examples where the stability of RNA was confirmed under conditions for Acbz-group deprotection reaction, amide cyclization reaction by native chemical ligation, and subsequent desulfurization reaction

Whether RNAs exist stably under the series of reaction conditions described above, the Acbz-group deprotection reaction, the amide cyclization reaction by native chemical ligation, and the subsequent desulfurization reaction, was confirmed. That is, the mRNA-puromycin linker ligation product (sequence number: R-17) was subjected to the series of reaction conditions, and then analyzed by gel electrophoresis.

Specifically, 150 mM TCEP cyclization reaction reagent solution (15 µL) was mixed into a buffer (1 mM GTP, 1 mM ATP, 20 mM creatine phosphate, 50 mM HEPES-KOH (pH 7.6), 100 mM potassium acetate, 9 mM magnesium acetate, 2 mM spermidine, 1 mM dithiothreitol, and 0.1 mM 10-HCO-H4 folate) (60 µL) containing 1 µM mRNA-puromycin linker ligation product (sequence number: R-17), and this mixture was incubated at 37°C for one hour. 150 mM TCEP cyclization reaction reagent solution was mixed with a TCEP solution (pH 7.0) (500 mM, 12 µL), a Tris solution (pH 8.3) (2 M, 3 µL), an EDTA solution (pH 8.0) (500 mM, Nacalai Tesque, product number 14362-24, 5 µL), and an aqueous potassium hydroxide solution (200 mM, 20 µL) to prepare an Acbz deprotection cyclization solution.

Furthermore, to the mixed solution (75 µL), a TCEP solution (pH 7.0) (500 mM, 75 µL) and a glutathione (GSH) solution (250 mM, 10.7 µL) were added, this was incubated at 42°C for one minute, and then a 2,2'-azobis[2-(2-imidazolin-2-yl)propane]dihydrochloride salt (VA-044) solution (1 M, 42.9 µL) and an aqueous potassium hydroxide solution (1 M, 10.7 µL) were added to the reaction mixture, and this was incubated at 42°C for three hours.

Subsequently, the solution was purified by RNeasy MinElute kit (Qiagen) to obtain a solution of the mRNA-puromycin linker ligation product, and then the ligation product was analyzed by electrophoresis (Fig. 7, Lane 1).

On the other hand, 139.3 µL of water was added to the Acbz deprotection cyclization solution (75 µL) described above, and this was incubated at 42°C for three hours. As described above, the mRNA-puromycin linker ligation product was purified using RNeasy MinElute kit (Qiagen), and this was prepared as a solution not subjected to desulfurization reaction conditions and used for comparison by electrophoresis (Fig. 7, Lane 2).

Furthermore, as a standard solution, a 0.5 µM solution of the mRNA-puromycin linker ligation product (sequence number: R-17) not subjected to the reaction conditions was used for comparison by electrophoresis (Fig. 7, Lane 3). TrackIt 10 bp ladder (Life Technologies, product no. 10488-019) was used as the marker (Fig. 7, Lane 4).

10 µL of Novex® TBE-urea sample buffer (2x) (Invitrogen) was added to 10 µL of each of the above-mentioned reaction sample solutions. 4 µL of each mixture was subjected to electrophoresis using 10% TBE-urea gel and stained with SYBR gold nucleic acid stain (Invitrogen). As a result, compared to the standard solution (Fig. 7, Lane 3), both reaction samples (Fig. 7, Lanes 1 and 2) did not show change in band patterns that may be derived from decomposition products. This indicated that RNA is not degraded and stably exists under the series of reaction conditions, which are the Acbz-group deprotection reaction, the amide cyclization reaction by native chemical ligation, and the subsequent desulfurization reaction.

**[Table 20]**

| Lane No. | Explanation on Samples |
|---|---|
| 1 | Sample subjected to deprotection reaction of Acbz group and amide cyclization reaction by native chemical ligation (30 mM TCEP, pH 7.9, 37°C, 1 hour) of mRNA-Puromycin linker ligation product (Sequence number: R-17) followed by desulfurization reaction (200 mM VA-044, 200 mM TCEP, 12.5 mM GSH, pH 6.7, 42°C, 3 hour) |
| 2 | Sample subjected to deprotection reaction of Acbz group and amide cyclization reaction by native chemical ligation (30 mM TCEP, pH 7.9, 37°C, 1 hour) of mRNA-Puromycin linker ligation product (Sequence number: R-17) |
| 3 | Standard product: mRNA-Puromycin linker ligation product (Sequence number: R-17) |
| Marker | TrackIt 10 bp ladder (Life Technologies Co., Product #10488-019) |

### [Example 7] Synthesis of pCpA-amino acids for use in a cell-free translation system

### Definition of the terms

- Azoc: azidomethoxycarbonyl group

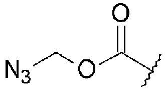

- HOGly: glycolic acid
- DMT: di(*p*-methoxyphenyl)phenylmethyl group

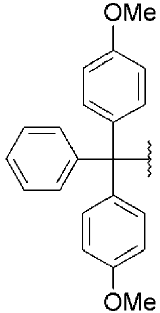

### 7-1. Synthesis of pCpA-amino acids for translationally introducing an amino acid analog at the N terminus by the initiation suppression method

### Synthesis of 2-(tert-butyldisulfanyl)ethan-1-amine hydrochloride salt (Compound nk31)

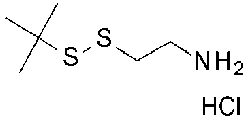

A 4N hydrochloric acid/1,4-dioxane solution (1.0 mL) was added to *tert*-butyl (2-(*tert*-butyldisulfanyl)ethyl)carbamate (53 mg, 0.20 mmol) synthesized by the method described in the literature (WO 2013/100132A), and this was stirred at room temperature for 90 minutes. The reaction solution was concentrated under reduced pressure to give 2-(*tert*-butyldisulfanyl)ethan-1-amine hydrochloride salt (Compound nk31) (40 mg).
LCMS (ESI) m/z = 166 (M+H)+
Retention time: 0.36 minutes (analysis condition SQDFA05)

### Synthesis of N-(((4-azidobenzyl)oxy)carbonyl)-N-(2-(tert-butyldisulfanyl)ethyl)glycine (Compound nk32)

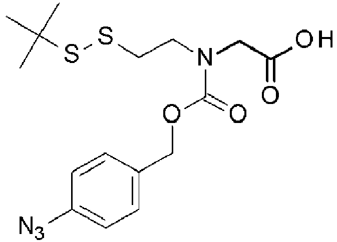

2-(*tert*-butyldisulfanyl)ethan-1-amine hydrochloride salt (Compound nk31) (40 mg, 0.20 mmol) obtained in the previous step and *N,N*-diisopropylethylamine (0.140 mL, 0.80 mmol) were dissolved in acetonitrile (1.0 mL). After cooling the mixture in an ice bath, *tert*-butyl 2-bromoacetate (0.029 mL, 0.20 mmol) was added. The reaction mixture was stirred for five minutes, and then stirred at room temperature for 90 minutes. *tert*-Butyl 2-bromoacetate (0.006 mL, 0.040 mmol) was further added and stirred at room temperature for 16.5 hours. The reaction solution was concentrated under reduced pressure to give a concentrated residue. The obtained concentrated residue was dissolved in DCM (1 mL), the mixture was cooled in an ice bath, and then TFA (1 mL) was added. After stirring the reaction mixture at room temperature for 60 minutes, the reaction solution was concentrated under reduced pressure.

Under nitrogen atmosphere, DMF (0.40 mL) was added at room temperature to a mixture of the obtained concentrated residue and 4-azidobenzyl (4-nitrophenyl)carbonate (62.9 mg, 0.20 mmol). The mixture was cooled in an ice bath, and then triethylamine (84 µL, 0.60 mmol) was added to it. The reaction mixture was stirred at 25°C for three days and then purified by reverse-phase silica gel column chromatography (0.1% aqueous formic acid solution/0.1% formic acid-acetonitrile solution) to give *N*-(((4-azidobenzyl)oxy)carbonyl)-*N*-(2-(*tert*-butyldisulfanyl)ethyl)glycine (Compound nk32) (53.9 mg, 68%).
LCMS (ESI) m/z = 397 (M-H)-
Retention time: 0.90 minutes (analysis condition SQDFA05)

### Synthesis of cyanomethyl N-(((4-azidobenzyl)oxy)carbonyl)-N-(2-(tert-butyldisulfanyl)ethyl)glycinate (Compound nk33)

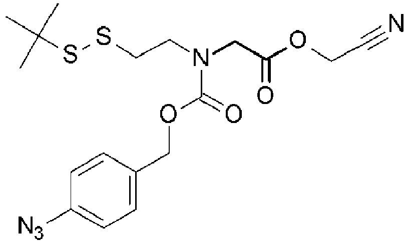

Under nitrogen atmosphere, *N*-(((4-azidobenzyl)oxy)carbonyl)-N-(2-(*tert*-butyldisulfanyl)ethyl)glycine (Compound nk32) (53.9mg, 0.135 mmol) and *N,N*-diisopropylethyl amine (DIPEA) (0.047 mL, 0.271 mmol) were dissolved in acetonitrile (541 µL), 2-bromoacetonitrile (0.014 mL, 0.203 mmol) was added, and the mixture was stirred at room temperature for 3.5 hours. The reaction solution was concentrated to give cyanomethyl *N*-(((4-azidobenzyl)oxy)carbonyl)-*N*-(2-(*tert*-butyldisulfanyl)ethyl)glycinate (Compound nk33) as a crude product. Cyanomethyl *N-*(((4-azidobenzyl)oxy)carbonyl)-*N*-(2-(*tert*-butyldisulfanyl)ethyl)glycinate (Compound nk33) obtained as a crude product was dissolved in acetonitrile (0.675 mL) and was directly used in the next step.
LCMS (ESI) m/z = 438 (M+H)+
Retention time: 0.99 minutes (analysis condition SQDFA05)

### Synthesis of (2R,3S,4R,5R)-2-((((((2R,3S,4R,5R)-5-(4-amino-2-oxopyrimidin-1(2H)-yl)-4-hydroxy-2-((phos phonooxy)methyl)tetrahydrofuran-3-yl)oxy)(hydroxy)phosphoryl)oxy)methyl)-5-(6-amino-9H-p urin-9-yl)-4-hydroxytetrahydrofuran-3-yl N-(((4-azidobenzyl)oxy)carbonyl)-N-(2-(tert-butyldisulfanyl)ethyl)glycinate (Compound nk34)

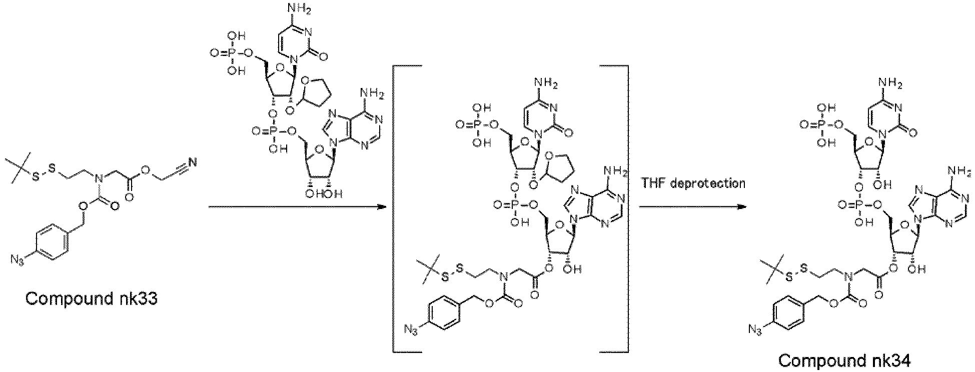

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (24.6 mg, 0.034 mmol) was dissolved in Buffer A (16.4 mL), a solution of cyanomethyl *N*-(((4-azidobenzyl)oxy)carbonyl)-*N*-(2-(*tert*-butyldisulfanyl)ethyl)glycinate (Compound nk33) in 0.2 M acetonitrile (0.339 mL, 0.068 mmol) was added to it in three portions (0.113-mL each was added at the start of the reaction, and ten minutes and 30 minutes after starting the reaction, three times additions in total), and the mixture was stirred at room temperature for 90 minutes. The reaction solution was cooled to 0°C, and then trifluoroacetic acid (0.82 mL) was added to it. After stirring the reaction solution at 0°C for ten minutes, this was purified by reverse-phase silica gel column chromatography (0.05% aqueous trifluoroacetic acid solution/0.05% trifluoroacetic acid-acetonitrile solution) to give the titled compound (Compound nk34) (16.2 mg, 46%).
LCMS (ESI) m/z = 1033 (M+H)+
Retention time: 0.61 minutes (analysis condition SQDFA05)

### Synthesis of 3-((((4-azidobenzyl)oxy)carbonyl)(2-(tert-butyldisulfanyl)ethyl)amino)propanoic acid (Compound nk35)

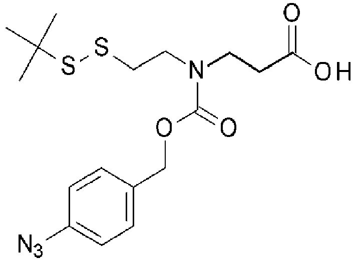

2-(*tert*-Butyldisulfanyl)ethan-1-amine hydrochloride salt (Compound nk31) (40 mg, 0.20 mmol) and *N,N*-diisopropylethylamine (0.279 mL, 1.60 mmol) were dissolved in DCM (1.0 mL). After cooling the mixture in an ice bath, *tert*-butyl 3-bromopropanoate (0.10 mL, 0.60 mmol) was added, and this was stirred for five minutes. The reaction mixture was stirred at room temperature for 18 hours, and then the reaction solution was concentrated under reduced pressure to give a concentrated residue. The resulting concentrated residue was dissolved in DCM (1 mL), the mixture was cooled in an ice bath, and then TFA (1 mL) was added. After stirring the reaction mixture at room temperature for 15 minutes, the reaction solution was concentrated under reduced pressure.

Under nitrogen atmosphere, DMF (0.4 mL) was added at room temperature to a mixture of the obtained concentrated residue and 4-azidobenzyl (4-nitrophenyl)carbonate (62.9 mg, 0.20 mmol). The mixture was cooled in an ice bath, and then triethylamine (139 µL, 1.00 mmol) was added to it. The reaction mixture was stirred at 35°C for one day and then purified by reverse-phase silica gel column chromatography (0.1% aqueous formic acid solution/0.1% formic acid-acetonitrile solution) to give 3-((((4-azidobenzyl)oxy)carbonyl)(2-(*tert*-butyldisulfanyl)ethyl)amino)propanoic acid (Compound nk35) (47.5 mg, 58%).
LCMS (ESI) m/z = 411 (M-H)-
Retention time: 0.91 minutes (analysis condition SQDFA05)

### Synthesis of cyanomethyl 3-((((4-azidobenzyl)oxy)carbonyl)(2-(tert-butyldisulfanyl)ethyl)amino)propanoate (Compound nk36)

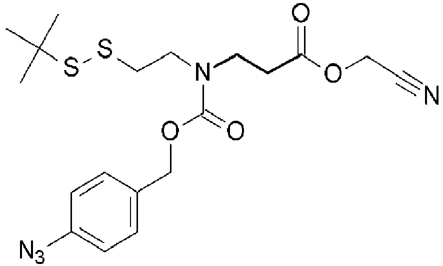

Under nitrogen atmosphere, 3-((((4-azidobenzyl)oxy)carbonyl)(2-(*tert*-butyldisulfanyl)ethyl)amino)propanoic acid (Compound nk35) (47.5 mg, 0.115 mmol) and *N,N*-diisopropylethylamine (DIPEA) (0.040 mL, 0.230 mmol) were dissolved in acetonitrile (461 µL), 2-bromoacetonitrile (0.012 mL, 0.173 mmol) was added, and the mixture was stirred at room temperature for 16 hours. The reaction solution was concentrated to give cyanomethyl 3-((((4-azidobenzyl)oxy)carbonyl)(2-(*tert*-butyldisulfanyl)ethyl)amino)propanoate (Compound nk36) as a crude product. Cyanomethyl 3-((((4-azidobenzyl)oxy)carbonyl)(2-(*tert*-butyldisulfanyl)ethyl)amino)propanoate (Compound nk36) obtained as a crude product was dissolved in acetonitrile (0.575 mL) and was directly used in the next step.
LCMS (ESI) m/z = 452 (M+H)+
Retention time: 1.00 minute (analysis condition SQDFA05)

### Synthesis of (2R,3S,4R,5R)-2-((((((2R,3S,4R,5R)-5-(4-amino-2-oxopyrimidin-1(2H)-yl)-4-hydroxy-2-((phos phonooxy)methyl)tetrahydrofuran-3-yl)oxy)(hydroxy)phosphoryl)oxy)methyl)-5-(6-amino-9H-p urin-9-yl)-4-hydroxytetrahydrofuran-3-yl 3-((((4-azidobenzyl)oxy)carbonyl)(2-(tert-butyldisulfanyl)ethyl)amino)propanoate (Compound nk37)

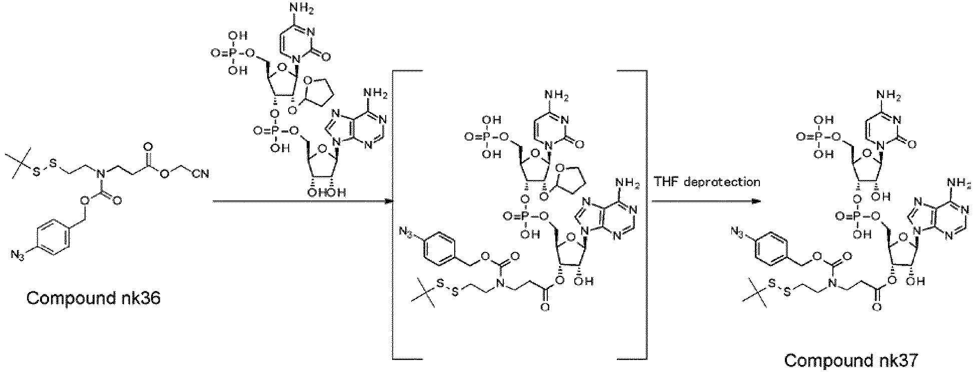

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (41.5 mg, 0.058 mmol) was dissolved in Buffer A (27.7 mL), a solution of cyanomethyl 3-((((4-azidobenzyl)oxy)carbonyl)(2-(*tert*-butyldisulfanyl)ethyl)amino)propanoate (Compound nk36) in 0.2 M acetonitrile (0.576 mL, 0.115 mmol) was added to it in three portions (0.192-mL each was added at the start of the reaction, and ten minutes and 30 minutes after starting the reaction, three times additions in total), and the mixture was stirred at room temperature for 90 minutes. The reaction solution was cooled to 0°C, and then trifluoroacetic acid (1.39 mL) was added to it. After stirring the reaction solution at 0°C for ten minutes, this was purified by reverse-phase silica gel column chromatography (0.05% aqueous trifluoroacetic acid solution/0.05% trifluoroacetic acid-acetonitrile solution) to give the titled compound (Compound nk37) (28.1 mg, 47%).
LCMS (ESI) m/z = 1047.5 (M+H)+
Retention time: 0.63 minutes (analysis condition SQDFA05)

### Synthesis of tert-butyl (R)-(1-(tert-butyldisulfanyl)-4-diazo-3-oxobutan-2-yl)carbamate (Compound nk38)

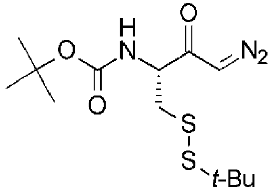

*N-*(*tert*-butoxycarbonyl)-S-(*tert*-butylthio)-L-cysteine (Boc-Cys(StBu)-OH) (309 mg, 1.00 mmol) was dissolved in THF (2 mL), *N,N*-diisopropylethylamine (DIPEA) (0.175 mL, 1.00 mmol) was added to it, this mixture was cooled to -15°C, and ethyl chloroformate (0.105 mL, 1.10 mmol) was added to it dropwise. The reaction mixture solution was stirred at -15°C for six hours, and then further stirred at room temperature for 12 hours. After cooling the reaction solution in an ice bath, acetic acid (0.114 mL, 2.00 mmol) was added to it, and this mixture was diluted using ethyl acetate and then washed with saturated saline solution. The organic layer was dried over anhydrous sodium sulfate, filtered, and then concentrated under reduced pressure. The resulting residue was purified by normal-phase silica gel column chromatography (hexane/ethyl acetate) to give *tert*-butyl (R)-(1-(*tert*-butyldisulfanyl)-4-diazo-3-oxobutan-2-yl)carbamate (Compound nk38) (55.1 mg, 17%).
LCMS (ESI) m/z = 334 (M+H)+
Retention time: 0.87 minutes (analysis condition SQDFA05)

### Synthesis of (R)-3-((tert-butoxycarbonyl)amino)-4-(tert-butyldisulfanyl)butanoic acid (Compound nk39)

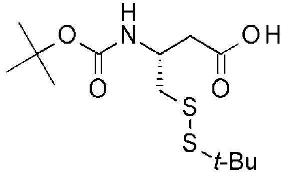

*tert*-Butyl (R)-(1-(*tert*-butyldisulfanyl)-4-diazo-3-oxobutan-2-yl)carbamate (Compound nk38) (85 mg, 0.255 mmol) was dissolved in THF (1.7 mL), *N,N*-diisopropylethylamine (DIPEA) (0.111 mL, 0.637 mmol) was added to it. After cooling the mixture solution in an ice bath, silver(I) trifluoroacetate (11.3 mg, 0.051 mmol) was added to it. Under a shaded condition, the reaction mixture solution was stirred at 0°C for two hours, and further stirred at room temperature for three days. The reaction solution was concentrated under reduced pressure, and the resulting residue was purified by reverse-phase silica gel column chromatography (0.1% aqueous formic acid solution/0.1% formic acid-acetonitrile solution) to give (R)-3-((*tert*-butoxycarbonyl)amino)-4-(*tert*-butyldisulfanyl)butanoic acid (Compound nk39) (30 mg, 36%).
LCMS (ESI) m/z = 322 (M-H)-
Retention time: 0.79 minutes (analysis condition SQDFA05)

### Synthesis of (R)-3-((((4-azidobenzyl)oxy)carbonyl)amino)-4-(tert-butyldisulfanyl)butanoic acid (Compound nk40)

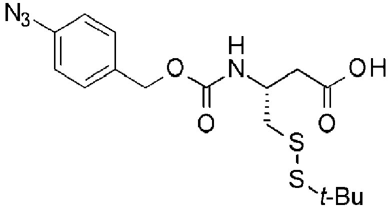

A 4N hydrochloric acid/1,4-dioxane solution (1.0 mL) was added to (R)-3-((*tert*-butoxycarbonyl)amino)-4-(*tert*-butyldisulfanyl)butanoic acid (Compound nk39) (30 mg, 0.093 mmol), the mixture was stirred at room temperature for 60 minutes, and then the reaction solution was concentrated under reduced pressure.

Under nitrogen atmosphere, DMF (0.93 mL) was added at room temperature to a mixture of the obtained concentrated residue and 4-azidobenzyl (4-nitrophenyl)carbonate (29.2 mg, 0.093 mmol). The mixture was cooled in an ice bath, and then triethylamine (32 µL, 0.233 mmol) was added to it. The reaction mixture was stirred at 30°C for three days and then purified by reverse-phase silica gel column chromatography (0.1% aqueous formic acid solution/0.1% formic acid-acetonitrile solution) to give (R)-3-((((4-azidobenzyl)oxy)carbonyl)amino)-4-(*tert*-butyldisulfanyl)butanoic acid (Compound nk40) (18.8 mg, 51%).
LCMS (ESI) m/z = 397 (M-H)-
Retention time: 0.84 minutes (analysis condition SQDFA05)

### Synthesis of cyanomethyl (R)-3-((((4-azidobenzyl)oxy)carbonyl)amino)-4-(tert-butyldisulfanyl)butanoate (Compound nk41)

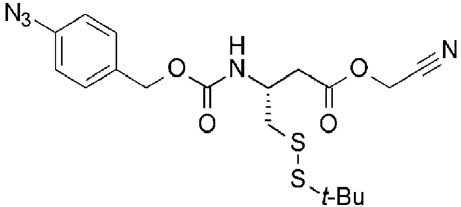

Under nitrogen atmosphere, (R)-3-((((4-azidobenzyl)oxy)carbonyl)amino)-4-(*tert*-butyldisulfanyl)butanoic acid (Compound nk40) (18.8 mg, 0.047 mmol) and *N,N*-diisopropylethylamine (DIPEA) (0.012 mL, 0.071 mmol) were dissolved in acetonitrile (236 µL), 2-bromoacetonitrile (0.005 mL, 0.071 mmol) was added, and the mixture was stirred at room temperature for six hours. The reaction solution was concentrated to give cyanomethyl (R)-3-((((4-azidobenzyl)oxy)carbonyl)amino)-4-(*tert*-butyldisulfanyl)butanoate (Compound nk41) as a crude product. Cyanomethyl (R)-3-((((4-azidobenzyl)oxy)carbonyl)amino)-4-(tert-butyldisulfanyl)butanoate (Compound nk41) obtained as a crude product was dissolved in acetonitrile (0.236 mL) and was directly used in the next step.
LCMS (ESI) m/z = 436 (M-H)-
Retention time: 0.93 minutes (analysis condition SQDFA05)

### Synthesis of (2R,3S,4R,5R)-2-((((((2R,3S,4R,5R)-5-(4-amino-2-oxopyrimidin-1(2H)-yl)-4-hydroxy-2-((phos phonooxy)methyl)tetrahydrofuran-3-yl)oxy)(hydroxy)phosphoryl)oxy)methyl)-5-(6-amino-9H-p urin-9-yl)-4-hydroxytetrahydrofuran-3-yl (3R)-3-((((4-azidobenzyl)oxy)carbonyl)amino)-4-(tert-butyldisulfanyl)butanoate (Compound nk42)

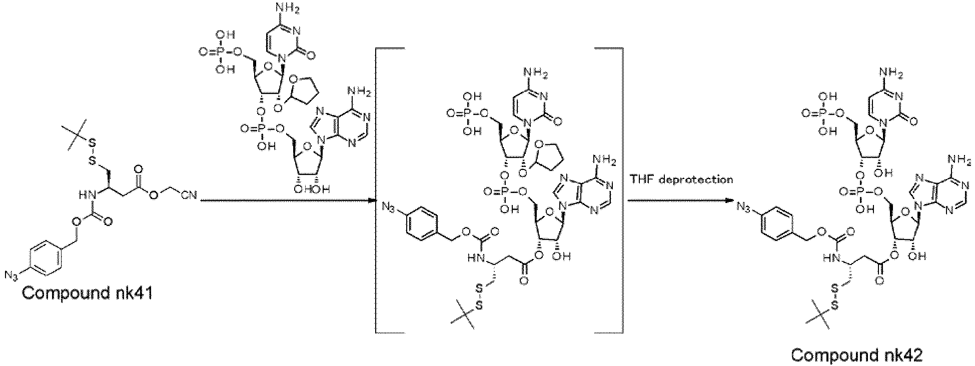

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (34.0 mg, 0.047 mmol) was dissolved in Buffer A (40 mL), a solution of cyanomethyl (R)-3-((((4-azidobenzyl)oxy)carbonyl)amino)-4-(*tert*-butyldisulfanyl)butanoate (Compound nk41) in 0.2 M acetonitrile (0.236 mL, 0.047 mmol) was added to it, and the mixture was stirred at room temperature for 90 minutes. The reaction solution was cooled to 0°C, and then trifluoroacetic acid (2 mL) was added to it. After stirring the reaction solution at 0°C for ten minutes, the reaction solution was purified by reverse-phase silica gel column chromatography (0.05% aqueous trifluoroacetic acid solution/0.05% trifluoroacetic acid-acetonitrile solution) to give the titled compound (Compound nk42) (10.3 mg, 21%).
LCMS (ESI) m/z = 1033.6 (M+H)+
Retention time: 0.58 minutes (analysis condition SQDFA05)

### Synthesis of (2S,4R)-1-(((9H-fluoren-9-yl)methoxy)carbonyl)-4-(methyldisulfanyl)pyrrolidin-2-carboxylic acid (Compound nk43)

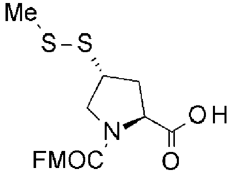

(2S,4R)-1-(((9*H*-fluoren-9-yl)methoxy)carbonyl)-4-(tritylthio)pyrrolidin-2-carboxylic acid (120 mg, 0.196 mmol) was dissolved in DCM (2 mL), TFA (0.100 mL, 1.30 mmol) and triethylsilane (0.060 mL, 0.38 mmol) were added to it, and the reaction mixture solution was stirred at room temperature for one hour. *S*-Methyl methanethiosulfonate (0.092 mL, 0.98 mmol) and *N,N*-diisopropylethylamine (DIPEA) (0.240 mL, 1.37 mmol) were further added to the reaction solution, and the reaction mixture solution was stirred at room temperature for one hour.

The reaction solution was concentrated under reduced pressure, and purified by reverse-phase silica gel column chromatography (0.1% aqueous formic acid solution/0.1% formic acid-acetonitrile solution) to give (2S,4R)-1-(((9*H*-fluoren-9-yl)methoxy)carbonyl)-4-(methyldisulfanyl)pyrrolidin-2-carboxylic acid (Compound nk43) (77.8 mg, 95%).
LCMS (ESI) m/z = 416 (M+H)+
Retention time: 0.87 minutes (analysis condition SQDFA05)

### Synthesis of (2S,4R)-4-(methyldisulfanyl)pyrrolidin-2-carboxylic acid (Compound nk44)

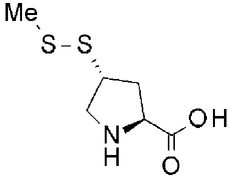

4-(3-Phenylpropyl)piperidine (0.102 mL, 0.481 mmol) was added to a solution of (2S,4R)-1-(((9*H*-fluoren-9-yl)methoxy)carbonyl)-4-(methyldisulfanyl)pyrrolidin-2-carboxylic acid (Compound nk43) (100 mg, 0.241 mmol) in DCM (0.96 mL), and this was stirred at room temperature for 60 minutes. The reaction solution was concentrated under reduced pressure, and purified by reverse-phase silica gel column chromatography (0.1% aqueous formic acid solution/0.1% formic acid-acetonitrile) to give (2S,4R)-4-(methyldisulfanyl)pyrrolidin-2-carboxylic acid (Compound nk44) (39.5 mg, 85%).
LCMS (ESI) m/z = 194 (M+H)+
Retention time: 0.25 minutes (analysis condition SQDFA05)

### Synthesis of (2S,4R)-1-(((4-azidobenzyl)oxy)carbonyl)-4-((R)-methylsulfinothioyl)pyrrolidin-2-carboxylic acid (Compound nk45)

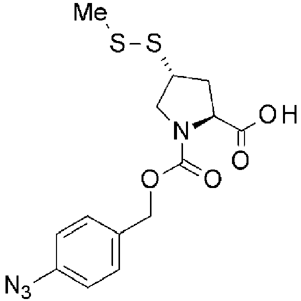

Under nitrogen atmosphere, DMSO (0.41 mL) was added to a mixture of (2S,4R)-4-(methyldisulfanyl)pyrrolidin-2-carboxylic acid (Compound nk44) (39.5 mg, 0.204 mmol) and 4-azidobenzyl (4-nitrophenyl)carbonate (64.2 mg, 0.204 mmol) at room temperature. The mixture was cooled in an ice bath, and then triethylamine (71.2 µL, 0.511 mmol) was added to it. The reaction mixture was stirred at 30°C for four hours and then purified by reverse-phase silica gel column chromatography (0.1% aqueous formic acid solution/0.1% formic acid-acetonitrile solution) to give (2S,4R)-1-(((4-azidobenzyl)oxy)carbonyl)-4-((R)-methylsulfinothioyl)pyrrolidin-2-carboxylic acid (Compound nk45) (74.8 mg, 99%).
LCMS (ESI) m/z = 367 (M-H)-
Retention time: 0.77 minutes (analysis condition SQDFA05)

### Synthesis of 2-(cyanomethyl) 1-(4-azidobenzyl) (2S,4R)-4-((R)-methylsulfinothioyl)pyrrolidin-1,2-dicarboxylate (Compound nk46)

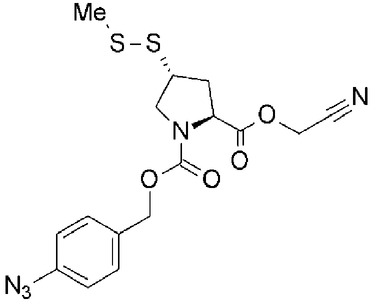

Under nitrogen atmosphere, (2S,4R)-1-(((4-azidobenzyl)oxy)carbonyl)-4-((R)-methylsulfinothioyl)pyrrolidin-2-carboxylic acid (Compound nk45) (74 mg, 0.20 mmol) and *N,N*-diisopropylethylamine (DIPEA) (0.052 mL, 0.30 mmol) were dissolved in acetonitrile (400 µL), 2-bromoacetonitrile (0.020 mL, 0.30 mmol) was added, and the mixture was stirred at room temperature for 17 hours. The reaction solution was concentrated to give 2-(cyanomethyl) 1-(4-azidobenzyl) (2S,4R)-4-((R)-methylsulfinothioyl)pyrrolidin-1,2-dicarboxylate (Compound nk46) as a crude product. 2-(Cyanomethyl) 1-(4-azidobenzyl) (2S,4R)-4-((R)-methylsulfinothioyl)pyrrolidin-1,2-dicarboxylate (Compound nk46) obtained as a crude product was dissolved in acetonitrile (1.0 mL) and was directly used in the next step.
LCMS (ESI) m/z = 408 (M+H)+
Retention time: 0.88 minutes (analysis condition SQDFA05)

### Synthesis of 1-(4-azidobenzyl) 2-((2R,3S,4R,5R)-2-((((((2R,3S,4R,5R)-5-(4-amino-2-oxopyrimidin-1(2H)-yl)-4-hydroxy-2-((ph osphonooxy)methyl)tetrahydrofuran-3-yl)oxy)(hydroxy)phosphoryl)oxy)methyl)-5-(6-amino-9H -purin-9-yl)-4-hydroxytetrahydrofuran-3-yl) (2S,4R)-4-(methyldisulfanyl)pyrrolidin-1,2-dicarboxylate (Compound nk47)

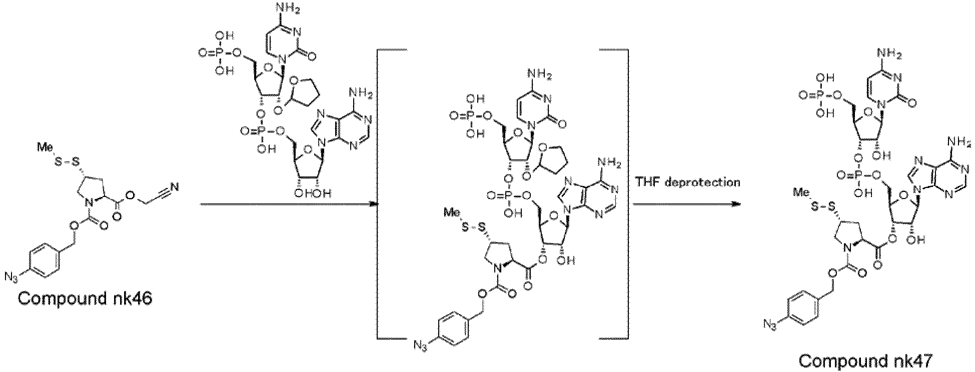

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (72.2 mg, 0.100 mmol) was dissolved in Buffer A (48 mL), a solution of 2-(cyanomethyl) 1-(4-azidobenzyl) (2S,4R)-4-((R)-methylsulfinothioyl)pyrrolidin-1,2-dicarboxylate (Compound nk46) in 0.2 M acetonitrile (1.00 L, 0.20 mmol) was added to it in three portions (0.33-mL each was added at the start of the reaction, and ten minutes and 30 minutes after starting the reaction, three times additions in total), and the mixture was stirred at room temperature for 90 minutes. The reaction solution was cooled to 0°C, and then trifluoroacetic acid (2.4 mL) was added to it. After stirring the reaction solution at 0°C for ten minutes and further stirred at room temperature for ten minutes, this was purified by reverse-phase silica gel column chromatography (0.05% aqueous trifluoroacetic acid solution/0.05% trifluoroacetic acid-acetonitrile solution) to give the titled compound (Compound nk47) (23.5 mg, 23%).
LCMS (ESI) m/z = 1001 (M-H)-
Retention time: 0.53 minutes (analysis condition SQDFA05)

### Synthesis of methyl 4-(((azidomethoxy)carbonyl)(2-(tert-butyldisulfanyl)ethyl)amino)butanoate (Compound nk50)

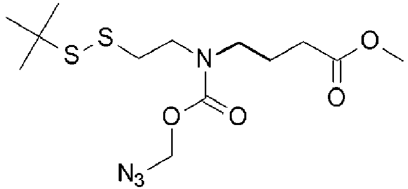

2-(*tert*-Butyldisulfanyl)ethan-1-amine hydrochloride salt (Compound nk31) (81 mg, 0.40 mmol) and *N,N*-diisopropylethylamine (0.279 mL, 1.60 mmol) were dissolved in acetonitrile (1.6 mL). After cooling the mixture in an ice bath, methyl 4-bromobutyrate (0.075 mL, 0.80 mmol) was added. The reaction mixture was stirred for five minutes and then stirred at 60°C for 25 hours. The reaction solution was then concentrated under reduced pressure to give a concentrated residue.

The obtained concentrated residue was dissolved in acetonitrile (1.6 mL), and *N,N*-diisopropylethylamine (0.14 mL, 0.80 mmol) was added to it. This mixture was cooled to -20°C, and chloromethyl chloroformate (51.6 mg, 0.400 mmol) was added to it. The reaction mixture was stirred at 0°C for 30 minutes and water (7.2 µL, 0.40 mmol) was added to it. The reaction mixture was then stirred at room temperature for ten minutes, and then the reaction solution was concentrated under reduced pressure. The resulting concentrated residue was dissolved in acetonitrile (1.6 mL), and tetrabutylammonium azide (569 mg, 2.00 mmol) was added to it. The reaction mixture was stirred at room temperature for three days, and purified by reverse-phase silica gel column chromatography (0.05% aqueous trifluoroacetic acid solution/0.05% trifluoroacetic acid-acetonitrile solution) to give methyl 4-(((azidomethoxy)carbonyl)(2-(*tert*-butyldisulfanyl)ethyl)amino)butanoate (Compound nk50) (89.7 mg, 62%).
LCMS (ESI) m/z = 365 (M+H)+
Retention time: 0.92 minutes (analysis condition SQDFA05)

### Synthesis of 4-(((azidomethoxy)carbonyl)(2-(tert-butyldisulfanyl)ethyl)amino)butanoic acid (Compound nk51)

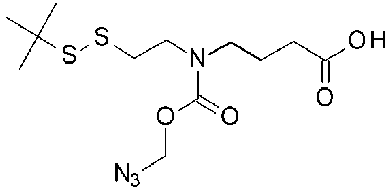

After dissolving methyl 4-(((azidomethoxy)carbonyl)(2-(*tert*-butyldisulfanyl)ethyl)amino)butanoate (Compound nk50) (84 mg, 0.23 mmol) in methanol (0.80 mL), a 0.6 M lithium hydroxide solution (methanol:water = 1:1, 0.768 mL) was added, and the reaction solution was stirred at room temperature for two hours. After cooling the reaction mixture in an ice bath, 1 M hydrochloric acid (0.5 mL) was added, and then this was purified by reverse-phase silica gel column chromatography (0.1% aqueous formic acid solution/0.1% formic acid-acetonitrile solution) to give 4-(((azidomethoxy)carbonyl)(2-(*tert*-butyldisulfanyl)ethyl)amino)butanoic acid (Compound nk51) (75.6 mg, 94%).
LCMS (ESI) m/z = 349 (M-H)-
Retention time: 0.78 minutes (analysis condition SQDFA05)

### Synthesis of cyanomethyl 4-(((azidomethoxy)carbonyl)(2-(tert-butyldisulfanyl)ethyl)amino)butanoate (Compound nk52)

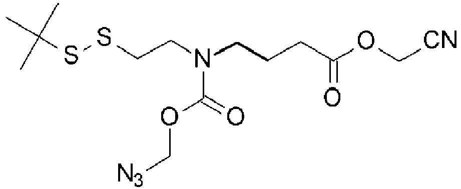

Under nitrogen atmosphere, 4-(((azidomethoxy)carbonyl)(2-(*tert*-butyldisulfanyl)ethyl)amino)butanoic acid (Compound nk51) (75.6 mg, 0.216 mmol) and *N,N*-diisopropylethylamine (DIPEA) (0.094 mL, 0.54 mmol) were dissolved in acetonitrile (539 µL), 2-bromoacetonitrile (0.029 mL, 0.43 mmol) was added, and the mixture was stirred at room temperature for two hours. The reaction solution was concentrated to give cyanomethyl 4-(((azidomethoxy)carbonyl)(2-(*tert*-butyldisulfanyl)ethyl)amino)butanoate (Compound nk52) as a crude product. Cyanomethyl 4-(((azidomethoxy)carbonyl)(2-(*tert*-butyldisulfanyl)ethyl)amino)butanoate (Compound nk52) obtained as a crude product was dissolved in acetonitrile (1.08 mL) and was directly used in the next step.
LCMS (ESI) m/z = 390 (M+H)+
Retention time: 0.89 minutes (analysis condition SQDFA05)

### Synthesis of (2R,3S,4R,5R)-2-((((((2R,3S,4R,5R)-5-(4-amino-2-oxopyrimidin-1(2H)-yl)-4-hydroxy-2-((phos phonooxy)methyl)tetrahydrofuran-3-yl)oxy)(hydroxy)phosphoryl)oxy)methyl)-5-(6-amino-9H-p urin-9-yl)-4-hydroxytetrahydrofuran-3-yl 4-(((azidomethoxy)carbonyl)(2-(tert-butyldisulfanyl)ethyl)amino)butanoate (Compound nk53)

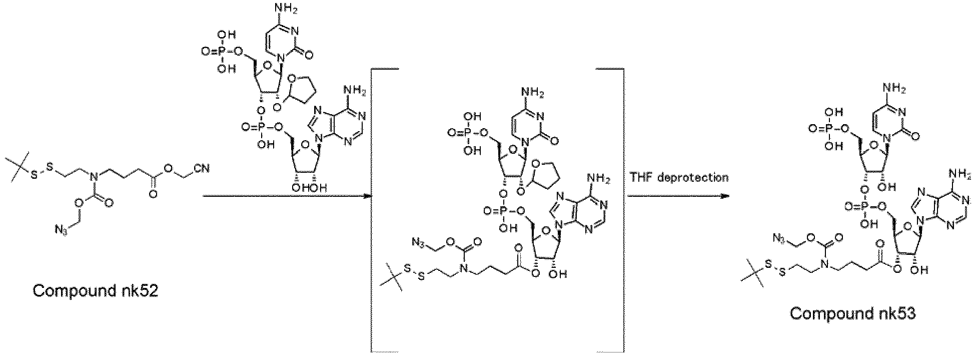

((2R,3R,4R,5R)-5-(4-amino-2-oxopyrimidin-1(2*H*)-yl)-3-(((((2R,3S,4R,5R)-5-(6-amino -9*H*-purin-9-yl)-3,4-dihydroxytetrahydrofuran-2-yl)methoxy)(hydroxy)phosphoryl)oxy)-4-((tetra hydrofuran-2-yl)oxy)tetrahydrofuran-2-yl)methyl dihydrogenphosphate (Compound pc01) (67 mg, 0.092 mmol) was dissolved in Buffer A (45 mL), a solution of cyanomethyl 4-(((azidomethoxy)carbonyl)(2-(*tert*-butyldisulfanyl)ethyl)amino)butanoate (Compound nk52) in 0.2 M acetonitrile (0.92 mL, 0.18 mmol) was added to it in three portions (0.31-mL each was added at the start of the reaction, and ten minutes and 30 minutes after starting the reaction, three times additions in total), and the mixture was stirred at room temperature for 90 minutes. The reaction solution was cooled to 0°C, and then trifluoroacetic acid (2.3 mL) was added to it. The reaction solution was stirred at 0°C for ten minutes and further stirred at room temperature for ten minutes, and then the reaction solution was purified by reverse-phase silica gel column chromatography (0.05% aqueous trifluoroacetic acid solution/0.05% trifluoroacetic acid-acetonitrile solution) to give the titled compound (Compound nk53) (31.0 mg, 34%).
LCMS (ESI) m/z = 985.5 (M+H)+
Retention time: 0.54 minutes (analysis condition SQDFA05)

### [Example 8] Synthesis of aminoacyl-tRNAs for translation initiation, which have a protecting group on the amino group

### 8-1. Synthesis of aminoacyl-tRNA for incorporation of an amino acid analog at the N terminus

10x Ligation buffer (500 mM HEPES-KOH (pH 7.5), 200 mM MgCl₂) (4 µL), 10 mM ATP (4 µL), and nuclease free water (5.6 µL) were added to 50 µM transcribed tRNAfMetCAU(-CA) (sequence number: R-1) (20 µL). The mixture was heated at 95°C for two minutes and then incubated at room temperature for five minutes to perform refolding of tRNA. Ten units/µL T4 RNA ligase (New England Biolabs) (2.4 µL) and a 5 mM solution of aminoacylated pCpA (nk-34, 37, 42, 47, or 53) in DMSO (4 µL) were added, and ligation reaction was carried out at 16°C for 45 minutes. Aminoacylated tRNA (Compound AAtR-10, 11, 12, 13, or 14) was collected by phenol-chloroform extraction followed by ethanol precipitation. Aminoacylated tRNA (Compound AAtR-10, 11, 12, or 13) was dissolved in 1 mM sodium acetate immediately before adding to the translation mixture.

### Compound AAtR-10

Acbz-(tBuSSEt)Gly-tRNAfMetCAU (SEQ ID NO: 3)

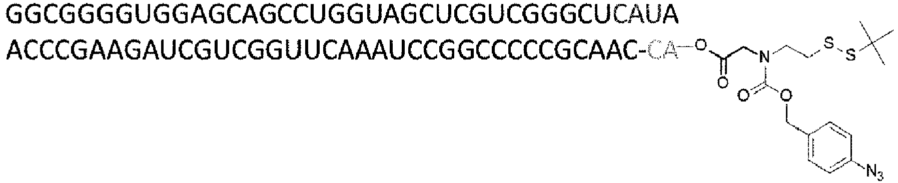
GGCGGGGUGGAGCAGCCUGGUAGCUCGUCGGGCUCAUA
ACCCGAAGAUCGUCGGUUCAAAUCCGGCCCCCGCAAC-CA

### Compound AAtR-11

Acbz-(tBuSSEt)βAla-tRNAfMetCAU (SEQ ID NO: 3)

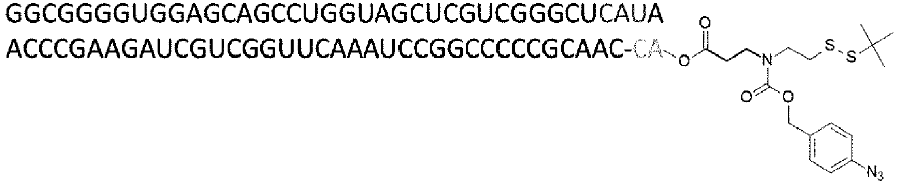
GGCGGGGUGGAGCAGCCUGGUAGCUCGUCGGGCUCAUA
ACCCGAAGAUCGUCGGUUCAAAUCCGGCCCCCGCAAC-CA
O
O
N
S
S
O
O
N3

### Compound AAtR-12

Acbz-βhCys(StBu)-tRNAfMetCAU (SEQ ID NO: 3)

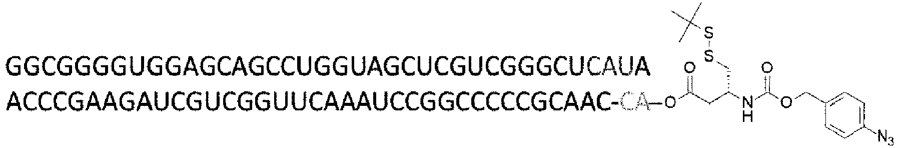
GGCGGGGUGGAGCAGCCUGGUAGCUCGUCGGGCUCAUA
ACCCGAAGAUCGUCGGUUCAAAUCCGGCCCCCGCAAC-CA-

### Compound AAtR-13

Acbz-Pro(SSMe)-tRNAfMetCAU (SEQ ID NO: 3)

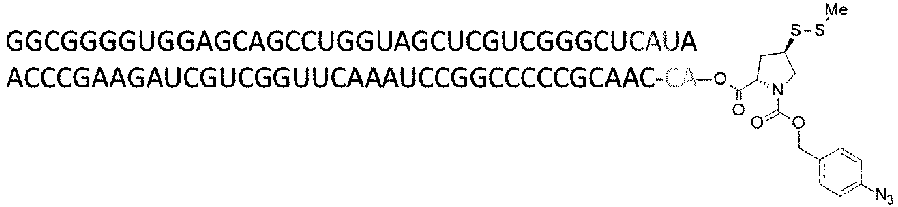
GGCGGGGUGGAGCAGCCUGGUAGCUCGUCGGGCUCAUA
ACCCGAAGAUCGUCGGUUCAAAUCCGGCCCCCGCAAC-CA-

### Compound AAtR-14

Azoc-(tBuSSEt)GABA-tRNAfMetCAU (SEQ ID NO: 3)

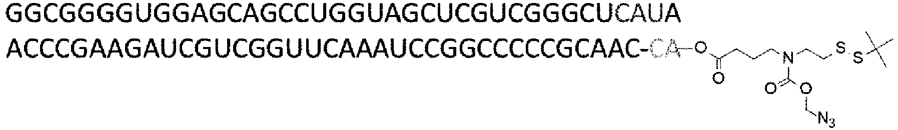
GGCGGGGUGGAGCAGCCUGGUAGCUCGUCGGGCUCAUA
ACCCGAAGAUCGUCGGUUCAAAUCCGGCCCCCGCAAC-CA-

### [Example 9]

### 9-1. Ribosomal synthesis of a peptide having at its N terminus an amino acid carrying protecting groups at each of the amino group and thiol group, using the initiation suppression method

Similarly to 3-2 mentioned above, tRNAfMet aminoacylated with an amino acid carrying protecting groups at each of the amino group and thiol group was added to a cell-free translation system and ribosomal synthesis was carried out using the initiation suppression method. The translation system used was PURE system, an *E. coli*-derived reconstituted cell-free protein synthesis system. Specifically, the synthesis was carried out as follows: 1 µM template mRNA and 250 µM each of Thr, Arg, Lys, Ala, Tyr, Trp, Ser, Leu, Pro, and Gly were added to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 10 mM magnesium acetate, 2 mM spermidine, 1 mM dithiothreitol, 0.5 mg/mL *E. coli* MRE600 (RNase-negative)-derived tRNA (Roche), 0.1 mM 10-HCO-H4 folate, 4 µg/ml creatine kinase, 3 µg/ml myokinase, 2 units/mL inorganic pyrophosphatase, 1.1 µg/mL nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), a tRNAfMet aminoacylated with an amino acid carrying protecting groups at each of the amino group and thiol group was added to the translation solution mixture, and this was incubated at 37°C for one hour.

### <Detection by mass spectrometry>

MALDI-TOF MS was used for mass spectrometric analyses of the peptides ribosomally synthesized in the cell-free translation system. Specifically, the analyses were carried out as follows: a solution to which 1 µM template mRNA (R-4) and Thr, Arg, Lys, Ala, Tyr, Trp, Ser, Leu, Pro, and Gly as each amino acid (each at a final concentration of 250 µM) were added to the above-described cell-free translation system was prepared, and tRNAfMet aminoacylated with an amino acid carrying protecting groups at each of the amino group and thiol group (Acbz-(tBuSSEt)Gly-tRNAfMetCAU (Compound AAtR-10),
Acbz-(*t*BuSSEt)βAla-tRNAfMetCAU (Compound AAtR-11),
Acbz-βhCys(StBu)-tRNAfMetCAU (Compound AAtR-12), Acbz-Pro(SSMe)-tRNAfMetCAU (Compound AAtR-13), or Azoc-(*t*BuSSEt)GABA-tRNAfMetCAU (Compound AAtR-14)) (final concentration of 25 µM) was added to the translation solution mixture, and this was incubated at 37°C for one hour.

Tris(2-carboxyethyl)phosphine (TCEP) (final concentration of 20 mM) was added to the obtained translation reaction product, and then this was incubated at 37°C for one hour. Translation products were analyzed by MALDI-TOF MS using α-cyano-4-hydroxycinnamic acid (CHCA) as the matrix.

As a result, MS peaks indicating the peptides introduced with a thiol group-carrying amino acid at the N terminus (Table 21, Peptide sequence numbers: Pep-61, Pep-62, Pep-63, Pep-64, and Pep-65) were observed.

**[Table 21]**

| Aminoacyl -tRNAfMetCAU (Compound number) | Peptide sequence number | Peptide sequence | MS theoretical value | MS measured value[M+H]+ |
|---|---|---|---|---|
| | | [Xaa]ThrArgThrLysAlaTyrTrpSerLeuProGly | | |
| Acbz-(tBuSSEt)Gly (AAtR-10) | Pep-61 | [HSEt-Gly]ThrArgThrLysAlaTyrTrpSerLeuProGly | 1395.7 | 1396.4 |
| Acbz-(tBuSSEt)*β* Ala (AAtR-11) | Pep-62 | [HSEt-βAla]ThrArgThrLysAlaTyrTrpSerLeuProGl y | 1409.7 | 1410.4 |
| Acbz-*β*hCys(StBu) (AAtR-12) | Pep-63 | [*βh*Cys]ThrArgThrLysAlaTyrTrpSerLeuProGly | 1395.7 | 1396.4 |
| Acbz-Pro(SSMe) (AAtR-13) | Pep-64 | [Pro(SH)]ThrArgThrLysAlaTyrTrpSerLeuProGly | 1407.7 | 1409.0 |
| Azoc-(tBuSSEt)GABA (AAtR-14) | Pep-65 | [HSEtGABA]ThrArgThrLysAlaTyrTrpSerLeuProGly | 1423.7 | 1425.0 |

### <Detection by electrophoresis>

For detection of peptides into which an amino acid carrying protecting groups at each of the amino group and thiol group is ribosomally introduced at the N terminus, radioisotope-labeled aspartic acid was used to perform peptide translation experiments. Specifically, the experiments were carried out as follows: a solution in which 1 µM template mRNA (R-4D) and each of amino acids, Thr, Arg, Lys, Ala, Tyr, Trp, Ser, Leu, Pro, and Gly (each at a final concentration of 250 µM), and ¹⁴C-aspartic acid (final concentration of 37 µM, Moravek Biochemicals, MC139) were added to the above-described cell-free translation system was prepared, and tRNAfMet aminoacylated with an amino acid carrying protecting groups at each of the amino group and thiol group (Acbz-(*t*BuSSEt)Gly-tRNAfMetCAU (Compound AAtR-10), Acbz-(*t*BuSSEt)βAla-tRNAfMetCAU (Compound AAtR-11), Acbz-βhCys(StBu)-tRNAfMetCAU (Compound AAtR-12), Acbz-Pro(SSMe)-tRNAfMetCAU (Compound AAtR-13), or Azoc-(*t*BuSSEt)GABA-tRNAfMetCAU (Compound AAtR-14)) (final concentration of 25 µM) was further added to the translation solution mixture, this was incubated at 37°C for one hour. Furthermore, as a control experiment, a translation reaction solution to which 250 µM Met was added instead of aminoacyl-tRNA for translation initiation, and a translation reaction solution to which aminoacyl-tRNA for translation initiation was not added were prepared, and they were incubated at 37°C for one hour.

A 2x sample buffer (TEFCO, cat No. 06-323) equivalent in amount to the obtained translation reaction solution was added, this was heated at 95°C for three minutes, and then subjected to electrophoresis (16% Peptide-PAGE mini, TEFCO, TB-162). The electrophoresed gel was dried using a Clear Dry Quick Dry Starter KIT (TEFCO, 03-278), exposed for 24 hours to an Imaging Plate (GE Healthcare, 28-9564-75), subjected to detection using a bioanalyzer system (Typhoon FLA 7000, GE Healthcare), and analyzed using ImageQuantTL (GE Healthcare).

As a result, for Acbz-(*t*BuSSEt)Gly, the production ratio of the peptide of interest (Peptide sequence number: Pep-66) and the side product (Peptide sequence number: Pep-9) was 96:10 (this shows the relative translation efficiency when the translation efficiency of a peptide of which translation was initiated by formylmethionine (Peptide sequence number: Pep-8), a control experiment, is regarded as 100) (Fig. 8). Furthermore, for Acbz-(*t*BuSSEt)βAla, the production ratio of the peptide of interest (Peptide sequence number: Pep-67) and the side product (Peptide sequence number: Pep-9) was 92:12; and for Acbz-βhCys(StBu), the production ratio of the peptide of interest (Peptide sequence number: Pep-68) and the side product (Peptide sequence number: Pep-9) was 104:11.

Furthermore, for Acbz-Pro(SSMe), the production ratio of the peptide of interest (Peptide sequence number: Pep-69) and the side product (Peptide sequence number: Pep-9) was 93:9; and for Azoc-(*t*BuSSEt)GABA, the production ratio of the peptide of interest (Peptide sequence number: Pep-70) and the side product (Peptide sequence number: Pep-9) was 108:10 (Fig. 9).

**[Table 22]**

| Aminoacyl -tRNAfMetCAU (Compound number) | Peptide sequence number | Peptide sequence |
|---|---|---|
| | | [Xaa]ThrArgThrLysAlaTyrTrpSerLeuProGlyAspAspAsp |
| | Pep-8 | MetThrArgThrLysAlaTyrTrpSerLeuProGlyAspAspAsp |
| | Pep-9 | ThrArgThrLysAlaTyrTrpSerLeuProGlyAspAspAsp |
| Acbz-(tBuSSEt)Gly (AAtR-10) | Pep-66 | [Acbz-(tBuSSEt)Gly]ThrArgThrLysAlaTyrTrpSerLeuProGlyAspAspAsp |
| Acbz-(tBuSSEt)*β*Ala (AAtR-11) | Pep-67 | [Acbz-(tBuSSEt)*β*Ala]ThrArgThrLysAlaTyrTrpSerLeuProGlyAspAspAsp |
| Acbz-*β*hCys(StBu) (AAtR-12) | Pep-68 | [Acbz-*β*hCys(StBu)]ThrArgThrLysAlaTyrTrpSerLeuProGlyAspAspAsp |
| Acbz-Pro(SSMe) (AAtR-13) | Pep-69 | [Acbz-Pro(SSMe)]ThrArgThrLysAlaTyrTrpSerLeuProGlyAspAspAsp |
| Azoc-(tBuSSEt)GABA (AAtR-14) | Pep-70 | [Azoc-(tBuSSEt)GABA]ThrArgThrLysAlaTyrTrpSerLeuProGlyAspAspAsp |

### [Example 10] Production of branched peptides from translational products

### 10-1. Synthesis of acyl-pCpA which enables production of branched peptides from translational products

### Synthesis of (2R,3S,4R,5R)-2-((((((2R,3R,4R,5R)-5-(4-amino-2-oxopyrimidin-1(2H)-yl)-2-((phosphonooxy) methyl)-4-((tetrahydrofuran-2-yl)oxy)tetrahydrofuran-3-yl)oxy)(hydroxy)phosphoryl)oxy)methy l)-5-(6-amino-9H-purin-9-yl)-4-hydroxytetrahydrofuran-3-yl 2-(bis(4-methoxyphenyl)(phenyl)methoxy)acetate(Compound nk48, DMT-HOGly-pCpA)

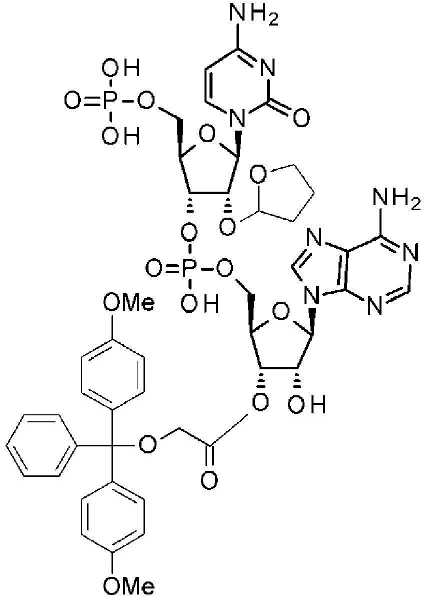

Under nitrogen atmosphere, cyanomethyl 2-(*bis*(4-methoxyphenyl)(phenyl)methoxy)acetate (115 mg, 0.28 mmol) synthesized by the method described in the literature (J. Am. Chem. Soc. 2007, 129, 6180) and ((2R,3R,4R,5R)-5-(4-amino-2-oxopyrimidin-1(2*H*)-yl)-3-(((((2R,3S,4R,5R)-5-(6-amino-9*H*-puri n-9-yl)-3,4-dihydroxytetrahydrofuran-2-yl)methoxy)(hydroxy)phosphoryl)oxy)-4-((tetrahydrofur an-2-yl)oxy)tetrahydrofuran-2-yl)methyl dihydrogenphosphate (Compound pc01) (167 mg, 0.14 mmol) were dissolved in DMSO (1.5 mL), and triethylamine (193 µL, 1.38 mmol) was added to this solution. The reaction mixture was stirred at 35°C for three hours and then purified by reverse-phase silica gel column chromatography (0.1% aqueous formic acid solution/0.1% formic acid-acetonitrile solution) to give (2R,3S,4R,5R)-2-((((((2R,3R,4R,5R)-5-(4-amino-2-oxopyrimidin-1(2*H*)-yl)-2-((phosphonooxy) methyl)-4-((tetrahydrofuran-2-yl)oxy)tetrahydrofuran-3-yl)oxy)(hydroxy)phosphoryl)oxy)methy 1)-5-(6-amino-9*H*-purin-9-yl)-4-hydroxytetrahydrofuran-3-yl 2-(*bis*(4-methoxyphenyl)(phenyl)methoxy)acetate (Compound nk48, DMT-HOGly-pCpA) (61.2 mg, 41%).
LCMS (ESI) m/z = 1081.6 (M-H)-
Retention time: 0.84 minutes (analysis condition SQDFA05)

### Synthesis of (2R,3S,4R,5R)-2-((((((2R,3S,4R,5R)-5-(4-amino-2-oxopyrimidin-1(2H)-yl)-4-hydroxy-2-((phos phonooxy)methyl)tetrahydrofuran-3-yl)oxy)(hydroxy)phosphoryl)oxy)methyl)-5-(6-amino-9H-p urin-9-yl)-4-hydroxytetrahydrofuran-3-yl 2-hydroxyacetate (Compound nk49, HOGly-pCpA)

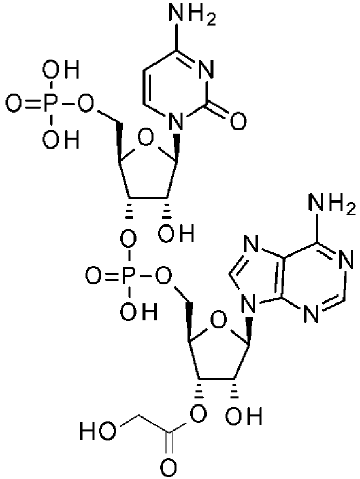

An 80% aqueous acetic acid solution (316 µL) was added to (2R,3S,4R,5R)-2-((((((2R,3R,4R,5R)-5-(4-amino-2-oxopyrimidin-1(2*H*)-yl)-2-((phosphonooxy) methyl)-4-((tetrahydrofuran-2-yl)oxy)tetrahydrofuran-3-yl)oxy)(hydroxy)phosphoryl)oxy)methy 1)-5-(6-amino-9*H*-purin-9-yl)-4-hydroxytetrahydrofuran-3-yl 2-(*bis*(4-methoxyphenyl)(phenyl)methoxy)acetate (Compound nk48, DMT-HOGly-pCpA) (15.8 mg, 0.015 mmol) obtained in the previous step, and this mixture was stirred at room temperature for 90 minutes and then purified by reverse-phase silica gel column chromatography (0.05% aqueous trifluoroacetic acid solution/0.05% trifluoroacetic acid-acetonitrile solution) to give the titled compound (Compound nk49, HOGly-pCpA) (8.4 mg, 81%).
LCMS (ESI) m/z = 709 (M-H)-
Retention time: 0.13 minutes (analysis condition SQDFA05)

### 10-2. Synthesis of acyl-tRNA using HOGly-pCpA

10x ligation buffer (500 mM HEPES-KOH (pH 7.5), 200 mM MgCl₂) (4 µL), 10 mM ATP (4 µL), and Nuclease free water (5.6 µL) were added to 50 µM transcribed tRNAGluCUG(-CA) (sequence number: R-2) (20 µL). The mixture was heated at 95°C for two minutes and then incubated at room temperature for five minutes to perform refolding of the tRNA. 10 units/µL T4 RNA ligase (New England Biolabs) (2.4 µL) and a 5 mM solution of HOGly-pCpA (nk-49) in DMSO (4 µL) were added, and ligation reaction was carried out at 16°C for 45 minutes. Acylated tRNA (Compound AAtR-15) was collected by phenol-chloroform extraction followed by ethanol precipitation. Acylated tRNA (Compound AAtR-15) was dissolved in 1 mM sodium acetate immediately before adding to the translation mixture.

### Compound AAtR-15

HOGly-tRNAGluCUG (SEQ ID NO: 10)

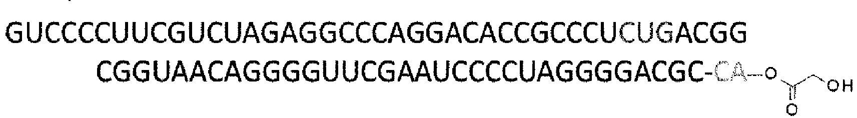
GUCCCCUUCGUCUAGAGGCCCAGGACACCGCCCUCUGACGG
CGGUAACAGGGGUUCGAAUCCCCUAGGGGACGC-CA-O
O
OH

To synthesize a branched peptide from a translated peptide, a peptide carrying Acbz-Cys(StBu) at its N terminus, a Cys-Pro-HOGly sequence, and a lysine with a side-chain amino group was ribosomally synthesized using the initiation suppression method as shown in the reaction formula below. Furthermore, after Acbz deprotection, a cyclic peptide was synthesized through cyclization reaction by native chemical ligation, and a branched peptide was synthesized by subsequent second reaction.

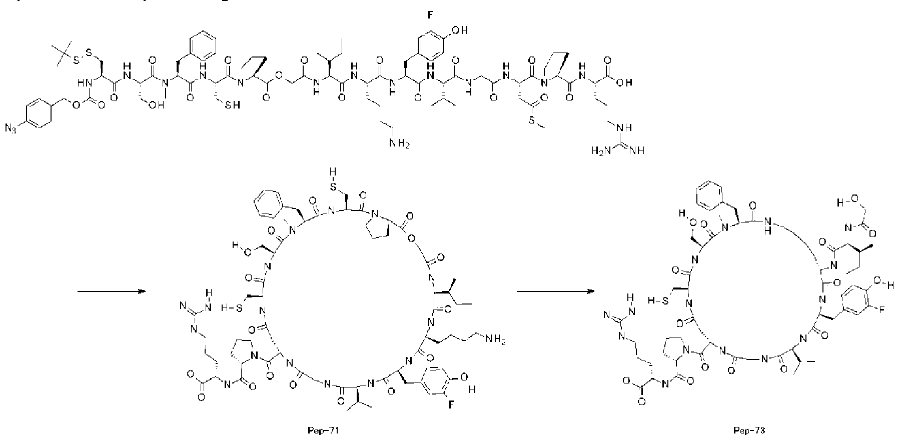

### 10-3. Ribosomal synthesis of a peptide having an N-terminal cysteine, a cysteinyl prolyl ester sequence, and a side-chain amino group using the initiation suppression method, followed by synthesis of a cyclic peptide by native chemical ligation

Template mRNA sequence number: R-18 (SEQ ID NO: 37)

GGGUUAACUUUAAGAAGGAGAUAUACAUAUGAGUUUUUGCCCGCAGAUUAAAUA
CGUUGGUCUUCCGCGUUAAGCUUCG

The translation system used was PURE system, an *E. coli*-derived reconstituted cell-free protein synthesis system. Specifically, the synthesis was carried out as follows: 1 µM template mRNA (R-18) and 250 µM each of amino acids, Ala, Arg, Cys, Gly, His, Ile, Lys, Pro, Ser, Thr, Trp, Val, and 3-fluoro-L-tyrosine (Tyr(3-F)), were added to a cell-free translation solution (1% (v/v) RNasein Ribonuclease inhibitor (Promega, N2111), 1 mM GTP, 1 mM ATP, 20 mM creatine phosphate, 50 mM HEPES-KOH (pH 7.6), 100 mM potassium acetate, 10 mM magnesium acetate, 2 mM spermidine, 1 mM dithiothreitol, 1.5 mg/mL *E. coli* MRE600 (RNase-negative)-derived tRNA (Roche), 4 µg/ml creatine kinase, 3 µg/ml myokinase, 2 units/mL inorganic pyrophosphatase, 1.1 µg/mL nucleoside diphosphate kinase, 0.6 µM methionyl-tRNA transformylase, 0.26 µM EF-G, 0.24 µM RF2, 0.17 µM RF3, 0.5 µM RRF, 2.7 µM IF1, 0.4 µM IF2, 1.5 µM IF3, 40 µM EF-Tu, 44 µM EF-Ts, 1.2 µM ribosome, 0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.4 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS (self-prepared proteins were basically prepared as His-tagged proteins)), 5 mM *N*-methyl phenylalanine (MePhe) was added. 20 µM AspSMe-tRNAGluAAG (Compound AAtR-9), 20 µM HOGly-tRNAGluCUG (Compound AAtR-15), and 20 µM Acbz-Cys(StBu)-tRNAfMetCAU (Compound AAtR-1) were further added to the translation solution mixture, and this was incubated at 37°C for one hour.

Next, a TCEP solution (pH 7.0) (500 mM, 1.2 µL), *N*-methyl-2-pyrrolidone (NMP) (10 µL), an EDTA solution (pH 8.0) (500 mM, Nacalai Tesque; product number 14362-24, 0.5 µL), and Nuclease-free water (0.5 µL) were mixed into the translation reaction solution (7.8 µL), and this was incubated at 37°C for 30 minutes. Progress of the Acbz deprotection reaction and cyclization reaction by native chemical ligation was confirmed by MALDI-TOF MS (Fig. 10-1). Analysis by MALDI-TOF MS was carried out by sampling 1 µL of the reaction solution and purifying the sample using SPE C-TIP (Nikkyo Technos) and using α-cyano-4-hydroxycinnamic acid (CHCA) as the matrix.

Compound obtained by ribosomal synthesis from template mRNA sequence number: R-18, followed by Acbz-group deprotection reaction and cyclization reaction by native chemical ligation

### [Cys*]Ser[MePhe]CysProHOGlyIleLys[Tyr(3-F)]ValGly[Asp*]ProArg (cyclized at the two * sites) (Pep-71)

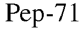

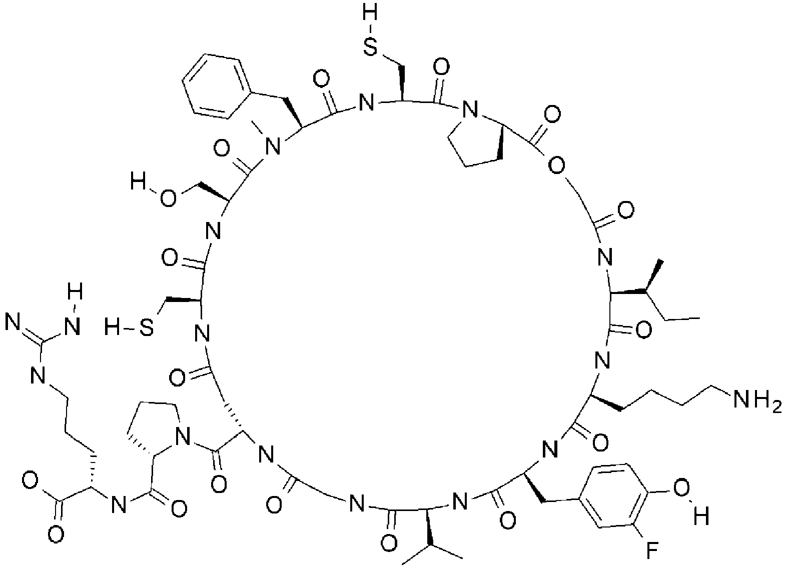

MALDI-TOF MS
m/z = 1556.4 (M+H)+ Calc. 1555.7

Side product generated by ribosomal synthesis from template mRNA sequence number: R-18, followed by Acbz-group deprotection reaction and cyclization reaction by native chemical ligation

### HOGlyIleLys[Tyr(3-F)]ValGly[Asp(SMe)]ProArg (Pep-72)

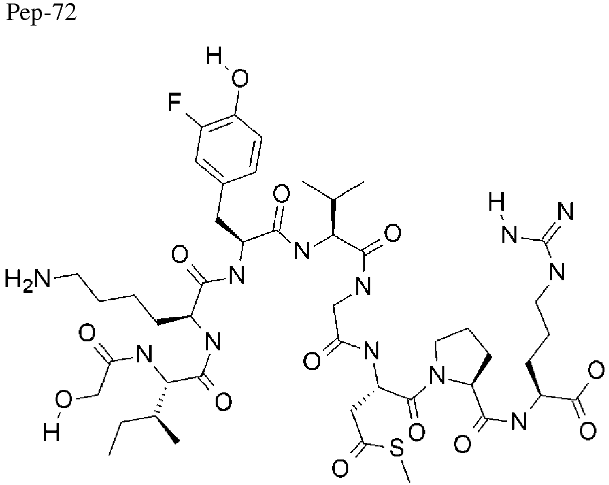

MALDI-TOF MS
m/z = 1053.3 (M+H)+ Calc. 1052.5

### 10-4. Reaction of producing an intramolecular branched peptide using the cyclic peptide Pep-71 synthesized from a translated peptide

*N*-methyl-2-pyrrolidone (NMP) (20 µL), a TCEP solution (pH 7.0) (500 mM, 5 µL), a bicine solution (pH 9.2) (2 M, 9 µL), an aqueous potassium hydroxide solution (5 M, 6 µL), and 4-(trifluoromethyl)benzenethiol (6.8 µL, 50 µL) were mixed into the aforementioned Pep-71-containing cyclization reaction solution (10 µL), and this mixture was incubated at 30°C for 24 hours.

Production of the branched peptide was confirmed by MALDI-TOF MS (Fig. 10-2, upper panel). As a result, the MS intensity ratio for desired branched peptide (Pep-73), side product (Pep-74), and cyclic peptide (Pep-71) was 20:6:74.

Analysis by MALDI-TOF MS was carried out by sampling 2 µL of reaction solution and purifying using SPE-C-TIP (Nikkyo Technos) and using α-cyano-4-hydroxycinnamic acid (CHCA) as the matrix.

Compound obtained by ribosomal synthesis from template mRNA sequence number: R-18 followed by Acbz-group deprotection reaction and cyclization reaction by native chemical ligation (Pep-73) 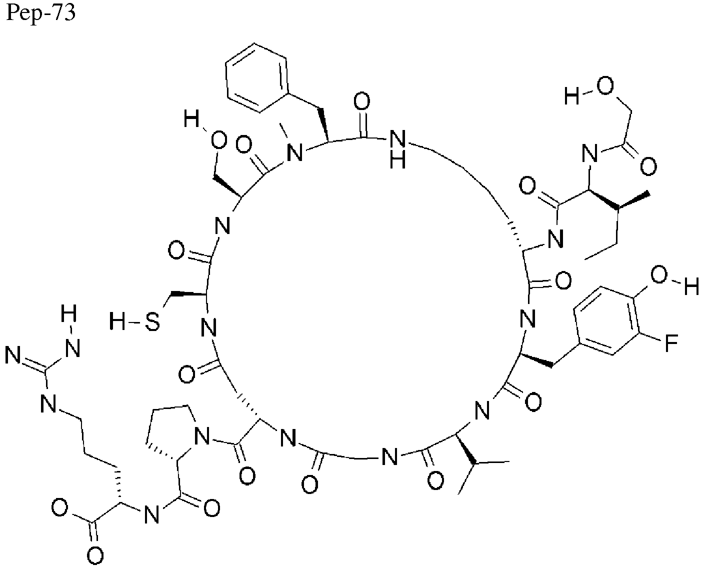 MALDI-TOF MS
m/z = 1356.3 (M+H)+ Calc. 1355.6

Side product produced in reaction for synthesizing the intramolecular branched peptide HOGlyIle[Lys*][Tyr(3-F)]ValGly[Asp*]ProArg (cyclized at the two * sites) (Pep-74) 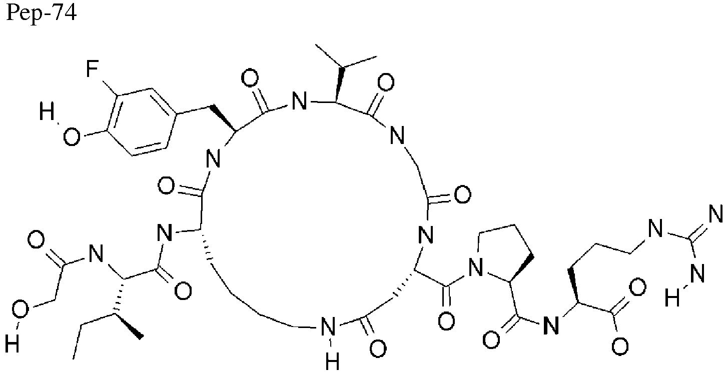 MALDI-TOF MS
m/z = 1005.3 (M+H)+ Calc. 1004.5

The results described above showed that while the branched peptide (Pep-73) was generated, the cyclic peptide which is an intermediate (Pep-71) was the major peak. Therefore, there was room for improvement. For more efficient synthesis of branched peptides, the branching reaction was further improved.

### 10-5. Reaction for synthesizing intramolecular branched peptide using cyclic peptide Pep-71 synthesized from a translated peptide, which is carried out under conditions where the branching reaction proceeds satisfactorily

*N*-methyl-2-pyrrolidone (NMP) (20 µL), a TCEP solution (1 M, 5 µL), a bicine solution (pH 9.2) (2 M, 8 µL), an aqueous potassium hydroxide solution (5 M, 7 µL), and 4-(trifluoromethyl)benzenethiol (6.8 µL, 50 µL) were mixed into the Pep-71-containing cyclization reaction solution (10 µL), and this mixture was incubated at 45°C for 14 hours.

The TCEP solution (1 M) was prepared by mixing TCEP hydrochloride salt (73.2 mg, 0.256 mmol) and an aqueous potassium hydroxide solution (8 M, 128 µL), using Nuclease free water.

Production of the branched peptide was confirmed by MALDI-TOF MS (Fig. 10-2, lower panel). As a result, the MS intensity ratio for the desired branched peptide (Pep-73), the side product (Pep-74), and the cyclic peptide (Pep-71) was 85:9:6. Analysis by MALDI-TOF MS was carried out by sampling 2 µL of the reaction solution and purifying the sample using SPE C-TIP (Nikkyo Technos) and using α-cyano-4-hydroxycinnamic acid (CHCA) as the matrix.

The results described above show that by improving the reaction conditions for branching, reaction to the branched peptide of interest through the series of reactions starting from the translation product proceeds in good yield.

On the other hand, since RNA was sufficiently stable under such series of reaction conditions according to the literature (WO 2013/100132A), the branching reaction can proceed in good yield also in peptide-RNA complexes without the RNA degradation.

### [Example 11] Construction of a display library cyclized by amide cyclization using the initiation suppression method and panning of target-binding peptides

A display library of cyclic peptides was produced using the initiation suppression method, and panning was carried out to perform an experiment to obtain binding peptides to GTPase KRas (KRAS).

### 11-1. Synthesis of pCpA-amino acids for display library construction

### Synthesis of (S)-cyanomethyl 3-(4-chlorophenyl)-2-(N-methylpenta-4-enamide)propanoate (Compound nk56, Pen-MePhe(4-Cl)-OCH₂CN)

The synthesis was carried out according to the scheme shown below.

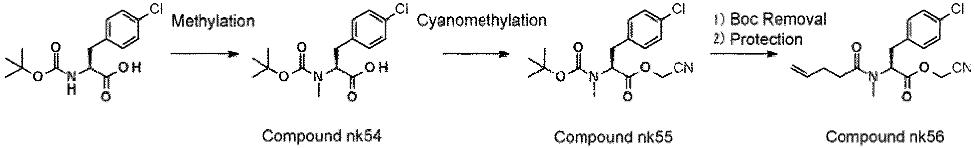

(S)-2-((*tert*-butoxycarbonyl)(methyl)amino)-3-(4-chlorophenyl)propanoic acid (Boc-Phe (4-Cl)-OH) (4.00 g, 13.3 mmol) was dissolved in tetrahydrofuran (THF) (120 mL) and then, sodium hydride (1.60 g, 40.0 mmol, 60% oil dispersion) and methyl iodide (9.48 g, 66.8 mmol) were added at 0°C. Using an oil bath, the reaction solution was stirred at 30°C for two days, and then the reaction was quenched with ice-water (50 mL). The mixed solution was washed three times with ethyl acetate, then pH of the aqueous layer was adjusted to 3 to 4 using sodium hydrogen sulfate, and this was extracted three times with ethyl acetate. The organic layers were combined and dried over anhydrous sodium sulfate, filtered, and concentrated. The resulting residue was purified by normal-phase silica gel column chromatography (dichloromethane/methanol) to give (S)-2-((*tert*-butoxycarbonyl)(methyl)amino)-3-(4-chlorophenyl)propanoic acid (Compound nk54, Boc-MePhe(4-Cl)-OH) (2.20 g, 53%).

Under nitrogen atmosphere, (S)-2-((*tert*-butoxycarbonyl)(methyl)amino)-3-(4-chlorophenyl)propanoic acid (Compound nk54, Boc-MePhe(4-Cl)-OH) (1.50 g, 4.78 mmol) obtained in the previous step was dissolved in dichloromethane (80 mL), *N*-ethyl-isopropylpropan-2-amine (DIPEA) (1.24 g, 9.59 mmol) and 2-bromoacetonitrile (2.28 g, 19.0 mmol) were added to the solution, and this was stirred at 25°C for 16 hours. The reaction solution was concentrated, and the resulting residue was purified by normal-phase silica gel column chromatography (petroleum ether/ethyl acetate) to give cyanomethyl (S)-2-((*tert*-butoxycarbonyl)(methyl)amino)-3-(4-chlorophenyl)propanoate (Compound nk55, Boc-MePhe(4-Cl)-OCH₂CN) (1.45 g, 86%).

Cyanomethyl (S)-2-((*tert*-butoxycarbonyl)(methyl)amino)-3-(4-chlorophenyl)propanoate (Compound nk55, Boc-MePhe(4-Cl)-OCH₂CN) (1.30 g, 3.68 mmol) obtained in the previous step was dissolved in dichloromethane (50 mL), and the solution was bubbled with hydrochloric acid gas and stirred at 20°C for one hour. The reaction solution was concentrated to give cyanomethyl (S)-3-(4-chlorophenyl)-2-(methylamino)propanoate (1.06 g) as a mixture.

The mixture (0.96 g) of cyanomethyl (S)-3-(4-chlorophenyl)-2-(methylamino)propanoate-containing obtained in the previous step was dissolved in dichloromethane (50 mL), triethylamine (840 mg, 8.30 mmol) was added at 0°C, and then to this mixture, a solution of penta-4-enoyl chloride (472 mg, 3.98 mmol) in dichloromethane (10 mL) was added dropwise. The reaction solution was stirred at 20°C for two hours and then concentrated, and the resulting residue was purified by normal-phase silica gel column chromatography (petroleum ether/ethyl acetate) to give (S)-cyanomethyl 3-(4-chlorophenyl)-2-(*N*-methylpenta-4-enamide)propanoate (Compound nk56, Pen-MePhe(4-Cl)-OCH₂CN) (0.918 g, 83%, two steps).
LCMS (ESI) m/z = 335 (M+H)+
Retention time: 2.21 minutes (analysis condition SMD method 5)

### Synthesis of (2S)-(2R,3S,4R,5R)-2-((((((2R,3R,4R,5R)-5-(4-amino-2-oxopyrimidin-1(2H)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9H-purin-9-yl)-4-hydroxytetrahydrofuran-3-yl 3-(4-chlorophenyl)-2-(N-methylpenta-4-enamide)propanoate (Compound nk57)

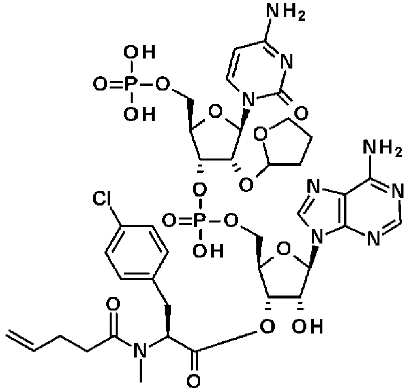

An aqueous solution (6.25 mL) of ((2R,3R,4R,5R)-5-(4-amino-2-oxopyrimidin-1(2*H*)-yl)-3-(((((2R,3S,4R,5R)-5-(6-amino-9*H*-puri n-9-yl)-3,4-dihydroxytetrahydrofuran-2-yl)methoxy)(hydroxy)phosphoryl)oxy)-4-((tetrahydrofur an-2-yl)oxy)tetrahydrofuran-2-yl)methyl dihydrogenphosphate (Compound pc01) (200 mg, 0.277 mmol) and a solution of cyanomethyl (S)-3-(3-chlorophenyl)-2-(penta-4-enamide)propanoate (Compound nk56, Pen-Phe(3-Cl)-OCH₂CN) (371 mg, 1.11 mmol) in acetonitrile (3.1 mL) were added to Buffer A (115 mL), and this was stirred at room temperature for two hours. The reaction solution was lyophilized, the resulting residue was purified by reverse-phase silica gel column chromatography (0.1% aqueous formic acid solution/0.1% formic acid-acetonitrile solution) to give a mixture (277 mg) of the titled compound (Compound nk57) and *N,N,N*-trimethylhexadecan-1-aminium chloride.
LCMS (ESI) m/z = 1000 (M+H)+
Retention time: 0.58 minutes (analysis condition SQDFA05)

### Synthesis of (2S)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 3-(4-chlorophenyl)-2-(N-methylpenta-4-enamide)propanoate (Compound nk58)

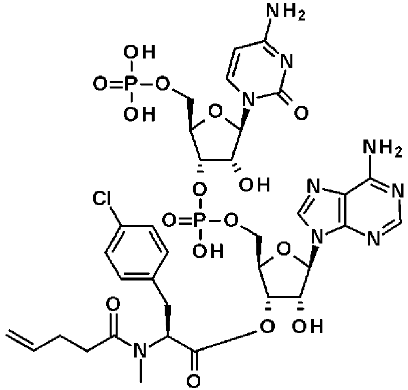

80% Aqueous acetic acid solution (4.0 mL) was added to a mixture (277 mg) of (2S)-(2R,3S,4R,5R)-2-((((((2R,3R,4R,5R)-5-(4-amino-2-oxopyrimidin-1(2*H*)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9*H*-purin-9-yl)-4-hydroxytetrahydrofuran-3-yl 3-(4-chlorophenyl)-2-(*N*-methylpenta-4-enamide)propanoate (Compound nk57) and *N,N,N*-trimethylhexadecane-1-aminium chloride obtained in the previous step, and stirred at room temperature for four hours. The reaction solution was purified by reverse-phase silica gel column chromatography (0.05% aqueous trifluoroacetic acid solution/0.05% trifluoroacetic acid-acetonitrile) to give the titled compound (Compound nk58, Pen-MePhe(4-Cl)-pCpA) (119 mg, 46%, two steps).
LCMS (ESI) m/z = 928 (M-H)-
Retention time: 0.52 minutes (analysis condition SQDFA05)

### Synthesis of (S)-4-(methylsulfonyl)-2-(penta-4-enamide)butanoic acid (Compound nk59, Pen-Met(O2)-OH)

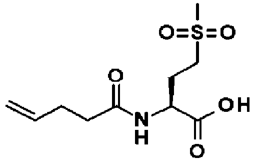

A 20% solution of piperidine in DMF (80 mL) was added to (S)-2-((((9*H*-fluoren-9-yl)methoxy)carbonyl)amino)-4-(methylsulfonyl)butanoic acid (Fmoc-Met(O2)-OH) (5.00 g 12.4 mmol), and then the reaction solution was stirred at 25°C for three hours. Next, diethyl ether (50 mL) was added to the reaction solution, this mixture was filtered, and the obtained solids were washed with diethyl ether and hexane to give (S)-2-amino-4-(methylsulfonyl)-butanoic acid (H-Met(O2)-OH) (2.88 g) as a crude product.

(S)-2-Amino-4-(methylsulfonyl)-butanoic acid (H-Met(O2)-OH) obtained as a crude product (2.88 g), and 2,5-dioxopyrrolidin-1-yl pent-4-enoate (3.76 g, 19.1 mmol) and sodium hydrogen carbonate (2.68 g, 31.9 mmol) were added to a mixed solvent of water (40 mL) and 1,4-dioxane (40 mL), and then the reaction solution was stirred at 25°C for four hours. The reaction solution was washed twice with ethyl acetate, and then pH of the aqueous layer was adjusted to 2 with 1 M aqueous hydrochloric acid solution and then extracted four times with dichloromethane. The obtained organic layers were combined, washed with saturated saline solution, dried over anhydrous sodium sulfate, filtered and then concentrated under reduced pressure. The resulting residue was purified by normal-phase silica gel column chromatography (dichloromethane/methanol) to give (S)-4-(methylsulfonyl)-2-(penta-4-enamide)butanoic acid (Compound nk59, Pen-Met(O2)-OH) (2.4 g, 74%, two steps).
LCMS (ESI) m/z = 264 (M+H)+
Retention time: 1.00 minute (analysis condition SMD method 4)

### Synthesis of (S)-cyanomethyl 4-(methylsulfonyl)-2-(penta-4-enamide)butanoate (Compound nk60, Pen-Met(O2)-OCH₂CN)

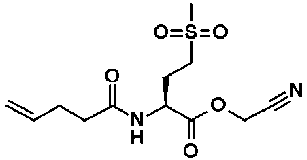

Under nitrogen atmosphere, (S)-4-(methylsulfonyl)-2-(penta-4-enamide)butanoic acid (Compound nk59, Pen-Met(O2)-OH) (1.80 g, 6.84 mmol) and *N*-ethyl-isopropylpropan-2-amine (DIPEA) (3.00 g, 23.2 mmol) were dissolved in dichloromethane (30 mL), 2-bromoacetonitrile (3.28 g, 27.4 mmol) was added, and the mixture was stirred at 25°C for 16 hours. The reaction solution was concentrated and the resulting residue was purified by normal-phase silica gel column chromatography (petroleum ether/ethyl acetate) to give (S)-cyanomethyl 4-(methylsulfonyl)-2-(penta-4-enamide)butanoate (Compound nk60, Pen-Met(O2)-OCH₂CN) (1.5 g, 73%).
LCMS (ESI) m/z = 303 (M+H)+
Retention time: 0.50 minutes (analysis condition SQDAA05)

### Synthesis of (2S)-(2R,3S,4R,5R)-2-((((((2R,3R,4R,5R)-5-(4-amino-2-oxopyrimidin-1(2H)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9H-purin-9-yl)-4-hydroxytetrahydrofuran-3-yl 4-(methylsulfonyl)-2-(penta-4-enamide)butanoate (Compound nk61)

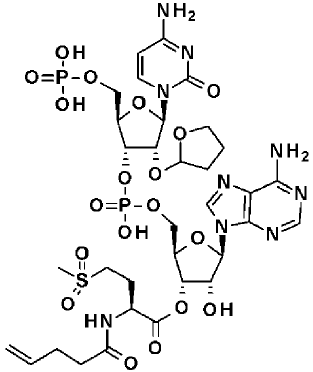

An aqueous solution (3.6 mL) of ((2R,3R,4R,5R)-5-(4-amino-2-oxopyrimidin-1(2*H*)-yl)-3-(((((2R,3S,4R,5R)-5-(6-amino-9*H*-puri n-9-yl)-3,4-dihydroxytetrahydrofuran-2-yl)methoxy)(hydroxy)phosphoryl)oxy)-4-((tetrahydrofur an-2-yl)oxy)tetrahydrofuran-2-yl)methyl dihydrogenphosphate (Compound pc01) (300 mg, 0.415 mmol) and a solution of (S)-cyanomethyl 4-(methylsulfonyl)-2-(penta-4-enamide)butanoate (Compound nk60, Pen-Met(O2)-OCH₂CN) (502 mg, 1.66 mmol) in acetonitrile (3.6 mL) were added to Buffer A (120 mL), and this was stirred at room temperature for two hours. The reaction solution was lyophilized, and the resulting residue was purified by reverse-phase silica gel column chromatography (10 mM aqueous ammonium acetate solution/methanol) to give a mixture (136 mg) of the titled compound (Compound nk61) and *N,N,N*-trimethylhexadecan-1-aminium chloride.
LCMS (ESI) m/z = 968.5 (M+H)+
Retention time: 0.44 minutes (analysis condition SQDAA05)

### Synthesis of (2S)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 4-(methylsulfonyl)-2-(penta-4-enamide)butanoate (Compound nk62, Pen-Met(O2)-pCpA)

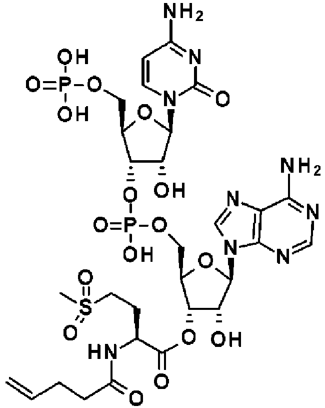

80% Aqueous acetic acid solution (1.0 mL) was added to a mixture (70 mg) of (2S)-(2R,3S,4R,5R)-2-((((((2R,3R,4R,5R)-5-(4-amino-2-oxopyrimidin-1(2*H*)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9*H*-purin-9-yl)-4-hydroxytetrahydrofuran-3-yl 4-(methylsulfonyl)-2-(penta-4-enamide)butanoate (Compound nk61) and *N,N,N*-trimethylhexadecane-1-aminium chloride obtained in the previous step, and stirred at room temperature for two hours. The reaction solution was lyophilized, and the resulting residue was purified by reverse-phase silica gel column chromatography (0.05% aqueous trifluoroacetic acid solution/0.05% trifluoroacetic acid-acetonitrile) to give the titled compound (Compound nk62, Pen-Met(O2)-pCpA) (12.7 mg, 7%, two steps).
LCMS (ESI) m/z = 898 (M+H)+
Retention time: 0.29 minutes (analysis condition SQDFA05)

### Synthesis of cyanomethyl (S)-3-(3-chlorophenyl)-2-(penta-4-enamide)propanoate (Compound nk63, Pen-Phe(3-Cl)-OCH₂CN)

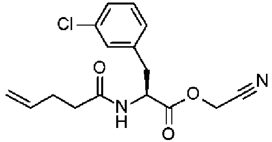

A 20% solution of piperidine in DMF (76 mL) was added to (S)-2-((((9*H*-fluoren-9-yl)methoxy)carbonyl)amino)-3-(3-chlorophenyl)propanoic acid (Fmoc-Phe(3-Cl)-OH) (5.00 g, 11.9 mmol), and then this reaction solution was stirred at 25°C for 16 hours. After concentrating the reaction solution, diethyl ether was added to the resulting residue, and by filtering this mixture, (S)-2-amino-3-(3-chlorophenyl)propanoic acid (H-Phe(3-Cl)-OH) (2.22 g) was obtained as a crude product.

(S)-2-Amino-3-(3-chlorophenyl)propanoic acid (H-Phe(3-Cl)-OH) obtained as a crude product (2.22 g), 2,5-dioxopyrrolidin-1-yl pent-4-enoate (2.62 g, 13.3 mmol), and sodium hydrogen carbonate (1.86 g, 22.1 mmol) were added to a mixed solvent of water (28 mL) and 1,4-dioxane (28 mL), and then the reaction solution was stirred at 25°C for 16 hours. The reaction solution was washed twice with ethyl acetate, and then pH of the aqueous layer was adjusted to 2 with 1 M aqueous hydrochloric acid solution and then extracted three times with dichloromethane. The obtained organic layers were combined, washed with saturated saline solution, dried over anhydrous sodium sulfate, filtered and then concentrated under reduced pressure. The resulting residue was purified by normal-phase silica gel column chromatography (dichloromethane/methanol) to give (S)-3-(3-chlorophenyl)-2-(penta-4-enamide)propanoic acid (Pen-Phe(3-Cl)-OH) (1.5 g, 45%, two steps).

Under nitrogen atmosphere, (S)-3-(3-chlorophenyl)-2-(penta-4-enamide)propanoic acid (Pen-Phe(3-Cl)-OH) (1.50 g, 5.32 mmol) and *N*-ethyl-isopropylpropan-2-amine (DIPEA) (1.37 g, 10.6 mmol) were dissolved in dichloromethane (24 mL), 2-bromoacetonitrile (2.55 g, 21.3 mmol) was added, and the mixture was stirred at 25°C for 16 hours. The reaction solution was concentrated and the resulting residue was purified by normal-phase silica gel column chromatography (petroleum ether/ethyl acetate) to give cyanomethyl (S)-3-(3-chlorophenyl)-2-(penta-4-enamide)propanoate (Compound nk63, Pen-Phe (3-Cl)-OCH₂CN) (0.767 g, 45%).
LCMS (ESI) m/z = 321 (M+H)+
Retention time: 0.87 minutes (analysis condition SQDAA05)

### Synthesis of (2S)-(2R,3S,4R,5R)-2-((((((2R,3R,4R,5R)-5-(4-amino-2-oxopyrimidin-1(2H)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9H-purin-9-yl)-4-hydroxytetrahydrofuran-3-yl 3-(3-chlorophenyl)-2-(penta-4-enamide)propanoate (Compound nk64)

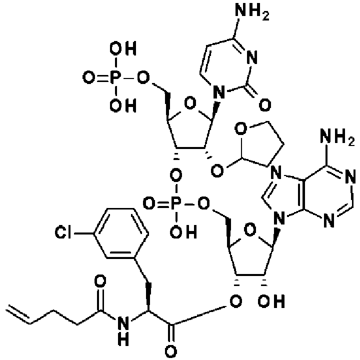

An aqueous solution (3.0 mL) of ((2R,3R,4R,5R)-5-(4-amino-2-oxopyrimidin-1(2*H*)-yl)-3-(((((2R,3S,4R,5R)-5-(6-amino-9*H*-puri n-9-yl)-3,4-dihydroxytetrahydrofuran-2-yl)methoxy)(hydroxy)phosphoryl)oxy)-4-((tetrahydrofur an-2-yl)oxy)tetrahydrofuran-2-yl)methyl dihydrogenphosphate (Compound pc01) (200 mg, 0.277 mmol) and a solution of cyanomethyl (S)-3-(3-chlorophenyl)-2-(penta-4-enamide)propanoate (Compound nk63, Pen-Phe(3-Cl)-OCH₂CN) (355 mg, 1.11 mmol) in acetonitrile (3.0 mL) were added to Buffer A (120 mL), and this was stirred at room temperature for two hours. The reaction solution was lyophilized, and the resulting residue was purified by reverse-phase silica gel column chromatography (10 mM aqueous ammonium acetate solution/methanol) to give a mixture (75 mg) of the titled compound (Compound nk64) and *N,N,N*-trimethylhexadecan-1-aminium chloride .
LCMS (ESI) m/z = 986 (M+H)+
Retention time: 0.77 minutes (analysis condition SQDAA05)

### Synthesis of (2S)-(2R,3S,4R,5R)-2-((((((2R,3S,4R,5R)-5-(4-amino-2-oxopyrimidin-1(2H)-yl)-4-hydroxy-2-(( phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl 3-(3-chlorophenyl)-2-(penta-4-enamide)propanoate (Compound nk65, Pen-Phe(3-Cl)-pCpA)

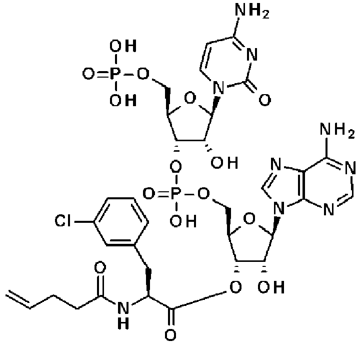

80% Aqueous acetic acid solution (0.80 mL) was added to a mixture (40 mg) of (2S)-(2R,3S,4R,5R)-2-((((((2R,3R,4R,5R)-5-(4-amino-2-oxopyrimidin-1(2*H*)-yl)-2-((phosphono oxy)methyl)-4-((tetrahydrofuran-2-yl)oxy)tetrahydrofuran-3-yl)oxy)(hydroxy)phosphoryl)oxy)m ethyl)-5-(6-amino-9*H*-purin-9-yl)-4-hydroxytetrahydrofuran-3-yl 3-(3-chlorophenyl)-2-(penta-4-enamide)propanoate (Compound nk64) and *N,N,N*-trimethylhexadecane-1-aminium chloride obtained in the previous step, and stirred at room temperature for six hours. The reaction solution was purified by reverse-phase silica gel column chromatography (10 mM aqueous ammonium acetate solution/methanol), and purified again by reverse-phase silica gel column chromatography (0.05% aqueous trifluoroacetic acid solution/0.05% trifluoroacetic acid-acetonitrile) to give the titled compound (Compound nk65, Pen-Phe(3-Cl)-pCpA) (7.5 mg, 6%, two steps).
LCMS (ESI) m/z = 914 (M-H)-
Retention time: 0.47 minutes (analysis condition SQDFA05)

### 11-2. Synthesis of acylated tRNA

Acylated tRNAs used for panning were prepared by methods described in the patent document (WO 2013/100132 A1). The nucleotide sequences of the tRNAs (lacking CA) that were used are shown in Table 23. Elongator aminoacylated tRNA mixtures shown in Table 24 were prepared. Thereafter, for use in translation, the translation solutions were prepared such that the final concentrations became the concentrations indicated in Table 24. Preparation of an initiator aminoacylated tRNA was carried out by ligation reaction followed by performing the operations from the phenol extraction and onwards, without performing the deprotection operation. The three species shown in Table 25 were prepared individually, and for use in translation, any one of them was added to the translation solution at the final concentration indicated in the Table.

**[Table 23]**

| RNA Name | RNA sequence number | RNA sequence |
|---|---|---|
| tRNAGluCUA (Lacking CA) | T - 1 | GUCCCCUUCGUCUAGAGGCCCAGGACACCGCCCUcuaACGGCGGUAACAGGGGUUCGAAUCCCCU AGGGGACGC（SEQ ID NO:3 8） |
| tRNAGluCAA (Lacking CA) | T - 2 | GUCCCCUUCGUCUAGAGGCCCAGGACACCGCCCUcaaACGGCGGUAACAGGGGUUCGAAUCCCCU AGGGGACGC (SEQ ID NO:39) |
| tRNAGluUAG (Lacking CA) | T - 3 | GUCCCCUUCGUCUAGAGGCCCAGGACACCGCCCUuagACGGCGGUAACAGGGGUUCGAAUCCCCU AGGGGACGC (SEQ ID NO:40) |
| tRNAGluCUG (Lacking CA) | T - 4 | GUCCCCUUCGUCUAGAGGCCCAGGACACCGCCCUcugACGGCGGUAACAGGGGUUCGAAUCCCCU AGGGGACGC (SEQ ID NO: 8) |
| tRNAGluCCG (Lacking CA) | T - 5 | GUCCCCUUCGUCUAGAGGCCCAGGACACCGCCCUccgACGGCGGUAACAGGGGUUCGAAUCCCCU AGGGGACGC (SEQ ID NO:4 1) |
| tRNAGluCCU (Lacking CA) | T - 6 | GUCCCCUUCGUCUAGAGGCCCAGGACACCGCCCUccuACGGCGGUAACAGGGGUUCGAAUCCCCU AGGGGACGC (SEQ ID NO: 4 2) |
| tRNAfMetCAU (Lacking CA) | T - 10 | GGCGGGGUGGAGCAGCCUGGUAGCUCGUCGGGCUcauAACCCGAAGAUCGUCGGUUCAAAUCCGG CCCCCGCAAC (SEQ ID NO:4 3) |

**[Table 24]**

| Name of contained Acylated tRNA | Final concentration at translation [*µ*M] |
|---|---|
| Asp(SMe)-tRNAGluCUA | 20 |
| Phe(3-Cl)-tRNAGluCAA | 20 |
| MeGly-tRNAGluUAG | 20 |
| MeAla(4-Thz)-tRNAGluCUG | 20 |
| MePhe(4-Cl)-tRNAGluCCG | 20 |
| Met(02)-tRNAGluCCU | 20 |

**[Table 25]**

| Name of Acylated tRNA | Final concentration at translation [*µ*M] |
|---|---|
| Acbz-Cys(StBu)-tRNAfMetCAU | 25 |
| Acbz-MeCys(StBu)-tRNAfMetCAU | 25 |
| Acbz-D-MeCys(StBu)-tRNAfMetCAU | 25 |

### 11-3. Library of randomized double-stranded DNAs encoding a peptide library

A DNA library was constructed by the method described in the patent document (WO 2013/100132A1). DNAs in which any one of the codons of TTT, TTG, CTA, ATT, GTT, CCG, ACT, GCT, TAC, CAT, CAG, TGG, CGG, AGT, AGG, and GGT repeatedly appears nine times were prepared.

### 11-4. Preparation of the biotinylated target protein

GTPase KRas (KRAS) expressed in and purified from *E. coli* was used as the target protein used for panning. The biotinylated protein was prepared using the method of the non-patent documents BMC biotechnology, 2008, 8, 41 and Protein Science, 1999, 8, 921-929.

### 11-5. Translation solution used for panning

Translation solution used for panning was composed of the following components: 1 mM GTP, 1 mM ATP, 20 mM creatine phosphate, 50 mM HEPES-KOH (pH 7.6), 100 mM potassium acetate, 10 mM magnesium acetate, 2 mM spermidine, 1 mM dithiothreitol, 0.5 mg/ml *E. coli* MRE600 (RNase-negative)-derived tRNA (Roche), 4 µg/ml creatine kinase, 3 µg/ml myokinase, 2 units/ml inorganic pyrophosphatase, 1.1 µg/ml nucleoside diphosphate kinase, 0.26 µM EF-G, 2.7 µM IF1, 0.4 µM IF2, 1.5 µM IF3, 40 µM EF-Tu, 44 µM EF-Ts, 1.2 µM ribosome, 2.73 µM AlaRS, 0.09 µM GlyRS, 0.4 µM IleRS, 0.5 µM mutant PheRS05 (Patent Document: WO 2016/148044), 0.16 µM ProRS, 1 µM mutant SerRS37 (Patent Document: WO 2016/148044), 0.09 µM ThrRS, 0.01 µM TrpRS, 0.02 µM TyrRS, 1 µM mutant ValRS13 (Patent Document: WO 2016/148044), 0.11 µM LysRS, 0.33 µM HisRS, 3 µM *in vitro* transcribed E. *coli* tRNA Ala1B, 3 µM purified *E. coli* tRNA His QUG (purified according to the method of the non-patent document: Nucleic Acids Res. 2010 Apr; 38(6): e89), 250 µM glycine, 250 µM isoleucine, 250 µM proline, 250 µM threonine, 250 µM tryptophan, 250 µM lysine, 5 mM *N*-methyl valine, 5 mM *N*-methyl serine, 5 mM *N*-methyl alanine, 5 mM *N*-methyl phenylalanine, 250 µM 3-fluorotyrosine, 5 mM *N*-methyl histidine, and a mixture of elongator aminoacylated tRNAs shown in Table 23. Furthermore, one of the initiator aminoacylated tRNAs (Table 24) was added to prepare the solution.

### 11-6. Panning

The above-described double-stranded DNA library and the translation solution were used to perform panning according to the patent document (WO 2013/100132A1).

### 11-7. Analysis of the concentrated sequences and confirmation of binding to the target

After panning, the DNA pool was subjected to nucleotide sequence analysis, and solid-phase peptide syntheses were performed according to the patent document (WO 2013/100132 A1) for the sequences showing high frequency of appearance. Biacore was used to confirm the binding ability towards the target protein. The results showed that multiple sequences bind to the target.

These target-binding sequences were peptides having a cyclic portion containing a plurality of amino acid analogs. The results indicated that cyclic peptides containing a plurality of amino acid analogs, which bind to GTPase KRas (KRAS), can be obtained using the initiation suppression method.

### Industrial Applicability

The present invention is useful for providing peptides having a drug-like cyclic portion containing a plurality of amino acid analogs, complexes of the peptides and nucleic acids, and display libraries of the complexes. The peptides can be synthesized with higher efficiency when using the display libraries constructed using the present invention than when using display libraries obtained by conventional methods. Furthermore, the display libraries constructed using the present invention are expected to contain greater variety of compounds compared to display libraries obtained by conventional methods. Therefore, use of the methods of the present invention further increases the probability of obtaining a greater variety of drug-like hit compounds.

## Claims

1. A method for preparing a peptide comprising two or more amino acid analog residues and a cyclic portion, a complex of said peptide and a nucleic acid, or a library comprising said complex,
wherein the method comprises the step of forming an amide bond by reacting a first reaction point with a second reaction point in a peptide, or in a complex of the peptide and a nucleic acid, wherein the peptide is synthesized by translating a nucleic acid encoding a noncyclic peptide which comprises at its N terminus an amino acid residue represented by general formula (I) or general formula (II) shown below comprising the first reaction point, and which comprises at a position at least four residues apart from the N terminus to the C-terminal side an amino acid residue comprising the second reaction point in one of its side chains: 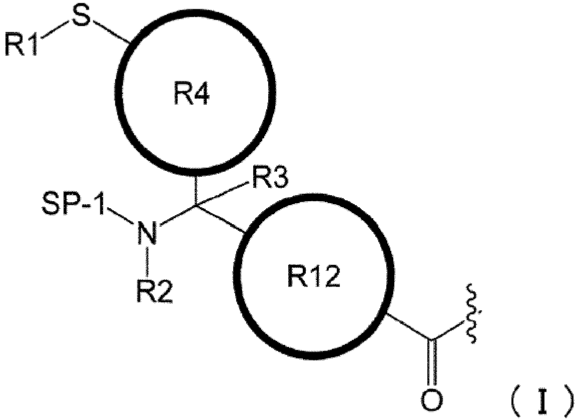 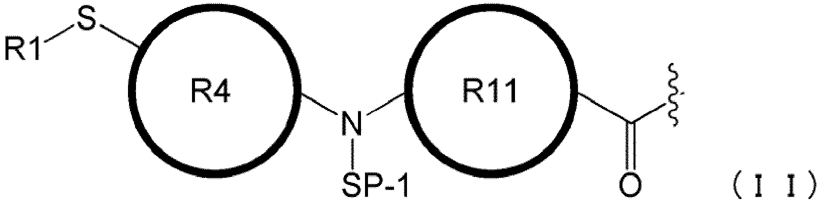 wherein,
R1 is a thiol group-protecting group which can be ribosomally synthesized;
R2 and R3 each independently are a hydrogen atom, an alkyl group, an alkenyl group, an alkynyl group, an aryl group, a heteroaryl group, an aralkyl group, or a cycloalkyl group, each of which is optionally substituted; or R2 and R3 form a ring together with atoms to which they are bonded; or R2 or R3 form a ring together with R4 and atoms to which they are bonded; with the proviso that when R2 forms an azido group (-N₃) together with the N atom to which it is bonded and SP-1, the above-mentioned definition does not apply to R2;
R4 represents an alkylene, an arylene, a heteroarylene, an alkylenearylene, an alkyleneheteroarylene, an arylenealkylene, or a heteroarylenealkylene, each of which is optionally substituted;
R11 and R12 each independently are a single bond, an alkylene, an arylene, a heteroarylene, an alkylene arylene, an alkyleneheteroarylene, an arylenealkylene, or a heteroarylenealkylene, each of which is optionally substituted;
SP-1 forms an azido group together with the N atom to which it is bonded and R2, or is an amino group-protecting group represented by the following formula: 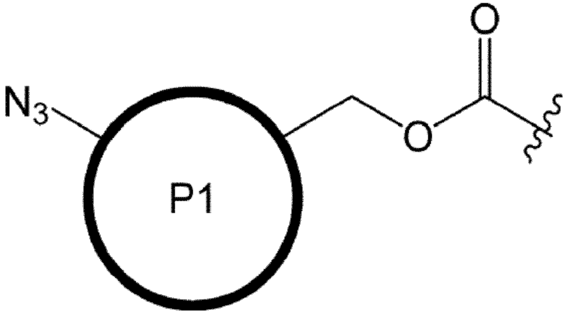
wherein, PI is a single bond, an arylene, or a heteroarylene,
or represented by the following formula: 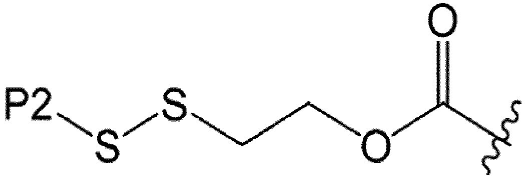 wherein, P2 is an alkyl, an aryl, or a heteroaryl.

2. The method of claim 1, wherein R1 is selected from the group consisting of S-R23, wherein R23 is an alkyl, an alkenyl, an alkynyl, an aryl, a heteroaryl or an aralkyl, each of which is optionally substituted; a sulfonate (-SO₃⁻); and a thiosulfonate (-S₂O₃⁻).

3. The method of claim 2, wherein R23 is a methyl, an ethyl, an isopropyl, a tert-butyl, a phenyl, a *p*-trifluoromethylphenyl, a *p*-fluorophenyl, a benzyl, or a phenethyl, each of which is optionally substituted.

4. The method of any one of claims 1 to 3, wherein R2 and R3 each independently are a hydrogen atom, a C1-C4 alkyl optionally substituted with a halogen or a C1-C4 alkoxy optionally substituted with a halogen.

5. The method of any one of claims 1 to 4, wherein R4 is selected from the group consisting of the following: 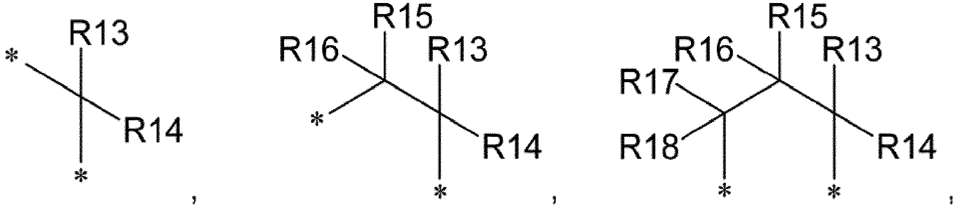 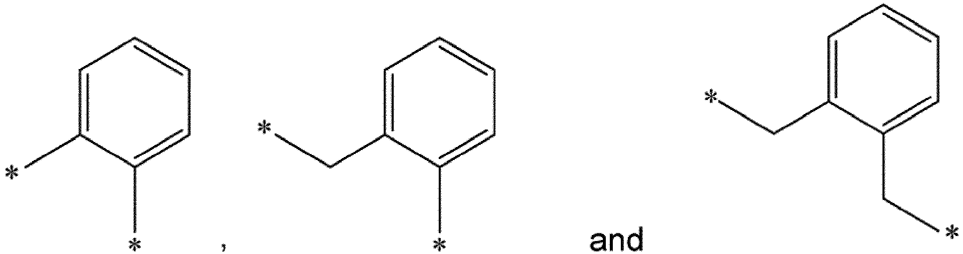 wherein R13 to R18 each independently are a hydrogen atom, an optionally substituted alkyl, or an optionally substituted alkoxy.

6. The method of claim 5, wherein R13 to R18 each independently are a hydrogen atom or a methyl.

7. The method of any one of claims 1 to 6, wherein R11 and R12 each independently are a single bond or are selected from the group consisting of the following: 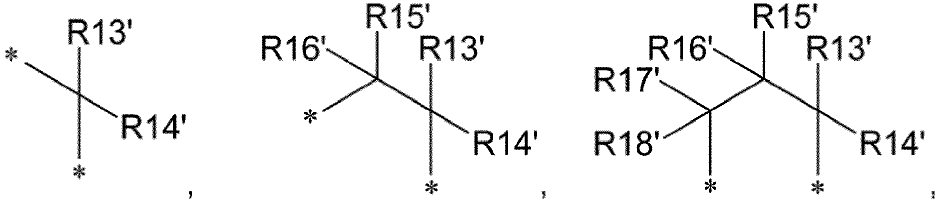 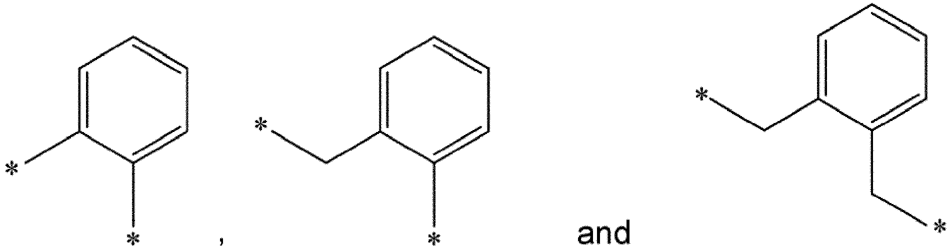 wherein R13' to R18' each independently are a hydrogen atom, an optionally substituted alkyl, or an optionally substituted alkoxy.

8. The method of claim 7, wherein R13' to R18' each independently are a hydrogen atom or a methyl group.

9. The method of any one of claims 1 to 8, wherein the amino acid residue represented by general formula (I) is an amino acid residue represented by any one of the following general formulas: 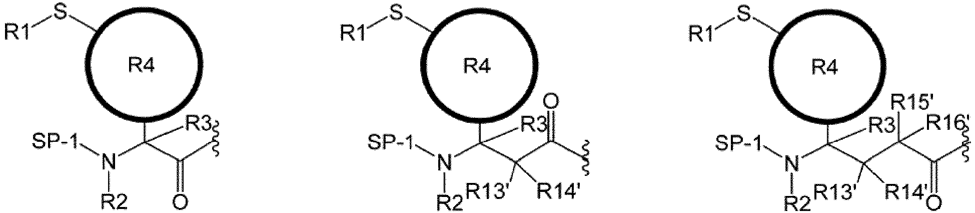
wherein R1 to R4 and SP-1 have the same meaning as R1 to R4 and SP-1 of claim 1, respectively, and
R13' to R16' have the same meaning as R13' to R16' of claim 7, respectively; or
the amino acid residue represented by general formula (II) is an amino acid residue represented by any one of the following general formulas: 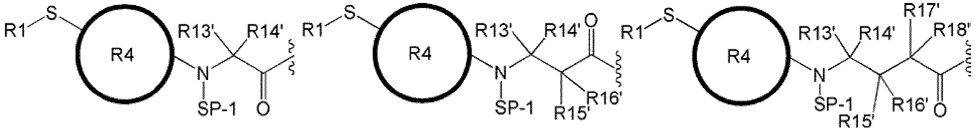
wherein R1, R4, and SP-1 have the same meaning as R1, R4, and SP-1 of claim 1, respectively, and
R13' to R18' have the same meaning as R13' to R18' of claim 7, respectively.

10. The method of any one of claims 1 to 9, wherein the amino acid residue represented by general formula (I) is an amino acid residue represented by any one of the following general formulas: 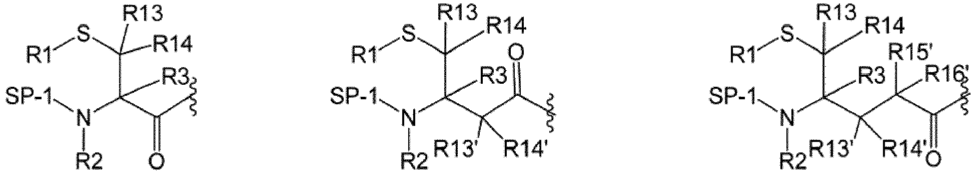 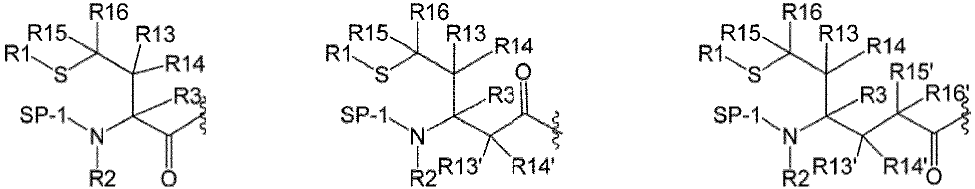
wherein R1 to R3 and SP-1 have the same meaning as R1 to R3 and SP-1 of claim 1, respectively;
R13 to R16 have the same meaning as R13 to R16 of claim 5, respectively; and
R13' to R16' have the same meaning as R13' to R16' of claim 7, respectively; or
R2 forms a ring together with R13, R14, R15, or R16 and atoms to which they are bonded, with the proviso that when R2 forms an azido group (-N₃) together with the N atom to which it is bonded and SP-1, the above-mentioned definition does not apply to R2; or
the amino acid residue represented by general formula (II) is an amino acid residue represented by any one of the following general formulas: 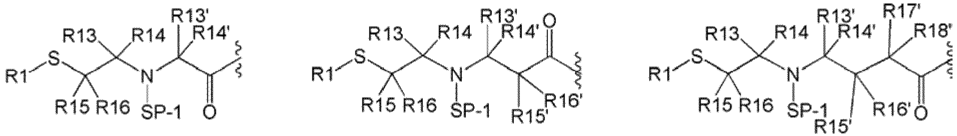 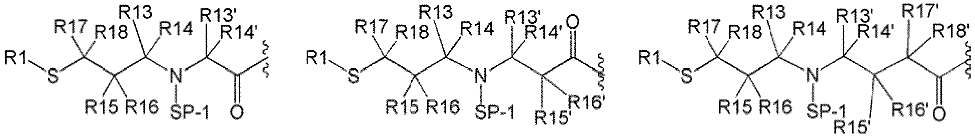
wherein R1 and SP-1 have the same meaning as R1 and SP-1 of claim 1, respectively;
R13 to R18 have the same meaning as R13 to R18 of claim 5, respectively; and
R13' to R18' have the same meaning as R13' to R18' of claim 7, respectively.

11. The method of any one of claims 1 to 10, wherein SP-1 is a 4-azidobenzyloxycarbonyl (p-Acbz), a 2-azidobenzyloxycarbonyl (o-Acbz), an azidomethoxycarbonyl (Azoc), a phenyldisulfanylethyloxycarbonyl (Phdec), a 2-pyridyldisulfanylethyloxycarbonyl (Pydec), or a 2-(*t*-butyldisulfanyl)ethyloxycarbonyl (Tbeoc); or SP-1 forms an azido group together with the N atom to which it is bonded and R2.

12. The method of any one of claims 1 to 11, wherein the amino acid residue comprising the second reaction point in one of the side chains is represented by the following general formula: 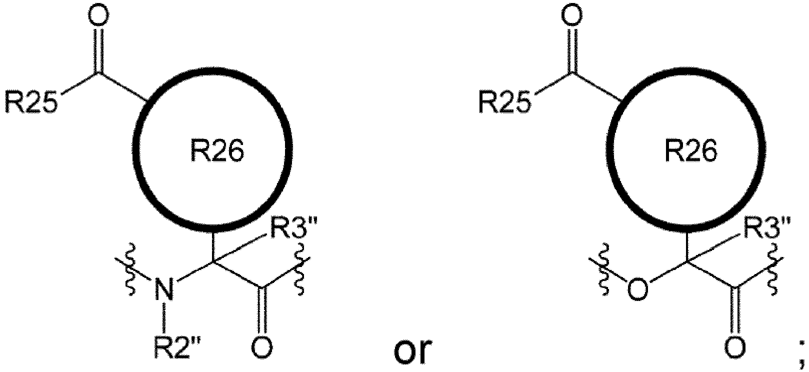 wherein
R2" and R3" each independently are a hydrogen atom, an alkyl, an alkenyl, an alkynyl, an aryl, a heteroaryl, an aralkyl, or a cycloalkyl, each of which is optionally substituted; or R2" and R3" form a ring together with atoms to which they are bonded; or R2" or R3" form a ring together with R26 and atoms to which they are bonded;
R25 is a hydroxyl group or forms an active ester with CO to which it is bonded; and
R26 is an alkylene, an arylene, a heteroarylene, an arylenealkylene, or a heteroarylenealkylene, each of which is optionally substituted;

13. The method of any one of claims 1 to 12, wherein R26 is selected from the group consisting of the following: 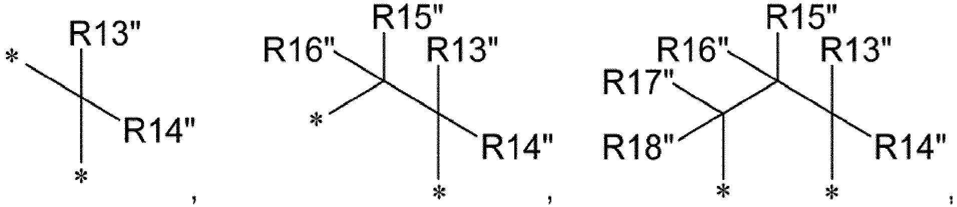 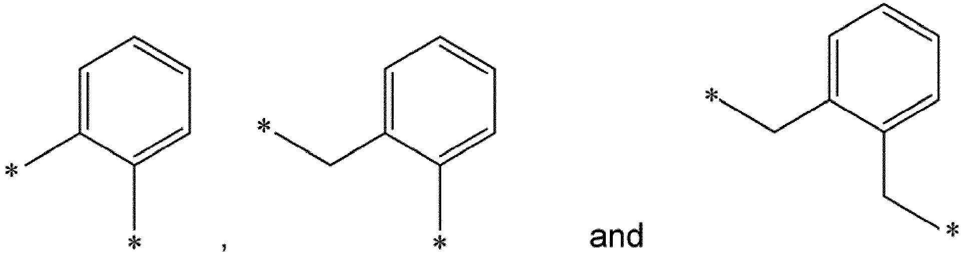 wherein, R13" to R18" each independently are a hydrogen atom, an optionally substituted alkyl, or an optionally substituted alkoxy.

14. The method of any one of claims 1 to 13, wherein the amino acid residue comprising the second reaction point in one of the side chains is represented by the following general formula: 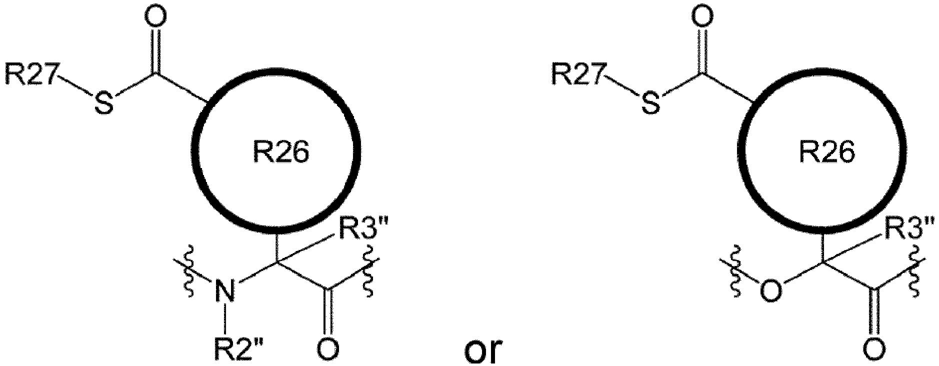 wherein
R2" has the same meaning as R2" of claim 12;
R3" is a hydrogen atom or an optionally substituted C1-C4 alkyl group; and
R27 is selected from among a hydrogen atom, an optionally substituted alkyl group, an optionally substituted alkenyl group, an optionally substituted alkynyl group, an optionally substituted aryl group, an optionally substituted heteroaryl group, an optionally substituted cycloalkyl group, and an optionally substituted aralkyl group.

15. The method of any one of claims 1 to 14, wherein the amino acid residue comprising the second reaction point in one of the side chains is represented by the following general formula: 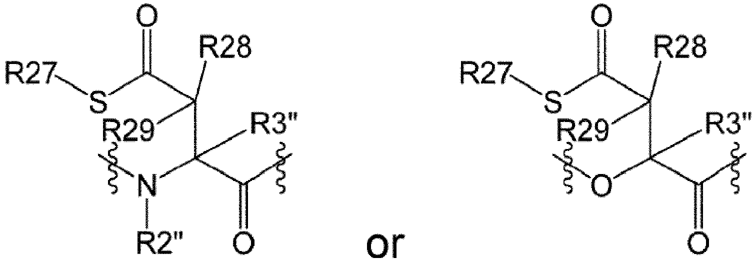 wherein, R2" has the same meaning as R2" of claim 12;
R3" is a hydrogen atom or an optionally substituted C1-C4 alkyl group;
R28 and R29 each independently are a hydrogen atom, an optionally substituted C1-C6 alkyl group, an optionally substituted C2-C6 alkenyl group, an optionally substituted C2-C6 alkynyl group, an optionally substituted aryl group, an optionally substituted heteroaryl group, an optionally substituted aralkyl group, or an optionally substituted cycloalkyl group; and
R27 is selected from among a hydrogen atom, an optionally substituted alkyl group, an optionally substituted alkenyl group, an optionally substituted alkynyl group, an optionally substituted aryl group, an optionally substituted heteroaryl group, an optionally substituted cycloalkyl group, and an optionally substituted aralkyl group.

16. The method of any one of claims 1 to 15, which further comprises the step of desulfurizing an -SH group present in the cyclic portion.

17. The method of any one of claims 1 to 16, wherein the complex of cyclic portion-comprising peptide and nucleic acid comprises a linker between the peptide and the nucleic acid.

18. The method of any one of claims 1 to 17, wherein the peptide is synthesized by translation in a cell-free translation system not containing at least one of methionine, methionyl-tRNA synthetase (MetRS), translation initiation tRNA for methionine, formyl donor, and methionyl-tRNA transferase.

19. A compound represented by the following general formula (IA) or general formula (IIA): 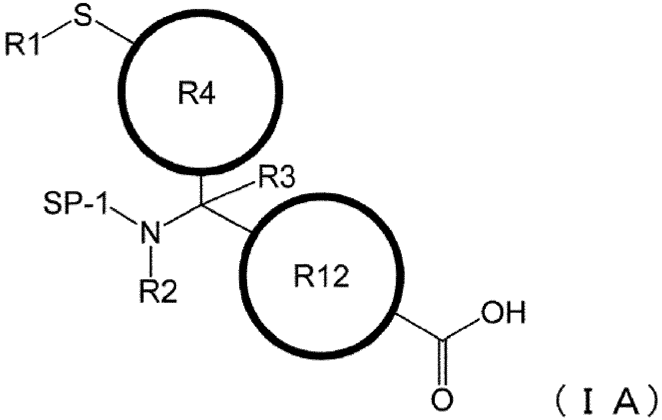 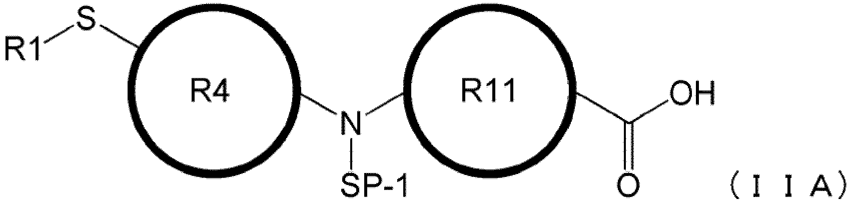 wherein R1 to R4, R11, R12, and SP-1 have the same meaning as R1 to R4, R11, R12, and SP-1 of claim 1, respectively.

20. A compound represented by the following general formula (IB) or general formula (IIB): 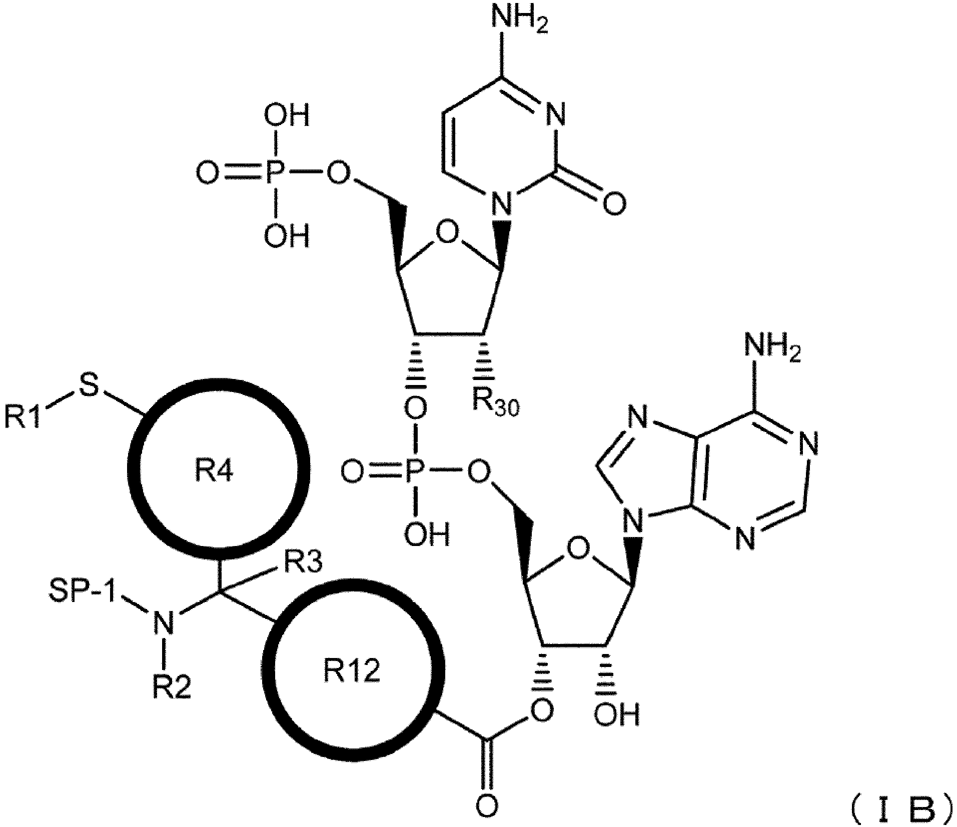 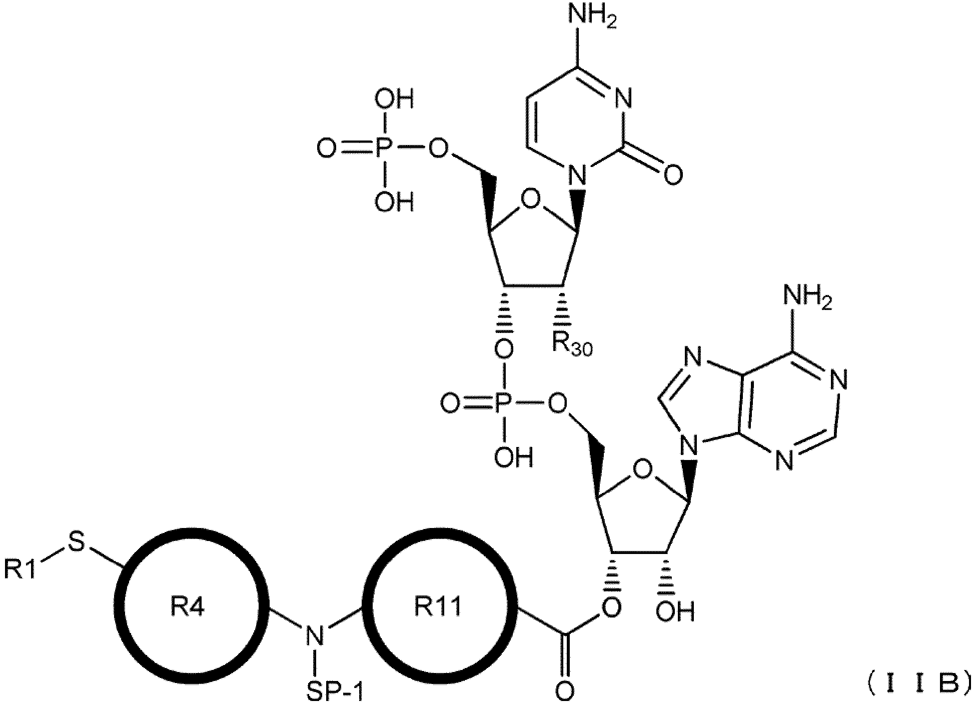 wherein, R1 to R4, R11, R12, and SP-1 have the same meaning as R1 to R4, R11, R12, and SP-1 of claim 1, respectively; and R30 represents H or a hydroxyl group.

21. An aminoacyl-tRNA formed by bonding the compound of claim 19 and tRNA.

22. A noncyclic peptide or a complex of the peptide and a nucleic acid, wherein the peptide comprises at its N terminus an amino acid residue represented by general formula (I) or general formula (II) shown below comprising a first reaction point, and which comprises at a position at least four residues apart from the N terminus to the C-terminal side an amino acid residue comprising a second reaction point in one of its side chains: 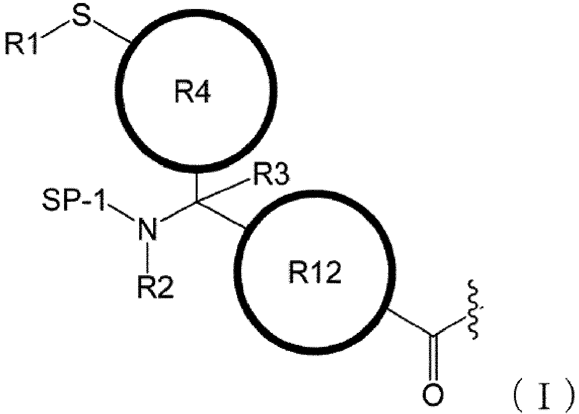 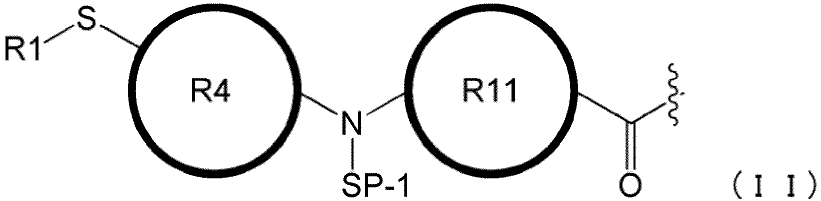 wherein R1 to R4, R11, R12, and SP-1 have the same meaning as R1 to R4, R11, R12, and SP-1 of claim 1, respectively.

23. The peptide or the complex of claim 22, wherein the noncyclic peptide is obtained by a method comprising the step of synthesizing the noncyclic peptide by translating a nucleic acid encoding the noncyclic peptide which comprises at its N terminus an amino acid residue represented by general formula (I) or general formula (II) comprising a first reaction point, and which comprises at a position at least four residues apart from the N terminus to the C-terminal side an amino acid residue comprising a second reaction point in one of its side chains: 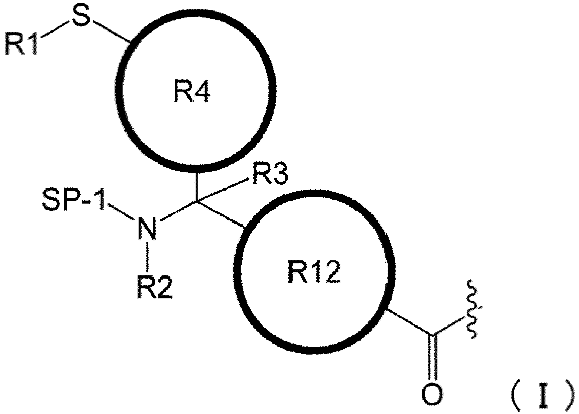 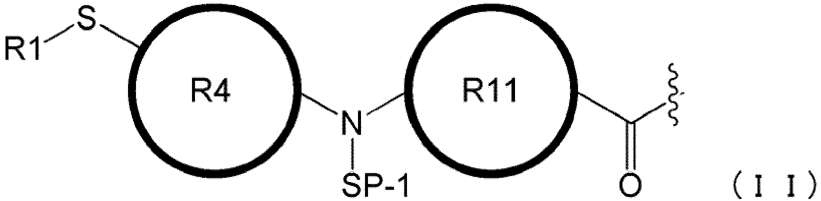 wherein, R1 to R4, R11, R12, and SP-1 have the same meaning as R1 to R4, R11, R12, and SP-1 of claim 1, respectively.

24. A method of producing a peptide comprising two or more amino acid analog residues and having a cyclic portion, a complex of the peptide and a nucleic acid, or a library comprising the complex using the peptide or the complex of claim 22 or 23.
